# EUROPEAN PATENT APPLICATION

(11) **EP 2 325 310 A1**
(43) Date of publication of application: **25.05.2011**
(21) Application number: 09805016.4
(22) Date of filing: 06.08.2009
(51) Int. Cl.: C12N 15/09, C07K 16/44, C12Q 1/02, C12Q 1/68, G01N 33/48, G01N 33/53

(54) **METHOD FOR OBTAINING PURKINJE PROGENITOR CELL BY USING NEPH3(65B13) AND E-CADHERIN**

(30) Priority: 08.08.2008 JP 2008206309
(71) Applicant: Eisai R&D Management Co., Ltd., Tokyo 112-8088 (JP)
(72) Inventor: ONO, Yuichi, Kobe-shi Hyogo 650-0047 (JP); NAKAGAWA, Yasuko, Kobe-shi Hyogo 650-0047 (JP); MIZUHARA, Eri, Kobe-shi Hyogo 650-0047 (JP)
(74) Representative: Grünecker, Kinkeldey, Stockmair & Schwanhäusser Anwaltssozietät
(86) International application number: PCT/JP2009/063915
(87) International publication number: WO 2010/016533

(57) **Abstract**

65B13 was discovered to be a useful marker for isolating GABA neuron progenitor cells including Purkinje cells. Furthermore, E-cadherin was revealed to be a useful marker for isolating Purkinje progenitor cells from a 65B13-positive cell population. Specifically, when used in combination with 65B 13, E-cadherin was found to be a useful marker for isolating Purkinje progenitor cells.

SEQUENCE LISTING

<110>  EISAI R&D MANAGEMENT CO., LTD.

<120>  Method for isolating purukinje precursor cells using genetic markers,
Neph3(65B13) and E-cadherin

<130>  E1-A0801P

<150>  JP 2008-206309
<151>  2008-08-08

<160>  59

<170>  PatentIn version 3.3

<210>  1
<211>  2876
<212>  DNA
<213>  Mus musculus

<400>  1
gatgagccag atttcgggga ctctgggcca gacataaaat cttccagccc ggagagaatt      60

gtgtgcagag aggggctcca gtccagcgtg gtgtgagagg cgtgctatca agaaagaagt     120

tggaggggaa ccagtgcaac cctaactcta cgagatcttg gggtacacac actcgggatg     180

ctggcctccg ccctcctcgt tttcctttgc tgtttcaaag gacatgcagg ctcatcgccc     240

catttcctac aacagccaga ggacatggtg gtgctgttgg gggaggaagc ccggctgccc     300

tgcgctctgg gcgcgtacag ggggctcgtg cagtggacta aggatgggct ggctctaggg     360

ggcgaaagag accttccagg gtggtcccgg tactggatat cggggaattc agccagtggc     420

cagcatgacc tccacattaa gcctgtggaa ttggaagatg aggcatcgta tgagtgccag     480

gcttcgcaag caggtctccg atcacgacca gcccaactgc acgtgatggt ccccccagaa     540

gctccccagg tactaggcgg cccctctgtg tctctggttg ctggagttcc tggaaatctg     600

acctgtcgga gtcgtgggga ttcccgacct gcccctgaac tactgtggtt ccgagatggg     660

atccggctgg atgcgagcag cttccaccag accacgctga aggacaaggc cactggaaca     720

gtggaaaaca ccttattcct gaccccttcc agtcatgatg atggcgccac cttgatctgc     780

agagcgcgaa gccaggccct gcccacaggg agggacacag ctgttacact gagccttcag     840

tatcccccaa tggtgactct gtctgctgag ccccagactg tgcaggaggg agagaaggtg     900

actttcctgt gtcaagccac tgcccagcct cctgtcactg gctacaggtg ggcgaagggg     960

ggatccccgg tgctcggggc acgtgggcca aggttggagg tcgttgcaga tgccactttc    1020

ctgactgagc cggtgtcctg cgaggtcagc aacgcggtcg gaagcgccaa ccgcagcacg    1080

gcgctggaag tgttgtatgg acccattctg caggcaaaac ctaagtccgt gtccgtggac    1140

gtggggaaag atgcctcctt cagctgtgtc tggcgcggga acccacttcc acggataacc    1200

tggacccgca tgggtggctc tcaggtgctg agctccgggc ccacgctgcg gcttccgtcc 1260

gtggcactgg aggatgcggg cgactatgta tgcagggctg agccgaggag aacgggtctg 1320

ggaggcggca aagcgcaggc gaggctgact gtgaacgcac cccctgtagt gacagccctg 1380

caacctgcac cagcctttct gaggggtcct gctcgcctcc agtgtgtggt gtttgcctcc 1440

cctgccccag actcggtggt ttggtcttgg gacgagggct tcttggaggc aggctcactg 1500

ggcaggttcc tagtggaagc cttcccagcc ccggaagtgg aggggggaca gggccctggc 1560

cttatttctg tgctacacat ttccggaacc caggagtccg actttaccac cggcttcaac 1620

tgcagtgccc gcaaccggct aggagaggga cgagtccaga tccacttggg ccgtagagat 1680

ttgctgccta ctgtccggat tgtggctggt gcagcatctg cagccacctc tctccttatg 1740

gtcatcactg gagtggtcct ctgctgctgg cgccatggct ctctctctaa gcaaaagaac 1800

ttggtccgga tcccaggaag cagcgagggt tccagttcac gtggccctga ggaggagaca 1860

ggcagcagtg aggaccgggg tcccattgtg cacaccgacc acagtgattt ggttcttgag 1920

gaaaaagagg ctctggagac aaaggatcca accaacggtt actacaaggt tcgaggggtc 1980

agtgtgagcc ttagccttgg ggaagctcct ggaggaggcc tcttcttgcc accgccctct 2040

ccgatcggtc tcccagggac tcctacttac tatgacttca agccacatct ggacttagtc 2100

cctccctgca gactgtacag agcgagggca ggttatctta ccacccccca tccccgtgcc 2160

ttcaccagct acatgaaacc cacatccttt ggacccccag atttgagctc tggaactccc 2220

cccttcccgt atgctacctt gtctccaccc agccaccagc gtctccagac tcatgtgtga 2280

atccatctct ccaagtgaag ggtcttggaa tcttctgttt gccatatagt gtgttgtcca 2340

gatttctggg gagtcagaac aagttgatga ccaacccctc caaaactgaa cattgaagga 2400

gggaaagatc attacaagca tcaggactgt tggtgtacac tcagttcagc caaagtggat 2460

tctccaagtg ggagcaatat ggccgctttc ccatgagaaa gacattcaag atggtgacta 2520

aatgactaaa tactttgcag agggacaaag atgggaacta gggatacgga tggaagtagt 2580

agagaagata tatgaccatc tgcatcaaga ggaaggataa catatgacaa atcaagatga 2640

aagaaataat ccaccccacc cccaccgcgt cctggccaat aagtatagcc tacatggctg 2700

ttcattatct gggaaccaaa atggccacta tcttgactcc ttccttaaag atacagaaag 2760

aattgaatcc aaggaatggg gtagggtgga aatagaagaa atgaagggga ctcttgggct 2820

aagaatactt atgtttaata ataaaagggg gaggcaaaga tgcaaaaaaa aaaaaa 2876

<210> 2
<211> 700
<212> PRT

<213>  Mus musculus

<400>  2

Met Leu Ala Ser Ala Leu Leu Val Phe Leu Cys Cys Phe Lys Gly His
1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly
        115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Ala Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys

225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys
        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu
545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Lys Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Asp Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  3
<211>  2243
<212>  DNA

<213>  Mus musculus

<400>  3
gagagaattg tgtgcagaga gaggctccag tccagcgtgg tgtgagaggc gtgctatcaa      60

gaaagaagtt ggaggggaac cagtgcaacc ctaactctac gagatcttgg ggtacacaca     120

ctcgggatgc tggcctccgc cctcctcgtt ttcctttgct gtttcaaagg acatgcaggg     180

tggtcccggt actggatatc ggggaattca gccagtggcc agcatgacct ccacattaag     240

cctgtggaat tggaagatga ggcatcgtat gagtgccagg cttcgcaagc aggtctccga     300

tcacgaccag cccaactgca cgtgatggtc cccccagaag ctccccaggt actaggcggc     360

ccctctgtgt ctctggttgc tggagttcct ggaaatctga cctgtcggag tcgtggggat     420

tcccgacctg cccctgaact actgtggttc cgagatggga tccggctgga tgcgagcagc     480

ttccaccaga ccacgctgaa ggacaaggcc actggaacag tggaaaacac cttattcctg     540

accccttcca gtcatgatga tggcgccacc ttgatctgca gagcgcgaag ccaggccctg     600

cccacaggga gggacacagc tgttacactg agccttcagt atcccccaat ggtgactctg     660

tctgctgagc cccagactgt gcaggaggga gagaaggtga ctttcctgtg tcaagccact     720

gcccagcctc ctgtcactgg ctacaggtgg gcgaaggggg gatccccggt gctcggggca     780

cgtgggccaa ggttggaggt cgttgcagat gccactttcc tgactgagcc ggtgtcctgc     840

gaggtcagca acgcggtcgg aagcgccaac cgcagcacgg cgctggaagt gttgtatgga     900

cccattctgc aggcaaaacc taagtccgtg tccgtggacg tggggaaaga tgcctccttc     960

agctgtgtct ggcgcgggaa cccacttcca cggataacct ggacccgcat gggtggctct    1020

caggtgctga gctccgggcc cacgctgcgg cttccgtccg tggcactgga ggatgcgggc    1080

gactatgtat gcagggctga gccgaggaga acgggtctgg gaggcggcaa agcgcaggcg    1140

aggctgactg tgaacgcacc ccctgtagtg acagccctgc aacctgcacc agcctttctg    1200

aggggtcctg ctcgcctcca gtgtgtggtg tttgcctccc ctgccccaga ctcggtggtt    1260

tggtcttggg acgagggctt cttggaggca ggctcactgg gcaggttcct agtggaagcc    1320

ttcccagccc cggaagtgga ggggggacag ggccctggcc ttatttctgt gctacacatt    1380

tccggaaccc aggagtccga ctttaccacc ggcttcaact gcagtgcccg caaccggcta    1440

ggagagggac gagtccagat ccacttgggc cgtagagatt tgctgcctac tgtccggatt    1500

gtggctggtg cagcatctgc agccacctct ctccttatgg tcatcactgg agtggtcctc    1560

tgctgctggc gccatggctc tctctctaag caaaagaact tggtccggat cccaggaagc    1620

agcgagggtt ccagttcacg tggccctgag gaggagacag gcagcagtga ggaccggggt    1680

cccattgtgc acaccgacca cagtgatttg gttcttgagg aaaaagaggc tctggagaca    1740

aaggatccaa ccaacggtta ctacaaggtt cgaggggtca gtgtgagcct tagccttggg 1800

gaagctcctg gaggaggcct cttcttgcca ccgccctctc cgatcggtct cccagggact 1860

cctacttact atgacttcaa gccacatcag gacttagtcc ctccctgcag actgtacaga 1920

gcgagggcag gttatcttac caccccccat ccccgtgcct tcaccagcta catgaaaccc 1980

acatcctttg gacccccaga tttgagctct ggaactcccc ccttcccgta tgctaccttg 2040

tctccaccca gccaccagcg tctccagact catgtgtgaa tccatctctc caagtgaagg 2100

gtcttggaat cttctgtttg ccatatagtg tgttgtccag atttctgggg agtcagaaca 2160

agttgatgac caacccctcc aaaactgaac attgaaggag ggaaagatca ttacaagcat 2220

caggactgtt ggtgtacact cag 2243

<210> 4
<211> 650
<212> PRT
<213> Mus musculus

<400> 4

Met Leu Ala Ser Ala Leu Leu Val Phe Leu Cys Cys Phe Lys Gly His
1               5                   10                  15

Ala Gly Trp Ser Arg Tyr Trp Ile Ser Gly Asn Ser Ala Ser Gly Gln
            20                  25                  30

His Asp Leu His Ile Lys Pro Val Glu Leu Glu Asp Glu Ala Ser Tyr
        35                  40                  45

Glu Cys Gln Ala Ser Gln Ala Gly Leu Arg Ser Arg Pro Ala Gln Leu
    50                  55                  60

His Val Met Val Pro Pro Glu Ala Pro Gln Val Leu Gly Gly Pro Ser
65                  70                  75                  80

Val Ser Leu Val Ala Gly Val Pro Gly Asn Leu Thr Cys Arg Ser Arg
                85                  90                  95

Gly Asp Ser Arg Pro Ala Pro Glu Leu Leu Trp Phe Arg Asp Gly Ile
            100                 105                 110

Arg Leu Asp Ala Ser Ser Phe His Gln Thr Thr Leu Lys Asp Lys Ala
        115                 120                 125

Thr Gly Thr Val Glu Asn Thr Leu Phe Leu Thr Pro Ser Ser His Asp
    130                 135                 140

Asp Gly Ala Thr Leu Ile Cys Arg Ala Arg Ser Gln Ala Leu Pro Thr
145                 150                 155                 160

Gly Arg Asp Thr Ala Val Thr Leu Ser Leu Gln Tyr Pro Pro Met Val
                165                 170                 175

Thr Leu Ser Ala Glu Pro Gln Thr Val Gln Glu Gly Glu Lys Val Thr
            180                 185                 190

Phe Leu Cys Gln Ala Thr Ala Gln Pro Pro Val Thr Gly Tyr Arg Trp
        195                 200                 205

Ala Lys Gly Gly Ser Pro Val Leu Gly Ala Arg Gly Pro Arg Leu Glu
    210                 215                 220

Val Val Ala Asp Ala Thr Phe Leu Thr Glu Pro Val Ser Cys Glu Val
225                 230                 235                 240

Ser Asn Ala Val Gly Ser Ala Asn Arg Ser Thr Ala Leu Glu Val Leu
                245                 250                 255

Tyr Gly Pro Ile Leu Gln Ala Lys Pro Lys Ser Val Ser Val Asp Val
            260                 265                 270

Gly Lys Asp Ala Ser Phe Ser Cys Val Trp Arg Gly Asn Pro Leu Pro
        275                 280                 285

Arg Ile Thr Trp Thr Arg Met Gly Gly Ser Gln Val Leu Ser Ser Gly
    290                 295                 300

Pro Thr Leu Arg Leu Pro Ser Val Ala Leu Glu Asp Ala Gly Asp Tyr
305                 310                 315                 320

Val Cys Arg Ala Glu Pro Arg Arg Thr Gly Leu Gly Gly Gly Lys Ala
                325                 330                 335

Gln Ala Arg Leu Thr Val Asn Ala Pro Pro Val Val Thr Ala Leu Gln
            340                 345                 350

Pro Ala Pro Ala Phe Leu Arg Gly Pro Ala Arg Leu Gln Cys Val Val
        355                 360                 365

Phe Ala Ser Pro Ala Pro Asp Ser Val Val Trp Ser Trp Asp Glu Gly
    370                 375                 380

Phe Leu Glu Ala Gly Ser Leu Gly Arg Phe Leu Val Glu Ala Phe Pro
385                 390                 395                 400

Ala Pro Glu Val Glu Gly Gly Gln Gly Pro Gly Leu Ile Ser Val Leu
                405                 410                 415

His Ile Ser Gly Thr Gln Glu Ser Asp Phe Thr Thr Gly Phe Asn Cys
            420                 425                 430

Ser Ala Arg Asn Arg Leu Gly Glu Gly Arg Val Gln Ile His Leu Gly
        435                 440                 445

Arg Arg Asp Leu Leu Pro Thr Val Arg Ile Val Ala Gly Ala Ala Ser
    450                 455                 460

Ala Ala Thr Ser Leu Leu Met Val Ile Thr Gly Val Val Leu Cys Cys
465                 470                 475                 480

Trp Arg His Gly Ser Leu Ser Lys Gln Lys Asn Leu Val Arg Ile Pro
                485                 490                 495

Gly Ser Ser Glu Gly Ser Ser Ser Arg Gly Pro Glu Glu Glu Thr Gly
            500                 505                 510

Ser Ser Glu Asp Arg Gly Pro Ile Val His Thr Asp His Ser Asp Leu
        515                 520                 525

Val Leu Glu Glu Lys Glu Ala Leu Glu Thr Lys Asp Pro Thr Asn Gly
    530                 535                 540

Tyr Tyr Lys Val Arg Gly Val Ser Val Ser Leu Ser Leu Gly Glu Ala
545                 550                 555                 560

Pro Gly Gly Gly Leu Phe Leu Pro Pro Pro Ser Pro Ile Gly Leu Pro
                565                 570                 575

Gly Thr Pro Thr Tyr Tyr Asp Phe Lys Pro His Gln Asp Leu Val Pro
            580                 585                 590

Pro Cys Arg Leu Tyr Arg Ala Arg Ala Gly Tyr Leu Thr Thr Pro His
        595                 600                 605

Pro Arg Ala Phe Thr Ser Tyr Met Lys Pro Thr Ser Phe Gly Pro Pro
    610                 615                 620

Asp Leu Ser Ser Gly Thr Pro Pro Phe Pro Tyr Ala Thr Leu Ser Pro

625                 630                 635                 640

Pro Ser His Gln Arg Leu Gln Thr His Val
                645                 650

<210>  5
<211>  2750
<212>  DNA
<213>  Mus musculus

<400>  5
aacagccagg agcagtgacc gagccgctgg agctggggag agacgcgcgg aagactgggc      60

caggagacta gggaccgagg gacgcgcgcc tggggagagc caacaaggaa cccgcgggcc     120

ggacagcgac accggcaatc cgcgccaaac tgttccagcc gctggccttc tatagccgca     180

gccccaggac attctaaagc tctccaagac gccccctccc ctggcttctc gcgttgacca     240

aggaaaagaa aaagggatgg aaaaagaaag gaaggagact agaaagaaaa cccagatttg     300

ccaccgcaca aaaagagagg tgggggggac aaggaaaaaa aaaaaagtcg agcgactgtg     360

gggccggaac acaggcagcg ggatcgtggg ccgagcgatg caaggctgcg cgcccaagcg     420

gccgcgagtt gtgactgaag ccaggatgct cgtccaggcg cagtgaagag ccagaccgtg     480

ttgcctcccc aggagtccaa gcgcagggag ggccgctcgg aggacgcggc agactgcctg     540

gcaggccacc ggccgaggtg acagggctgg ggcggtgggg agcgagcgag tgcgcccggc     600

tgcgtccgcc cgaagcggac ggtccctttc catttttgac tggcacaaaa aagaaaactc     660

tccaaagggg tgggggctac ctaagcaaca actacaatca acaaaatatc ctacccaacc     720

cgccatctcc cccacacctc ggtctgcccc cgccccctcc ccaggcccag cgcgggcgcc     780

cagagcgtcc caactcactg caagaaaccg gcaatgtagg atccaaagct ttctactccc     840

gtgttctttt ctttccgtgt tttttttaaa ggggaaaacc cggtggtggg cagtctgaca     900

cgcacacaac ctgccttcat actctgacaa aagcagatgc actttgactt ctgacagctc     960

tacctcaagc tggagagaac ccagctttcc cgaatcctga gctcttggcg tcttcctttt    1020

cgtctgtttc cattttattt atttacgtcc cgccgcctct cacggtgacc ttcactcctt    1080

cgcgggcttt gagcagaaga gccgctttct agcccgcttg agactgattt tcctcgcccg    1140

gtgagctgag gtggcgctgc tccatcccgt tgccccggga ctccggggct gccctctacc    1200

agcctggtct ctcccccttt tgatttgcta gtacgggttt tttgcttgcc caactagaga    1260

gggtttcttc tttttggagg agctggttgt cttcagaagt catcccctcg actctaattg    1320

ccctgtcgct ccgggcctca ccggaccaaa ccaaagacca tggtgcactg tgcgggctgc    1380

aaaaggccca tcctggaccg tttcctcttg aacgtgttgg acagggcctg gcacgtcaag    1440

tgcgtccagt gctgtgaatg taaatgcaac ctgaccgaga agtgcttctc ccgggaaggc 1500

aagctctact gtaaaaacga cttcttccga tgtttcggta ccaaatgcgc cggttgtgcg 1560

cagggcatct ctccaagcga tctggttcgc agagcgcgaa gcaaagtgtt tcacctcaac 1620

tgcttcacct gcatgatgtg taacaagcag ctctccaccg gcgaggagct ctacatcata 1680

gacgagaaca agttcgtttg taaagaggat tacctgagta acagcagtgt cgccaaagag 1740

aacagcctcc actcggccac cacaggcagt gaccctagtt tatctccgga ttcccaagat 1800

ccatcgcagg atgatgccaa ggactctgaa agtgccaacg tctcagataa ggaaggtggt 1860

agtaatgaga atgatgatca gaacctaggt gccaaacgta ggggaccccg gaccacgatc 1920

aaagccaagc aactggagac gttgaaggca gcctttgcag ctacacccaa gcccacacgc 1980

catatccgtg agcaactggc ccaggagact ggcctcaaca tgcgtgttat ccaggtctgg 2040

ttccagaatc gacgctccaa ggagcgaagg atgaaacagc taagcgcgct aggcgcgcgg 2100

cgccacgcct ttttccgcag tcctcgtcgg atgcggccgc tggtggaccg cctggagccg 2160

ggcgaactca tccccaacgg ccccttctcc ttttacggag attaccagag tgagtactac 2220

ggtcccggag gcaactacga cttcttcccg caaggaccgc catcctctca ggctcagacg 2280

ccagtggacc taccctttgt gccatcatct ggcccttcgg ggacgcccct tggaggtctg 2340

gaccacccgc tgcctggtca ccacccttcc agtgaggcgc agcgatttac tgacatcctg 2400

gcacatcccc caggggactc ccctagtcct gagcccagct tgcccgggcc tctccactcc 2460

atgtcagcgg aggtcttcgg gcccagtcca cctttctcat ctctgtcggt caatggtgga 2520

gccagctacg ggaaccattt gtctcaccct cctgaaatga acgaggcagc cgtgtggtag 2580

cggggtctcg catgggccac gggagctcgt ggttgtacag agacgagctt ttatttcaga 2640

aaaatagatt aaaaagacaa aaaaaaaaaa acccccaaaa caaaaaagca agcctcctgc 2700

tccacttcct tcagcctcgg ggaccagtct gtttggggag actggatagc 2750

<210> 6
<211> 406
<212> PRT
<213> Mus musculus

<400> 6

Met Val His Cys Ala Gly Cys Lys Arg Pro Ile Leu Asp Arg Phe Leu
1               5                   10                  15

Leu Asn Val Leu Asp Arg Ala Trp His Val Lys Cys Val Gln Cys Cys
            20                  25                  30

Glu Cys Lys Cys Asn Leu Thr Glu Lys Cys Phe Ser Arg Glu Gly Lys

35                  40                  45

Leu Tyr Cys Lys Asn Asp Phe Phe Arg Cys Phe Gly Thr Lys Cys Ala
    50                  55                  60

Gly Cys Ala Gln Gly Ile Ser Pro Ser Asp Leu Val Arg Arg Ala Arg
65                  70                  75                  80

Ser Lys Val Phe His Leu Asn Cys Phe Thr Cys Met Met Cys Asn Lys
                85                  90                  95

Gln Leu Ser Thr Gly Glu Glu Leu Tyr Ile Ile Asp Glu Asn Lys Phe
            100                 105                 110

Val Cys Lys Glu Asp Tyr Leu Ser Asn Ser Ser Val Ala Lys Glu Asn
        115                 120                 125

Ser Leu His Ser Ala Thr Thr Gly Ser Asp Pro Ser Leu Ser Pro Asp
    130                 135                 140

Ser Gln Asp Pro Ser Gln Asp Asp Ala Lys Asp Ser Glu Ser Ala Asn
145                 150                 155                 160

Val Ser Asp Lys Glu Gly Gly Ser Asn Glu Asn Asp Asp Gln Asn Leu
                165                 170                 175

Gly Ala Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Val Asp Arg Leu Glu Pro Gly Glu Leu Ile Pro
            260                 265                 270

Asn Gly Pro Phe Ser Phe Tyr Gly Asp Tyr Gln Ser Glu Tyr Tyr Gly
        275                 280                 285

Pro Gly Gly Asn Tyr Asp Phe Phe Pro Gln Gly Pro Pro Ser Ser Gln
    290                 295                 300

Ala Gln Thr Pro Val Asp Leu Pro Phe Val Pro Ser Ser Gly Pro Ser
305                 310                 315                 320

Gly Thr Pro Leu Gly Gly Leu Asp His Pro Leu Pro Gly His His Pro
                325                 330                 335

Ser Ser Glu Ala Gln Arg Phe Thr Asp Ile Leu Ala His Pro Pro Gly
            340                 345                 350

Asp Ser Pro Ser Pro Glu Pro Ser Leu Pro Gly Pro Leu His Ser Met
        355                 360                 365

Ser Ala Glu Val Phe Gly Pro Ser Pro Pro Phe Ser Ser Leu Ser Val
    370                 375                 380

Asn Gly Gly Ala Ser Tyr Gly Asn His Leu Ser His Pro Pro Glu Met
385                 390                 395                 400

Asn Glu Ala Ala Val Trp
                405

<210>  7
<211>  2283
<212>  DNA
<213>  Homo sapiens

<400>  7
cggccgcgag ttgtgactgg agccacgatg cacggccagg cgcggtgaga agccagcccg      60

tagtgcctcc cgaaggagcc cgggcgcagg gagggtcgcc ctgaggacac ggaggccgcc     120

aggcaggcca agggccgagg tgactgggct ggggcggtag ggaaggagcg agtgcgcctg     180

gctgcctccg cacggagttg tccctctctg ttttcgattg acacaaacac ttctccaaaa     240

gcggggaaac ctaagcaaca acagcaatca acaccaagat cttcctccta ccctcccctc     300

tttcccttct cccgcggtcg gccctcgccc cctcccccag gcccagcgcg ggcgctcggc     360

gcgtccagac ccgcggcgcg atgccggcag tttaggatcc aaagcttctc tgctcctttt     420

gttctttcct tccctttttt aaaaaaagag gggggaaatc ccagtggtgg gcagcctggc     480

acgcacacag tcgccctcat accccgacaa aagcagatgc actttgactt ctgacagctc     540

tacctcaagc cccggagaac tcagcggcgc tttcctcgca acccgagctc ggcgagtcgt     600

cgtcttcttc ttctccgttt ttatttattt atttccgttc ccgccgccgt tctcgctgac     660

cttcactcct ccgcgggctc tgagcagaag ggtcgcattc tctcccgcct gagacttctt 720

ttcctcgccc cgggagctca ggcggcgccg ctccagcccg gggccccggg actccccggc 780

tgcacacttc actgagacgc ccccccaggc cccgatcagc ctcgtttcct ccaccctact 840

ttgatttcct ggtgcgagtt ttggcttgca cggccgagtg tgtgtcctct ttttggagag 900

actggggagc tcgtgccgat tgtcttcagg agtcatcccc tgggctctac tttgcccctc 960

tctctctctg ggcctcatca gaccaaacca aagaccatgg ttcactgtgc cggctgcaaa 1020

aggcccatcc tggaccgctt tctcttgaac gtgctggaca gggcctggca cgtcaagtgc 1080

gtccagtgct gtgaatgtaa atgcaacctg accgagaagt gcttctccag ggaaggcaaa 1140

ctctactgca agaacgactt cttccggtgt ttcggtacca aatgcgcagg ctgcgctcag 1200

ggcatctccc ctagcgacct ggtgcggaga gcgcggagca aagtgtttca cctgaactgc 1260

ttcacctgca tgatgtgtaa caagcagctc tccactggcg aggaactcta catcatcgac 1320

gagaataagt tcgtctgcaa agaggattac ctaagtaaca gcagtgttgc caaagagaac 1380

agccttcact cggccaccac gggcagtgac cccagtttgt ctccggattc ccaagacccg 1440

tcgcaggacg acgccaagga ctcggagagc gccaacgtgt cggacaagga agcgggtagc 1500

aacgagaatg acgaccagaa cctgggcgcc aagcggcggg gaccgcgcac caccatcaaa 1560

gccaagcagc tggagacgct gaaggccgcc ttcgctgcta cacccaagcc cacccgccac 1620

atccgcgagc agctggcgca ggagaccggc ctcaacatgc gcgtcattca ggtctggttc 1680

cagaaccggc gctccaagga gcggaggatg aagcagctga gcgccctggg cgcccggcgc 1740

cacgccttct tccgcagtcc gcgccggatg cggccgctgg tggaccgcct ggagccgggc 1800

gagctcatcc ccaatggtcc cttctccttc tacggagatt accagagcga gtactacggg 1860

cccgggggca actacgactt cttcccgcaa ggccccccgt cctcgcaggc ccagacacca 1920

gtggacctac ccttcgtgcc gtcatctggg ccgtccggga cgcccctggg tggcctggag 1980

cacccgctgc cgggccacca cccgtcgagc gaggcgcagc ggtttaccga catcctggcg 2040

cacccacccg gggactcgcc cagccccgag cccagcctgc ccgggcctct gcactccatg 2100

tcggccgagg tcttcggacc cagcccgccc ttctcgtcgc tgtcggtcaa cggtggggcg 2160

agctacggaa accacctgtc ccaccccccc gaaatgaacg aggcggccgt gtggtagcgg 2220

ggtctcgcac ggtctgcgga gttcgtggtt gtacagaaat gaacctttat ttaagaaaaa 2280

tag 2283

<210> 8
<211> 406
<212> PRT

<213>  Homo sapiens

<400>  8

Met Val His Cys Ala Gly Cys Lys Arg Pro Ile Leu Asp Arg Phe Leu
1               5                   10                  15

Leu Asn Val Leu Asp Arg Ala Trp His Val Lys Cys Val Gln Cys Cys
            20                  25                  30

Glu Cys Lys Cys Asn Leu Thr Glu Lys Cys Phe Ser Arg Glu Gly Lys
        35                  40                  45

Leu Tyr Cys Lys Asn Asp Phe Phe Arg Cys Phe Gly Thr Lys Cys Ala
    50                  55                  60

Gly Cys Ala Gln Gly Ile Ser Pro Ser Asp Leu Val Arg Arg Ala Arg
65                  70                  75                  80

Ser Lys Val Phe His Leu Asn Cys Phe Thr Cys Met Met Cys Asn Lys
                85                  90                  95

Gln Leu Ser Thr Gly Glu Glu Leu Tyr Ile Ile Asp Glu Asn Lys Phe
            100                 105                 110

Val Cys Lys Glu Asp Tyr Leu Ser Asn Ser Ser Val Ala Lys Glu Asn
        115                 120                 125

Ser Leu His Ser Ala Thr Thr Gly Ser Asp Pro Ser Leu Ser Pro Asp
    130                 135                 140

Ser Gln Asp Pro Ser Gln Asp Asp Ala Lys Asp Ser Glu Ser Ala Asn
145                 150                 155                 160

Val Ser Asp Lys Glu Ala Gly Ser Asn Glu Asn Asp Asp Gln Asn Leu
                165                 170                 175

Gly Ala Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln

225                 230                 235                 240

Leu Ser Ala Leu Gly Ala Arg Arg His Ala Phe Phe Arg Ser Pro Arg
                245                 250                 255

Arg Met Arg Pro Leu Val Asp Arg Leu Glu Pro Gly Glu Leu Ile Pro
            260                 265                 270

Asn Gly Pro Phe Ser Phe Tyr Gly Asp Tyr Gln Ser Glu Tyr Tyr Gly
        275                 280                 285

Pro Gly Gly Asn Tyr Asp Phe Phe Pro Gln Gly Pro Pro Ser Ser Gln
    290                 295                 300

Ala Gln Thr Pro Val Asp Leu Pro Phe Val Pro Ser Ser Gly Pro Ser
305                 310                 315                 320

Gly Thr Pro Leu Gly Gly Leu Glu His Pro Leu Pro Gly His His Pro
                325                 330                 335

Ser Ser Glu Ala Gln Arg Phe Thr Asp Ile Leu Ala His Pro Pro Gly
            340                 345                 350

Asp Ser Pro Ser Pro Glu Pro Ser Leu Pro Gly Pro Leu His Ser Met
        355                 360                 365

Ser Ala Glu Val Phe Gly Pro Ser Pro Pro Phe Ser Ser Leu Ser Val
    370                 375                 380

Asn Gly Gly Ala Ser Tyr Gly Asn His Leu Ser His Pro Pro Glu Met
385                 390                 395                 400

Asn Glu Ala Ala Val Trp
                405

<210>  9
<211>  2405
<212>  DNA
<213>  Mus musculus

<400>  9
aggacacctg ctcgaagctg gagcgagcgc ccggtcgcga cccgtgacat gaggctgtga      60

ccgctgccgc cctccacgcc actctgggca gtgcagcgcc aggccggaga gcgtcggagg     120

acttgacccc gagaagtctt ggttgatccg taacggactc gcccctacag actcgcctac     180

agactaggaa ggctgagagc cacagcagcg gggaccgaga gggcctaagg gcccaggggc     240

cccaaggagg acgaggcggc ccgagccgcc ggggcgcgcg gctatgatgg tgcactgtgc 300

tggctgtgag cggcccatcc tcgaccgctt tctgctgaac gtactagacc gcgcgtggca 360

tatcaaatgt gttcaatgct gcgagtgcaa aaccaacctc tcggagaagt gcttctcacg 420

ggaaggcaag ctatactgta aaaacgactt tttcaggcgc tttggcacaa agtgcgccgg 480

ctgcgcgcaa ggtatctctc cgagcgacct ggtacggaag gcccggagca aagtcttcca 540

cctcaactgc ttcacctgta tggtgtgcaa taagcagcta tccaccggag aggagctcta 600

cgtgatcgac gagaacaagt ttgtgtgcaa ggacgactac ttaagctcct ctagcctcaa 660

ggaaggaagt ctcaactcgg tgtcgtcctg tacggaccgc agtttgtccc cggacctcca 720

ggatccgtta caggacgacc ccaaagagac cgacaattcg acctcatcgg acaaggaaac 780

cgctaacaac gagaatgagg aacagaactc cggcaccaaa cggcgcggcc cgcgcaccac 840

catcaaggcc aagcagctgg agacgctcaa ggcagccttc gcagccacgc ccaagcccac 900

gcgccacatt cgcgaacagc tggcacagga gacgggcctc aacatgaggg tcattcaggt 960

gtggtttcag aaccgaaggt ccaaagaacg ccgcatgaaa cagctgagcg ctctgggcgc 1020

gcggagacac gccttcttcc ggagtccgcg gcgcatgcgt cccctgggcg gccgcttgga 1080

cgagtctgag atgttggggt ctaccccata cacttattac ggagactacc aaagtgacta 1140

ctacgctccg ggaggcaatt acgatttctt cgcgcacggt ccgccgtcac aggcgcagtc 1200

ccctgccgac tccagcttcc tggcagcatc gggacctggc tcgacgccgc tgggcgcgct 1260

ggaaccgccg ctggccgggc ctcacggcgc ggacaacccc agattcaccg acatgatctc 1320

gcatccggac acgccgagcc ccgagccggg cctgcccggt gcgctgcacc ccatgccggg 1380

agaggtgttc agcggcgggc ccagcccgcc cttccccatg agcggcacca gcggctacag 1440

tggacccctg tcgcacccca accctgagct caacgaagcg gccgtatggt aaggccgagg 1500

ggctgagttg tccccctgcc accaagcccg ggacgggacg ccgcctgggt aagcctcaag 1560

agtcctctcg tgggttcgca cccaaccagg ccactcgcat caccacccct cagagctttg 1620

gcacgcgcct gcgcaatttc tcgggaccaa agtcaatatt ctgaagggtc gagattccaa 1680

gcacatctta gaagccctcc ggatccccca cccatcatca cctccttgaa ctaagagagg 1740

gggatgaggc caaggagcgg agaccatggc actacccctc cctgcgagcc gaggcattgt 1800

gaaatcctat ttctcacttt ctcttttaaa aaaagaaaga aagaaggaag gaaggaagga 1860

aggaaagaaa gaaagagagg ttgaaagggg gagagaaaga gagagagaga gagagagaga 1920

gagagagaga gagagagaga gagagagaga gagagagaga gagagagaga gagagagaga 1980

gcgcgagcga gctgaggaaa gctcagccag agaagaaaaa tgaggaggac cgccggtgaa 2040

tggtggcttt gcaggaagac aaatccacct gcgagttggc gccccctggt ggctcaatgt 2100

cagcttgtct aggaaggtgc gcggtctggg cctggccttc ctgagcccaa ctccctgctc 2160

ctccatactt tgagtctgag gggcctggca ggattcgaac cctcccaccc tgctctgacc 2220

ctgagccggg cgccaagtca ttgagcattt gcccacggat cactctccct gcccgggacc 2280

tggaggctgg gccatcagga cgaacagtat tatacttttt tggaagtcgg acgcttctag 2340

tttccttatt ttgtataaag aagaaacaaa taaagtatgt ttttgtgaaa aaaaaaaaaa 2400

aaaaa 2405

<210> 10
<211> 402
<212> PRT
<213> Mus musculus

<400> 10

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Pro Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly
            260                 265                 270

Ser Thr Pro Tyr Thr Tyr Tyr Gly Asp Tyr Gln Ser Asp Tyr Tyr Ala
        275                 280                 285

Pro Gly Gly Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Gly Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Ala Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala
385                 390                 395                 400

Val Trp

<210>  11
<211>  2084
<212>  DNA
<213>  Homo sapiens

<400>  11
aaccaggtac aagctaatac tcaacaatac tgatgccttg ttttttttgc tctgtccgga      60

cagcaacgct gtagccaatt tagatatgct ataaatttaa gaggttgcca tggccacggt     120

gcgcccattg gccgctgggc cccctacgtg cagcgccacg tcaccaaatc tgaataagga     180

tgcgcgaatt acgcggcgac cagacaaaga tgaggatccg gaccgcttga aagtggggga     240

aagtgccggc gcctccgcca ccggggaaag ccgctccgca gcgccgaggc cagcagccac     300

ccgagatacc tggggaagcc cggaacaggc gccggggcgt gcggcccgtg gcatgaggtt     360

gtgaacgcca cccgcccccc accaccccac tccgggcagc ccagcgccag gccagagatt     420

gcccaaggac tggaccggct gagtcttggt ccggaccaga ctcgccctgc agctgctgag     480

acaagaggcg aagggcagcg gagggcccgg caggcccgag ggccaggggc ccaaagggag     540

ggcaaggcgg ccgaagccgc cggggcgcgg ggctatgatg gtgcactgcg ccggttgcga     600

gcggcccatc ctcgaccgct ttctgctgaa cgtgctggac cgcgcgtggc acatcaaatg     660

tgttcagtgc tgcgagtgca aaaccaacct ctcggagaag tgcttctcgc gcgagggcaa     720

gctctactgc aaaaatgact ttttcaggcg ctttggcacg aaatgcgccg gctgcgcgca     780

aggcatctcg cccagcgacc tggtgcgcaa ggcccggagc aaagtctttc acctcaactg     840

tttcacctgc atggtgtgta acaagcagct gtccaccggc gaggagctct acgtcatcga     900

cgagaacaag ttcgtgtgca aagacgacta cctgagctca tccagcctca aggagggcag     960

cctcaactca gtgtcatcct gtacggaccg cagtttgtcc ccggacctcc aggacgcact    1020

gcaggacgac cccaaagaga cggacaactc gacctcgtcg gacaaggaga cggccaacaa    1080

cgagaacgag gagcagaact cgggcaccaa gcggcgcggc ccccgcacca ccatcaaggc    1140

caagcagctg gagacgctca aggctgcctt cgccgccacg cccaagccca cgcgccacat    1200

ccgcgagcag ctggcgcagg agaccggcct caacatgcgc gtcatccagg tgtggtttca    1260

gaaccgacgg tccaaagaac gccggatgaa acagctgagc gccctaggcg cccggaggca    1320

cgccttcttc cggagtccgc ggcgcatgcg tccgctgggc ggccgcttgg acgagtctga    1380

gatgttgggg tccaccccgt acacctacta cggagactac caaggcgact actacgcgcc    1440

gggaagcaac tacgacttct tcgcgcacgg cccgccttcg caggcgcagt ccccggccga    1500

ctccagcttc ctggcggcct ctggccccgg ctcgacgccg ctgggagcgc tggaaccgcc    1560

gctcgccggc ccgcacgccg cggacaaccc caggttcacc gacatgatct cgcacccgga 1620

cacaccgagc cccgagccag gcctgccggg cacgctgcac cccatgcccg gcgaggtatt 1680

cagcggcggg cccagcccgc ccttcccaat gagcggcacc agcggctaca gcggacccct 1740

gtcgcatccc aaccccgagc tcaacgaagc cgccgtgtgg taaggccgcc gggccgcccc 1800

ccgcgctcgg cccccggggg ccccgccccg aagcagcctc ctgaaaccaa aacgcccgac 1860

gcagacgcgg tgggagacgt gggtgtccct cgggggttct ctctcgggtc cgcactcaac 1920

tggcagctgc tcctcggctg ggcgccgagg gggggccgac ccccatctcc accccgcggg 1980

ctctccagga gcctcagccc accgccagta ctctcccagc aaccgcgagc aatttcttgg 2040

gaccaaagtc aatactccgg agggtcaaga gatttcgagc acgc 2084

<210> 12
<211> 402
<212> PRT
<213> Homo sapiens

<400> 12

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Ala Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly
            260                 265                 270

Ser Thr Pro Tyr Thr Tyr Tyr Gly Asp Tyr Gln Gly Asp Tyr Tyr Ala
        275                 280                 285

Pro Gly Ser Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Ala Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Thr Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala
385                 390                 395                 400

Val Trp

<210>  13
<211>  1914
<212>  DNA
<213>  Rattus norvegicus

<400>  13
cggcgcctcc gccaaccggg aaagccgctc gccagtgccg aagccagcag cctccgagga      60

cacctgctcg aagctggagc gagcgcccag acgcgacccg tgacatgagg ctgtgaccgc     120

cgccgccctc cacgccactc tgggcagtgc agtgccaggc cggagagcgt cggaggactt     180

gaccccgaga agtcttggtt gatctgtatc ggactcgacc ctaccgaaga ccacagacca     240

ggggggctga gagccacaga ccaggggggc tgagagccac agcagcgggg accgagaggc     300

cctaagggcc caggggcccc aaggaggacg aggcggcccg agccgccggg gcgcgcggct     360

atgatggtgc actgtgccgg ctgtgagcgg cccatcctcg accgctttct gctgaacgtg     420

ctggaccgcg cgtggcatat caaatgtgtt caatgctgcg agtgcaaaac caacctctcg     480

gagaagtgtt tctcccggga gggcaagctg tactgtaaaa acgacttctt caggcgcttt     540

ggcacaaagt gcgccggctg tgcgcaaggt atctctccca gcgacctggt gcgcaaggcc     600

cggagcaaag tcttccacct caactgcttc acctgcatgg tgtgcaacaa gcagctgtcc     660

accggagaag aactctacgt gatcgacgag aacaagtttg tgtgcaagga cgactactta     720

agctcctcta gtctcaaaga gggaagcctc aactcagtgt catcctgtac ggaccgcagt     780

ttgtctccgg acctccaaga tccgttacag gacgacccca aagagaccga caactcgacc     840

tcatcggaca aggagaccgc taacaacgag aatgaggaac agaactccgg caccaaacgg     900

cgcggcccgc gcactaccat caaggccaag cagctggaga cgctcaaggc agccttcgca     960

gccacgccca agcccacgcg ccacatccgc gaacagctgg cacaagagac cggcctcaac    1020

atgagggtca ttcaggtgtg gtttcagaac cgaaggtcca aagaacgccg catgaaacag    1080

ctgagcgctc tgggcgcccg gagacacgcc ttcttccgga gtccgcggcg catgcgtccc    1140

ctgggcggcc gcttggacga gtctgagatg ttggggtcta ccccatatac ttattatgga    1200

gactaccaaa gcgactacta cgctccggga ggcaactacg atttcttcgc gcacggcccg    1260

ccgtcgcagg cacagtctcc ggccgactca agcttcttgg cagcatcggg acctggctcg    1320

acgccgcttg gcgcgctgga accaccgctg gctgggcctc acggcgcgga caaccctagg    1380

ttcaccgaca tgatctcgca cccggacacg cccagtccgg agccaggctt gcccggagcg    1440

ctgcacccca tgccgggaga ggtgttcagc ggcgggccca gcccgccctt ccccatgagc 1500

ggcaccagcg gctacagcgg acccctgtcg caccccaatc ctgagctcaa cgaagcggcc 1560

gtatggtaag gccgaggggc cgagttgacc cctgccacca agccccggac gccgcctggg 1620

taagccacaa gagtcttctc ttgagtttgc acccaccagg caactcgcat caccacccct 1680

cagagcttcg gcacgcgcct gcacagtttc tcgggaccaa agtcaatatt ctggagggtc 1740

gagattccaa gcacacccta gaagccctcc ggacccccac ccaaccatca cctctttgaa 1800

ttaagagggg gaggggatga gacaaggaac ggagatcgtg gtactacccc tccctgcgag 1860

ccgaggcatt gtggaatcct atttctcgct ttctcttttt aaaaagggga attc 1914

<210> 14
<211> 402
<212> PRT
<213> Rattus norvegicus

<400> 14

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Pro Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly
            260                 265                 270

Ser Thr Pro Tyr Thr Tyr Tyr Gly Asp Tyr Gln Ser Asp Tyr Tyr Ala
        275                 280                 285

Pro Gly Gly Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Gly Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Ala Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala

385                 390                 395                 400

Val Trp

<210>  15
<211>  4156
<212>  DNA
<213>  Mus musculus

<400>  15
gagttcagag acagagaaag gcgctgtcag actacgctcc acttggggct cgcttgccgc      60

gggtttccgc gcacccacac tttctgtggt ggtggggaca ccctgcccca atccaggccc     120

ccattctgcc caccaccccc ccgccgggat ttgagcgcca ttagctcgcg ccagtaccgg     180

gatcttcgct cagctcgcct gagtcccgcc cgctggattc gaggacccgc ttggatgctt     240

cgcctccgag cgccctcgga agatgggccg gcagctacaa gcctaaagac gtcaagggct     300

taccagtctc caaggcaaac ccggagctct aagcccgctg ttcttccgtg gaggagatgg     360

cttccagccc actgccaggg cccaatgata tcctacttgc atcgccatcg agcgccttcc     420

agcccgacgc attgagccaa ccgcggccgg gtcacgccaa ccttaaaccc aaccaggtgg     480

gccaggtgat cctctatggc attcccatcg tgtccttggt gatcgacggg caggagcgcc     540

tgtgcctggc gcagatctcc aacactcttc tcaagaactt cagctataac gagatccaca     600

accgccgagt ggctctgggc atcacgtgcg tgcagtgcac acctgtccaa ctggagatcc     660

tgcggcgggc cggggccatg cccatctctt cgcggcgttg tggcatgatc accaagcgtg     720

aggccgagcg cctttgtaag tcattcctgg gcgaaaatcg gccacccaag ctgccggaca     780

acttcgcctt cgacgtgtcg cacgagtgtg cctggggctg ccgcggcagc ttcatcccgg     840

cgcgctacaa cagctctcgc gccaagtgca tcaagtgtag ctactgcaac atgtacttct     900

cccccaacaa gttcattttc cactcccacc gcaccccaga cgccaagtac acacagccag     960

acgcggctaa cttcaattcc tggcgccgcc atctcaagct cacagacaag agccctcagg    1020

acgagctagt cttcgcctgg gaggacgtca aggccatgtt caacggtggc agccgcaagc    1080

gcgcgctacc tcaacccagc gcgcacccgg cctgtcaccc actcagttcc gtcaaggctg    1140

ctgcggtggc ggccgcagcc gcagtggccg gggcggaggg cctgctgggc ccgcacctgc    1200

tgggggctcc acccccgcca ccgccgccac cacccttggc tgagctggcg ggcgcacctc    1260

acgcccatca caagcggccg cgcttcgacg acgatgacga ttccctccag gaagctgccg    1320

tggtggctgc agccagcctc tcggcagccg cagccagcct ctcggtggcc gcagccacag    1380

gcggcgccgg gccaggcgca ggtggccccg ggggtggctg cgtggccggc gtgggcgtgg    1440

gtgccagtgc gggggctggt gcagcagctg gcaccaaagg tccacgcagc tacccagtca 1500
tcccagtgcc cagcaagggt tcgttcgggg gcgtgctaca gaagttcccg ggctgcgggg 1560
gcctcttccc gcatccttac accttcccgg cagcggccgc agccttcggc ttgtgccaca 1620
agaaggagga cgcggggaca gcggccgagg ccctgggggg agcgggcgcg gggagcgcag 1680
gtgcggcgcc caaggcaggg ctgtcgggtc tcttctggcc cgcgggtcgc aaggacgcct 1740
tctaccctcc cttctgcatg ttctggccac cgcggacccc cggcgggctg cccgtgccca 1800
cctacctaca gcccccgccg cagcccccgt ctgcgctcgg ctgcgcgctg ggtgatagcc 1860
cggccctgct gcgtcaggcc ttcctggacc tggccgagcc gggcggtgca ggtggcagcg 1920
ccgaggcagc gccccctccg ggccaacctc cccccgtggt ggccaatggc cctggctccg 1980
gtcctccagc tactgggggc actggagcac gcgacacgct cttcgagtcg cccccgggcg 2040
gcagcggcgg ggactgcagc gccgggtcca cgccacccgc agagcaagga gtgacgtccg 2100
ggaccgggtc tgcgtcctcc ggagcaggct ctgtgggcac ccgagtgccg gctccccatc 2160
acccgcacct cctggaaggg cgcaaggcgg gcggcggcag ctaccaccat tccagcgcct 2220
tccgtccggt gggcggcaag gacgacgcag aaagcctggc caagctgcac ggggcgtcgg 2280
cgggcacacc ccactcagcc ccagcgcatc accatcacca ccaccatcac ccgcaccatc 2340
accaccatca ccctccgcag ccgccgtcgc cactgctgct gttgcagccc cagcccgatg 2400
agccggggtc ggagcgccac cacccagccc cgccaccccc gccaccgccg ccccctctgg 2460
ccccgcagcc gcaccaccga ggccttctgt cccccgaggg caccagctgc agctacccca 2520
gtgaggacag ctctgaagac gaggaggacg aggaggaaga gcaggaggtg gacgtggagg 2580
gccacaagcc actcgaaggc gaggaagagg aggacggtcg cgatcctgaa gatgaggagg 2640
aagaagatga ggagacccgg gtccttctag gagactccct ggttggcggt ggccggttcc 2700
tccagggccg agggctatcg gagaagggga gcggtcggga ccgcacgacg cccgccgtgg 2760
gtgctttccc tctagcgctg aactcctcca ggctgctaca agaggatggg aaactggggg 2820
actctggagg ctcggacctg ccggcgcccc cgcccccacc cctggccccc cagaaagcaa 2880
gcagcagtgg gggcagcagg ccaggcagcc ctgtccacca tccatcactg gaggaggagc 2940
cctcgtacaa agataatcag aaacctaagg aaaacaacca agttattata tctacaaagg 3000
atgacaactt ctcagataag aacaagggac atggcttctt catcacagat tctgattctt 3060
ctggagactt ttggagagaa agatcaggtg aacatacaca agaaaccaat tcacctcatt 3120
cgctcaaaaa ggatgtagaa aacatggaaa aagaggaact tcagaaggtt ttgtttgagc 3180
aaatagattt gcggaggcgg ctggagcaag aattccaagt gttaaaagga aatacgtcct 3240
tcccagtctt caataacttt caggatcaga tgaaaaggga actggcttac cgagaagaga 3300

tggtgcaaca gttacaaatc atcccctatg cagcaagctt gatcaggaaa gagaagcttg 3360

gcgcccatct cagcaaaagc taaaaggcga cagacccact cactcttgtt ctgtaagata 3420

cagcctacca ctgagcactt cggacctgca gaaagaagaa ctgcaaactg aaagggcttg 3480

ggcaccaaaa ccaggttcga gagaagccaa ggacaagtga cctcgcgcca gcatgggcaa 3540

ccatgtaaaa tagactgtgg cggccattca ttaggagggg ggggaggggg gagccaacca 3600

gagcggtcaa tgcttgtcac atcttttggt ggaaccaaag tttgaatatt tgtgttttga 3660

aagcatagct gatcccagag ggtaggaagt gctgagtggg gaaatgtttg tgtggtctct 3720

ttggcgccat acgattatcc tttgctcttc cggaagtaat tcatagtgga aagaggacag 3780

aatggggact taggtttaga caaacctgct ttccatacca agctggacag acacacatgg 3840

ccccttcctc tctggtactt tgcctgctgt atgaagattg tattcctctg gaaatatttt 3900

acagtttaat attgagtgta attaagaata taatcatgtt atcaaaaatg gtatttaact 3960

ctgttgtagt ttctttaaca ttcatgtgga taaaaagttt ataataaaaa aactatgacg 4020

taatagatgt gttcatgtag ttaagtgcat atatgcttgg gggcaactca gaaacgtaat 4080

gctttttaga gttattttgg cataaagtat ttgaatataa ttatttttga aaacaaaaaa 4140

aaaaaaaaaa aaaaaa 4156

<210> 16
<211> 1008
<212> PRT
<213> Mus musculus

<400> 16

Met Ala Ser Ser Pro Leu Pro Gly Pro Asn Asp Ile Leu Leu Ala Ser
1               5                   10                  15

Pro Ser Ser Ala Phe Gln Pro Asp Ala Leu Ser Gln Pro Arg Pro Gly
            20                  25                  30

His Ala Asn Leu Lys Pro Asn Gln Val Gly Gln Val Ile Leu Tyr Gly
        35                  40                  45

Ile Pro Ile Val Ser Leu Val Ile Asp Gly Gln Glu Arg Leu Cys Leu
    50                  55                  60

Ala Gln Ile Ser Asn Thr Leu Leu Lys Asn Phe Ser Tyr Asn Glu Ile
65                  70                  75                  80

His Asn Arg Arg Val Ala Leu Gly Ile Thr Cys Val Gln Cys Thr Pro
                85                  90                  95

Val Gln Leu Glu Ile Leu Arg Arg Ala Gly Ala Met Pro Ile Ser Ser
            100                 105                 110

Arg Arg Cys Gly Met Ile Thr Lys Arg Glu Ala Glu Arg Leu Cys Lys
        115                 120                 125

Ser Phe Leu Gly Glu Asn Arg Pro Pro Lys Leu Pro Asp Asn Phe Ala
    130                 135                 140

Phe Asp Val Ser His Glu Cys Ala Trp Gly Cys Arg Gly Ser Phe Ile
145                 150                 155                 160

Pro Ala Arg Tyr Asn Ser Ser Arg Ala Lys Cys Ile Lys Cys Ser Tyr
                165                 170                 175

Cys Asn Met Tyr Phe Ser Pro Asn Lys Phe Ile Phe His Ser His Arg
            180                 185                 190

Thr Pro Asp Ala Lys Tyr Thr Gln Pro Asp Ala Ala Asn Phe Asn Ser
        195                 200                 205

Trp Arg Arg His Leu Lys Leu Thr Asp Lys Ser Pro Gln Asp Glu Leu
    210                 215                 220

Val Phe Ala Trp Glu Asp Val Lys Ala Met Phe Asn Gly Gly Ser Arg
225                 230                 235                 240

Lys Arg Ala Leu Pro Gln Pro Ser Ala His Pro Ala Cys His Pro Leu
                245                 250                 255

Ser Ser Val Lys Ala Ala Ala Val Ala Ala Ala Ala Ala Val Ala Gly
            260                 265                 270

Gly Gly Gly Leu Leu Gly Pro His Leu Leu Gly Ala Pro Pro Pro Pro
        275                 280                 285

Pro Pro Pro Pro Pro Leu Ala Glu Leu Ala Gly Ala Pro His Ala His
    290                 295                 300

His Lys Arg Pro Arg Phe Asp Asp Asp Asp Asp Ser Leu Gln Glu Ala
305                 310                 315                 320

Ala Val Val Ala Ala Ala Ser Leu Ser Ala Ala Ala Ala Ser Leu Ser
                325                 330                 335

Val Ala Ala Ala Thr Gly Gly Ala Gly Pro Gly Ala Gly Gly Pro Gly
            340                 345                 350

Gly Gly Cys Val Ala Gly Val Gly Val Gly Ala Ser Ala Gly Ala Gly
        355                 360                 365

Ala Ala Ala Gly Thr Lys Gly Pro Arg Ser Tyr Pro Val Ile Pro Val
    370                 375                 380

Pro Ser Lys Gly Ser Phe Gly Gly Val Leu Gln Lys Phe Pro Gly Cys
385                 390                 395                 400

Gly Gly Leu Phe Pro His Pro Tyr Thr Phe Pro Ala Ala Ala Ala Ala
                405                 410                 415

Phe Gly Leu Cys His Lys Lys Glu Asp Ala Gly Thr Ala Ala Glu Ala
            420                 425                 430

Leu Gly Gly Ala Gly Ala Gly Ser Ala Gly Ala Ala Pro Lys Ala Gly
        435                 440                 445

Leu Ser Gly Leu Phe Trp Pro Ala Gly Arg Lys Asp Ala Phe Tyr Pro
    450                 455                 460

Pro Phe Cys Met Phe Trp Pro Pro Arg Thr Pro Gly Gly Leu Pro Val
465                 470                 475                 480

Pro Thr Tyr Leu Gln Pro Pro Pro Gln Pro Pro Ser Ala Leu Gly Cys
                485                 490                 495

Ala Leu Gly Asp Ser Pro Ala Leu Leu Arg Gln Ala Phe Leu Asp Leu
            500                 505                 510

Ala Glu Pro Gly Gly Ala Gly Gly Ser Ala Glu Ala Ala Pro Pro Pro
        515                 520                 525

Gly Gln Pro Pro Pro Val Val Ala Asn Gly Pro Gly Ser Gly Pro Pro
    530                 535                 540

Ala Thr Gly Gly Thr Gly Ala Arg Asp Thr Leu Phe Glu Ser Pro Pro
545                 550                 555                 560

Gly Gly Ser Gly Gly Asp Cys Ser Ala Gly Ser Thr Pro Pro Ala Glu
                565                 570                 575

Gln Gly Val Thr Ser Gly Thr Gly Ser Ala Ser Ser Gly Ala Gly Ser
            580                 585                 590

Val Gly Thr Arg Val Pro Ala Pro His His Pro His Leu Leu Glu Gly
        595                 600                 605

Arg Lys Ala Gly Gly Gly Ser Tyr His His Ser Ser Ala Phe Arg Pro
    610                 615                 620

Val Gly Gly Lys Asp Asp Ala Glu Ser Leu Ala Lys Leu His Gly Ala
625                 630                 635                 640

Ser Ala Gly Thr Pro His Ser Ala Pro Ala His His His His His His
                645                 650                 655

His His Pro His His His His His His Pro Pro Gln Pro Pro Ser Pro
            660                 665                 670

Leu Leu Leu Leu Gln Pro Gln Pro Asp Glu Pro Gly Ser Glu Arg His
        675                 680                 685

His Pro Ala Pro Pro Pro Pro Pro Pro Pro Pro Pro Leu Ala Pro Gln
    690                 695                 700

Pro His His Arg Gly Leu Leu Ser Pro Glu Gly Thr Ser Cys Ser Tyr
705                 710                 715                 720

Pro Ser Glu Asp Ser Ser Glu Asp Glu Glu Asp Glu Glu Glu Glu Gln
                725                 730                 735

Glu Val Asp Val Glu Gly His Lys Pro Leu Glu Gly Glu Glu Glu Glu
            740                 745                 750

Asp Gly Arg Asp Pro Glu Asp Glu Glu Glu Glu Asp Glu Glu Thr Arg
        755                 760                 765

Val Leu Leu Gly Asp Ser Leu Val Gly Gly Gly Arg Phe Leu Gln Gly
    770                 775                 780

Arg Gly Leu Ser Glu Lys Gly Ser Gly Arg Asp Arg Thr Thr Pro Ala
785                 790                 795                 800

Val Gly Ala Phe Pro Leu Ala Leu Asn Ser Ser Arg Leu Leu Gln Glu
                805                 810                 815

Asp Gly Lys Leu Gly Asp Ser Gly Gly Ser Asp Leu Pro Ala Pro Pro

            820                 825                 830

Pro Pro Pro Leu Ala Pro Gln Lys Ala Ser Ser Ser Gly Gly Ser Arg
        835                 840                 845

Pro Gly Ser Pro Val His His Pro Ser Leu Glu Glu Glu Pro Ser Tyr
    850                 855                 860

Lys Asp Asn Gln Lys Pro Lys Glu Asn Asn Gln Val Ile Ile Ser Thr
865                 870                 875                 880

Lys Asp Asp Asn Phe Ser Asp Lys Asn Lys Gly His Gly Phe Phe Ile
                885                 890                 895

Thr Asp Ser Asp Ser Ser Gly Asp Phe Trp Arg Glu Arg Ser Gly Glu
            900                 905                 910

His Thr Gln Glu Thr Asn Ser Pro His Ser Leu Lys Lys Asp Val Glu
        915                 920                 925

Asn Met Gly Lys Glu Glu Leu Gln Lys Val Leu Phe Glu Gln Ile Asp
    930                 935                 940

Leu Arg Arg Arg Leu Glu Gln Glu Phe Gln Val Leu Lys Gly Asn Thr
945                 950                 955                 960

Ser Phe Pro Val Phe Asn Asn Phe Gln Asp Gln Met Lys Arg Glu Leu
                965                 970                 975

Ala Tyr Arg Glu Glu Met Val Gln Gln Leu Gln Ile Ile Pro Tyr Ala
            980                 985                 990

Ala Ser Leu Ile Arg Lys Glu Lys Leu Gly Ala His Leu Ser Lys Ser
        995                 1000                1005

<210> 17
<211> 3045
<212> DNA
<213> Homo sapiens

<400> 17
atggcttcca gtccgctgcc agggcccaac gacatcctgc tggcgtcgcc gtcgagcgcc 60

ttccagcccg acacgctgag ccagccgcgg ccagggcacg ccaacctcaa acccaaccag 120

gtgggccagg tgatcctcta cggcattccc atcgtgtcgt tggtgatcga cgggcaagag 180

cgcctgtgcc tggcgcagat ctccaacact ctgctcaaga acttcagcta caacgagatc 240

cacaaccgtc gcgtggcact gggcatcacg tgtgtgcagt gcacgccggt gcaactggag 300
atcctgcggc gtgccggggc catgcccatc tcatcgcgcc gctgcggcat gatcaccaaa 360
cgcgaggccg agcgtctgtg caagtcgttc ctgggcgaaa acaggccgcc caagctgcca 420
gacaatttcg ccttcgacgt gtcacacgag tgcgcctggg gctgccgcgg cagcttcatt 480
cccgcgcgct acaacagctc gcgcgccaag tgcatcaaat gcagctactg caacatgtac 540
ttctcgccca acaagttcat tttccactcc caccgcacgc ccgacgccaa gtacactcag 600
ccagacgcag ccaacttcaa ctcgtggcgc cgtcatctca agctcaccga caagagtccc 660
caggacgagc tggtcttcgc ctgggaggac gtcaaggcca tgttcaacgg cggcagccgc 720
aagcgcgcac tgccccagcc gggcgcgcac cccgcctgcc acccgctcag ctctgtgaag 780
gcggccgccg tggccgccgc ggccgcggtg gccggaggcg ggggtctgct gggcccccac 840
ctgctgggtg cgcccccgcc gccgccgccg ccaccgccgc ccttggcaga gctggctggt 900
gccccgcacg cccatcacaa gcggccgcgc ttcgacgacg acgacgactc cttgcaggag 960
gccgccgtag tggccgccgc cagcctctcg gccgcagccg ccagcctctc tgtggctgct 1020
gcttcgggcg gcgcggggac tggtgggggc ggcgctgggg gtggctgtgt ggccggcgtg 1080
ggcgtgggcg cgggcgcggg ggcgggtgcc ggggcagggg ccaaaggccc gcgcagctac 1140
ccagtcatcc cggtgcccag caagggctcg ttcgggggcg tcctgcagaa gttcccgggc 1200
tgcggcgggc tcttcccgca cccctacacc ttccctgccg cggccgccgc cttcagcttg 1260
tgccataaga aagaggatgc gggtgccgcc gctgaggccc tggggggcgc gggcgcaggc 1320
ggcgcgggcg cggcgcccaa ggccggcttg tccggcctct tctggcccgc gggccgcaag 1380
gacgccttct atccgccctt ctgcatgttc tggccgccgc ggacccctgg cgggctcccg 1440
gtgcccacct acctgcagcc cccgcctcag ccgccctcgg cgctaggctg cgcgctaggc 1500
gaaagcccgg ccctgctgcg ccaggccttc ctggacctgg ctgagccagg cggtgctgct 1560
gggagcgccg aggccgcgcc cccgccgggg cagcccccgc aggtagtggc caacggcccg 1620
ggctccggcc cacctcctcc tgccgggggc gccggctctc gcgacgcgct cttcgagtcg 1680
cccccgggcg gcagcggcgg ggactgcagc gcgggctcca cgccgcccgc ggactctgtg 1740
gcagctgccg gggcaggggc cgcggccgcc gggtctggcc ccgcgggctc ccgggttccg 1800
gcgccccacc atccgcacct tctggagggg cgcaaagcgg gcggtggcag ctaccaccat 1860
tccagcgcct tccggccagt gggcggcaag gacgacgcgg agagcctggc caagctgcac 1920
ggggcgtcgg cgggcgcgcc ccactcggcc cagacgcatc cccaccacca tcaccaccct 1980
caccaccacc accaccacca ccaccccccg cagccgccgt cgccgcttct gctgctgccc 2040
ccgcagcccg acgagccggg ttccgagcgc caccacccgg ccccgccgcc gccgccgccg 2100

ccgcccccgc cgccccctct ggcccagcac ccgcaccacc gaggccttct gtctcccggg 2160

ggaaccagct gctgctaccc cagcgaggac agctccgagg acgaggacga cgaggaagaa 2220

gagcaggagg tggacgtgga gggccacaag ccccccgagg gcgaggaaga ggaggaaggt 2280

cgagaccctg acgacgacga ggaagaggac gaggagacgg aggtcctact cggcgacccc 2340

ttagtcgggg gcggccggtt cctccagggc cgagggccgt cggagaaggg gagcagccgg 2400

gaccgcgcgc cggccgtcgc gggcgcgttc ccgctcggcc tgaactcctc caggctgctg 2460

caggaagacg ggaaactcgg ggaccccggc tcggacctgc ccccgccccc gccgccgccc 2520

ctggcccccc agaaggcgag tggcggcggc agcagcagcc cgggcagccc agttcaccat 2580

ccatcactgg aggagcagcc ctcctacaaa gatagtcaga aaactaagga aaataaccaa 2640

gtaattgtat ctacaaagga tgacaacgtt ctagataaga acaaggagca tagctttttc 2700

atcacagact ctgatgcttc tggaggagat ttttggagag aaagatcagg tgaacataca 2760

caagaaacca actcacctca ttcactgaaa aaggatgtag aaaatatggg gaaagaagaa 2820

cttcagaagg ttttatttga acaaatagat ttacggagac gactggaaca agaattccag 2880

gtgttaaaag gaaacacatc tttcccagta ttcaataatt ttcaggatca gatgaaaagg 2940

gagctagcct accgagaaga aatggtgcaa cagttacaaa ttatccccta tgcagcaagt 3000

ttgatcagga aagaaaagct tggcgcccat ctcagcaaaa gctaa 3045

<210> 18
<211> 1014
<212> PRT
<213> Homo sapiens

<400> 18

Met Ala Ser Ser Pro Leu Pro Gly Pro Asn Asp Ile Leu Leu Ala Ser
1               5                   10                  15

Pro Ser Ser Ala Phe Gln Pro Asp Thr Leu Ser Gln Pro Arg Pro Gly
            20                  25                  30

His Ala Asn Leu Lys Pro Asn Gln Val Gly Gln Val Ile Leu Tyr Gly
        35                  40                  45

Ile Pro Ile Val Ser Leu Val Ile Asp Gly Gln Glu Arg Leu Cys Leu
    50                  55                  60

Ala Gln Ile Ser Asn Thr Leu Leu Lys Asn Phe Ser Tyr Asn Glu Ile
65                  70                  75                  80

His Asn Arg Arg Val Ala Leu Gly Ile Thr Cys Val Gln Cys Thr Pro
                85                  90                  95

Val Gln Leu Glu Ile Leu Arg Arg Ala Gly Ala Met Pro Ile Ser Ser
            100                 105                 110

Arg Arg Cys Gly Met Ile Thr Lys Arg Glu Ala Glu Arg Leu Cys Lys
        115                 120                 125

Ser Phe Leu Gly Glu Asn Arg Pro Pro Lys Leu Pro Asp Asn Phe Ala
    130                 135                 140

Phe Asp Val Ser His Glu Cys Ala Trp Gly Cys Arg Gly Ser Phe Ile
145                 150                 155                 160

Pro Ala Arg Tyr Asn Ser Ser Arg Ala Lys Cys Ile Lys Cys Ser Tyr
                165                 170                 175

Cys Asn Met Tyr Phe Ser Pro Asn Lys Phe Ile Phe His Ser His Arg
            180                 185                 190

Thr Pro Asp Ala Lys Tyr Thr Gln Pro Asp Ala Ala Asn Phe Asn Ser
        195                 200                 205

Trp Arg Arg His Leu Lys Leu Thr Asp Lys Ser Pro Gln Asp Glu Leu
    210                 215                 220

Val Phe Ala Trp Glu Asp Val Lys Ala Met Phe Asn Gly Gly Ser Arg
225                 230                 235                 240

Lys Arg Ala Leu Pro Gln Pro Gly Ala His Pro Ala Cys His Pro Leu
                245                 250                 255

Ser Ser Val Lys Ala Ala Ala Val Ala Ala Ala Ala Ala Val Ala Gly
            260                 265                 270

Gly Gly Gly Leu Leu Gly Pro His Leu Leu Gly Ala Pro Pro Pro Pro
        275                 280                 285

Pro Pro Pro Pro Pro Pro Leu Ala Glu Leu Ala Gly Ala Pro His Ala
    290                 295                 300

His His Lys Arg Pro Arg Phe Asp Asp Asp Asp Asp Ser Leu Gln Glu
305                 310                 315                 320

Ala Ala Val Val Ala Ala Ala Ser Leu Ser Ala Ala Ala Ala Ser Leu

                325                 330                 335

Ser Val Ala Ala Ala Ser Gly Gly Ala Gly Thr Gly Gly Gly Gly Ala
            340                 345                 350

Gly Gly Gly Cys Val Ala Gly Val Gly Val Gly Ala Gly Ala Gly Ala
        355                 360                 365

Gly Ala Gly Ala Gly Ala Lys Gly Pro Arg Ser Tyr Pro Val Ile Pro
    370                 375                 380

Val Pro Ser Lys Gly Ser Phe Gly Gly Val Leu Gln Lys Phe Pro Gly
385                 390                 395                 400

Cys Gly Gly Leu Phe Pro His Pro Tyr Thr Phe Pro Ala Ala Ala Ala
                405                 410                 415

Ala Phe Ser Leu Cys His Lys Lys Glu Asp Ala Gly Ala Ala Ala Glu
            420                 425                 430

Ala Leu Gly Gly Ala Gly Ala Gly Gly Ala Gly Ala Ala Pro Lys Ala
        435                 440                 445

Gly Leu Ser Gly Leu Phe Trp Pro Ala Gly Arg Lys Asp Ala Phe Tyr
    450                 455                 460

Pro Pro Phe Cys Met Phe Trp Pro Pro Arg Thr Pro Gly Gly Leu Pro
465                 470                 475                 480

Val Pro Thr Tyr Leu Gln Pro Pro Pro Gln Pro Pro Ser Ala Leu Gly
                485                 490                 495

Cys Ala Leu Gly Glu Ser Pro Ala Leu Leu Arg Gln Ala Phe Leu Asp
            500                 505                 510

Leu Ala Glu Pro Gly Gly Ala Ala Gly Ser Ala Glu Ala Ala Pro Pro
        515                 520                 525

Pro Gly Gln Pro Pro Gln Val Val Ala Asn Gly Pro Gly Ser Gly Pro
    530                 535                 540

Pro Pro Pro Ala Gly Gly Ala Gly Ser Arg Asp Ala Leu Phe Glu Ser
545                 550                 555                 560

Pro Pro Gly Gly Ser Gly Gly Asp Cys Ser Ala Gly Ser Thr Pro Pro
                565                 570                 575

Ala Asp Ser Val Ala Ala Ala Gly Ala Gly Ala Ala Ala Ala Gly Ser
            580                 585                 590

Gly Pro Ala Gly Ser Arg Val Pro Ala Pro His His Pro His Leu Leu
        595                 600                 605

Glu Gly Arg Lys Ala Gly Gly Gly Ser Tyr His His Ser Ser Ala Phe
    610                 615                 620

Arg Pro Val Gly Gly Lys Asp Asp Ala Glu Ser Leu Ala Lys Leu His
625                 630                 635                 640

Gly Ala Ser Ala Gly Ala Pro His Ser Ala Gln Thr His Pro His His
                645                 650                 655

His His His Pro His His His His His His His His Pro Pro Gln Pro
            660                 665                 670

Pro Ser Pro Leu Leu Leu Leu Pro Pro Gln Pro Asp Glu Pro Gly Ser
        675                 680                 685

Glu Arg His His Pro Ala Pro Pro Pro Pro Pro Pro Pro Pro Pro Pro
    690                 695                 700

Pro Pro Leu Ala Gln His Pro His His Arg Gly Leu Leu Ser Pro Gly
705                 710                 715                 720

Gly Thr Ser Cys Cys Tyr Pro Ser Glu Asp Ser Ser Glu Asp Glu Asp
                725                 730                 735

Asp Glu Glu Glu Glu Gln Glu Val Asp Val Glu Gly His Lys Pro Pro
            740                 745                 750

Glu Gly Glu Glu Glu Glu Glu Gly Arg Asp Pro Asp Asp Asp Glu Glu
        755                 760                 765

Glu Asp Glu Glu Thr Glu Val Leu Leu Gly Asp Pro Leu Val Gly Gly
    770                 775                 780

Gly Arg Phe Leu Gln Gly Arg Gly Pro Ser Glu Lys Gly Ser Ser Arg
785                 790                 795                 800

Asp Arg Ala Pro Ala Val Ala Gly Ala Phe Pro Leu Gly Leu Asn Ser
                805                 810                 815

Ser Arg Leu Leu Gln Glu Asp Gly Lys Leu Gly Asp Pro Gly Ser Asp
            820                 825                 830

Leu Pro Pro Pro Pro Pro Pro Pro Leu Ala Pro Gln Lys Ala Ser Gly
        835                 840                 845

Gly Gly Ser Ser Ser Pro Gly Ser Pro Val His His Pro Ser Leu Glu
    850                 855                 860

Glu Gln Pro Ser Tyr Lys Asp Ser Gln Lys Thr Lys Glu Asn Asn Gln
865                 870                 875                 880

Val Ile Val Ser Thr Lys Asp Asp Asn Val Leu Asp Lys Asn Lys Glu
                885                 890                 895

His Ser Phe Phe Ile Thr Asp Ser Asp Ala Ser Gly Gly Asp Phe Trp
            900                 905                 910

Arg Glu Arg Ser Gly Glu His Thr Gln Glu Thr Asn Ser Pro His Ser
        915                 920                 925

Leu Lys Lys Asp Val Glu Asn Met Gly Lys Glu Glu Leu Gln Lys Val
    930                 935                 940

Leu Phe Glu Gln Ile Asp Leu Arg Arg Arg Leu Glu Gln Glu Phe Gln
945                 950                 955                 960

Val Leu Lys Gly Asn Thr Ser Phe Pro Val Phe Asn Asn Phe Gln Asp
                965                 970                 975

Gln Met Lys Arg Glu Leu Ala Tyr Arg Glu Glu Met Val Gln Gln Leu
            980                 985                 990

Gln Ile Ile Pro Tyr Ala Ala Ser  Leu Ile Arg Lys Glu  Lys Leu Gly
        995                 1000                 1005

Ala His  Leu Ser Lys Ser
    1010

<210>  19
<211>  3123
<212>  DNA
<213>  Mus musculus

<400>  19
gctctctccg acccgcaggc ccacgggagc ctgagctccg cctccccagg gcgcggaagc      60

tggcgaagcc ccagggattc ccatttatag cttggtttcc actcagctca gtccctccag 120

gactcgggct gagcaagttt cttccattcc cttctctcct ccctccaccc ccttctcctc 180

ctccttctcc ttcttttctt cctcctcatt cccgcctccc cttcaacctc agcagggtgc 240

aggtgtccaa ctcgaacaag ggccccaact tggactcaga tgttcccact ctcagacccc 300

ctgataatgc aggggcgccc gcctgctgcg cggacagcta ccctgagcat ccgtagccgt 360

ccgcacacaa ggcgcgggag tttctcaatg ggaagaggcc gggactctag gaggcggggc 420

gaataggatt cctcccgcct agtgggtccc tcgcagtcct agggttgcaa cccttgagcg 480

gtagagaaca ccggagactg cggatgagcc agatttcggg gacataaaat cttccagccc 540

ggagagaatt gtgtgcagag aggggctcca gtccagcgtg gtgtgagagg cgtgctatca 600

agaaagaagt tggaggggaa ccagtgcaac cctaactctg cgagatcttg gggtacacac 660

actcgggatg ctggcctccg ccctcctcgt tttcctttgc tgtttcaaag gacatgcagg 720

ctcatcgccc catttcctac aacagccaga ggacatggtg gtgctgttgg gggaggaagc 780

ccggctgccc tgcgctctgg gcgcgtacag gggcctcgtg cagtggacta aggatgggct 840

ggctctaggg ggcgaaagag accttccagg gtggtcccgg tactggatat cggggaattc 900

agccagtggc cagcatgacc tccacattaa gcctgtggaa ttggaagatg aggcatcgta 960

tgagtgccag gcttcgcaag caggtctccg atcacgacca gcccaactgc acgtgatggt 1020

ccccccagaa gctccccagg tactaggcgg cccctctgtg tctctggttg ctggagttcc 1080

tggaaatctg acctgtcgga gtcgtgggga ttcacgacct gcccctgaac tactgtggtt 1140

ccgagatggg atccggctgg atgggagcag cttccaccag accacgctga aggacaaggc 1200

cactggaaca gtggaaaaca ccttattcct gaccccttcc agtcatgatg atggtgccac 1260

cttgatctgc agagcgcgaa gccaggccct gcccacaggg agggacacag ctgttacact 1320

gagccttcag tatcccccaa tggtgactct gtctgctgag ccccagactg tgcaggaggg 1380

agagaaggtg actttcctgt gtcaagccac tgcccagcct cctgtcactg gctacaggtg 1440

ggcgaagggg ggatccccgg tgcttggggc acgtgggcca aggttggagg tcgttgcaga 1500

tgccactttc ctgactgagc cggtgtcctg cgaggtcagc aacgcggtcg gaagcgccaa 1560

ccgcagcacc gcgctggaag tgttgtatgg acccattctg caggcaaaac ctaagtccgt 1620

gtccgtggac gtggggaaag atgcctcctt cagctgtgtc tggcgcggga acccacttcc 1680

acggataacc tggacccgca tgggtggctc tcaggtgctg agctccgggc ccacgctgcg 1740

gcttccgtcc gtggcactgg aggatgcggg cgactatgta tgcagggctg agccgaggag 1800

aacgggtctg ggaggcggca aagcgcaggc gaggctgact gtgaacgcac cccctgtagt 1860

gacagccctg caacctgcac cagcctttct gaggggtcct gctcgcctcc agtgtgtggt 1920

gtttgcctcc cctgccccag actcggtggt ttggtcttgg gacgagggct tcttggaggc 1980

aggctcactg ggcaggttcc tagtggaagc cttcccagcc ccggaagtgg aggggggaca 2040

gggccctggc cttatttctg tgctacacat ttccggaacc caggagtccg actttaccac 2100

cggcttcaac tgcagtgccc gcaaccggct aggagaggga cgagtccaga tccacttggg 2160

ccgtagagac ttgctgccta ctgtccggat tgtggctggt gcagcatctg cagccacctc 2220

tctccttatg gtcatcactg gagtggtcct ctgctgctgg cgccatggct ctctctctaa 2280

gcaaaagaac ttggtccgga tcccgggaag cagcgagggt tccagttcac gtggccctga 2340

ggaggagaca ggcagcagtg aggaccgggg tcccattgtg cacaccgacc acagtgattt 2400

ggttcttgag gaaaaagagg ctctggagac aaaggatcca accaacggtt actacagggt 2460

tcgaggggtc agtgtgagcc ttagccttgg ggaagctcct ggaggaggcc tcttcttgcc 2520

accgccctct ccgatcggtc tcccagggac tcctacttac tatgacttca agccacatct 2580

ggacttagtc cctccctgca gactgtacag agcgagggca ggttatctta ccacccccca 2640

tccccgtgcc ttcaccagct acatgaaacc cacatccttt ggacccccag aattgagctc 2700

tggaactccc cccttcccgt atgctacctt gtctccaccc agccaccagc gtctccagac 2760

tcatgtgtga atccatctct ccaagtgaag ggtcttggaa tcttctgttt gccatatagt 2820

gtgttgtcca gatttctggg gagtcagaac aagttgatga ccaacccctc caaaactgaa 2880

cattgaagga gggaaagatc attacaagca tcaggactgt tggtgtacac tcagttcagc 2940

caaagtggat tctccaagtg ggagcaatat ggccgctttc ccatgagaaa gacattcaag 3000

atggtgacta aatgactaaa tactttgcag agggacaaag atgggaacta gggatatgga 3060

tggaagtagt agagaagata tatgaccatc tgcatcaaga gaaaggataa cataagacaa 3120

atc 3123

<210> 20
<211> 700
<212> PRT
<213> Mus musculus

<400> 20

Met Leu Ala Ser Ala Leu Leu Val Phe Leu Cys Cys Phe Lys Gly His
1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly
        115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Gly Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys
225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys
        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu
545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Arg Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Glu Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  21
<211>  2847
<212>  DNA
<213>  Mus musculus

<400>  21
ggtccggaat tcccgggatg agaggggctc cagtccagcg tggtgtgaga ggcgtgctat      60

caagaaagaa gttggagggg aaccagtgca accctaactc tgcgagatct tggggtacac     120

acactcggga tgctggcctc cgccctcctc gttttccttt gctgtttcaa aggacatgca     180

ggctcatcgc cccatttcct acaacagcca gaggacatgg tggtgctgtt gggggaggaa     240

gcccggctgc cctgcgctct gggcgcgtac agggggctcg tgcagtggac taaggatggg     300

ctggctctag ggggcgaaag agaccttcca gggtggtccc ggtactggat atcggggaat     360

tcagccagtg gccagcatga cctccacatt aagcctgtgg aattggaaga tgaggcatcg 420

tatgagtgcc aggcttcgca agcaggtctc cgatcacgac cagcccaact gcacgtgatg 480

gtccccccag aagctcccca ggtactaggc ggcccctctg tgtctctggt tgctggagtt 540

cctggaaatc tgacctgtcg gagtcgtggg gattcacgac ctgcccctga actactgtgg 600

ttccgagatg ggatccggct ggatgggagc agcttccacc agaccacgct gaaggacaag 660

gccactggaa cagtggaaaa caccttattc ctgacccctt ccagtcatga tgatggtgcc 720

accttgatct gcagagcgcg aagccaggcc ctgcccacag ggagggacac agctgttaca 780

ctgagccttc agtatccccc aatggtgact ctgtctgctg agccccagac tgtgcaggag 840

ggagagaagg tgactttcct gtgtcaagcc actgcccagc ctcctgtcac tggctacagg 900

tgggcgaagg ggggatcccc ggtgcttggg gcacgtgggc caaggttgga ggtcgttgca 960

gatgccactt tcctgactga gccggtgtcc tgcgaggtca gcaacgcggt cggaagcgcc 1020

aaccgcagca ccgcgctgga agtgttgtat ggacccattc tgcaggcaaa acctaagtcc 1080

gtgtccgtgg acgtggggaa agatgcctcc ttcagctgtg tctggcgcgg gaacccactt 1140

ccacggataa cctggacccg catgggtggc tctcaggtgc tgagctccgg gcccacgctg 1200

cggcttccgt ccgtggcact ggaggatgcg ggcgactatg tatgcagggc tgagccgagg 1260

agaacgggtc tgggaggcgg caaagcgcag gcgaggctga ctgtgaacgc accccctgta 1320

gtgacagccc tgcaacctgc accagccttt ctgaggggtc ctgctcgcct ccagtgtgtg 1380

gtgtttgcct cccctgcccc agactcggtg gtttggtctt gggacgaggg cttcttggag 1440

gcaggctcac tgggcaggtt cctagtggaa gccttcccag ccccggaagt ggagggggga 1500

cagggccctg gccttatttc tgtgctacac atttccggaa cccaggagtc cgactttacc 1560

accggcttca actgcagtgc ccgcaaccgg ctaggagagg gacgagtcca gatccacttg 1620

ggccgtagag acttgctgcc tactgtccgg attgtggctg gtgcagcatc tgcagccacc 1680

tctctcctta tggtcatcac tggagtggtc ctctgctgct ggcgccatgg ctctctctct 1740

aagcaaaaga acttggtccg gatcccagga agcagcgagg gttccagttc acgtggccct 1800

gaggaggaga caggcagcag tgaggaccgg ggtcccattg tgcacaccga ccacagtgat 1860

ttggttcttg aggaaaaaga ggctctggag acaaaggatc caaccaacgg ttactacagg 1920

gttcgagggg tcagtgtgag ccttagcctt ggggaagctc ctggaggagg cctcttcttg 1980

ccaccgccct ctccgatcgg tctcccaggg actcctactt actatgactt caagccacat 2040

ctggacttag tccctccctg cagactgtac agagcgaggg caggttatct taccaccccc 2100

catccccgtg ccttcaccag ctacatgaaa cccacatcct ttggaccccc agaattgagc 2160

tctggaactc cccccttccc gtatgctacc ttgtctccac ccagccacca gcgtctccag 2220

actcatgtgt gaatccatct ctccaagtga agggtcttgg aatcttctgt ttgccatata 2280

gtgtgttgtc cagatttctg gggagtcaga acaagttgat gaccaacccc tccaaaactg 2340

aacattgaag gagggaaaga tcattacaag catcaggact gttggtgtac actcagttca 2400

gccaaagtgg attctccaag tgggagcaat atggccgctt tcccatgaga aagacattca 2460

agatggtgac taaatgacta aatactttgc agagggacaa agatgggaac tagggatatg 2520

gatggaagta gtagagaaga tatatgacca tctgcatcaa gagaaaggat aacataagac 2580

aaatcaagat gaaagaaata atccacaccc ccccccccac cgcgtcctgg ccaataagta 2640

tagcctacat ggctgttcat tatctgggaa ccaaaatggc cactatcttg actccttcct 2700

taaagataca gaaagaattg aatccaagga atggggtagg gtggaaatag aagaaatgaa 2760

ggggactctt gggctaagaa tacttatgtt taataataaa agggggaggc aaagaaaaaa 2820

aaaaaaaaaa aaaaaaaaaa aaaaaaa 2847

<210> 22
<211> 700
<212> PRT
<213> Mus musculus

<400> 22

Met Leu Ala Ser Ala Leu Leu Val Phe Leu Cys Cys Phe Lys Gly His
1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly

115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Gly Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys
225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys
        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu
545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Arg Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Glu Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  23
<211>  2153
<212>  DNA
<213>  Homo sapiens

<400>  23
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg cccgtcgccc catttcctgc aacagccaga ggacctggtg     300

gtgctgctgg gggaggaagc ccggctgccg tgtgctctgg gcgcctactg ggggctagtt     360

cagtggacta agagtgggct ggccctaggg ggccaaaggg acctaccagg gtggtcccgg     420

tactggatat cagggaatgc agccaatggc cagcatgacc tccacattag gcccgtggag     480

ctagaggatg aagcatcata tgaatgtcag gctacacaag caggcctccg ctccagacca     540

gcccaactgc acgtgctggt ccccccagaa gccccccagg tgctgggcgg cccctctgtg     600

tctctggttg ctggagttcc tgcgaacctg acatgtcgga gccgtgggga tgcccgccct     660

acccctgaat tgctgtggtt ccgagatggg gtcctgttgg atggagccac cttccatcag     720

accctgctga aggaagggac ccctgggtca gtggagagca ccttaaccct gacccctttc     780

agccatgatg atggagccac ctttgtctgc cgggcccgga gccaggccct gcccacagga     840

agagacacag ctatcacact gagcctgcag taccccccag aggtgactct gtctgcttcg     900

ccacacactg tgcaggaggg agagaaggtc attttcctgt gccaggccac agcccagcct 960

cctgtcacag gctacaggtg ggcaaaaggg ggctctccgg tgctcggggc ccgcgggcca 1020

aggttagagg tcgtggcaga cgcctcgttc ctgactgagc ccgtgtcctg cgaggtcagc 1080

aacgccgtgg gtagcgccaa ccgcagtact gcgctggatg tgctgtttgg gccgattctg 1140

caggcaaagc cggagcccgt gtccgtggac gtgggggaag acgcttcctt cagctgcgcc 1200

tggcgcggga acccgcttcc acgggtaacc tggacccgcc gcggtggcgc gcaggtgctg 1260

ggctctggag ccacactgcg tcttccgtcg gtggggcccg aggacgcagg cgactatgtg 1320

tgcagagctg aggctgggct atcgggcctg cggggcggcg ccgcggaggc tcggctgact 1380

gtgaacgctc ccccagtagt gaccgccctg cactctgcgc ctgccttcct gaggggccct 1440

gctcgcctcc agtgtctggt tttcgcctct cccgccccag atgccgtggt ctggtcttgg 1500

gatgagggct tcctggaggc ggggtcgcag ggccggttcc tggtggagac attccctgcc 1560

ccagagagcc gcgggggact gggtccgggc ctgatctctg tgctacacat ttcggggacc 1620

caggagtctg actttagcag gagctttaac tgcagtgccc ggaaccggct gggcgaggga 1680

ggtgcccagg ccagcctggg ccgtagagac ttgctgccca ctgtgcggat agtggccgga 1740

gtggccgctg ccaccacaac tctccttatg gtcatcactg gggtggccct ctgctgctgg 1800

cgccacagca aggcctcagc ctctttctcc gagcaaaaga acctgatgcg aatccctggc 1860

agcagcgacg gctccagttc acgaggtcct gaagaagagg agacaggcag ccgcgaggac 1920

cggggcccca ttgtgcacac tgaccacagt gatctggttc tggaggagga agggactctg 1980

gagaccaagg acccaaccaa cggttactac aaggtccgag gagtcagtcc acccgcgtct 2040

ccagactcac gtgtgacatc tttccaatgg aagagtcctg ggatctccaa cttgccataa 2100

tggattgttc tgatttctga ggagccagga caagttggcg accttactcc tcc 2153

<210> 24
<211> 633
<212> PRT
<213> Homo sapiens

<400> 24

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser

530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Pro Pro Ala Ser Pro Asp Ser Arg Val Thr Ser Phe Gln Trp
    610                 615                 620

Lys Ser Pro Gly Ile Ser Asn Leu Pro
625                 630

<210>  25
<211>  2979
<212>  DNA
<213>  Homo sapiens

<400>  25
ccgcggaact ggcaggcgtt tcagagcgtc agaggctgcg gatgagcaga cttggaggac      60

tccaggccag agactaggct gggcgaagag tcgagcgtga agggggctcc gggccagggt      120

gacaggaggc gtgcttgaga ggaagaagtt gacgggaagg ccagtgcgac ggcaaatctc      180

gtgaaccttg ggggacgaat gctcaggatg cgggtccccg ccctcctcgt cctcctcttc      240

tgcttcagag ggagagcagg cccgtcgccc catttcctgc aacagccaga ggacctggtg      300

gtgctgctgg gggaggaagc ccggctgccg tgtgctctgg gcgcctactg ggggctagtt      360

cagtggacta agagtgggct ggccctaggg ggccaaaggg acctaccagg gtggtcccgg      420

tactggatat cagggaatgc agccaatggc cagcatgacc tccacattag gcccgtggag      480

ctagaggatg aagcatcata tgaatgtcag gctacacaag caggcctccg ctccagacca      540

gcccaactgc acgtgctggt ccccccagaa gccccccagg tgctgggcgg cccctctgtg      600

tctctggttg ctggagttcc tgcgaacctg acatgtcgga gccgtgggga tgcccgccct      660

acccctgaat tgctgtggtt ccgagatggg gtcctgttgg atggagccac cttccatcag      720

accctgctga aggaagggac ccctgggtca gtggagagca ccttaaccct gacccctttc      780

agccatgatg atggagccac ctttgtctgc cgggcccgga gccaggccct gcccacagga      840

agagacacag ctatcacact gagcctgcag taccccccag aggtgactct gtctgcttcg 900
ccacacactg tgcaggaggg agagaaggtc attttcctgt gccaggccac agcccagcct 960
cctgtcacag gctacaggtg ggcaaaaggg ggctctccgg tgctcggggc ccgcgggcca 1020
aggttagagg tcgtggcaga cgcctcgttc ctgactgagc ccgtgtcctg cgaggtcagc 1080
aacgccgtgg gtagcgccaa ccgcagtact gcgctggatg tgctgtttgg gccgattctg 1140
caggcaaagc cggagcccgt gtccgtggac gtgggggaag acgcttcctt cagctgcgcc 1200
tggcgcggga acccgcttcc acgggtaacc tggacccgcc gcggtggcgc gcaggtgctg 1260
ggctctggag ccacactgcg tcttccgtcg gtggggcccg aggacgcagg cgactatgtg 1320
tgcagagctg aggctgggct atcgggcctg cggggcggcg ccgcggaggc tcggctgact 1380
gtgaacgctc ccccagtagt gaccgccctg cactctgcgc ctgccttcct gaggggccct 1440
gctcgcctcc agtgtctggt tttcgcctct cccgccccag atgccgtggt ctggtcttgg 1500
gatgagggct tcctggaggc ggggtcgcag ggccggttcc tggtggagac attccctgcc 1560
ccagagagcc gcgggggact gggtccgggc ctgatctctg tgctacacat ttcggggacc 1620
caggagtctg actttagcag gagctttaac tgcagtgccc ggaaccggct gggcgaggga 1680
ggtgcccagg ccagcctggg ccgtagagac ttgctgccca ctgtgcggat agtggccgga 1740
gtggccgctg ccaccacaac tctccttatg gtcatcactg gggtggccct ctgctgctgg 1800
cgccacagca aggcctcagc ctctttctcc gagcaaaaga acctgatgcg aatccctggc 1860
agcagcgacg gctccagttc acgaggtcct gaagaagagg agacaggcag ccgcgaggac 1920
cggggcccca ttgtgcacac tgaccacagt gatctggttc tggaggagga agggactctg 1980
gagaccaagg acccaaccaa cggttactac aaggtccgag gagtcagtgt gagcctgagc 2040
cttggcgaag cccctggagg aggtctcttc ctgccaccac cctcccccct tgggccccca 2100
gggaccccta ccttctatga cttcaaccca cacctgggca tggtcccccc ctgcagactt 2160
tacagagcca gggcaggcta tctcaccaca ccccaccctc gagctttcac cagctacatc 2220
aaacccacat cctttgggcc cccagatctg gcccccggga ctcccccctt cccatatgct 2280
gccttcccca cacctagcca cccgcgtctc cagactcacg tgtgacatct ttccaatgga 2340
agagtcctgg gatctccaac ttgccataat ggattgttct gatttctgag gagccaggac 2400
aagttggcga ccttactcct ccaaaactga acacaagggg agggaaagat cattacattt 2460
gtcaggagca tttgtataca gtcagctcag ccaaaggaga tgccccaagt gggagcaaca 2520
tggccaccca atatgcccac ctattccccg gtgtaaaaga gattcaagat ggcaggtagg 2580
ccctttgagg agagatgggg acagggcagt gggtgttggg agtttggggc cgggatggaa 2640
gttgtttcta gccactgaaa gaagatattt caagatgacc atctgcattg agaggaaagg 2700

tagcatagga tagatgaaga tgaagagcat accaggcccc accctggctc tccctgaggg   2760

gaactttgct cggccaatgg aaatgcagcc aagatggcca tatactccct aggaacccaa   2820

gatggccacc atcttgattt tactttcctt aaagactcag aaagacttgg acccaaggag   2880

tggggataca gtgagaatta ccactgttgg ggcaaaatat tgggataaaa atatttatgt   2940

ttaataataa aaaaaagtca aagaggcaaa aaaaaaaaa                          2979

<210>  26
<211>  708
<212>  PRT
<213>  Homo sapiens

<400>  26

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Val Ser Leu Ser Leu Gly Glu Ala Pro Gly Gly Gly Leu Phe
    610                 615                 620

Leu Pro Pro Pro Ser Pro Leu Gly Pro Pro Gly Thr Pro Thr Phe Tyr
625                 630                 635                 640

Asp Phe Asn Pro His Leu Gly Met Val Pro Pro Cys Arg Leu Tyr Arg
                645                 650                 655

Ala Arg Ala Gly Tyr Leu Thr Thr Pro His Pro Arg Ala Phe Thr Ser
            660                 665                 670

Tyr Ile Lys Pro Thr Ser Phe Gly Pro Pro Asp Leu Ala Pro Gly Thr
        675                 680                 685

Pro Pro Phe Pro Tyr Ala Ala Phe Pro Thr Pro Ser His Pro Arg Leu
    690                 695                 700

Gln Thr His Val
705

<210>  27
<211>  2973
<212>  DNA
<213>  Homo sapiens

<400>  27
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg cccgtcgccc catttcctgc aacagccaga ggacctggtg     300

gtgctgctgg gggaggaagc ccggctgccg tgtgctctgg gcgcctactg ggggctagtt     360

cagtggacta agagtgggct ggccctaggg ggccaaaggg acctaccagg gtggtcccgg     420

tactggatat cagggaatgc agccaatggc cagcatgacc tccacattag gcccgtggag     480

ctagaggatg aagcatcata tgaatgtcag gctacacaag caggcctccg ctccagacca     540

gcccaactgc acgtgctggt ccccccagaa gccccccagg tgctgggcgg cccctctgtg     600

tctctggttg ctggagttcc tgcgaacctg acatgtcgga gccgtgggga tgcccgccct     660

acccctgaat tgctgtggtt ccgagatggg gtcctgttgg atggagccac ctttcatcag     720

accctgctga aggaagggac ccctgggtca gtggagagca ccttaaccct gacccctttc     780

agccatgatg atggagccac ctttgtctgc cgggcccgga gccaggccct gcccacagga     840

agagacacag ctatcacact gagcctgcag taccccccag aggtgactct gtctgcttcg     900

ccacacactg tgcaggaggg agagaaggtc attttcctgt gccaggccac agcccagcct     960

cctgtcacag gctacaggtg ggcaaaaggg ggctctccgg tgctcggggc ccgcgggcca    1020

aggttagagg tcgtggcaga cgcctcgttc ctgactgagc ccgtgtcctg cgaggtcagc    1080

aacgccgtgg gtagcgccaa ccgcagtact gcgctggatg tgctgtttgg gccgattctg    1140

caggcaaagc cggagcccgt gtccgtggac gtgggggaag acgcttcctt cagctgcgcc    1200

tggcgcggga acccgcttcc acgggtaacc tggacccgcc gcggtggcgc gcaggtgctg 1260

ggctctggag ccacactgcg tcttccgtcg gtggggcccg aggacgcagg cgactatgtg 1320

tgcagagctg aggctgggct atcgggcctg cggggcggcg ccgcggaggc tcggctgact 1380

gtgaacgctc ccccagtagt gaccgccctg cactctgcgc ctgccttcct gaggggccct 1440

gctcgcctcc agtgtctggt tttcgcctct cccgccccag atgccgtggt ctggtcttgg 1500

gatgagggct tcctggaggc ggggtcgcag ggccggttcc tggtggagac attccctgcc 1560

ccagagagcc gcgggggact gggtccgggc ctgatctctg tgctacacat ttcggggacc 1620

caggagtctg actttagcag gagctttaac tgcagtgccc ggaaccggct gggcgaggga 1680

ggtgcccagg ccagcctggg ccgtagagac ttgctgccca ctgtgcggat agtggccgga 1740

gtggccgctg ccaccacaac tctccttatg gtcatcactg gggtggccct ctgctgctgg 1800

cgccacagca aggcctcagc ctctttctcc gagcaaaaga acctgatgcg aatccctggc 1860

agcagcgacg gctccagttc acgaggtcct gaagaagagg agacaggcag ccgcgaggac 1920

cggggcccca ttgtgcacac tgaccacagt gatctggttc tggaggagga agggactctg 1980

gagaccaagg acccaaccaa cggttactac aaggtccgag gagtcagtgt gagcctgagc 2040

cttggcgaag cccctggagg aggtctcttc ctgccaccac cctcccccct tgggccccca 2100

gggaccccta ccttctatga cttcaaccca cacctgggca tggtcccccc ctgcagactt 2160

tacagagcca gggcaggcta tctcaccaca ccccaccctc gagctttcac cagctacatc 2220

aaacccacat cctttgggcc cccagatctg gcccccggga ctcccccctt cccatatgct 2280

gccttcccca cacctagcca cccgcgtctc cagactcacg tgtgacatct ttccaatgga 2340

agagtcctgg gatctccaac ttgccataat ggattgttct gatttctgag gagccaggac 2400

aagttggcga ccttactcct ccaaaactga acacaagggg agggaaagat cattacattt 2460

gtcaggagca tttgtataca gtcagctcag ccaaaggaga tgccccaagt gggagcaaca 2520

tggccaccca atatgcccac ctattccccg gtgtaaaaga gattcaagat ggcaggtagg 2580

ccctttgagg agagatgggg acagggcagt gggtgttggg agtttggggc cgggatggaa 2640

gttgtttcta gccactgaaa gaagatattt caagatgacc atctgcattg agaggaaagg 2700

tagcatagga tagatgaaga tgaagagcat accaggcccc accctggctc tccctgaggg 2760

gaactttgct cggccaatgg aaatgcagcc aagatggcca tatactccct aggaacccaa 2820

aatggccacc atcttgattt tactttcctt aaagactcag aaagacttgg acccaaggag 2880

tggggataca gtgagaatta ccactgttgg ggcaaaatat tgggataaaa atatttatgt 2940

ttaataataa aaaaaagtca aagagaaaaa aaa 2973

<210>  28
<211>  708
<212>  PRT
<213>  Homo sapiens

<400>  28

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln

210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Val Ser Leu Ser Leu Gly Glu Ala Pro Gly Gly Gly Leu Phe
    610                 615                 620

Leu Pro Pro Pro Ser Pro Leu Gly Pro Pro Gly Thr Pro Thr Phe Tyr
625                 630                 635                 640

Asp Phe Asn Pro His Leu Gly Met Val Pro Pro Cys Arg Leu Tyr Arg
                645                 650                 655

Ala Arg Ala Gly Tyr Leu Thr Thr Pro His Pro Arg Ala Phe Thr Ser
            660                 665                 670

Tyr Ile Lys Pro Thr Ser Phe Gly Pro Pro Asp Leu Ala Pro Gly Thr
        675                 680                 685

Pro Pro Phe Pro Tyr Ala Ala Phe Pro Thr Pro Ser His Pro Arg Leu
    690                 695                 700

Gln Thr His Val
705

<210> 29
<211> 1969
<212> DNA
<213> Homo sapiens

<400> 29
accttggggg acgaatgctc aggatgcggg tccccgccct cctcgtcctc ctcttctgct 60

tcagagggag agcaggcccg tcgccccatt tcctgcaaca gccagaggac ctggtggtgc 120

tgctggggga ggaagcccgg ctgccgtgtg ctctgggcgc ctactggggg ctagttcagt 180

ggactaagag tgggctggcc ctagggggcc aaagggacct accagggtgg tcccggtact 240

ggatatcagg gaatgcagcc aatggccagc atgacctcca cattaggccc gtggagctag 300

aggatgaagc atcatatgaa tgtcaggcta cacaagcagg cctccgctcc agaccagccc 360

aactgcacgt gctggtcccc ccagaagccc cccaggtgct gggcggcccc tctgtgtctc 420

tggttgctgg agttcctgcg aacctgacat gtcggagccg tggggatgcc cgccctaccc 480

ctgaattgct gtggttccga gatggggtcc tgttggatgg agccaccttc catcagaccc 540

tgctgaagga agggacccct gggtcagtgg agagcacctt aaccctgacc cctttcagcc 600

atgatgatgg agccaccctt gtctgccggg cccggagcca ggccctgccc acaggaagag 660

acacagctat cacactgagc ctgcagtacc ccccagaggt gactctgtct gcttcgccac 720

acactgtgca ggagggagag aaggtcattt tcctgtgcca ggccacagcc cagcctcctg 780

tcacaggcta caggtgggca aaagggggct ctccggtgct cggggcccgc gggccaaggt 840

tagaggtcgt ggcagacgcc tcgttcctga ctgagcccgt gtcctgcgag gtcagcaacg 900

ccgtgggtag cgccaaccgc agtactgcgc tggatgtgct gtttgggccg attctgcagg 960

caaagccgga gcccgtgtcc gtggacgtgg gggaagacgc ttccttcagc tgcgcctggc 1020

gcgggaatcc gcttccacgg gtaacctgga cccgccgcgg tggcgcgcag gtgctgggct 1080

ctggagccac actgcgtctt ccgtcggtgg ggcccgagga cgcagacgac tatgtgtgca 1140

gagctgaggc tgggctatcg ggcctgcggg gcggcgccgc ggaggctcgg ctgactgtga 1200

acgctccccc agtagtgacc gccctgcact ctgcgcctgc cttcctgagg ggccctgctc 1260

gcctccagtg tctggttttc gcctctcccg ccccagatgc cgtggtctgg tcttgggatg 1320

agggcttcct ggaggcgggg tcgcagggtc ggttcctggt ggagacattc cctgccccag 1380

agagccgcgg gggactgggt ccgggcctga tctctgtgct acacatttcg gggacccagg 1440

agtctgactt tagcaggagc tttaactgca gtgcccggaa ccggctgggc gagggaggtg 1500

cccaggccag cctgggccgt agagacttgc tgcccactgt gcggatagtg gccggagtgg 1560

tcgctgccac cacaactctc cttatggtca tcactggggt ggccctctgc tgctggcgcc 1620

acagcaaggc ctcagcctct ttctccgagc aaaagaacct gatgcgaatc cctggcagca 1680

gcgacggctc cagttcacga ggtcctgaag aagaggagac aggcagccgc gaggaccggg 1740

gccccattgt gcacactgac cacagtgatc tggttctgga ggaggaaggg actctggaga 1800

ccaaggaccc aaccaacggt tactacaagg tccgaggagt cagtccaccc gcgtctccag 1860

actcacgtgt gacatctttc caatggaaga gtcctgggat ctccaacttg ccataatgga 1920

ttgttctgat ttctgaggag ccaggacaag ttggcgacct tactcctcc 1969

<210> 30
<211> 633
<212> PRT
<213> Homo sapiens

<400> 30

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp

145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Leu Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Asp Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Val Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Pro Pro Ala Ser Pro Asp Ser Arg Val Thr Ser Phe Gln Trp
    610                 615                 620

Lys Ser Pro Gly Ile Ser Asn Leu Pro
625                 630

<210>  31
<211>  2003
<212>  DNA
<213>  Homo sapiens

<400>  31
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg gtggtcccgg tactggatat cagggaatgc agccaatggc     300

cagcatgacc tccacattag gcccgtggag ctagaggatg aagcatcata tgaatgtcag     360

gctacacaag caggcctccg ctccagacca gcccaactgc acgtgctggt ccccccagaa     420

gccccccagg tgctgggcgg cccctctgtg tctctggttg ctggagttcc tgcgaacctg     480

acatgtcgga gccgtgggga tgcccgccct acccctgaat tgctgtggtt ccgagatggg     540

gtcctgttgg atggagccac cttccatcag accctgctga aggaagggac ccctgggtca     600

gtggagagca ccttaaccct gacccctttc agccatgatg atggagccac ctttgtctgc     660

cgggcccgga gccaggccct gcccacagga agagacacag ctatcacact gagcctgcag     720

taccccccag aggtgactct gtctgcttcg ccacacactg tgcaggaggg agagaaggtc     780

attttcctgt gccaggccac agcccagcct cctgtcacag gctacaggtg ggcaaaaggg     840

ggctctccgg tgctcggggc ccgcgggcca aggttagagg tcgtggcaga cgcctcgttc     900

ctgactgagc ccgtgtcctg cgaggtcagc aacgccgtgg gtagcgccaa ccgcagtact     960

gcgctggatg tgctgtttgg gccgattctg caggcaaagc cggagcccgt gtccgtggac    1020

gtgggggaag acgcttcctt cagctgcgcc tggcgcggga acccgcttcc acgggtaacc    1080

tggacccgcc gcggtggcgc gcaggtgctg ggctctggag ccacactgcg tcttccgtcg    1140

gtggggcccg aggacgcagg cgactatgtg tgcagagctg aggctgggct atcgggcctg    1200

cggggcggcg ccgcggaggc tcggctgact gtgaacgctc ccccagtagt gaccgccctg    1260

cactctgcgc ctgccttcct gaggggccct gctcgcctcc agtgtctggt tttcgcctct    1320

cccgccccag atgccgtggt ctggtcttgg gatgagggct tcctggaggc ggggtcgcag    1380

ggccggttcc tggtggagac attccctgcc ccagagagcc gcgggggact gggtccgggc    1440

ctgatctctg tgctacacat ttcggggacc caggagtctg actttagcag gagctttaac    1500

tgcagtgccc ggaaccggct gggcgaggga ggtgcccagg ccagcctggg ccgtagagac    1560

ttgctgccca ctgtgcggat agtggccgga gtggccgctg ccaccacaac tctccttatg    1620

gtcatcactg gggtggccct ctgctgctgg cgccacagca aggcctcagc ctctttctcc 1680

gagcaaaaga acctgatgcg aatccctggc agcagcgacg gctccagttc acgaggtcct 1740

gaagaagagg agacaggcag ccgcgaggac cggggcccca ttgtgcacac tgaccacagt 1800

gatctggttc tggaggagga agggactctg gagaccaagg acccaaccaa cggttactac 1860

aaggtccgag gagtcagtcc acccgcgtct ccagactcac gtgtgacatc tttccaatgg 1920

aagagtcctg ggatctccaa cttgccataa tggattgttc tgatttctga ggagccagga 1980

caagttggcg accttactcc tcc 2003

<210> 32
<211> 583
<212> PRT
<213> Homo sapiens

<400> 32

Met Leu Arg Met Arg Val Pro Ala Leu Leu Val Leu Leu Phe Cys Phe
1               5                   10                  15

Arg Gly Arg Ala Gly Trp Ser Arg Tyr Trp Ile Ser Gly Asn Ala Ala
            20                  25                  30

Asn Gly Gln His Asp Leu His Ile Arg Pro Val Glu Leu Glu Asp Glu
        35                  40                  45

Ala Ser Tyr Glu Cys Gln Ala Thr Gln Ala Gly Leu Arg Ser Arg Pro
    50                  55                  60

Ala Gln Leu His Val Leu Val Pro Pro Glu Ala Pro Gln Val Leu Gly
65                  70                  75                  80

Gly Pro Ser Val Ser Leu Val Ala Gly Val Pro Ala Asn Leu Thr Cys
                85                  90                  95

Arg Ser Arg Gly Asp Ala Arg Pro Thr Pro Glu Leu Leu Trp Phe Arg
            100                 105                 110

Asp Gly Val Leu Leu Asp Gly Ala Thr Phe His Gln Thr Leu Leu Lys
        115                 120                 125

Glu Gly Thr Pro Gly Ser Val Glu Ser Thr Leu Thr Leu Thr Pro Phe
    130                 135                 140

Ser His Asp Asp Gly Ala Thr Phe Val Cys Arg Ala Arg Ser Gln Ala
145                 150                 155                 160

Leu Pro Thr Gly Arg Asp Thr Ala Ile Thr Leu Ser Leu Gln Tyr Pro
                165                 170                 175

Pro Glu Val Thr Leu Ser Ala Ser Pro His Thr Val Gln Glu Gly Glu
            180                 185                 190

Lys Val Ile Phe Leu Cys Gln Ala Thr Ala Gln Pro Pro Val Thr Gly
        195                 200                 205

Tyr Arg Trp Ala Lys Gly Gly Ser Pro Val Leu Gly Ala Arg Gly Pro
    210                 215                 220

Arg Leu Glu Val Val Ala Asp Ala Ser Phe Leu Thr Glu Pro Val Ser
225                 230                 235                 240

Cys Glu Val Ser Asn Ala Val Gly Ser Ala Asn Arg Ser Thr Ala Leu
                245                 250                 255

Asp Val Leu Phe Gly Pro Ile Leu Gln Ala Lys Pro Glu Pro Val Ser
            260                 265                 270

Val Asp Val Gly Glu Asp Ala Ser Phe Ser Cys Ala Trp Arg Gly Asn
        275                 280                 285

Pro Leu Pro Arg Val Thr Trp Thr Arg Arg Gly Gly Ala Gln Val Leu
    290                 295                 300

Gly Ser Gly Ala Thr Leu Arg Leu Pro Ser Val Gly Pro Glu Asp Ala
305                 310                 315                 320

Gly Asp Tyr Val Cys Arg Ala Glu Ala Gly Leu Ser Gly Leu Arg Gly
                325                 330                 335

Gly Ala Ala Glu Ala Arg Leu Thr Val Asn Ala Pro Pro Val Val Thr
            340                 345                 350

Ala Leu His Ser Ala Pro Ala Phe Leu Arg Gly Pro Ala Arg Leu Gln
        355                 360                 365

Cys Leu Val Phe Ala Ser Pro Ala Pro Asp Ala Val Val Trp Ser Trp
    370                 375                 380

Asp Glu Gly Phe Leu Glu Ala Gly Ser Gln Gly Arg Phe Leu Val Glu
385                 390                 395                 400

Thr Phe Pro Ala Pro Glu Ser Arg Gly Gly Leu Gly Pro Gly Leu Ile
                405                 410                 415

Ser Val Leu His Ile Ser Gly Thr Gln Glu Ser Asp Phe Ser Arg Ser
            420                 425                 430

Phe Asn Cys Ser Ala Arg Asn Arg Leu Gly Glu Gly Gly Ala Gln Ala
        435                 440                 445

Ser Leu Gly Arg Arg Asp Leu Leu Pro Thr Val Arg Ile Val Ala Gly
    450                 455                 460

Val Ala Ala Ala Thr Thr Thr Leu Leu Met Val Ile Thr Gly Val Ala
465                 470                 475                 480

Leu Cys Cys Trp Arg His Ser Lys Ala Ser Ala Ser Phe Ser Glu Gln
                485                 490                 495

Lys Asn Leu Met Arg Ile Pro Gly Ser Ser Asp Gly Ser Ser Ser Arg
            500                 505                 510

Gly Pro Glu Glu Glu Glu Thr Gly Ser Arg Glu Asp Arg Gly Pro Ile
        515                 520                 525

Val His Thr Asp His Ser Asp Leu Val Leu Glu Glu Glu Gly Thr Leu
    530                 535                 540

Glu Thr Lys Asp Pro Thr Asn Gly Tyr Tyr Lys Val Arg Gly Val Ser
545                 550                 555                 560

Pro Pro Ala Ser Pro Asp Ser Arg Val Thr Ser Phe Gln Trp Lys Ser
                565                 570                 575

Pro Gly Ile Ser Asn Leu Pro
            580

<210>  33
<211>  1819
<212>  DNA
<213>  Homo sapiens

<400>  33
accttggggg acgaatgctc tggatgcggg tccccgccct cctcgtcctc ctcttctgct      60

tcagagggag agcagggtgg tcccggtact ggatatcagg gaatgcagcc aatggccagc     120

atgacctcca cattaggccc gtggagctag aggatgaagc atcatatgaa tgtcaggcta     180

cacaagcagg cctccgctcc agaccagccc aactgcacgt gctggtcccc ccagaagccc     240

cccaggtgct gggcggcccc tctgtgtctc tggttgctgg agttcctgcg aacctgacat 300

gtcggagccg tggggatgcc cgccctaccc ctgaattgct gtggttccga gatggggtcc 360

tgttggatgg agccaccttc catcagaccc tgctgaagga agggacccct gggtcagtgg 420

agagcacctt aaccctgacc cctttcagcc atgatgatgg agccaccttt gtctgccggg 480

cccggagcca ggccctgccc acaggaagag acacagctat cacactgagc ctgcagtacc 540

ccccagaggt gactctgtct gcttcgccac acactgtgca ggagggagag aaggtcattt 600

tcctgtgcca ggccacagcc cagcctcctg tcacaggcta caggtgggca aaggggggct 660

ctccggtgct cggggcccgc gggccaaggt tagaggtcgt ggcagacgcc tcgttcctga 720

ctgagcccgt gtcctgcgag gtcagcaacg ccgtgggtag cgccaaccgc agtactgcgc 780

tggatgtgct gtttgggccg attctgcagg caaagccgga gcccgtgtcc gtggacgtgg 840

gggaagacgc ttccttcagc tgcgcctggc gcgggaatcc gcttccacgg gtaacctgga 900

cccgccgcgg tggcgcgcag gtgctgggct ctggagccac actgcgtctt ccgtcggtgg 960

ggcccgagga cgcagacgac tatgtgtgca gagctgaggc tgggctatcg ggtctgcggg 1020

gcggcgccgt ggaggctcgg ctgactgtgg acgctccccc agtagtgacc gccctgcact 1080

ctgcgcctgc cttcctgagg ggccctgctc gcctccagtg tctggttttc gcctctcccg 1140

ccccagatgc cgtggtctgg tcttgggatg agggcttcct ggaggcgggg tcgcagggtc 1200

ggttcctggt ggagacattc cctgccccag agagccgcgg gggactgggt ccgggcctga 1260

tctctgtgct acacatttcg gggacccagg agtctgactt tagcaggagc tttaactgca 1320

gtgcccggaa ccggctgggc gagggaggtg cccaggccag cctgggccgt agagacttgc 1380

tgcccactgt gcggatagtg gccggagtgg tcgctgccac cacaactctc cttatggtca 1440

tcactggggt ggccctctgc tgctggcgcc acagcaaggc ctcagcctct ttctccgagc 1500

aaaagaacct gatgcgaatc cctggcagca gcgacggctc cagttcacga ggtcctgaag 1560

aagaggagac aggcagccgc gaggaccggg gccccattgt gcacactgac cacagtgatc 1620

tggttctgga ggaggaaggg actctggaga ccaaggaccc aaccaacggt tactacaagg 1680

tccgaggagt cagtccaccc gcgtctccag actcacgtgt gacatctttc caatggaaga 1740

gtcctgggat ctccaacttg ccataatgga ttgttctgat ttctgaggag ccaggacaag 1800

ttggcgacct tactcctcc 1819

<210> 34
<211> 583
<212> PRT
<213> Homo sapiens

<400>  34

<210>  35
<211>  2959
<212>  DNA
<213>  Homo sapiens

<400>  35
atgcacttga gtgcaccttg agtctccagc ctctcaagga accgggagat caggccatca      60

gcgtctcagc cagcaaaggc ctgaaccacc agtcccttat aaccctgcgt ttcagagcgt     120

cagaggcgtg cttgagagga agaagttgac gggaaggcca gtgcgacggc aaatctcgtg     180

aaccttgggg gacgaatgct caggatgcgg gtccccgccc tcctcgtcct cctcttctgc     240

ttcagaggga gagcaggccc gtcgccccat ttcctgcaac agccagagga cctggtggtg     300

ctgctggggg aggaagcccg gctgccgtgt gctctgggcg cctactgggg gctagttcag     360

tggactaaga gtgggctggc cctagggggc caaaggacct taccagggtg gtcccggtac     420

tggatatcag ggaatgcagc caatggccag catgacctcc acattaggcc cgtggagcta     480

gaggatgaag catcatatga atgtcaggct acacaagcag gcctccgctc cagaccagcc     540

caactgcacg tgctggtccc cccagaagcc ccccaggtgc tgggcggccc ctctgtgtct     600

ctggttgctg gagttcctgc gaacctgaca tgtcggagcc gtggggatgc ccgccctacc     660

cctgaattgc tgtggttccg agatggggtc ctgttggatg gagccacctt ccatcagacc     720

ctgctgaagg aagggacccc tgggtcagtg gagagcacct taaccctgcc cacaggaaga     780

gacacagcta tcacactgag cctgcagtac cccccagagg tgactctgtc tgcttcgcca     840

cacactgtgc aggagggaga gaaggtcatt ttcctgtgcc aggccacagc ccagcctcct 900
gtcacaggct acaggtgggc aaaagggggc tctccggtgc tcggggcccg cgggccaagg 960
ttagaggtcg tggcagacgc ctcgttcctg actgagcccg tgtcctgcga ggtcagcaac 1020
gccgtgggta gcgccaaccg cagtactgcg ctggatgtgc tgtttgggcc gattctgcag 1080
gcaaagccgg agcccgtgtc cgtggacgtg ggggaagacg cttccttcag ctgcgcctgg 1140
cgcgggaacc cgcttccacg ggtaacctgg acccgccgcg gtggcgcgca ggtgctgggc 1200
tctggagcca cactgcgtct tccgtcggtg gggcccgagg acgcaggcga ctatgtgtgc 1260
agagctgagg ctgggctatc gggcctgcgg ggcggcgccg cggaggctcg gctgactgtg 1320
aacgctcccc cagtagtgac cgccctgcac tctgcgcctg ccttcctgag gggccctgct 1380
cgcctccagt gtctggtttt cgcctctccc gccccagatg ccgtggtctg gtcttgggat 1440
gagggcttcc tggaggcggg gtcgcagggc cggttcctgg tggagacatt ccctgcccca 1500
gagagccgcg ggggactggg tccgggcctg atctctgtgc tacacatttc ggggacccag 1560
gagtctgact ttagcaggag ctttaactgc agtgcccgga accggctggg cgagggaggt 1620
gcccaggcca gcctgggccg tagagacttg ctgcccactg tgcggatagt ggccggagtg 1680
gccgctgcca ccacaactct ccttatggtc atcactgggg tggccctctg ctgctggcgc 1740
cacagcaagg cctcagcctc tttctccgag caaaagaacc tgatgcgaat ccctggcagc 1800
agcgacggct ccagttcacg aggtcctgaa gaagaggaga caggcagccg cgaggaccgg 1860
ggccccattg tgcacactga ccacagtgat ctggttctgg aggaggaagg gactctggag 1920
accaaggacc caaccaacgg ttactacaag gtccgaggag tcagtgtgag cctgagcctt 1980
ggcgaagccc ctggaggagg tctcttcctg ccaccaccct ccccccttgg gcccccaggg 2040
acccctacct tctatgactt caacccacac ctgggcatgg tccccccctg cagactttac 2100
agagccaggg caggctatct caccacaccc caccctcgag ctttcaccag ctacatcaaa 2160
cccacatcct ttgggccccc agatctggcc cccgggactc cccccttccc atatgctgcc 2220
ttccccacac ctagccaccc gcgtctccag actcacgtgt gacatctttc caatggaaga 2280
gtcctgggat ctccaacttg ccataatgga ttgttctgat ttctgaggag ccaggacaag 2340
ttggcgacct tactcctcca aaactgaaca caaggggagg gaaagatcat tacatttgtc 2400
aggagcattt gtatacagtc agctcagcca aaggagatgc cccaagtggg agcaacatgg 2460
ccacccaata tgcccaccta ttccccggtg taaaagagat tcaagatggc aggtaggccc 2520
tttgaggaga gatggggaca gggcagtggg tgttgggagt ttggggccgg gatggaagtt 2580
gtttctagcc actgaaagaa gatatttcaa gatgaccatc tgcattgaga ggaaaggtag 2640

cataggatag atgaagatga agagcatacc aggccccacc ctggctctcc ctgaggggaa 2700

ctttgctcgg ccaatggaaa tgcagccaag atggccatat actccctagg aacccaagat 2760

ggccaccatc ttgattttac tttccttaaa gactcagaaa gacttggacc caaggagtgg 2820

ggatacagtg agaattacca ctgttggggc aaaatattgg gataaaaata tttatgttta 2880

ataataaaaa aaagtcaaag aggaaaaaaa aaaaaaaaaa aaaaaaaaaa aaaaaaaaaa 2940

aaaaaaaaaa aaaaaaaaa 2959

<210> 36
<211> 688
<212> PRT
<213> Homo sapiens

<400> 36

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu

                165                 170                 175

Leu Lys Glu Gly Thr Pro Gly Ser Val Glu Ser Thr Leu Thr Leu Pro
            180                 185                 190

Thr Gly Arg Asp Thr Ala Ile Thr Leu Ser Leu Gln Tyr Pro Pro Glu
        195                 200                 205

Val Thr Leu Ser Ala Ser Pro His Thr Val Gln Glu Gly Glu Lys Val
    210                 215                 220

Ile Phe Leu Cys Gln Ala Thr Ala Gln Pro Pro Val Thr Gly Tyr Arg
225                 230                 235                 240

Trp Ala Lys Gly Gly Ser Pro Val Leu Gly Ala Arg Gly Pro Arg Leu
                245                 250                 255

Glu Val Val Ala Asp Ala Ser Phe Leu Thr Glu Pro Val Ser Cys Glu
            260                 265                 270

Val Ser Asn Ala Val Gly Ser Ala Asn Arg Ser Thr Ala Leu Asp Val
        275                 280                 285

Leu Phe Gly Pro Ile Leu Gln Ala Lys Pro Glu Pro Val Ser Val Asp
    290                 295                 300

Val Gly Glu Asp Ala Ser Phe Ser Cys Ala Trp Arg Gly Asn Pro Leu
305                 310                 315                 320

Pro Arg Val Thr Trp Thr Arg Arg Gly Gly Ala Gln Val Leu Gly Ser
                325                 330                 335

Gly Ala Thr Leu Arg Leu Pro Ser Val Gly Pro Glu Asp Ala Gly Asp
            340                 345                 350

Tyr Val Cys Arg Ala Glu Ala Gly Leu Ser Gly Leu Arg Gly Gly Ala
        355                 360                 365

Ala Glu Ala Arg Leu Thr Val Asn Ala Pro Pro Val Val Thr Ala Leu
    370                 375                 380

His Ser Ala Pro Ala Phe Leu Arg Gly Pro Ala Arg Leu Gln Cys Leu
385                 390                 395                 400

Val Phe Ala Ser Pro Ala Pro Asp Ala Val Val Trp Ser Trp Asp Glu
                405                 410                 415

Gly Phe Leu Glu Ala Gly Ser Gln Gly Arg Phe Leu Val Glu Thr Phe
            420                 425                 430

Pro Ala Pro Glu Ser Arg Gly Gly Leu Gly Pro Gly Leu Ile Ser Val
        435                 440                 445

Leu His Ile Ser Gly Thr Gln Glu Ser Asp Phe Ser Arg Ser Phe Asn
    450                 455                 460

Cys Ser Ala Arg Asn Arg Leu Gly Glu Gly Gly Ala Gln Ala Ser Leu
465                 470                 475                 480

Gly Arg Arg Asp Leu Leu Pro Thr Val Arg Ile Val Ala Gly Val Ala
                485                 490                 495

Ala Ala Thr Thr Thr Leu Leu Met Val Ile Thr Gly Val Ala Leu Cys
            500                 505                 510

Cys Trp Arg His Ser Lys Ala Ser Ala Ser Phe Ser Glu Gln Lys Asn
        515                 520                 525

Leu Met Arg Ile Pro Gly Ser Ser Asp Gly Ser Ser Ser Arg Gly Pro
    530                 535                 540

Glu Glu Glu Glu Thr Gly Ser Arg Glu Asp Arg Gly Pro Ile Val His
545                 550                 555                 560

Thr Asp His Ser Asp Leu Val Leu Glu Glu Glu Gly Thr Leu Glu Thr
                565                 570                 575

Lys Asp Pro Thr Asn Gly Tyr Tyr Lys Val Arg Gly Val Ser Val Ser
            580                 585                 590

Leu Ser Leu Gly Glu Ala Pro Gly Gly Gly Leu Phe Leu Pro Pro Pro
        595                 600                 605

Ser Pro Leu Gly Pro Pro Gly Thr Pro Thr Phe Tyr Asp Phe Asn Pro
    610                 615                 620

His Leu Gly Met Val Pro Pro Cys Arg Leu Tyr Arg Ala Arg Ala Gly
625                 630                 635                 640

Tyr Leu Thr Thr Pro His Pro Arg Ala Phe Thr Ser Tyr Ile Lys Pro
                645                 650                 655

Thr Ser Phe Gly Pro Pro Asp Leu Ala Pro Gly Thr Pro Pro Phe Pro
            660                 665                 670

Tyr Ala Ala Phe Pro Thr Pro Ser His Pro Arg Leu Gln Thr His Val
        675                 680                 685

<210>  37
<211>  2591
<212>  DNA
<213>  Pan troglodytes

<400>  37
atgctcagga tgcgggtccc cgccctcctc gtcctcctct tctgcttcag agggagcgca      60

ggcccatcgc cccatttcct gcaacagcca gaggacctgg tggtgctgct gggggaggaa     120

gcccggctgc cgtgtgctct gggcgcctac tgggggctag ttcagtggac taagagtggg     180

ctggccctag ggggccaaag ggacctacca gggtggtccc ggtactggat atcagggaat     240

gcagccaatg gccagcatga cctccacatt aggcccgtgg agctagagga tgaagcatca     300

tatgaatgtc aggctacaca agcaggcctc cgctccagac cagcccaact gcacgtgctg     360

gtacccccag aagcccccca ggtgctgggc ggcccctctg tgtctctggt tgctggagtt     420

cctgcgaacc tgacatgtcg gagccgtggg gatgcccgcc ctacccctga attgctgtgg     480

ttccgagatg gggtcctgtt ggatggaacc accttccatc agaccctgct gaaggaaggg     540

acccctgggt cagtggagag caccttaacc ctgacccctt tcagccatga tgatggagcc     600

acctttgtct gccgggcccg gagccaggcc ctgccctcag gaagagacac ggctatcaca     660

ctgagcctgc agtacccccc agaggtgact ctgtctgctt cgccacacac tgtgcaggag     720

ggagagaagg tcattttcct gtgccaggcc acagaccagc ctcctgtcac aggctacagg     780

gaggacgaag acccagaatc caccacaatg gggaccaggc ttcctacaaa tccggcttct     840

gacgcccctt ccctgtcgca ggtgctgggc tctggagcca cactgcatct tccgtcggta     900

gggcccgagg acgcaggcga ctatgtgtgc agagctgagc ctgggctatc gggcctgggg     960

ggcggcgccg cggaggctcg gctgactgtg aacgctcccc cagtagtgac cgccctgcac    1020

tctgcgcctg ccttcctgag gggccctgct cgcctccagt gtctggtttt cgcctctccc    1080

gccccagatg ccgtggtctg gtcttgggat gagggcttcc tggaggcggg gtcgcagggc    1140

cggttcctgg tggagacatt ccctgcccca gagagccgcg ggggactggg tccgggcctg    1200

atctctgtgc tacacatttc ggggacccag gagtctgact ttagcaggag ctttaactgc    1260

agtgcccgga accggctggg cgagggaggt gcccaggcca gcctgggccg tagagacttg    1320

ctgcccactg tgcggatagt ggccggagtg gccgctgcca ccacgactct ccttatggtc    1380

atcactgggg tggccctctg ctgctggcgc cacagcaagg cctcagcctc tttctccgag 1440

caaaagaacc tgatgcgaat ccctggcagc agcgacggct ccagttcgcg aggtcctgaa 1500

gaagaggaga caggcagccg cgaggaccgg ggccccattg tgcacactga ccacagtgat 1560

ctggttctgg aggaggaagg gactctggag accaaggacc caaccaacgg ttactacaag 1620

gtccgaggag tcagtgtgag cctgagcctt ggcgaagccc ctggaggagg cctcttcctg 1680

ccaccaccct ccccccttgg gcccccaggg acccctacct tctatgactt caacccacac 1740

ctgggcatgg tccccccctg cagactttac agagccaggg caggctatct caccacaccc 1800

caccctcgag ctttcaccag ctacatcaaa cccacatcct ttgggccccc agatctggcc 1860

cccgggactc cccccttccc atgtgctgcc ttccccacac ctagccaccc gcgtctccag 1920

actcatgtgt gacatctttc cagtggaaga gtcctgggat ctccaacttg ccataatgga 1980

ttgttctgat ttctgaggag ccaggacaag ttggcgacct tactcctcca aaactgaaca 2040

cagggggagg gaaagatcat tacatttgtc aggagcattt gtgtacagtc agctcagcca 2100

aaggagatgc cccaagtggg agcaacgtgg ccacccaata tgcccaccta ttccccggtg 2160

taaaagagat tcaagatggc aggtaggccc tttgaggaga gatggggaca gggcagtggg 2220

tgttgggagt ttggggccgg gatggaagtt gtttctagcc actgaaagaa gatatttcaa 2280

gatgaccatc tgcattgaga ggaaaggtag cataggatag atgaagatga agagcatacc 2340

aggccccacc ctggctctcc ctgaggggaa ttttgctcgg ccaatggaaa tgcagccaag 2400

atggccatat actccctagg aacccaagat ggccaccatc ttgattttac tttccttaaa 2460

gactcagaaa gacttggact caaggagtgg ggatacagtg agaattacca ctgttggggc 2520

aaaggggatt cttgggataa aaatatttat gtttaataat aaaaaaaagt caaagaggca 2580

agtgtgtctt a 2591

<210> 38
<211> 643
<212> PRT
<213> Pan troglodytes

<400> 38

Met Leu Arg Met Arg Val Pro Ala Leu Leu Val Leu Leu Phe Cys Phe
1               5                   10                  15

Arg Gly Ser Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Thr Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Ser Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Asp Gln Pro Pro Val
                245                 250                 255

Thr Gly Tyr Arg Glu Asp Glu Asp Pro Glu Ser Thr Thr Met Gly Thr
            260                 265                 270

Arg Leu Pro Thr Asn Pro Ala Ser Asp Ala Pro Ser Leu Ser Gln Val
        275                 280                 285

Leu Gly Ser Gly Ala Thr Leu His Leu Pro Ser Val Gly Pro Glu Asp
    290                 295                 300

Ala Gly Asp Tyr Val Cys Arg Ala Glu Pro Gly Leu Ser Gly Leu Gly
305                 310                 315                 320

Gly Gly Ala Ala Glu Ala Arg Leu Thr Val Asn Ala Pro Pro Val Val
                325                 330                 335

Thr Ala Leu His Ser Ala Pro Ala Phe Leu Arg Gly Pro Ala Arg Leu
            340                 345                 350

Gln Cys Leu Val Phe Ala Ser Pro Ala Pro Asp Ala Val Val Trp Ser
        355                 360                 365

Trp Asp Glu Gly Phe Leu Glu Ala Gly Ser Gln Gly Arg Phe Leu Val
    370                 375                 380

Glu Thr Phe Pro Ala Pro Glu Ser Arg Gly Gly Leu Gly Pro Gly Leu
385                 390                 395                 400

Ile Ser Val Leu His Ile Ser Gly Thr Gln Glu Ser Asp Phe Ser Arg
                405                 410                 415

Ser Phe Asn Cys Ser Ala Arg Asn Arg Leu Gly Glu Gly Gly Ala Gln
            420                 425                 430

Ala Ser Leu Gly Arg Arg Asp Leu Leu Pro Thr Val Arg Ile Val Ala
        435                 440                 445

Gly Val Ala Ala Ala Thr Thr Thr Leu Leu Met Val Ile Thr Gly Val
    450                 455                 460

Ala Leu Cys Cys Trp Arg His Ser Lys Ala Ser Ala Ser Phe Ser Glu
465                 470                 475                 480

Gln Lys Asn Leu Met Arg Ile Pro Gly Ser Ser Asp Gly Ser Ser Ser
                485                 490                 495

Arg Gly Pro Glu Glu Glu Glu Thr Gly Ser Arg Glu Asp Arg Gly Pro
            500                 505                 510

Ile Val His Thr Asp His Ser Asp Leu Val Leu Glu Glu Glu Gly Thr
        515                 520                 525

Leu Glu Thr Lys Asp Pro Thr Asn Gly Tyr Tyr Lys Val Arg Gly Val

530                 535                 540

Ser Val Ser Leu Ser Leu Gly Glu Ala Pro Gly Gly Gly Leu Phe Leu
545                 550                 555                 560

Pro Pro Pro Ser Pro Leu Gly Pro Pro Gly Thr Pro Thr Phe Tyr Asp
                565                 570                 575

Phe Asn Pro His Leu Gly Met Val Pro Pro Cys Arg Leu Tyr Arg Ala
            580                 585                 590

Arg Ala Gly Tyr Leu Thr Thr Pro His Pro Arg Ala Phe Thr Ser Tyr
        595                 600                 605

Ile Lys Pro Thr Ser Phe Gly Pro Pro Asp Leu Ala Pro Gly Thr Pro
    610                 615                 620

Pro Phe Pro Cys Ala Ala Phe Pro Thr Pro Ser His Pro Arg Leu Gln
625                 630                 635                 640

Thr His Val

<210>  39
<211>  3019
<212>  DNA
<213>  Bos taurus

<400>  39
atgggaagag ggcgggtctc ccagggggcg ggccggggcg gggcgagtgg aaaccagccc      60

tccagggtct ctccgagacc tggcgcgcct aactggcagg cgtttcagag cggcggagtc     120

tgcggcggag cagacgaggg gacctccagg tcagagagag gggagcttct agcctggggc     180

gaagagtcaa gagcggaggg gactccaggc cagagtggtg ggaagtgtgc tccgaggaag     240

gaactggcag ggaagccagt gcaacagcaa gtctgctgga tcttgggcga cacacttagg     300

atgctcgtgc ccgtactcct cgtcctctcc ttctgcctca gggggcgtgc aggcctggca     360

ccccactttc tgcaacagcc agaggaccag gtagttctgt tgggagacga ggcccgactg     420

ccctgtgccc tgggcgacta ccggggactg gttcagtgga ctaaggacgg gctggctcta     480

gggggcgaaa gggaccttcc aggctggtcc cggtactgga tatcagggaa tgcagcaagt     540

ggccagcatg acctccacat tagacccgtg gagctagagg atcaagcatc gtatgaatgt     600

caagcgacac aagccggcct ccgctctcga ccagcccaac tgcatgtgct ggtgcctcca     660

gacccccccc aggtgctggg cggcccctct gtgtctctgg ttgctggggt tcctgcgaat     720

ctgacctgtc ggagccgtgg tgatgcccac cctacccctg agctgctgtg gtttcgagat 780
gggatccgac tggacggggc catcttccgc cagactcctt tgaaggaagg aaccatcggg 840
tcagtggaga gcatcttgtc tttgacccct tccagccatg atgatggagc caccttggtt 900
tgccgggccc ggagccaggc cctgcctgca gggaaggaca cagctgtaac actaagcctg 960
cagtaccccc cagtggtgac tctgtctgca gaaccacaga cagtgcagga gggagagaag 1020
gtcactttcc tatgccaggc cacagcccag cctcctgtca ccggctatag atgggcaaag 1080
gggggctccc cggtgctcgg ggcccgaggg ccaatgctgg agatagtggc tgacgcttca 1140
ttcctgactg cgccggtgtc ctgcgaggtc agcaatgcag tgggtagcgc caaccgcagc 1200
acagcgctgg acgtgcagtt tggcccgatt ctgcaggcaa agccgaaagc cttgtcggtg 1260
gacgtaggag aagacgcctc cttcagctgt gtctggcgcg ggaatccgct cccacgggta 1320
acctggaccc gccgcgggga cgcacaggtg ctggcctccg ggcccacgct gcgtcttccc 1380
gcggtggggc ccgaggatgc aggcgactac gtgtgcaggg ccgagccggg gctctcaggc 1440
cggggcggtg gctctgcaga agctaggctg actgtgaacg ctcccccagt agtgaccgct 1500
ctgcactctg cgcccgcctt cctgaggggc cccgcccgcc tccagtgtct agtcttcgct 1560
tcccccaccc cagaagcagt ggtctggtct tgggatgagg gcttcctgac ggcggggtca 1620
cggggtcggt tcctggtgga gactttccca gccccagagg gcctcaaggg acagggtcca 1680
ggcctgatct ccgtgctaca catttcgggg acccaggagt ccgactttca ccggggcttc 1740
aactgcactg cccggaaccg attgggtgag ggaggcaccc aggtcagcct gggccgtaga 1800
gatttgctgc ccactgtgcg gattgtggcc ggagtggccg ccatggccat gactctcctt 1860
atgatcatca ctggggtggc cctctgctgc tggcgccatg gcaggggtca gtcctctttc 1920
tccaagcaaa agaacctggt gcgaatccca gggagcagtg atggctccag ttccaggggc 1980
cccgaggagg agacaggcat cagcaaagac cagggcccca tcgtgcacac ggaccacagt 2040
gacctggttc tggatgagga gggggctctg gagaccaagg acccaaccaa tggttactac 2100
aaggtccgag gagtcagtgt gagcctgagc cttggagaag cccccggagg aggtctcttc 2160
ctaccaccct cctcccctct tggacctcca ggaaccccta ccttctatga cttcaatcca 2220
catcttggca tggtccctcc ctgcagacta tatagatccc gggcaggcta tctcaccaca 2280
ccccatcctc gagcttttac cagctacatc aaacccacat cctttggacc cccagatctg 2340
gcccccagca ctcccccctt cccatatgct gccttcccca cgcccagcca cccacgtctc 2400
cagactcatg tgtgacatct ctccagctga agagtcctgg gatcttcaac ttgcacaatg 2460
gattgtcctg atttctgagt aaccagaaca aactggcaac cctatccttc caaaattgaa 2520
ccttgagggg agggaaagat cattgcagtt gcctgggtca ttgcgagaaa gccactgggc 2580

ttgcctcttc ctctgtataa aagagattca agagggcaga tgggcccttt gcagagggat 2640

ggggacaggg gttatgggag ttagggacag agaaggaaat tgcttccaaa cattgaaaga 2700

agagatttca agatggccac ccacattaag aagaaaggca acataaagca gatcaagata 2760

caggacctgt ccttgctatc cctgaggggt actttgtttc acccatggaa ttgctgccaa 2820

aatggccatt cattccctgg gaatccaaga tggccaccat cttgattctt tctaccttcc 2880

ttaaaggccc tggaagaacc ggacccaagg agtaaggaca gggtgaaagc tgttgtggtg 2940

gtgggaaggg ggaaaggagt ggtgttggaa tctgggaatg agaatattta tgttcaatta 3000

aaaaaaaaaa aagttgcaa 3019

<210> 40
<211> 804
<212> PRT
<213> Bos taurus

<400> 40

Met Gly Arg Gly Arg Val Ser Gln Gly Ala Gly Arg Gly Gly Ala Ser
1               5                   10                  15

Gly Asn Gln Pro Ser Arg Val Ser Pro Arg Pro Gly Ala Pro Asn Trp
            20                  25                  30

Gln Ala Phe Gln Ser Gly Gly Val Cys Gly Gly Ala Asp Glu Gly Thr
        35                  40                  45

Ser Arg Ser Glu Arg Gly Glu Leu Leu Ala Trp Gly Glu Glu Ser Arg
    50                  55                  60

Ala Glu Gly Thr Pro Gly Gln Ser Gly Gly Lys Cys Ala Pro Arg Lys
65                  70                  75                  80

Glu Leu Ala Gly Lys Pro Val Gln Gln Gln Val Cys Trp Ile Leu Gly
                85                  90                  95

Asp Thr Leu Arg Met Leu Val Pro Val Leu Leu Val Leu Ser Phe Cys
            100                 105                 110

Leu Arg Gly Arg Ala Gly Leu Ala Pro His Phe Leu Gln Gln Pro Glu
        115                 120                 125

Asp Gln Val Val Leu Leu Gly Asp Glu Ala Arg Leu Pro Cys Ala Leu
    130                 135                 140

Gly Asp Tyr Arg Gly Leu Val Gln Trp Thr Lys Asp Gly Leu Ala Leu
145                 150                 155                 160

Gly Gly Glu Arg Asp Leu Pro Gly Trp Ser Arg Tyr Trp Ile Ser Gly
                165                 170                 175

Asn Ala Ala Ser Gly Gln His Asp Leu His Ile Arg Pro Val Glu Leu
            180                 185                 190

Glu Asp Gln Ala Ser Tyr Glu Cys Gln Ala Thr Gln Ala Gly Leu Arg
        195                 200                 205

Ser Arg Pro Ala Gln Leu His Val Leu Val Pro Pro Asp Pro Pro Gln
    210                 215                 220

Val Leu Gly Gly Pro Ser Val Ser Leu Val Ala Gly Val Pro Ala Asn
225                 230                 235                 240

Leu Thr Cys Arg Ser Arg Gly Asp Ala His Pro Thr Pro Glu Leu Leu
                245                 250                 255

Trp Phe Arg Asp Gly Ile Arg Leu Asp Gly Ala Ile Phe Arg Gln Thr
            260                 265                 270

Pro Leu Lys Glu Gly Thr Ile Gly Ser Val Glu Ser Ile Leu Ser Leu
        275                 280                 285

Thr Pro Ser Ser His Asp Asp Gly Ala Thr Leu Val Cys Arg Ala Arg
    290                 295                 300

Ser Gln Ala Leu Pro Ala Gly Lys Asp Thr Ala Val Thr Leu Ser Leu
305                 310                 315                 320

Gln Tyr Pro Pro Val Val Thr Leu Ser Ala Glu Pro Gln Thr Val Gln
                325                 330                 335

Glu Gly Glu Lys Val Thr Phe Leu Cys Gln Ala Thr Ala Gln Pro Pro
            340                 345                 350

Val Thr Gly Tyr Arg Trp Ala Lys Gly Gly Ser Pro Val Leu Gly Ala
        355                 360                 365

Arg Gly Pro Met Leu Glu Ile Val Ala Asp Ala Ser Phe Leu Thr Ala
    370                 375                 380

Pro Val Ser Cys Glu Val Ser Asn Ala Val Gly Ser Ala Asn Arg Ser

385                 390                 395                 400

Thr Ala Leu Asp Val Gln Phe Gly Pro Ile Leu Gln Ala Lys Pro Lys
                405                 410                 415

Ala Leu Ser Val Asp Val Gly Glu Asp Ala Ser Phe Ser Cys Val Trp
            420                 425                 430

Arg Gly Asn Pro Leu Pro Arg Val Thr Trp Thr Arg Arg Gly Asp Ala
        435                 440                 445

Gln Val Leu Ala Ser Gly Pro Thr Leu Arg Leu Pro Ala Val Gly Pro
    450                 455                 460

Glu Asp Ala Gly Asp Tyr Val Cys Arg Ala Glu Pro Gly Leu Ser Gly
465                 470                 475                 480

Arg Gly Gly Gly Ser Ala Glu Ala Arg Leu Thr Val Asn Ala Pro Pro
                485                 490                 495

Val Val Thr Ala Leu His Ser Ala Pro Ala Phe Leu Arg Gly Pro Ala
            500                 505                 510

Arg Leu Gln Cys Leu Val Phe Ala Ser Pro Thr Pro Glu Ala Val Val
        515                 520                 525

Trp Ser Trp Asp Glu Gly Phe Leu Thr Ala Gly Ser Arg Gly Arg Phe
    530                 535                 540

Leu Val Glu Thr Phe Pro Ala Pro Glu Gly Leu Lys Gly Gln Gly Pro
545                 550                 555                 560

Gly Leu Ile Ser Val Leu His Ile Ser Gly Thr Gln Glu Ser Asp Phe
                565                 570                 575

His Arg Gly Phe Asn Cys Thr Ala Arg Asn Arg Leu Gly Glu Gly Gly
            580                 585                 590

Thr Gln Val Ser Leu Gly Arg Arg Asp Leu Leu Pro Thr Val Arg Ile
        595                 600                 605

Val Ala Gly Val Ala Ala Met Ala Met Thr Leu Leu Met Ile Ile Thr
    610                 615                 620

Gly Val Ala Leu Cys Cys Trp Arg His Gly Arg Gly Gln Ser Ser Phe
625                 630                 635                 640

Ser Lys Gln Lys Asn Leu Val Arg Ile Pro Gly Ser Ser Asp Gly Ser
                645                 650                 655

Ser Ser Arg Gly Pro Glu Glu Glu Thr Gly Ile Ser Lys Asp Gln Gly
            660                 665                 670

Pro Ile Val His Thr Asp His Ser Asp Leu Val Leu Asp Glu Glu Gly
        675                 680                 685

Ala Leu Glu Thr Lys Asp Pro Thr Asn Gly Tyr Tyr Lys Val Arg Gly
    690                 695                 700

Val Ser Val Ser Leu Ser Leu Gly Glu Ala Pro Gly Gly Gly Leu Phe
705                 710                 715                 720

Leu Pro Pro Ser Ser Pro Leu Gly Pro Pro Gly Thr Pro Thr Phe Tyr
                725                 730                 735

Asp Phe Asn Pro His Leu Gly Met Val Pro Pro Cys Arg Leu Tyr Arg
            740                 745                 750

Ser Arg Ala Gly Tyr Leu Thr Thr Pro His Pro Arg Ala Phe Thr Ser
        755                 760                 765

Tyr Ile Lys Pro Thr Ser Phe Gly Pro Pro Asp Leu Ala Pro Ser Thr
    770                 775                 780

Pro Pro Phe Pro Tyr Ala Ala Phe Pro Thr Pro Ser His Pro Arg Leu
785                 790                 795                 800

Gln Thr His Val

<210>  41
<211>  291
<212>  DNA
<213>  Artificial

<220>
<223>  an artificially synthesized sequence

<400>  41
aagctttcta gagggcccgt ttaaacccgc tgatcagcct cgactgtgcc ttctagttgc      60

cagccatctg ttgtttgccc ctcccccgtg ccttccttga ccctggaagg tgccactccc     120

actgtccttt cctaataaaa tgaggaaatt gcatcgcatt gtctgagtag gtgtcattct     180

attctggggg gtggggtggg gcaggacagc aagggggagg attgggaaga caatagcagg     240

catgctgggg atgcggtggg ctctatggct tctgaggcgg aaagactcga g              291


<210>  42
<211>  32
<212>  DNA
<213>  Artificial

<220>
<223>  an artificially synthesized sequence

<400>  42
gtaccactag tgctagcacg cgtgcggccg cg                                    32


<210>  43
<211>  32
<212>  DNA
<213>  Artificial

<220>
<223>  an artificially synthesized sequence

<400>  43
gatccgcggc cgcacgcgtg ctagcactag tg                                    32


<210>  44
<211>  3261
<212>  DNA
<213>  Mus musculus

<400>  44
atcgattagg agcctatggt ggcacttgtg agcaccagca gcttgtttgt tgctgtcttc      60

cctgagcctc tctctgtgtg ccctgctttt ctgttctttc aatctctttc taactcgccc     120

ttctctcttc cctccctccc tccctccctt ccttctttct cttatcttcc taccattgtc     180

tgtgactgtc gcagtctttc tgtcccggca tcgccttttc tgcccttttc tccccctttc     240

tcccccgtta acagttcttt ctctgagctc taaccccaag accttaaaaa tgcccccttc    300

tctctgtttt tctttactat cacctccctc tcacccactc acaggtactc aaagagcacc     360

ggctgctcta agtccagcag gttcacgaac ccaaagcagt cagccacagc actgagcatt     420

gcttggaccc agtgtgaact cattctgctg cttcccccaa gtccttcctg agtctgccca     480

gccgggcaat gaaatcccct gtcagcacat gccaggaaca ttctttggtg cctccccagc     540

ccgggtgacc ccaaggcccc gggagagcat ttctgatcag ggccatctcc agctcaggat     600

ggaacgcaga gctaggcctg tgtgcgtggg aaggctacaa cccccccccc aggacatcct     660

tgctctttgc agcccagact gtggcatgta ccgcacccct gctggtgaat atgcaggagt     720

gtgttgttaa accaagagag tatgggacag agagaagcca cttctgtggg tatgggtttc     780

agagtgtgcg tgttccaggg gagacgagag cagattttca tctgtggacg tatttggagt 840

gtctcatatg ggtgtgcaca gagcatccag acagtcagca ggactgtgtc cgcagacctc 900

agtgtgtgga aacctggtgt gctcagacgc agataaacat agggtggggg gtccctggga 960

gctgaagcac ctgaatccct gggccatgtg agggccttct gtttcctcaa ctcctccatc 1020

ttcccttgtg tgtgtgtgtg tgtgtgtgtg tatgtcttgc taggatgatt tatcttctta 1080

ttcttctcca cgtctttaat tccatctccc ccctcctcct cctcctccat tcctaccccc 1140

ttctcatttt ctgtcactct tgctgccccc tctgtgtctg gtccttgaat tttagaagca 1200

tatgttgact ggcctgccct cccaacctgg gtgcacagat gaattcgcct gggataagcc 1260

cctgctgcct gcttatctcc tgcaggaatt gcaactgttc tcttttgccc catattccct 1320

acaaacctct cacctcccgg ctgcttcttc tccaggaggc agtccaggca tctggcctga 1380

gcccccctca cagctctcta ccaagggtga gccctctggt ctttctttcc cacccagccc 1440

cattattgat tcattaccct tggtccacat tcccatctcc aaacctatca acacctcctg 1500

ggcaatggat tgcatacaga ccaagccagg gagtctgcta tggggccttt gccagatggt 1560

atctttgggg gccaaggtgt agagctgtca gttgctggag cctttaatta gtgcaaacct 1620

tctacactca cctttggctg ttttccttct ctgaatgctc tggggatccc aggctctcca 1680

tttttccatc ccgtggcccc agagtgccag gaaagggaag ctgtgtcagg tgtctggaaa 1740

aacagcctct cacctgactt cctcccgggg acttaggagt cctgggctca agtctgtcct 1800

atttcaaaac tcagaagcca ggagttcaga tttaggtgtg aggcctccta ggactctctt 1860

ttccaggccc ccagcctgca atccctccac ttctacagga ctcagcacag ctgttcagtc 1920

tgttggtttt cccatcctcg ggggccctag ggggtgagtg agaacgagac tggatgtcaa 1980

atccagtagc tttaagactt aagaccagac attctgaaac tggctcaact ccaccacact 2040

gcagcctgca aggcttctca gccagcagag aagtctgaac tatccccctg gggcaagtcc 2100

aaccctctga tatttagaag taccagtctg agccccaaat gtcatttatt tctgagaggt 2160

ccaggaatgt cagacccggg gtctcaggcc cccaacctcc ccccctccag ccccttcagt 2220

gagctcaggg tccctcccac ctgctctgcc agctgcactg cgtgggaacg cccagctggg 2280

ctgcaccgga gctgtcagga caagctgtgc ggttcccagc ctccctccct gcctgccaga 2340

gccagggcac tgctgcctcc cagccgtcgc ccggcaacca ctcaccactc ggatgggcca 2400

gggactgctg ggtggagaga ggggacgggt gggtgggtac tgctgggtcc tgggaaagga 2460

ggaactcctg gaggggaggg ggcgggctag attcctagat cttaaggtac gtgtatggct 2520

tgcagggata acagagcagc agggagtatt ttgggaaata gggagtattt gggaaatagg 2580

gagcagaaac cctcttctct caaggctcct aaatggtcct tcagcacctg ggtgccctct 2640

ctccgacccg caggcccacg ggagcctgag ctccgcctcc ccagggcgcg gaagctggcg 2700

aagccccagg gattcccatt tatagcttgg tttccactca gctcagtccc tccaggactc 2760

gggctgagca agtttcttcc attcccttct ctcctccctc cacccccttc tcctcctcct 2820

tctccttctt ttcttcctcc tcattcccgc ctccccttca acctcagcag ggtgcaggtg 2880

tccaactcga acaagggccc caacttggac tcagatgttc ccactctcag accccctgat 2940

aatgccgtcc gcacacaagg cgcgggagtt tctcaatggg aagaggccgg gactctagga 3000

ggcggggcga ataggattcc tcccgcctag tgggtccctc gcagtcctag ggttgcaacc 3060

cttgagcggt agagaacacc ggagactgcg gatgagccag atttcgggga cataaaatct 3120

tccagcccgg agagaattgt gtgcagagag gggctccagt ccagcgtggt gtgagaggcg 3180

tgctatcaag aaagaagttg gaggggaacc agtgcaaccc taactctgcg agatcttggg 3240

gtacacacac tcgggggtac c 3261

<210> 45
<211> 798
<212> DNA
<213> Mus musculus

<400> 45
ggtaccatgc cgcgctcctt cctggtggat tcccttgtgc tgcgggaagc cagcgacaag 60

aaggctccgg agggcagccc gccaccgctc ttcccctacg cggtcccgcc gccgcacgcg 120

ctccacggcc tctcgccggg cgcctgccac gcgcgcaagg ccggcttgct gtgcgtgtgt 180

cccctctgtg tcaccgcttc gcagctgcac gggccccccg ggccgccggc actgccgcta 240

ctcaaggcgt ccttccctcc cttcggatcg cagtactgcc acgcacccct gggccgccag 300

cactccgtgt cccctggagt cgcccacggc ccggccgcgg ccgcagcagc tgctgcactc 360

taccagacct cctacccgct gccggatccc agacagtttc actgcatctc tgtggacagc 420

agctcgaacc agctgcccag cagcaagagg atgcggacgg cgttcaccag cacacagctc 480

ctggagctgg agcgagagtt cgcctccaac atgtacctct cccgcctgcg gcgcatcgag 540

atcgcgacct atctgaacct gtccgagaag caggtgaaga tctggtttca gaaccgccgg 600

gtgaagcaca agaaagaagg caaaggcagt aaccaccgcg gcggagctgg ggcgggggcc 660

ggcgggggcg caccgcaagg ctgcaagtgc tcttcgctct cctcagccaa atgctcagag 720

gacgacgacg aattgcccat gtctccatct tcctccggga aggatgacag agatctcaca 780

gtcactccgt aggtcgac 798

<210> 46
<211> 4828

<212>  DNA
<213>  Homo sapiens

<400>  46
agtggcgtcg gaactgcaaa gcacctgtga gcttgcggaa gtcagttcag actccagccc      60

gctccagccc ggcccgaccc gaccgcaccc ggcgcctgcc ctcgctcggc gtccccggcc     120

agccatgggc ccttggagcc gcagcctctc ggcgctgctg ctgctgctgc aggtctcctc     180

ttggctctgc caggagccgg agccctgcca ccctggcttt gacgccgaga gctacacgtt     240

cacggtgccc cggcgccacc tggagagagg ccgcgtcctg ggcagagtga attttgaaga     300

ttgcaccggt cgacaaagga cagcctattt ttccctcgac acccgattca aagtgggcac     360

agatggtgtg attacagtca aaaggcctct acggtttcat aacccacaga tccatttctt     420

ggtctacgcc tgggactcca cctacagaaa gttttccacc aaagtcacgc tgaatacagt     480

ggggcaccac caccgccccc cgccccatca ggcctccgtt tctggaatcc aagcagaatt     540

gctcacattt cccaactcct ctcctggcct cagaagacag aagagagact gggttattcc     600

tcccatcagc tgcccagaaa atgaaaaagg cccatttcct aaaaacctgg ttcagatcaa     660

atccaacaaa gacaaagaag gcaaggtttt ctacagcatc actggccaag gagctgacac     720

accccctgtt ggtgtcttta ttattgaaag agaaacagga tggctgaagg tgacagagcc     780

tctggataga gaacgcattg ccacatacac tctcttctct cacgctgtgt catccaacgg     840

gaatgcagtt gaggatccaa tggagatttt gatcacggta accgatcaga atgacaacaa     900

gcccgaattc acccaggagg tctttaaggg gtctgtcatg gaaggtgctc ttccaggaac     960

ctctgtgatg gaggtcacag ccacagacgc ggacgatgat gtgaacacct acaatgccgc    1020

catcgcttac accatcctca gccaagatcc tgagctccct gacaaaaata tgttcaccat    1080

taacaggaac acaggagtca tcagtgtggt caccactggg ctggaccgag agagtttccc    1140

tacgtatacc ctggtggttc aagctgctga ccttcaaggt gaggggttaa gcacaacagc    1200

aacagctgtg atcacagtca ctgacaccaa cgataatcct ccgatcttca atcccaccac    1260

gtacaagggt caggtgcctg agaacgaggc taacgtcgta atcaccacac tgaaagtgac    1320

tgatgctgat gcccccaata ccccagcgtg ggaggctgta tacaccatat tgaatgatga    1380

tggtggacaa tttgtcgtca ccacaaatcc agtgaacaac gatggcattt tgaaaacagc    1440

aaagggcttg gattttgagg ccaagcagca gtacattcta cacgtagcag tgacgaatgt    1500

ggtacctttt gaggtctctc tcaccacctc cacagccacc gtcaccgtgg atgtgctgga    1560

tgtgaatgaa gcccccatct ttgtgcctcc tgaaaagaga gtggaagtgt ccgaggactt    1620

tggcgtgggc caggaaatca catcctacac tgcccaggag ccagacacat ttatggaaca    1680

gaaaataaca tatcggattt ggagagacac tgccaactgg ctggagatta atccggacac    1740

tggtgccatt tccactcggg ctgagctgga cagggaggat tttgagcacg tgaagaacag 1800

cacgtacaca gccctaatca tagctacaga caatggttct ccagttgcta ctggaacagg 1860

gacacttctg ctgatcctgt ctgatgtgaa tgacaacgcc cccataccag aacctcgaac 1920

tatattcttc tgtgagagga atccaaagcc tcaggtcata aacatcattg atgcagacct 1980

tcctcccaat acatctccct tcacagcaga actaacacac ggggcgagtg ccaactggac 2040

cattcagtac aacgacccaa cccaagaatc tatcattttg aagccaaaga tggccttaga 2100

ggtgggtgac tacaaaatca atctcaagct catggataac cagaataaag accaagtgac 2160

caccttagag gtcagcgtgt gtgactgtga aggggccgcc ggcgtctgta ggaaggcaca 2220

gcctgtcgaa gcaggattgc aaattcctgc cattctgggg attcttggag gaattcttgc 2280

tttgctaatt ctgattctgc tgctcttgct gtttcttcgg aggagagcgg tggtcaaaga 2340

gcccttactg cccccagagg atgacacccg ggacaacgtt tattactatg atgaagaagg 2400

aggcggagaa gaggaccagg actttgactt gagccagctg cacaggggcc tggacgctcg 2460

gcctgaagtg actcgtaacg acgttgcacc aaccctcatg agtgtccccc ggtatcttcc 2520

ccgccctgcc aatcccgatg aaattggaaa ttttattgat gaaaatctga aagcggctga 2580

tactgacccc acagccccgc cttatgattc tctgctcgtg tttgactatg aaggaagcgg 2640

ttccgaagct gctagtctga gctccctgaa ctcctcagag tcagacaaag accaggacta 2700

tgactacttg aacgaatggg gcaatcgctt caagaagctg gctgacatgt acggaggcgg 2760

cgaggacgac taggggactc gagagaggcg ggccccagac ccatgtgctg ggaaatgcag 2820

aaatcacgtt gctggtggtt tttcagctcc cttcccttga gatgagtttc tggggaaaaa 2880

aaagagactg gttagtgatg cagttagtat agctttatac tctctccact ttatagctct 2940

aataagtttg tgttagaaaa gtttcgactt atttcttaaa gctttttttt ttttcccatc 3000

actctttaca tggtggtgat gtccaaaaga tacccaaatt ttaatattcc agaagaacaa 3060

ctttagcatc agaaggttca cccagcacct tgcagatttt cttaaggaat tttgtctcac 3120

ttttaaaaag aaggggagaa gtcagctact ctagttctgt tgttttgtgt atataatttt 3180

ttaaaaaaaa tttgtgtgct tctgctcatt actacactgg tgtgtccctc tgcctttttt 3240

ttttttttta agacagggtc tcattctatc ggccaggctg gagtgcagtg gtgcaatcac 3300

agctcactgc agccttgtcc tcccaggctc aagctatcct tgcacctcag cctcccaagt 3360

agctgggacc acaggcatgc accactacgc atgactaatt ttttaaatat ttgagacggg 3420

gtctccctgt gttacccagg ctggtctcaa actcctgggc tcaagtgatc ctcccatctt 3480

ggcctcccag agtattggga ttacagacat gagccactgc acctgcccag ctccccaact 3540

ccctgccatt ttttaagaga cagtttcgct ccatcgccca ggcctgggat gcagtgatgt 3600

gatcatagct cactgtaacc tcaaactctg gggctcaagc agttctccca ccagcctcct 3660

ttttattttt ttgtacagat ggggtcttgc tatgttgccc aagctggtct taaactcctg 3720

gcctcaagca atccttctgc cttggccccc caaagtgctg ggattgtggg catgagctgc 3780

tgtgcccagc ctccatgttt taatatcaac tctcactcct gaattcagtt gctttgccca 3840

agataggagt tctctgatgc agaaattatt gggctctttt agggtaagaa gtttgtgtct 3900

ttgtctggcc acatcttgac taggtattgt ctactctgaa gacctttaat ggcttccctc 3960

tttcatctcc tgagtatgta acttgcaatg ggcagctatc cagtgacttg ttctgagtaa 4020

gtgtgttcat taatgtttat ttagctctga agcaagagtg atatactcca ggacttagaa 4080

tagtgcctaa agtgctgcag ccaaagacag agcggaacta tgaaaagtgg gcttggagat 4140

ggcaggagag cttgtcattg agcctggcaa tttagcaaac tgatgctgag gatgattgag 4200

gtgggtctac ctcatctctg aaaattctgg aaggaatgga ggagtctcaa catgtgtttc 4260

tgacacaaga tccgtggttt gtactcaaag cccagaatcc ccaagtgcct gcttttgatg 4320

atgtctacag aaaatgctgg ctgagctgaa cacatttgcc caattccagg tgtgcacaga 4380

aaaccgagaa tattcaaaat tccaaatttt ttcttaggag caagaagaaa atgtggccct 4440

aaagggggtt agttgagggg tagggggtag tgaggatctt gatttggatc tctttttatt 4500

taaatgtgaa tttcaacttt tgacaatcaa agaaaagact tttgttgaaa tagctttact 4560

gtttctcaag tgttttggag aaaaaaatca accctgcaat cactttttgg aattgtcttg 4620

atttttcggc agttcaagct atatcgaata tagttctgtg tagagaatgt cactgtagtt 4680

ttgagtgtat acatgtgtgg gtgctgataa ttgtgtattt tctttggggg tggaaaagga 4740

aaacaattca agctgagaaa agtattctca aagatgcatt tttataaatt ttattaaaca 4800

attttgttaa accataaaaa aaaaaaaa 4828

<210> 47
<211> 882
<212> PRT
<213> Homo sapiens

<400> 47

<210>  48
<211>  4413
<212>  DNA
<213>  Mus musculus

<400>  48
actggtgtgg gagccgcggc gcactactga gttcccaaga acttctgcta gactcctgcc      60

cggcctaacc cggccctgcc cgaccgcacc cgagctcagt gtttgctcgg cgtctgccgg     120

gtccgccatg ggagcccggt gccgcagctt ttccgcgctc ctgctcctgc tgcaggtctc     180

ctcatggctt tgccaggagc tggagcctga gtcctgcagt cccggcttca gttccgaggt     240

ctacaccttc ccggtgccgg agaggcacct ggagagaggc catgtcctgg gcagagtgag     300

atttgaagga tgcactggcc ggccaaggac agccttcttt tcggaagact cccgattcaa     360

agtggcgaca gacggcacca tcacagtgaa gcggcatcta aagctccaca agctggagac     420

cagtttcctc gtccgcgccc gggactccag tcatagggag ctgtctacca aagtgacgct     480

gaagtccatg gggcaccacc atcaccggca ccaccaccgc gaccctgcct ctgaatccaa     540

cccagagctg ctcatgtttc ccagcgtgta cccaggtctc agaagacaga aacgagactg     600

ggtcatccct cccatcagct gccccgaaaa tgaaaagggc gaatttccaa agaacctggt     660

tcagatcaaa tccaacaggg acaaagaaac aaaggttttc tacagcatca ccggccaagg     720

agctgacaaa ccccccgttg gcgttttcat cattgagagg gagacaggct ggctgaaagt 780

gacacagcct ctggatagag aagccattgc caagtacatc ctctattctc atgccgtgtc 840

atcaaatggg gaagcggtgg aggatcccat ggagatagtg atcacagtga cagatcagaa 900

tgacaacagg ccagagttta cccagccggt ctttgaggga ttcgttgcag aaggcgctgt 960

tccaggaacc tccgtgatga aggtctcagc caccgatgca gacgatgacg tcaacaccta 1020

caacgctgcc atcgcctaca ccatcgtcag ccaggatcct gagctgcctc acaaaaacat 1080

gttcactgtc aatagggaca ccggggtcat cagtgtgctc acctctgggc tggaccgaga 1140

gagttaccct acatacactc tggtggttca ggctgctgac cttcaaggtg aaggcttgag 1200

cacaacagcc aaggctgtga tcactgtcaa ggatattaat gacaacgctc ctgtcttcaa 1260

cccaagcacg tatcagggtc aagtgcctga gaatgaggtc aatgcccgga tcgccacact 1320

caaagtgacc gatgatgatg cccccaacac tccggcgtgg aaagctgtgt acaccgtagt 1380

caacgatcct gaccagcagt tcgttgttgt cacagacccc acgaccaatg atggcatttt 1440

gaaaacagcc aagggcttgg attttgaggc caagcagcaa tacatccttc atgtgagagt 1500

ggagaacgag gaaccctttg aggggtctct tgtcccttcc acagccactg tcactgtgga 1560

cgtggtagac gtgaatgaag cccccatctt tatgcctgcg gagaggagag tcgaagtgcc 1620

cgaagacttt ggtgtgggtc aggaaatcac atcttatacc gctcgagagc cggacacgtt 1680

catggatcag aagatcacgt atcggatttg gagggacact gccaactggc tggagattaa 1740

cccagagact ggtgccattt tcacgcgcgc tgagatggac agagaagacg ctgagcatgt 1800

gaagaacagc acatatgtag ctctcatcat cgccacagat gatggttcac ccattgccac 1860

tggcacgggc actcttctcc tggtcctgtt agacgtcaat gacaacgctc ccatcccaga 1920

acctcgaaac atgcagttct gccagaggaa cccacagcct catatcatca ccatcttgga 1980

tccagacctt ccccccaaca cgtccccctt tactgctgag ctaacccatg gggccagcgt 2040

caactggacc attgagtata atgacgcagc tcaagaatct ctcattttgc aaccaagaaa 2100

ggacttagag attggcgaat acaaaatcca tctcaagctc gcggataacc agaacaaaga 2160

ccaggtgacc acgttggacg tccatgtgtg tgactgtgaa gggacggtca acaactgcat 2220

gaaggcggga atcgtggcag caggattgca agttcctgcc atcctcggaa tccttggagg 2280

gatcctcgcc ctgctgattc tgatcctgct gctcctactg tttctacgga ggagaacggt 2340

ggtcaaagag cccctgctgc caccagatga tgatacccgg gacaatgtgt attactatga 2400

tgaagaagga ggtggagaag aagaccagga ctttgatttg agccagctgc acaggggcct 2460

ggatgcccga ccggaagtga ctcgaaatga tgtggctccc accctcatga gcgtgcccca 2520

gtatcgtccc cgtcctgcca atcctgatga aattggaaac ttcatcgatg aaaacctgaa 2580

ggcagccgac agcgacccca cggcaccccc ttacgactct ctgttggtgt tcgattacga 2640

gggcagtggt tctgaagccg ctagcctgag ctcactgaac tcctctgagt cggatcagga 2700

ccaggactac gattatctga acgagtgggg caaccgattc aagaagctgg cggacatgta 2760

cggcggtggt gaggacgact aggggactag caagtctccc ccgtgtggca ccatgggaga 2820

tgcagaataa ttatatcagt ggtctttcag ctccttccct gagtgtgtag aagagagact 2880

gatctgagaa gtgtgcagat tgcatagtgg tctcactctc cctactggac tgtctgtgtt 2940

aggatggttt tcactgattg ttgaaatctt tttttatttt ttatttttac agtgctgaga 3000

tataaactgt gccttttttt gtttgtttgt ttctgttttt gttcttttga gctatgatct 3060

gccccagaca caacagcccc aagcccctca cacctcacta attttttaca ttgtgtactt 3120

gccctcaatt accatgtttg ctgtattcta atagtcactc atgttcctga attctgttgc 3180

cctgcccagg tgatattcta ggatgcagaa atgcctgggc ccttttatgg tgagagacag 3240

gtatcttggt gtgggtgcaa ctgcgctgga tagtgtgtgt gttcccaaga tctttcgtgg 3300

tattccctct ccacctccag agaactcatt tacagtggca ttccttgttc ggctatgtgt 3360

ctggggcaga acaaaaaaaa gggaccacta tgcatgctgc acacgtctca gattcttagg 3420

tacacacctg attcttaggt gcatgccata gtgggatatg ttgctttgat cagaacctgc 3480

agggaggttt tcgggcacca cttaagtttc ttggcgtttc tttcaaacca aaactaaaga 3540

atggttgttc tctgagagag actggagtgc caccaccaaa gacagaggag agaaaaggag 3600

agaaaccaaa cttggggaca gcaacatcag cgaacccggc tagttggcac accgatggtg 3660

agggtacaca ggcggtgaga cctatcccac aagatttctg gaagactagg cttatctcaa 3720

ccaatgtttt ctggctggaa tctttgtcca tgtattcctg aagcccagga aatgcacccc 3780

tccaatgcct gctcttgatg gtagctacag aaaatgctgg ccgatttaaa cccaagttgc 3840

ccagttctga gtagaaaact gagactatgc tgtgtgtggc ggcgcgcacc tttaagccca 3900

gcactcagga ggcagaggca gtcagatctc cctgagttcg aggccaacct ggtatatata 3960

gtatagtaag cgaattctag gacagccagg gctacacaga gaaaccctgt ctctgcaaac 4020

caaaagagaa aactgagaat attacaaatt gtgcattttc tcaggaagca ggaagagaac 4080

attctaacgg gaaaaaggag acaagacctt tgagagtttt cattcaaaat gcaaatctca 4140

gctttttgat aaccactgga aagaatttta ttgaaagttc tgtacttacc taactttgga 4200

agaaaatgat gaccacaatc aactgtgaga actgttgatt tctctgtagt ttaatcatgt 4260

aatgttgcta gagtgacctt tgtatgtagt ttgagtgtat gtgtgtgggt gctgataatt 4320

ttgtattttg tggggggtgg aaaaggtaag ccattgaaac cgttctctaa gatgcatttt 4380

tatgaatttt attaaagagt tttgttaaac tgt                                    4413

<210>  49
<211>  884
<212>  PRT
<213>  Mus musculus

<400>  49

Met Gly Ala Arg Cys Arg Ser Phe Ser Ala Leu Leu Leu Leu Leu Gln
1               5                   10                  15

Val Ser Ser Trp Leu Cys Gln Glu Leu Glu Pro Glu Ser Cys Ser Pro
            20                  25                  30

Gly Phe Ser Ser Glu Val Tyr Thr Phe Pro Val Pro Glu Arg His Leu
        35                  40                  45

Glu Arg Gly His Val Leu Gly Arg Val Arg Phe Glu Gly Cys Thr Gly
    50                  55                  60

Arg Pro Arg Thr Ala Phe Phe Ser Glu Asp Ser Arg Phe Lys Val Ala
65                  70                  75                  80

Thr Asp Gly Thr Ile Thr Val Lys Arg His Leu Lys Leu His Lys Leu
                85                  90                  95

Glu Thr Ser Phe Leu Val Arg Ala Arg Asp Ser Ser His Arg Glu Leu
            100                 105                 110

Ser Thr Lys Val Thr Leu Lys Ser Met Gly His His His His Arg His
        115                 120                 125

His His Arg Asp Pro Ala Ser Glu Ser Asn Pro Glu Leu Leu Met Phe
    130                 135                 140

Pro Ser Val Tyr Pro Gly Leu Arg Arg Gln Lys Arg Asp Trp Val Ile
145                 150                 155                 160

Pro Pro Ile Ser Cys Pro Glu Asn Glu Lys Gly Glu Phe Pro Lys Asn
                165                 170                 175

Leu Val Gln Ile Lys Ser Asn Arg Asp Lys Glu Thr Lys Val Phe Tyr
            180                 185                 190

Ser Ile Thr Gly Gln Gly Ala Asp Lys Pro Pro Val Gly Val Phe Ile
        195                 200                 205

Ile Glu Arg Glu Thr Gly Trp Leu Lys Val Thr Gln Pro Leu Asp Arg
    210                 215                 220

Glu Ala Ile Ala Lys Tyr Ile Leu Tyr Ser His Ala Val Ser Ser Asn
225                 230                 235                 240

Gly Glu Ala Val Glu Asp Pro Met Glu Ile Val Ile Thr Val Thr Asp
                245                 250                 255

Gln Asn Asp Asn Arg Pro Glu Phe Thr Gln Pro Val Phe Glu Gly Phe
            260                 265                 270

Val Ala Glu Gly Ala Val Pro Gly Thr Ser Val Met Lys Val Ser Ala
        275                 280                 285

Thr Asp Ala Asp Asp Asp Val Asn Thr Tyr Asn Ala Ala Ile Ala Tyr
    290                 295                 300

Thr Ile Val Ser Gln Asp Pro Glu Leu Pro His Lys Asn Met Phe Thr
305                 310                 315                 320

Val Asn Arg Asp Thr Gly Val Ile Ser Val Leu Thr Ser Gly Leu Asp
                325                 330                 335

Arg Glu Ser Tyr Pro Thr Tyr Thr Leu Val Val Gln Ala Ala Asp Leu
            340                 345                 350

Gln Gly Glu Gly Leu Ser Thr Thr Ala Lys Ala Val Ile Thr Val Lys
        355                 360                 365

Asp Ile Asn Asp Asn Ala Pro Val Phe Asn Pro Ser Thr Tyr Gln Gly
    370                 375                 380

Gln Val Pro Glu Asn Glu Val Asn Ala Arg Ile Ala Thr Leu Lys Val
385                 390                 395                 400

Thr Asp Asp Asp Ala Pro Asn Thr Pro Ala Trp Lys Ala Val Tyr Thr
                405                 410                 415

Val Val Asn Asp Pro Asp Gln Gln Phe Val Val Val Thr Asp Pro Thr
            420                 425                 430

Thr Asn Asp Gly Ile Leu Lys Thr Ala Lys Gly Leu Asp Phe Glu Ala
        435                 440                 445

Lys Gln Gln Tyr Ile Leu His Val Arg Val Glu Asn Glu Glu Pro Phe
    450                 455                 460

Glu Gly Ser Leu Val Pro Ser Thr Ala Thr Val Thr Val Asp Val Val
465                 470                 475                 480

Asp Val Asn Glu Ala Pro Ile Phe Met Pro Ala Glu Arg Arg Val Glu
                485                 490                 495

Val Pro Glu Asp Phe Gly Val Gly Gln Glu Ile Thr Ser Tyr Thr Ala
            500                 505                 510

Arg Glu Pro Asp Thr Phe Met Asp Gln Lys Ile Thr Tyr Arg Ile Trp
        515                 520                 525

Arg Asp Thr Ala Asn Trp Leu Glu Ile Asn Pro Glu Thr Gly Ala Ile
    530                 535                 540

Phe Thr Arg Ala Glu Met Asp Arg Glu Asp Ala Glu His Val Lys Asn
545                 550                 555                 560

Ser Thr Tyr Val Ala Leu Ile Ile Ala Thr Asp Asp Gly Ser Pro Ile
                565                 570                 575

Ala Thr Gly Thr Gly Thr Leu Leu Leu Val Leu Leu Asp Val Asn Asp
            580                 585                 590

Asn Ala Pro Ile Pro Glu Pro Arg Asn Met Gln Phe Cys Gln Arg Asn
        595                 600                 605

Pro Gln Pro His Ile Ile Thr Ile Leu Asp Pro Asp Leu Pro Pro Asn
    610                 615                 620

Thr Ser Pro Phe Thr Ala Glu Leu Thr His Gly Ala Ser Val Asn Trp
625                 630                 635                 640

Thr Ile Glu Tyr Asn Asp Ala Ala Gln Glu Ser Leu Ile Leu Gln Pro
                645                 650                 655

Arg Lys Asp Leu Glu Ile Gly Glu Tyr Lys Ile His Leu Lys Leu Ala
            660                 665                 670

Asp Asn Gln Asn Lys Asp Gln Val Thr Thr Leu Asp Val His Val Cys
        675                 680                 685

Asp Cys Glu Gly Thr Val Asn Asn Cys Met Lys Ala Gly Ile Val Ala
    690                 695                 700

Ala Gly Leu Gln Val Pro Ala Ile Leu Gly Ile Leu Gly Gly Ile Leu
705                 710                 715                 720

Ala Leu Leu Ile Leu Ile Leu Leu Leu Leu Leu Phe Leu Arg Arg Arg
                725                 730                 735

Thr Val Val Lys Glu Pro Leu Leu Pro Pro Asp Asp Asp Thr Arg Asp
            740                 745                 750

Asn Val Tyr Tyr Tyr Asp Glu Glu Gly Gly Gly Glu Glu Asp Gln Asp
        755                 760                 765

Phe Asp Leu Ser Gln Leu His Arg Gly Leu Asp Ala Arg Pro Glu Val
    770                 775                 780

Thr Arg Asn Asp Val Ala Pro Thr Leu Met Ser Val Pro Gln Tyr Arg
785                 790                 795                 800

Pro Arg Pro Ala Asn Pro Asp Glu Ile Gly Asn Phe Ile Asp Glu Asn
                805                 810                 815

Leu Lys Ala Ala Asp Ser Asp Pro Thr Ala Pro Pro Tyr Asp Ser Leu
            820                 825                 830

Leu Val Phe Asp Tyr Glu Gly Ser Gly Ser Glu Ala Ala Ser Leu Ser
        835                 840                 845

Ser Leu Asn Ser Ser Glu Ser Asp Gln Asp Gln Asp Tyr Asp Tyr Leu
    850                 855                 860

Asn Glu Trp Gly Asn Arg Phe Lys Lys Leu Ala Asp Met Tyr Gly Gly
865                 870                 875                 880

Gly Glu Asp Asp

<210>  50
<211>  4396
<212>  DNA
<213>  Rattus norvegicus

<400>  50
ctggtgtggg agccgcggcg cactactgag ttctcaagaa cttctgctag acttcagccc      60

ggcctaaccc ggctctgccc gaccgcaccc gagctcagtg tttgctcggc gtttgcccgg     120

ccagccatgg gagcccggtg ccgcagcttc tccgcgctcc tgctcctgct gcaggtctcc 180

tcgtggcttt gtcagcagcc ggagtcggag tctgactcct gccgtcccgg cttcagttcc 240

gaggtctaca ccttcctggt gccggagagg cacctggaga gaggccacat cctgggcaga 300

gtgaaatttg aaggatgcac cggccgtcca aggacagcct tcttttctga agactcccga 360

ttcaaagtgt ctacagatgg cgtcatcaca gtcaaacggc atctaaagct tcacaagctg 420

gagaccagtt ttctcgtcca tgcctgggac tccagttaca ggaagctttc taccaaagtg 480

acactgaagt ccctgggcca ccaccaccac cggcatcacc acagagaccc tgtctctgaa 540

tccaacccag agctgctcac gtttcccagc tttcaccagg gtctgagaag acagaaacga 600

gactgggtca tccctcccat caactgcccg gaaaatcaaa agggcgaatt cccccagcga 660

ctggttcaga tcaaatccaa cagggacaaa gagacaacgg ttttctacag catcaccggc 720

ccaggagctg acaaaccccc tgttggcgtt ttcatcattg agagggagac aggctggctg 780

aaagtgacgc agcctctgga cagagaagcc attgacaagt accttctcta ctctcatgct 840

gtgtcatcaa atggggaagc cgtggaggat cccatggaga tagtggtcac agtcacagat 900

cagaatgaca acaggccaga gtttatccag gaggtctttg agggatctgt tgcagaaggc 960

gctcttccag gaacctccgt gatgcaggtc tcagccactg atgcagacga tgacataaac 1020

acctacaatg ctgccatcgc ctacaccatc ctcagccaag atcctgagct gcctcacaaa 1080

aacatgttca ctgtcaaccg ggacactggg gtcatcagtg tggtcacctc cggactggac 1140

cgagagagtt accctacata tactctggtg gttcaggctg ccgaccttca aggcgaaggc 1200

ctaagcacaa cagcaaaagc tgtgatcact gtcaaggata ttaatgacaa cgctcccatc 1260

ttcaacccaa gcacgtacca gggtcaagtg cttgagaatg aggtcggtgc ccgtattgcc 1320

acactcaagg tgactgatga tgatgccccc aacactccag cgtggaatgc tgtgtacacc 1380

gtagtcaatg atcctgatca tcagttcact gtcatcacag accccaagac caacgagggc 1440

attctgaaaa cagccaaggg cttggatttt gaggccaagc agcagtacat tctgcacgtg 1500

acagtggaaa atgaggagcc ctttgagggg tctcttgtcc cttccacagc cactgtcacc 1560

gtggatgtgg tagacgtgaa tgaagccccc atttttgtgc ctgcggagaa gagagtcgag 1620

gtgcctgagg actttggtgt gggtctggag atcgcatctt acactgcgcg agagccagac 1680

acattcatgg aacagaagat cacgtatcgg atttggaggg acactgccaa ttggctggag 1740

attaacccag agactggggt catttccact cgggctgaga tggacagaga agattcggag 1800

catgtgaaga acagcacgta tacagctctc atcattgcca cagatgatgg ttcacccatt 1860

gccactggca caggactctt tctcctggtc ctgtcagacg tcaacgacaa tgctcccatc 1920

ccagaacctc gaaatatgca gttctgccag agaaacccga agccccatgt catcaccatc 1980

ttggatccag accttccccc aaacacatcc cccttcactg cagagctcac ccatggggcc 2040

agcgtcaact ggaccattga gtacaatgac gcagaacaag aatctctcat tttgcaacca 2100

agaaaggact tagagattgg cgaatacaaa atcaatctca agctctcgga taaccagaat 2160

aaagaccagg tgaccacgtt ggaggtccac gtgtgtgact gtgaagggac cgtcaacaac 2220

tgcatgaagg cgatctccct ggaagcagga ttacaagttc ccgccatcct tggaatcctg 2280

ggagggatcc tggccctcct gattctgatc ctcctgctcc tactgtttct acggaggaga 2340

acggtggtca aagagccctt gctgccacca gatgacgata cccgggacaa tgtgtattac 2400

tatgatgaag agggaggtgg agaagaagac caggactttg atttgagcca gctgcacagg 2460

ggccttgatg ccagaccgga agtgattcga aatgatgtgg ctcccaccct catgagcatg 2520

ccccagtatc gtccccgtcc agccaatcct gatgaaatcg ggaacttcat cgatgaaaac 2580

ctgaaggcag cggacagtga ccccacagcg cccccttacg actctctgtt ggtgtttgac 2640

tatgagggga gtggttctga agctgcctcc ctgagctcgc tgaactcctc tgagtcagat 2700

caggaccagg actacgatta tctgaacgaa tggggcaacc ggttcaagaa gctggccgat 2760

atgtatggtg gcggcgaaga agactagaga gtcgttcctg tgtggcacca tgggagatgc 2820

agaatcatga tgtcagtggt ctttcagctc cttccctgac tttgtagaag agagactgat 2880

ctgagaagta tgcagattgc atactggtcc cactctacct accagtctgt ctgtgttagg 2940

agggttttca ctggttgttg gaatcttttt ctaaaatgtt tttgttttta cagtgctgtg 3000

atgtgatgaa ctgtaccctc tttttgtttt tgttttgagc tatgttctgc tccggacaca 3060

cagcccccaa gcccttcacc cctcactaat ttttttacat tgtatacttt cactcaatta 3120

ccatgtttat gttctgtatt ctaatagcca ctaagttcct gaattctgtt gcctggccca 3180

ggtgctattc tgtgacacag tagtgcctgg gcccttttat ggtaagagac aggtttcttg 3240

gtgtgggtgc aactgagctg gatagtgtat gtttcaaaca cctttcctgt gttctctccc 3300

cacctccaga gtgtctttac ttattcagct gtgtgtttgg ggcagaacaa aaaaataatg 3360

ggaccactat gcaagctgcg aagattctaa ggtgcacacc tgattcttag gcagatgcca 3420

tagtgagata tgttgctttg gttctctatc caatgctgtg accgggacct gcagcgaggt 3480

tttcggacac cgtggtttct tgcgtttctt tcaaaccagc agtaaaaaat ggttttttct 3540

gagagagact ggagtgccac caccaaagat agaggagaga agccaagctt ggggacagca 3600

agcatgccag tgaacctgac cactgtcatg agtcatgtgg gtggccacat gtccgtgaac 3660

ctggccagtt ggcacactga tggtgagggt acaaggaggc tagacctcgt cccacaaaat 3720

ttctggaaga attagggttg tctcagccaa tgtttcctag ctggaatcct gtccatgtat 3780

gtgttcctga agcccaggaa atacacccct ctagtgcctg cttttgatgg tagttataga 3840

aaagaccggc tgatttggac ctgagttgcc caatcttaag tacaaataga aaactgagac 3900

tatgctgggt gtgttggtgc acgcctttaa tcccggcact cgggaggcag agacagtctc 3960

agatctctct gagttcaagg tcagcctggt atagtaagtg aattccagga cagccagggc 4020

tacacagaaa ctctgtcttg gaaaaccaaa aaagaaaact gagaatatta gagattgtgc 4080

attttctcag aaagcaggaa gaaaacacca ctctgatggg aaaagggagg caaggccctt 4140

gagacttttc attgaaattg ctgtactcac ataattttgg aagcaaatga tgactgcaat 4200

caactgtgag aactgttggt ttctctgtag tttaattgtc taatgttgat agcgtgccct 4260

ttgtatgtag tttgagtgta tatgtgtgtg ggtgctgata attttgtatt ttgtggggag 4320

tggaaaaggc aagcaatcgg aactgttctc taagatgcat ttttatgaat tttattaaag 4380

agttttgtta aactgt 4396

<210> 51
<211> 886
<212> PRT
<213> Rattus norvegicus

<400> 51

Met Gly Ala Arg Cys Arg Ser Phe Ser Ala Leu Leu Leu Leu Leu Gln
1               5                   10                  15

Val Ser Ser Trp Leu Cys Gln Gln Pro Glu Ser Glu Ser Asp Ser Cys
            20                  25                  30

Arg Pro Gly Phe Ser Ser Glu Val Tyr Thr Phe Leu Val Pro Glu Arg
        35                  40                  45

His Leu Glu Arg Gly His Ile Leu Gly Arg Val Lys Phe Glu Gly Cys
    50                  55                  60

Thr Gly Arg Pro Arg Thr Ala Phe Phe Ser Glu Asp Ser Arg Phe Lys
65                  70                  75                  80

Val Ser Thr Asp Gly Val Ile Thr Val Lys Arg His Leu Lys Leu His
                85                  90                  95

Lys Leu Glu Thr Ser Phe Leu Val His Ala Trp Asp Ser Ser Tyr Arg
            100                 105                 110

Lys Leu Ser Thr Lys Val Thr Leu Lys Ser Leu Gly His His His His
        115                 120                 125

Arg His His His Arg Asp Pro Val Ser Glu Ser Asn Pro Glu Leu Leu
    130                 135                 140

Thr Phe Pro Ser Phe His Gln Gly Leu Arg Arg Gln Lys Arg Asp Trp
145                 150                 155                 160

Val Ile Pro Pro Ile Asn Cys Pro Glu Asn Gln Lys Gly Glu Phe Pro
                165                 170                 175

Gln Arg Leu Val Gln Ile Lys Ser Asn Arg Asp Lys Glu Thr Thr Val
            180                 185                 190

Phe Tyr Ser Ile Thr Gly Pro Gly Ala Asp Lys Pro Pro Val Gly Val
        195                 200                 205

Phe Ile Ile Glu Arg Glu Thr Gly Trp Leu Lys Val Thr Gln Pro Leu
    210                 215                 220

Asp Arg Glu Ala Ile Asp Lys Tyr Leu Leu Tyr Ser His Ala Val Ser
225                 230                 235                 240

Ser Asn Gly Glu Ala Val Glu Asp Pro Met Glu Ile Val Val Thr Val
                245                 250                 255

Thr Asp Gln Asn Asp Asn Arg Pro Glu Phe Ile Gln Glu Val Phe Glu
            260                 265                 270

Gly Ser Val Ala Glu Gly Ala Leu Pro Gly Thr Ser Val Met Gln Val
        275                 280                 285

Ser Ala Thr Asp Ala Asp Asp Asp Ile Asn Thr Tyr Asn Ala Ala Ile
    290                 295                 300

Ala Tyr Thr Ile Leu Ser Gln Asp Pro Glu Leu Pro His Lys Asn Met
305                 310                 315                 320

Phe Thr Val Asn Arg Asp Thr Gly Val Ile Ser Val Val Thr Ser Gly
                325                 330                 335

Leu Asp Arg Glu Ser Tyr Pro Thr Tyr Thr Leu Val Val Gln Ala Ala
            340                 345                 350

Asp Leu Gln Gly Glu Gly Leu Ser Thr Thr Ala Lys Ala Val Ile Thr
        355                 360                 365

Val Lys Asp Ile Asn Asp Asn Ala Pro Ile Phe Asn Pro Ser Thr Tyr
    370                 375                 380

Gln Gly Gln Val Leu Glu Asn Glu Val Gly Ala Arg Ile Ala Thr Leu
385                 390                 395                 400

Lys Val Thr Asp Asp Asp Ala Pro Asn Thr Pro Ala Trp Asn Ala Val
                405                 410                 415

Tyr Thr Val Val Asn Asp Pro Asp His Gln Phe Thr Val Ile Thr Asp
            420                 425                 430

Pro Lys Thr Asn Glu Gly Ile Leu Lys Thr Ala Lys Gly Leu Asp Phe
        435                 440                 445

Glu Ala Lys Gln Gln Tyr Ile Leu His Val Thr Val Glu Asn Glu Glu
    450                 455                 460

Pro Phe Glu Gly Ser Leu Val Pro Ser Thr Ala Thr Val Thr Val Asp
465                 470                 475                 480

Val Val Asp Val Asn Glu Ala Pro Ile Phe Val Pro Ala Glu Lys Arg
                485                 490                 495

Val Glu Val Pro Glu Asp Phe Gly Val Gly Leu Glu Ile Ala Ser Tyr
            500                 505                 510

Thr Ala Arg Glu Pro Asp Thr Phe Met Glu Gln Lys Ile Thr Tyr Arg
        515                 520                 525

Ile Trp Arg Asp Thr Ala Asn Trp Leu Glu Ile Asn Pro Glu Thr Gly
    530                 535                 540

Val Ile Ser Thr Arg Ala Glu Met Asp Arg Glu Asp Ser Glu His Val
545                 550                 555                 560

Lys Asn Ser Thr Tyr Thr Ala Leu Ile Ile Ala Thr Asp Asp Gly Ser
                565                 570                 575

Pro Ile Ala Thr Gly Thr Gly Thr Leu Leu Leu Val Leu Ser Asp Val
            580                 585                 590

Asn Asp Asn Ala Pro Ile Pro Glu Pro Arg Asn Met Gln Phe Cys Gln
        595                 600                 605

Arg Asn Pro Lys Pro His Val Ile Thr Ile Leu Asp Pro Asp Leu Pro
    610                 615                 620

Pro Asn Thr Ser Pro Phe Thr Ala Glu Leu Thr His Gly Ala Ser Val
625                 630                 635                 640

Asn Trp Thr Ile Glu Tyr Asn Asp Ala Glu Gln Glu Ser Leu Ile Leu
                645                 650                 655

Gln Pro Arg Lys Asp Leu Glu Ile Gly Glu Tyr Lys Ile Asn Leu Lys
            660                 665                 670

Leu Ser Asp Asn Gln Asn Lys Asp Gln Val Thr Thr Leu Glu Val His
        675                 680                 685

Val Cys Asp Cys Glu Gly Thr Val Asn Asn Cys Met Lys Ala Ile Ser
    690                 695                 700

Leu Glu Ala Gly Leu Gln Val Pro Ala Ile Leu Gly Ile Leu Gly Gly
705                 710                 715                 720

Ile Leu Ala Leu Leu Ile Leu Ile Leu Leu Leu Leu Leu Phe Leu Arg
                725                 730                 735

Arg Arg Thr Val Val Lys Glu Pro Leu Leu Pro Pro Asp Asp Asp Thr
            740                 745                 750

Arg Asp Asn Val Tyr Tyr Tyr Asp Glu Glu Gly Gly Gly Glu Glu Asp
        755                 760                 765

Gln Asp Phe Asp Leu Ser Gln Leu His Arg Gly Leu Asp Ala Arg Pro
    770                 775                 780

Glu Val Ile Arg Asn Asp Val Ala Pro Thr Leu Met Ser Met Pro Gln
785                 790                 795                 800

Tyr Arg Pro Arg Pro Ala Asn Pro Asp Glu Ile Gly Asn Phe Ile Asp
                805                 810                 815

Glu Asn Leu Lys Ala Ala Asp Ser Asp Pro Thr Ala Pro Pro Tyr Asp
            820                 825                 830

Ser Leu Leu Val Phe Asp Tyr Glu Gly Ser Gly Ser Glu Ala Ala Ser
        835                 840                 845

Leu Ser Ser Leu Asn Ser Ser Glu Ser Asp Gln Asp Gln Asp Tyr Asp

850                 855                 860

Tyr Leu Asn Glu Trp Gly Asn Arg Phe Lys Lys Leu Ala Asp Met Tyr
865                 870                 875                 880

Gly Gly Gly Glu Glu Asp
                885

<210>  52
<211>  4877
<212>  DNA
<213>  Pan troglodytes

<400>  52
agccaatcag cggtacgggg ggcggtgcct ccggggctca cctggcggca gccgcgcacc      60

ctctctcagt ggcgtcggaa ctgcaaagca cctgtgagct tgcggaagtc agttcagact     120

ccagcccgct ccagcccggc ccgacccgac cgcacccggc gcctgccctc tctcggcgtc     180

cccggccagc catgggccct tggagccgca gcctctcagc gctgctgctg ctgctgcagg     240

tctcctcttg gctctgccag gagccggagc cctgccaccc tggctttgac gccgagagct     300

acacgttcac ggtgccccgg ctccacctgg agagaggccg cgtcctgggc agagtgaatt     360

ttgaagattg caccggtcga caaaggacag cctatttttc cctcgacacc cgattcaaag     420

tgggcacaga tggtgtgatt acagtcaaaa ggcctctacg gtttcataac ccacagatcc     480

atttcttggt ctacgcctgg gactccacct acagaaagtt ttccaccaaa gtcacgctga     540

atacagtggg gcaccaccac cgccccccgc cccatcaggc ctctgtttct ggaatccaag     600

cagaattgct cacatttccc aactcctctc ctggcctcag aagacagaag agagactggg     660

ttattcctcc catcagctgc ccagaaaatg aaaaaggccc atttcctaaa aacctggttc     720

agatcaaatc caacaaagac aaagaaggca aggttttcta cagcatcact ggccaaggag     780

ctgacacacc ccctgttggt gtctttatta ttgaaagaga aacaggatgg ctgaaggtga     840

cagagcctct ggatagagaa cgcattgcca catacactct cttctctcat gctgtgtcat     900

ccaacgggaa tgccgttgag gatccaatgg agattttgat cacggtgacc gatcagaatg     960

acaacaaacc cgaattcacc caggaggtct ttaaggggtc tgtcatggaa ggtgctcttc    1020

caggaacctc tgtgatggag gtcacagcca cagacgcgga cgatgatgtg aacacctaca    1080

atgccgccat cgcttacacc atcctcagcc aagatcctga gctgcctgac aaaaatatgt    1140

tcaccattaa caggaacaca ggagtcatca gtgtggtcac cactgggctg gaccgagaga    1200

gtttccctac gtataccctg gtggttcaag ctgctgatct tcaaggtgag gggttaagca    1260

caacagcaac agctgtgatc acagtcactg acaccaacga taaccctccg atcttcaatc    1320

ccaccacgta caagggtcag gtgcctgaga acgaggctaa cgtcataatc accacactga 1380

aagtgactga tgctgatgcc cccaataccc cagcgtggga ggctgtatac accatattga 1440

atgatgatgg tggacaattt gtcgtcacca caaatccagt gaacaacgat ggcattttga 1500

aaacagcaaa gggcttggat tttgaggcca agcagcagta cattctacac gtagcagtga 1560

cgaatgtggt accttttgag gtctctctca ccacctccac agccaccgtc accgtggatg 1620

tgctggatgt gaatgaagcc cccatctttg tgcctcctga aaagagagtg gaagtgtcgg 1680

aggactttgg cgtgggccag gaaatcacat cctacactgc ccaggagcca gacacattta 1740

tggaacagaa aataacatat cggatttgga gagacactgc caactggctg gagattaatc 1800

cggacactgg tgccatttcc actcgggctg agctggacag ggaggatttg gagcacgtga 1860

agaacagcac gtacacagcc ctaatcatag ctacagacaa tggttctcca gttgctactg 1920

gaacagggac acttctgctg atcctgtctg atgtgaatga caatgccccc ataccagaac 1980

ctcgaactat attcttctgt gagaggaatc caaagcctca ggtcataaac atcattgatg 2040

cagaccttcc tcccaataca tctcccttca cagcagaact aacacacggg gcgagtgcca 2100

actggaccat tcagtacaac gacccaaccc aagaatctat cattttgaag ccaaagatgg 2160

ccttagaggt gggtgactac aaaatcaatc tcaagctcat ggataaccag aataaagacc 2220

aagtgaccac cttagaggtc agcgtgtgtg actgtgaagg ggccgccggc gtctgtagga 2280

aggcacagcc tgtcgaagca ggattgcaaa ttcctgccat tctggggatt cttggaggaa 2340

ttcttgcttt gctaattctg attctgctgc tcttgctgtt tcttcggagg agagcggtgg 2400

tcaaagagcc cttactgccc ccagaggatg acacccggga caacgtttat tactatgatg 2460

aagaaggagg cggagaagag gaccaggact ttgacttgag ccagttgcac aggggcctgg 2520

acgctcggcc tgaagtgact cgtaacgacg ttgcaccaac cctcatgagt gtcccccggt 2580

accttccccg ccctgccaat cccgatgaaa ttggaaattt tattgatgaa aatctgaaag 2640

cggctgatac tgaccccaca gccccgcctt atgattctct tctcgtgttt gactatgaag 2700

gaagcggttc cgaagctgct agtctgagct ccctgaactc ctcagagtca gacaaagacc 2760

aggactatga ctacttgaac gaatggggca atcgcttcaa gaagctggcg gacatgtacg 2820

gaggtggcga ggacgactag gggactcgag agaggcgggc cctagacccg tgtgctggga 2880

aatgcagaaa tcatgttgct ggtggttttt cagctccctt cccttgagat gagtttctgg 2940

ggaaaagaag agactggttg gtgatgcagt tagtatagct ttatgctctc tccactttat 3000

agctctaata agtgtgtgtt agaaaagttt tgacttattt cttgaagctt tttttttttt 3060

cccatcactc tacatggtgg tgatgtccaa aagataccca aattttaata ttccagaaga 3120

acaactttag catcagaagg ttcacccagc accttgcaga ttttcttaag gaattttgtc 3180

tcacttttaa aaagaagggg agaagtcagc tactctagtt ctgttgtttt gtgtatataa 3240

ttttttaaaa aaaatttgtg tgctcctgct cattgctaca ctggtgtgtc cctctgcctt 3300

tttttttttt aagacagggt ctcattctgt cggccaggct ggagtgcagt ggtgcaatca 3360

cagctcactg cagccttgtc ctcccaggct caagctatcc ttgcacctca gcctcccaag 3420

tagctgggac cacaggcatg caccactacg catgactaat tttttaaata tttgagacgg 3480

ggtctccctg tgttacccag gctggtctca aactcctggg ctcaagtgat cctcccatct 3540

tggcctccca gagtattggg attacagaca tgagccactg cacctgccca gctccccaac 3600

tccctgccat tttttaagag acagtttcgc tccattgccc aggcctggga tgcagtgatg 3660

tgatcatagc tcactgtaac ctcaaactct ggggctcaag caattctccc accagcctcc 3720

tttttatttt tttgtacaga tggggtcttg ctatgttgcc catgctggtc ttaaactcct 3780

ggcctcaagc aatcctcctg ccttggcccc ccaaagtgct gggattgcgg gcatgagctg 3840

ctgtgcccag cctccatgtt ttaatatcaa ctctccctcc tcaattcagt tgctttgccc 3900

aagataggag ttctccgatg cagaaattat tgggctcttt tagggtaaga agctcgtgtc 3960

ttcgtctggc cacatcttga ctaggtattg tctactctga agacctttaa tggcttccct 4020

ctttcatctc ctgagtatgt aacttgcaat gggcatctat ccagtgactt gttctgagta 4080

agtgtgttcg ttaatgttta tttagctctg aagcaagagt gatatactcc aggacttaga 4140

atagtgccta aagtgctgca gccaaagaca gagcggaact atgaaaattg ggcttggaga 4200

tggcaggaga gcttgtcatt gagcctggca atttagcaaa ctgatgctga ggatgattga 4260

ggtgggtcta cctcatctct gaaaattctg gaaggaatgg aggagtctca acatgtgttt 4320

ctgacacagg atctgtggtt tgtactcaaa ggccagaatc cccaagtgcc tgcttttgat 4380

gatgtctaca gaaaatgctg gctgagctga acacatttgc ccaattccag atgtgcatag 4440

aaaactgaga atattcaaaa ttccaaattt ttttcttagg agcaagaaga aaatgtggcc 4500

ctaaaggggg ttagttgagg ggtagggggt agtgaggatc ttgatttgga tctcttttta 4560

tttaaatgtg aatttcaact tttgacaatc aaagaaaaga gttttattga aatagcttta 4620

ctgtttctca agtgttttgg agaaaaaaat caaccctgca atcacttttt ggaattgtct 4680

tgatttttcg gcagttcaag ctatatcgaa tatagttctg tgtagagaat gtcactgtag 4740

ttttgagtgt atacatgtgt gggtgctgat aattgtgtat tttctctggg ggtggaaaag 4800

gaaaacgatt caagctgaga aaagtattct caaagatgca tttttataaa ttttattaaa 4860

gaattttgtt aaaccat 4877

<210> 53

<211>  882
<212>  PRT
<213>  Pan troglodytes

<400>  53

Met Gly Pro Trp Ser Arg Ser Leu Ser Ala Leu Leu Leu Leu Leu Gln
1               5                   10                  15

Val Ser Ser Trp Leu Cys Gln Glu Pro Glu Pro Cys His Pro Gly Phe
            20                  25                  30

Asp Ala Glu Ser Tyr Thr Phe Thr Val Pro Arg Leu His Leu Glu Arg
        35                  40                  45

Gly Arg Val Leu Gly Arg Val Asn Phe Glu Asp Cys Thr Gly Arg Gln
    50                  55                  60

Arg Thr Ala Tyr Phe Ser Leu Asp Thr Arg Phe Lys Val Gly Thr Asp
65                  70                  75                  80

Gly Val Ile Thr Val Lys Arg Pro Leu Arg Phe His Asn Pro Gln Ile
                85                  90                  95

His Phe Leu Val Tyr Ala Trp Asp Ser Thr Tyr Arg Lys Phe Ser Thr
            100                 105                 110

Lys Val Thr Leu Asn Thr Val Gly His His His Arg Pro Pro Pro His
        115                 120                 125

Gln Ala Ser Val Ser Gly Ile Gln Ala Glu Leu Leu Thr Phe Pro Asn
    130                 135                 140

Ser Ser Pro Gly Leu Arg Arg Gln Lys Arg Asp Trp Val Ile Pro Pro
145                 150                 155                 160

Ile Ser Cys Pro Glu Asn Glu Lys Gly Pro Phe Pro Lys Asn Leu Val
                165                 170                 175

Gln Ile Lys Ser Asn Lys Asp Lys Glu Gly Lys Val Phe Tyr Ser Ile
            180                 185                 190

Thr Gly Gln Gly Ala Asp Thr Pro Pro Val Gly Val Phe Ile Ile Glu
        195                 200                 205

Arg Glu Thr Gly Trp Leu Lys Val Thr Glu Pro Leu Asp Arg Glu Arg
    210                 215                 220

Ile Ala Thr Tyr Thr Leu Phe Ser His Ala Val Ser Ser Asn Gly Asn
225                 230                 235                 240

Ala Val Glu Asp Pro Met Glu Ile Leu Ile Thr Val Thr Asp Gln Asn
                245                 250                 255

Asp Asn Lys Pro Glu Phe Thr Gln Glu Val Phe Lys Gly Ser Val Met
            260                 265                 270

Glu Gly Ala Leu Pro Gly Thr Ser Val Met Glu Val Thr Ala Thr Asp
        275                 280                 285

Ala Asp Asp Asp Val Asn Thr Tyr Asn Ala Ala Ile Ala Tyr Thr Ile
    290                 295                 300

Leu Ser Gln Asp Pro Glu Leu Pro Asp Lys Asn Met Phe Thr Ile Asn
305                 310                 315                 320

Arg Asn Thr Gly Val Ile Ser Val Val Thr Thr Gly Leu Asp Arg Glu
                325                 330                 335

Ser Phe Pro Thr Tyr Thr Leu Val Val Gln Ala Ala Asp Leu Gln Gly
            340                 345                 350

Glu Gly Leu Ser Thr Thr Ala Thr Ala Val Ile Thr Val Thr Asp Thr
        355                 360                 365

Asn Asp Asn Pro Pro Ile Phe Asn Pro Thr Thr Tyr Lys Gly Gln Val
    370                 375                 380

Pro Glu Asn Glu Ala Asn Val Ile Ile Thr Thr Leu Lys Val Thr Asp
385                 390                 395                 400

Ala Asp Ala Pro Asn Thr Pro Ala Trp Glu Ala Val Tyr Thr Ile Leu
                405                 410                 415

Asn Asp Asp Gly Gly Gln Phe Val Val Thr Thr Asn Pro Val Asn Asn
            420                 425                 430

Asp Gly Ile Leu Lys Thr Ala Lys Gly Leu Asp Phe Glu Ala Lys Gln
        435                 440                 445

Gln Tyr Ile Leu His Val Ala Val Thr Asn Val Val Pro Phe Glu Val
    450                 455                 460

Ser Leu Thr Thr Ser Thr Ala Thr Val Thr Val Asp Val Leu Asp Val
465                 470                 475                 480

Asn Glu Ala Pro Ile Phe Val Pro Pro Glu Lys Arg Val Glu Val Ser
                485                 490                 495

Glu Asp Phe Gly Val Gly Gln Glu Ile Thr Ser Tyr Thr Ala Gln Glu
            500                 505                 510

Pro Asp Thr Phe Met Glu Gln Lys Ile Thr Tyr Arg Ile Trp Arg Asp
        515                 520                 525

Thr Ala Asn Trp Leu Glu Ile Asn Pro Asp Thr Gly Ala Ile Ser Thr
    530                 535                 540

Arg Ala Glu Leu Asp Arg Glu Asp Leu Glu His Val Lys Asn Ser Thr
545                 550                 555                 560

Tyr Thr Ala Leu Ile Ile Ala Thr Asp Asn Gly Ser Pro Val Ala Thr
                565                 570                 575

Gly Thr Gly Thr Leu Leu Leu Ile Leu Ser Asp Val Asn Asp Asn Ala
            580                 585                 590

Pro Ile Pro Glu Pro Arg Thr Ile Phe Phe Cys Glu Arg Asn Pro Lys
        595                 600                 605

Pro Gln Val Ile Asn Ile Ile Asp Ala Asp Leu Pro Pro Asn Thr Ser
    610                 615                 620

Pro Phe Thr Ala Glu Leu Thr His Gly Ala Ser Ala Asn Trp Thr Ile
625                 630                 635                 640

Gln Tyr Asn Asp Pro Thr Gln Glu Ser Ile Ile Leu Lys Pro Lys Met
                645                 650                 655

Ala Leu Glu Val Gly Asp Tyr Lys Ile Asn Leu Lys Leu Met Asp Asn
            660                 665                 670

Gln Asn Lys Asp Gln Val Thr Thr Leu Glu Val Ser Val Cys Asp Cys
        675                 680                 685

Glu Gly Ala Ala Gly Val Cys Arg Lys Ala Gln Pro Val Glu Ala Gly
    690                 695                 700

Leu Gln Ile Pro Ala Ile Leu Gly Ile Leu Gly Gly Ile Leu Ala Leu

705                 710                 715                 720

Leu Ile Leu Ile Leu Leu Leu Leu Leu Phe Leu Arg Arg Arg Ala Val
                725                 730                 735

Val Lys Glu Pro Leu Leu Pro Pro Glu Asp Asp Thr Arg Asp Asn Val
            740                 745                 750

Tyr Tyr Tyr Asp Glu Glu Gly Gly Gly Glu Glu Asp Gln Asp Phe Asp
        755                 760                 765

Leu Ser Gln Leu His Arg Gly Leu Asp Ala Arg Pro Glu Val Thr Arg
    770                 775                 780

Asn Asp Val Ala Pro Thr Leu Met Ser Val Pro Arg Tyr Leu Pro Arg
785                 790                 795                 800

Pro Ala Asn Pro Asp Glu Ile Gly Asn Phe Ile Asp Glu Asn Leu Lys
                805                 810                 815

Ala Ala Asp Thr Asp Pro Thr Ala Pro Pro Tyr Asp Ser Leu Leu Val
            820                 825                 830

Phe Asp Tyr Glu Gly Ser Gly Ser Glu Ala Ala Ser Leu Ser Ser Leu
        835                 840                 845

Asn Ser Ser Glu Ser Asp Lys Asp Gln Asp Tyr Asp Tyr Leu Asn Glu
    850                 855                 860

Trp Gly Asn Arg Phe Lys Lys Leu Ala Asp Met Tyr Gly Gly Gly Glu
865                 870                 875                 880

Asp Asp

<210>  54
<211>  26
<212>  DNA
<213>  Artificial

<220>
<223>  an artificially synthesized sequence

<400>  54
cagctccaca acctacatca ttccgt                                          26

<210>  55
<211>  12

<212>  DNA
<213>  Artificial

<220>
<223>  an artificially synthesized sequence

<400>  55
acggaatgat gt                                                          12


<210>  56
<211>  26
<212>  DNA
<213>  Artificial

<220>
<223>  an artificially synthesized sequence

<400>  56
ctccaatgcc tgctcttgat ggtagc                                           26


<210>  57
<211>  26
<212>  DNA
<213>  Artificial

<220>
<223>  an artificially synthesized sequence

<400>  57
tctctgtgta gccctggctg tcctag                                           26


<210>  58
<211>  26
<212>  DNA
<213>  Artificial

<220>
<223>  an artificially synthesized sequence

<400>  58
cttcccgtat gctaccttgt ctccac                                           26


<210>  59
<211>  26
<212>  DNA
<213>  Artificial

<220>
<223>  an artificially synthesized sequence

<400>  59
ccaacagtcc tgcatgcttg taatga                                           26

SEQUENCE LISTING

<110>  EISAI R&D MANAGEMENT CO., LTD.

<120>  Method for obtaining Purkinje progenitor cell by using Neph3(65B13) and
E-cadherin

<130>  EP73469FZapu

<140>  09 805 016.4
<141>  2009-08-06

<150>  JP 2008-206309
<151>  2008-08-08

<160>  59

<170>  PatentIn version 3.3

<210>  1
<211>  2876
<212>  DNA
<213>  Mus musculus

<400>  1
gatgagccag atttcgggga ctctgggcca gacataaaat cttccagccc ggagagaatt      60

gtgtgcagag aggggctcca gtccagcgtg gtgtgagagg cgtgctatca agaaagaagt     120

tggaggggaa ccagtgcaac cctaactcta cgagatcttg gggtacacac actcgggatg     180

ctggcctccg ccctcctcgt tttcctttgc tgtttcaaag gacatgcagg ctcatcgccc     240

catttcctac aacagccaga ggacatggtg gtgctgttgg gggaggaagc ccggctgccc     300

tgcgctctgg gcgcgtacag ggggctcgtg cagtggacta aggatgggct ggctctaggg     360

ggcgaaagag accttccagg gtggtcccgg tactggatat cggggaattc agccagtggc     420

cagcatgacc tccacattaa gcctgtggaa ttggaagatg aggcatcgta tgagtgccag     480

gcttcgcaag caggtctccg atcacgacca gcccaactgc acgtgatggt ccccccagaa     540

gctccccagg tactaggcgg cccctctgtg tctctggttg ctggagttcc tggaaatctg     600

acctgtcgga gtcgtgggga ttcccgacct gcccctgaac tactgtggtt ccgagatggg     660

atccggctgg atgcgagcag cttccaccag accacgctga aggacaaggc cactggaaca     720

gtggaaaaca ccttattcct gaccccttcc agtcatgatg atggcgccac cttgatctgc     780

agagcgcgaa gccaggccct gcccacaggg agggacacag ctgttacact gagccttcag     840

tatcccccaa tggtgactct gtctgctgag ccccagactg tgcaggaggg agagaaggtg     900

actttcctgt gtcaagccac tgcccagcct cctgtcactg gctacaggtg ggcgaagggg     960

ggatccccgg tgctcggggc acgtgggcca aggttggagg tcgttgcaga tgccactttc    1020

ctgactgagc cggtgtcctg cgaggtcagc aacgcggtcg gaagcgccaa ccgcagcacg    1080

gcgctggaag tgttgtatgg acccattctg caggcaaaac ctaagtccgt gtccgtggac    1140

gtggggaaag atgcctcctt cagctgtgtc tggcgcggga acccacttcc acggataacc    1200

tggacccgca tgggtggctc tcaggtgctg agctccgggc ccacgctgcg gcttccgtcc    1260

gtggcactgg aggatgcggg cgactatgta tgcagggctg agccgaggag aacgggtctg    1320

ggaggcggca aagcgcaggc gaggctgact gtgaacgcac cccctgtagt gacagccctg 1380
caacctgcac cagcctttct gaggggtcct gctcgcctcc agtgtgtggt gtttgcctcc 1440
cctgccccag actcggtggt ttggtcttgg gacgagggct tcttggaggc aggctcactg 1500
ggcaggttcc tagtggaagc cttcccagcc ccggaagtgg aggggggaca gggccctggc 1560
cttatttctg tgctacacat ttccggaacc caggagtccg actttaccac cggcttcaac 1620
tgcagtgccc gcaaccggct aggagaggga cgagtccaga tccacttggg ccgtagagat 1680
ttgctgccta ctgtccggat tgtggctggt gcagcatctg cagccacctc tctccttatg 1740
gtcatcactg gagtggtcct ctgctgctgg cgccatggct ctctctctaa gcaaaagaac 1800
ttggtccgga tcccaggaag cagcgagggt tccagttcac gtggccctga ggaggagaca 1860
ggcagcagtg aggaccgggg tcccattgtg cacaccgacc acagtgattt ggttcttgag 1920
gaaaaagagg ctctggagac aaaggatcca accaacggtt actacaaggt tcgaggggtc 1980
agtgtgagcc ttagccttgg ggaagctcct ggaggaggcc tcttcttgcc accgccctct 2040
ccgatcggtc tcccagggac tcctacttac tatgacttca agccacatct ggacttagtc 2100
cctccctgca gactgtacag agcgagggca ggttatctta ccacccccca tccccgtgcc 2160
ttcaccagct acatgaaacc cacatccttt ggacccccag atttgagctc tggaactccc 2220
cccttcccgt atgctacctt gtctccaccc agccaccagc gtctccagac tcatgtgtga 2280
atccatctct ccaagtgaag ggtcttggaa tcttctgttt gccatatagt gtgttgtcca 2340
gatttctggg gagtcagaac aagttgatga ccaacccctc caaaactgaa cattgaagga 2400
gggaaagatc attacaagca tcaggactgt tggtgtacac tcagttcagc caaagtggat 2460
tctccaagtg ggagcaatat ggccgctttc ccatgagaaa gacattcaag atggtgacta 2520
aatgactaaa tactttgcag agggacaaag atgggaacta gggatacgga tggaagtagt 2580
agagaagata tatgaccatc tgcatcaaga ggaaggataa catatgacaa atcaagatga 2640
aagaaataat ccaccccacc cccaccgcgt cctggccaat aagtatagcc tacatggctg 2700
ttcattatct gggaaccaaa atggccacta tcttgactcc ttccttaaag atacagaaag 2760
aattgaatcc aaggaatggg gtagggtgga aatagaagaa atgaagggga ctcttgggct 2820
aagaatactt atgtttaata ataaaagggg gaggcaaaga tgcaaaaaaa aaaaaa 2876

<210> 2
<211> 700
<212> PRT
<213> Mus musculus

<400> 2

Met Leu Ala Ser Ala Leu Leu Val Phe Leu Cys Cys Phe Lys Gly His
1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly
        115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Ala Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys
225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys
        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu
545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Lys Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Asp Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  3
<211>  2243
<212>  DNA
<213>  Mus musculus

<400>  3
gagagaattg tgtgcagaga gaggctccag tccagcgtgg tgtgagaggc gtgctatcaa      60

gaaagaagtt ggaggggaac cagtgcaacc ctaactctac gagatcttgg ggtacacaca     120

ctcgggatgc tggcctccgc cctcctcgtt ttcctttgct gtttcaaagg acatgcaggg     180

tggtcccggt actggatatc ggggaattca gccagtggcc agcatgacct ccacattaag     240

cctgtggaat tggaagatga ggcatcgtat gagtgccagg cttcgcaagc aggtctccga     300

tcacgaccag cccaactgca cgtgatggtc cccccagaag ctccccaggt actaggcggc     360

ccctctgtgt ctctggttgc tggagttcct ggaaatctga cctgtcggag tcgtggggat     420

tcccgacctg cccctgaact actgtggttc cgagatggga tccggctgga tgcgagcagc     480

ttccaccaga ccacgctgaa ggacaaggcc actggaacag tggaaaacac cttattcctg     540

accccttcca gtcatgatga tggcgccacc ttgatctgca gagcgcgaag ccaggccctg     600

cccacaggga gggacacagc tgttacactg agccttcagt atcccccaat ggtgactctg     660

tctgctgagc cccagactgt gcaggaggga gagaaggtga ctttcctgtg tcaagccact     720

gcccagcctc ctgtcactgg ctacaggtgg gcgaaggggg gatccccggt gctcggggca     780

cgtgggccaa ggttggaggt cgttgcagat gccactttcc tgactgagcc ggtgtcctgc     840

gaggtcagca acgcggtcgg aagcgccaac cgcagcacgg cgctggaagt gttgtatgga     900

cccattctgc aggcaaaacc taagtccgtg tccgtggacg tggggaaaga tgcctccttc 960

agctgtgtct ggcgcgggaa cccacttcca cggataacct ggacccgcat gggtggctct 1020

caggtgctga gctccgggcc cacgctgcgg cttccgtccg tggcactgga ggatgcgggc 1080

gactatgtat gcagggctga gccgaggaga acgggtctgg gaggcggcaa agcgcaggcg 1140

aggctgactg tgaacgcacc ccctgtagtg acagccctgc aacctgcacc agcctttctg 1200

aggggtcctg ctcgcctcca gtgtgtggtg tttgcctccc ctgccccaga ctcggtggtt 1260

tggtcttggg acgagggctt cttggaggca ggctcactgg gcaggttcct agtggaagcc 1320

ttcccagccc cggaagtgga ggggggacag ggccctggcc ttatttctgt gctacacatt 1380

tccggaaccc aggagtccga ctttaccacc ggcttcaact gcagtgcccg caaccggcta 1440

ggagagggac gagtccagat ccacttgggc cgtagagatt tgctgcctac tgtccggatt 1500

gtggctggtg cagcatctgc agccacctct ctccttatgg tcatcactgg agtggtcctc 1560

tgctgctggc gccatggctc tctctctaag caaaagaact tggtccggat cccaggaagc 1620

agcgagggtt ccagttcacg tggccctgag gaggagacag gcagcagtga ggaccggggt 1680

cccattgtgc acaccgacca cagtgatttg gttcttgagg aaaaagaggc tctggagaca 1740

aaggatccaa ccaacggtta ctacaaggtt cgaggggtca gtgtgagcct tagccttggg 1800

gaagctcctg gaggaggcct cttcttgcca ccgccctctc cgatcggtct cccagggact 1860

cctacttact atgacttcaa gccacatcag gacttagtcc ctccctgcag actgtacaga 1920

gcgagggcag gttatcttac caccccccat ccccgtgcct tcaccagcta catgaaaccc 1980

acatcctttg gacccccaga tttgagctct ggaactcccc ccttcccgta tgctaccttg 2040

tctccaccca gccaccagcg tctccagact catgtgtgaa tccatctctc caagtgaagg 2100

gtcttggaat cttctgtttg ccatatagtg tgttgtccag atttctgggg agtcagaaca 2160

agttgatgac caacccctcc aaaactgaac attgaaggag ggaaagatca ttacaagcat 2220

caggactgtt ggtgtacact cag 2243

<210> 4
<211> 650
<212> PRT
<213> Mus musculus

<400> 4

Met Leu Ala Ser Ala Leu Leu Val Phe Leu Cys Cys Phe Lys Gly His
1               5                   10                  15

Ala Gly Trp Ser Arg Tyr Trp Ile Ser Gly Asn Ser Ala Ser Gly Gln
            20                  25                  30

His Asp Leu His Ile Lys Pro Val Glu Leu Glu Asp Glu Ala Ser Tyr
        35                  40                  45

Glu Cys Gln Ala Ser Gln Ala Gly Leu Arg Ser Arg Pro Ala Gln Leu

50                  55                  60

His Val Met Val Pro Pro Glu Ala Pro Gln Val Leu Gly Gly Pro Ser
65                  70                  75                  80

Val Ser Leu Val Ala Gly Val Pro Gly Asn Leu Thr Cys Arg Ser Arg
                85                  90                  95

Gly Asp Ser Arg Pro Ala Pro Glu Leu Leu Trp Phe Arg Asp Gly Ile
            100                 105                 110

Arg Leu Asp Ala Ser Ser Phe His Gln Thr Thr Leu Lys Asp Lys Ala
        115                 120                 125

Thr Gly Thr Val Glu Asn Thr Leu Phe Leu Thr Pro Ser Ser His Asp
    130                 135                 140

Asp Gly Ala Thr Leu Ile Cys Arg Ala Arg Ser Gln Ala Leu Pro Thr
145                 150                 155                 160

Gly Arg Asp Thr Ala Val Thr Leu Ser Leu Gln Tyr Pro Pro Met Val
                165                 170                 175

Thr Leu Ser Ala Glu Pro Gln Thr Val Gln Glu Gly Glu Lys Val Thr
            180                 185                 190

Phe Leu Cys Gln Ala Thr Ala Gln Pro Pro Val Thr Gly Tyr Arg Trp
        195                 200                 205

Ala Lys Gly Gly Ser Pro Val Leu Gly Ala Arg Gly Pro Arg Leu Glu
    210                 215                 220

Val Val Ala Asp Ala Thr Phe Leu Thr Glu Pro Val Ser Cys Glu Val
225                 230                 235                 240

Ser Asn Ala Val Gly Ser Ala Asn Arg Ser Thr Ala Leu Glu Val Leu
                245                 250                 255

Tyr Gly Pro Ile Leu Gln Ala Lys Pro Lys Ser Val Ser Val Asp Val
            260                 265                 270

Gly Lys Asp Ala Ser Phe Ser Cys Val Trp Arg Gly Asn Pro Leu Pro
        275                 280                 285

Arg Ile Thr Trp Thr Arg Met Gly Gly Ser Gln Val Leu Ser Ser Gly
    290                 295                 300

Pro Thr Leu Arg Leu Pro Ser Val Ala Leu Glu Asp Ala Gly Asp Tyr
305                 310                 315                 320

Val Cys Arg Ala Glu Pro Arg Arg Thr Gly Leu Gly Gly Gly Lys Ala

                325                 330                 335

Gln Ala Arg Leu Thr Val Asn Ala Pro Pro Val Val Thr Ala Leu Gln
            340                 345                 350

Pro Ala Pro Ala Phe Leu Arg Gly Pro Ala Arg Leu Gln Cys Val Val
        355                 360                 365

Phe Ala Ser Pro Ala Pro Asp Ser Val Val Trp Ser Trp Asp Glu Gly
    370                 375                 380

Phe Leu Glu Ala Gly Ser Leu Gly Arg Phe Leu Val Glu Ala Phe Pro
385                 390                 395                 400

Ala Pro Glu Val Glu Gly Gly Gln Gly Pro Gly Leu Ile Ser Val Leu
                405                 410                 415

His Ile Ser Gly Thr Gln Glu Ser Asp Phe Thr Thr Gly Phe Asn Cys
            420                 425                 430

Ser Ala Arg Asn Arg Leu Gly Glu Gly Arg Val Gln Ile His Leu Gly
        435                 440                 445

Arg Arg Asp Leu Leu Pro Thr Val Arg Ile Val Ala Gly Ala Ala Ser
    450                 455                 460

Ala Ala Thr Ser Leu Leu Met Val Ile Thr Gly Val Val Leu Cys Cys
465                 470                 475                 480

Trp Arg His Gly Ser Leu Ser Lys Gln Lys Asn Leu Val Arg Ile Pro
                485                 490                 495

Gly Ser Ser Glu Gly Ser Ser Ser Arg Gly Pro Glu Glu Glu Thr Gly
            500                 505                 510

Ser Ser Glu Asp Arg Gly Pro Ile Val His Thr Asp His Ser Asp Leu
        515                 520                 525

Val Leu Glu Glu Lys Glu Ala Leu Glu Thr Lys Asp Pro Thr Asn Gly
    530                 535                 540

Tyr Tyr Lys Val Arg Gly Val Ser Val Ser Leu Ser Leu Gly Glu Ala
545                 550                 555                 560

Pro Gly Gly Gly Leu Phe Leu Pro Pro Pro Ser Pro Ile Gly Leu Pro
                565                 570                 575

Gly Thr Pro Thr Tyr Tyr Asp Phe Lys Pro His Gln Asp Leu Val Pro
            580                 585                 590

Pro Cys Arg Leu Tyr Arg Ala Arg Ala Gly Tyr Leu Thr Thr Pro His

595                 600                 605

Pro Arg Ala Phe Thr Ser Tyr Met Lys Pro Thr Ser Phe Gly Pro Pro
    610                 615                 620

Asp Leu Ser Ser Gly Thr Pro Pro Phe Pro Tyr Ala Thr Leu Ser Pro
625                 630                 635                 640

Pro Ser His Gln Arg Leu Gln Thr His Val
                645                 650

<210>  5
<211>  2750
<212>  DNA
<213>  Mus musculus

<400>  5
aacagccagg agcagtgacc gagccgctgg agctggggag agacgcgcgg aagactgggc      60
caggagacta gggaccgagg gacgcgcgcc tggggagagc caacaaggaa cccgcgggcc     120
ggacagcgac accggcaatc cgcgccaaac tgttccagcc gctggccttc tatagccgca     180
gccccaggac attctaaagc tctccaagac gccccctccc ctggcttctc gcgttgacca     240
aggaaaagaa aaagggatgg aaaaagaaag gaaggagact agaaagaaaa cccagatttg     300
ccaccgcaca aaaagagagg tgggggggac aaggaaaaaa aaaaaagtcg agcgactgtg     360
gggccggaac acaggcagcg ggatcgtggg ccgagcgatg caaggctgcg cgcccaagcg     420
gccgcgagtt gtgactgaag ccaggatgct cgtccaggcg cagtgaagag ccagaccgtg     480
ttgcctcccc aggagtccaa gcgcagggag ggccgctcgg aggacgcggc agactgcctg     540
gcaggccacc ggccgaggtg acagggctgg ggcggtgggg agcgagcgag tgcgcccggc     600
tgcgtccgcc cgaagcggac ggtccctttc catttttgac tggcacaaaa aagaaaactc     660
tccaaagggg tgggggctac ctaagcaaca actacaatca acaaaatatc ctacccaacc     720
cgccatctcc cccacacctc ggtctgcccc cgccccctcc ccaggcccag cgcgggcgcc     780
cagagcgtcc caactcactg caagaaaccg gcaatgtagg atccaaagct ttctactccc     840
gtgttctttt ctttccgtgt tttttttaaa ggggaaaacc cggtggtggg cagtctgaca     900
cgcacacaac ctgccttcat actctgacaa aagcagatgc actttgactt ctgacagctc     960
tacctcaagc tggagagaac ccagctttcc cgaatcctga gctcttggcg tcttcctttt    1020
cgtctgtttc cattttattt atttacgtcc cgccgcctct cacggtgacc ttcactcctt    1080
cgcgggcttt gagcagaaga gccgctttct agcccgcttg agactgattt tcctcgcccg    1140
gtgagctgag gtggcgctgc tccatcccgt tgccccggga ctccggggct gccctctacc    1200
agcctggtct ctcccccttt tgatttgcta gtacgggttt tttgcttgcc caactagaga    1260
gggtttcttc tttttggagg agctggttgt cttcagaagt catcccctcg actctaattg    1320
ccctgtcgct ccgggcctca ccggaccaaa ccaaagacca tggtgcactg tgcgggctgc    1380
aaaaggccca tcctggaccg tttcctcttg aacgtgttgg acagggcctg gcacgtcaag    1440

tgcgtccagt gctgtgaatg taaatgcaac ctgaccgaga agtgcttctc ccgggaaggc 1500

aagctctact gtaaaaacga cttcttccga tgtttcggta ccaaatgcgc cggttgtgcg 1560

cagggcatct ctccaagcga tctggttcgc agagcgcgaa gcaaagtgtt tcacctcaac 1620

tgcttcacct gcatgatgtg taacaagcag ctctccaccg gcgaggagct ctacatcata 1680

gacgagaaca agttcgtttg taaagaggat tacctgagta acagcagtgt cgccaaagag 1740

aacagcctcc actcggccac cacaggcagt gaccctagtt tatctccgga ttcccaagat 1800

ccatcgcagg atgatgccaa ggactctgaa agtgccaacg tctcagataa ggaaggtggt 1860

agtaatgaga atgatgatca gaacctaggt gccaaacgta ggggaccccg gaccacgatc 1920

aaagccaagc aactggagac gttgaaggca gcctttgcag ctacacccaa gcccacacgc 1980

catatccgtg agcaactggc ccaggagact ggcctcaaca tgcgtgttat ccaggtctgg 2040

ttccagaatc gacgctccaa ggagcgaagg atgaaacagc taagcgcgct aggcgcgcgg 2100

cgccacgcct ttttccgcag tcctcgtcgg atgcggccgc tggtggaccg cctggagccg 2160

ggcgaactca tccccaacgg ccccttctcc ttttacggag attaccagag tgagtactac 2220

ggtcccggag gcaactacga cttcttcccg caaggaccgc catcctctca ggctcagacg 2280

ccagtggacc taccctttgt gccatcatct ggcccttcgg ggacgcccct tggaggtctg 2340

gaccacccgc tgcctggtca ccacccttcc agtgaggcgc agcgatttac tgacatcctg 2400

gcacatcccc caggggactc ccctagtcct gagcccagct tgcccgggcc tctccactcc 2460

atgtcagcgg aggtcttcgg gcccagtcca cctttctcat ctctgtcggt caatggtgga 2520

gccagctacg ggaaccattt gtctcaccct cctgaaatga acgaggcagc cgtgtggtag 2580

cggggtctcg catgggccac gggagctcgt ggttgtacag agacgagctt ttatttcaga 2640

aaaatagatt aaaaagacaa aaaaaaaaaa acccccaaaa caaaaaagca agcctcctgc 2700

tccacttcct tcagcctcgg ggaccagtct gtttggggag actggatagc 2750

<210> 6
<211> 406
<212> PRT
<213> Mus musculus

<400> 6

Met Val His Cys Ala Gly Cys Lys Arg Pro Ile Leu Asp Arg Phe Leu
1               5                   10                  15

Leu Asn Val Leu Asp Arg Ala Trp His Val Lys Cys Val Gln Cys Cys
            20                  25                  30

Glu Cys Lys Cys Asn Leu Thr Glu Lys Cys Phe Ser Arg Glu Gly Lys
        35                  40                  45

Leu Tyr Cys Lys Asn Asp Phe Phe Arg Cys Phe Gly Thr Lys Cys Ala
    50                  55                  60

Gly Cys Ala Gln Gly Ile Ser Pro Ser Asp Leu Val Arg Arg Ala Arg
65                  70                  75                  80

Ser Lys Val Phe His Leu Asn Cys Phe Thr Cys Met Met Cys Asn Lys
                85                  90                  95

Gln Leu Ser Thr Gly Glu Glu Leu Tyr Ile Ile Asp Glu Asn Lys Phe
            100                 105                 110

Val Cys Lys Glu Asp Tyr Leu Ser Asn Ser Ser Val Ala Lys Glu Asn
        115                 120                 125

Ser Leu His Ser Ala Thr Thr Gly Ser Asp Pro Ser Leu Ser Pro Asp
    130                 135                 140

Ser Gln Asp Pro Ser Gln Asp Asp Ala Lys Asp Ser Glu Ser Ala Asn
145                 150                 155                 160

Val Ser Asp Lys Glu Gly Gly Ser Asn Glu Asn Asp Asp Gln Asn Leu
                165                 170                 175

Gly Ala Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Val Asp Arg Leu Glu Pro Gly Glu Leu Ile Pro
            260                 265                 270

Asn Gly Pro Phe Ser Phe Tyr Gly Asp Tyr Gln Ser Glu Tyr Tyr Gly
        275                 280                 285

Pro Gly Gly Asn Tyr Asp Phe Phe Pro Gln Gly Pro Pro Ser Ser Gln
    290                 295                 300

Ala Gln Thr Pro Val Asp Leu Pro Phe Val Pro Ser Ser Gly Pro Ser
305                 310                 315                 320

Gly Thr Pro Leu Gly Gly Leu Asp His Pro Leu Pro Gly His His Pro
                325                 330                 335

Ser Ser Glu Ala Gln Arg Phe Thr Asp Ile Leu Ala His Pro Pro Gly
            340                 345                 350

Asp Ser Pro Ser Pro Glu Pro Ser Leu Pro Gly Pro Leu His Ser Met
        355                 360                 365

Ser Ala Glu Val Phe Gly Pro Ser Pro Pro Phe Ser Ser Leu Ser Val
    370                 375                 380

Asn Gly Gly Ala Ser Tyr Gly Asn His Leu Ser His Pro Pro Glu Met
385                 390                 395                 400

Asn Glu Ala Ala Val Trp
                405

<210>  7
<211>  2283
<212>  DNA
<213>  Homo sapiens

<400>  7
cggccgcgag ttgtgactgg agccacgatg cacggccagg cgcggtgaga agccagcccg      60

tagtgcctcc cgaaggagcc cgggcgcagg gagggtcgcc ctgaggacac ggaggccgcc     120

aggcaggcca agggccgagg tgactgggct ggggcggtag ggaaggagcg agtgcgcctg     180

gctgcctccg cacggagttg tccctctctg ttttcgattg acacaaacac ttctccaaaa     240

gcggggaaac ctaagcaaca acagcaatca acaccaagat cttcctccta ccctcccctc     300

tttcccttct cccgcggtcg gccctcgccc cctcccccag gcccagcgcg ggcgctcggc     360

gcgtccagac ccgcggcgcg atgccggcag tttaggatcc aaagcttctc tgctcctttt     420

gttctttcct tccctttttt aaaaaaagag gggggaaatc ccagtggtgg gcagcctggc     480

acgcacacag tcgccctcat accccgacaa aagcagatgc actttgactt ctgacagctc     540

tacctcaagc cccggagaac tcagcggcgc tttcctcgca acccgagctc ggcgagtcgt     600

cgtcttcttc ttctccgttt ttatttattt atttccgttc ccgccgccgt tctcgctgac     660

cttcactcct ccgcgggctc tgagcagaag ggtcgcattc tctcccgcct gagacttctt     720

ttcctcgccc cgggagctca ggcggcgccg ctccagcccg gggccccggg actccccggc     780

tgcacacttc actgagacgc ccccccaggc cccgatcagc ctcgtttcct ccaccctact     840

ttgatttcct ggtgcgagtt ttggcttgca cggccgagtg tgtgtcctct ttttggagag      900

actggggagc tcgtgccgat tgtcttcagg agtcatcccc tgggctctac tttgcccctc     960

tctctctctg ggcctcatca gaccaaacca aagaccatgg ttcactgtgc cggctgcaaa    1020

aggcccatcc tggaccgctt tctcttgaac gtgctggaca gggcctggca cgtcaagtgc    1080

gtccagtgct gtgaatgtaa atgcaacctg accgagaagt gcttctccag ggaaggcaaa    1140

ctctactgca agaacgactt cttccggtgt ttcggtacca aatgcgcagg ctgcgctcag    1200

ggcatctccc ctagcgacct ggtgcggaga gcgcggagca aagtgtttca cctgaactgc    1260

ttcacctgca tgatgtgtaa caagcagctc tccactggcg aggaactcta catcatcgac 1320

gagaataagt tcgtctgcaa agaggattac ctaagtaaca gcagtgttgc caaagagaac 1380

agccttcact cggccaccac gggcagtgac cccagtttgt ctccggattc ccaagacccg 1440

tcgcaggacg acgccaagga ctcggagagc gccaacgtgt cggacaagga agcgggtagc 1500

aacgagaatg acgaccagaa cctgggcgcc aagcggcggg gaccgcgcac caccatcaaa 1560

gccaagcagc tggagacgct gaaggccgcc ttcgctgcta cacccaagcc cacccgccac 1620

atccgcgagc agctggcgca ggagaccggc ctcaacatgc gcgtcattca ggtctggttc 1680

cagaaccggc gctccaagga gcggaggatg aagcagctga gcgccctggg cgcccggcgc 1740

cacgccttct tccgcagtcc gcgccggatg cggccgctgg tggaccgcct ggagccgggc 1800

gagctcatcc ccaatggtcc cttctccttc tacggagatt accagagcga gtactacggg 1860

cccgggggca actacgactt cttcccgcaa ggccccccgt cctcgcaggc ccagacacca 1920

gtggacctac ccttcgtgcc gtcatctggg ccgtccggga cgcccctggg tggcctggag 1980

cacccgctgc cgggccacca cccgtcgagc gaggcgcagc ggtttaccga catcctggcg 2040

cacccacccg gggactcgcc cagccccgag cccagcctgc ccgggcctct gcactccatg 2100

tcggccgagg tcttcggacc cagcccgccc ttctcgtcgc tgtcggtcaa cggtggggcg 2160

agctacggaa accacctgtc ccaccccccc gaaatgaacg aggcggccgt gtggtagcgg 2220

ggtctcgcac ggtctgcgga gttcgtggtt gtacagaaat gaacctttat ttaagaaaaa 2280

tag 2283

<210> 8
<211> 406
<212> PRT
<213> Homo sapiens

<400> 8

Met Val His Cys Ala Gly Cys Lys Arg Pro Ile Leu Asp Arg Phe Leu
1               5                   10                  15

Leu Asn Val Leu Asp Arg Ala Trp His Val Lys Cys Val Gln Cys Cys
            20                  25                  30

Glu Cys Lys Cys Asn Leu Thr Glu Lys Cys Phe Ser Arg Glu Gly Lys
        35                  40                  45

Leu Tyr Cys Lys Asn Asp Phe Phe Arg Cys Phe Gly Thr Lys Cys Ala
    50                  55                  60

Gly Cys Ala Gln Gly Ile Ser Pro Ser Asp Leu Val Arg Arg Ala Arg
65                  70                  75                  80

Ser Lys Val Phe His Leu Asn Cys Phe Thr Cys Met Met Cys Asn Lys
                85                  90                  95

Gln Leu Ser Thr Gly Glu Glu Leu Tyr Ile Ile Asp Glu Asn Lys Phe
            100                 105                 110

Val Cys Lys Glu Asp Tyr Leu Ser Asn Ser Ser Val Ala Lys Glu Asn
        115                 120                 125

Ser Leu His Ser Ala Thr Thr Gly Ser Asp Pro Ser Leu Ser Pro Asp
    130                 135                 140

Ser Gln Asp Pro Ser Gln Asp Asp Ala Lys Asp Ser Glu Ser Ala Asn
145                 150                 155                 160

Val Ser Asp Lys Glu Ala Gly Ser Asn Glu Asn Asp Asp Gln Asn Leu
                165                 170                 175

Gly Ala Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Val Asp Arg Leu Glu Pro Gly Glu Leu Ile Pro
            260                 265                 270

Asn Gly Pro Phe Ser Phe Tyr Gly Asp Tyr Gln Ser Glu Tyr Tyr Gly
        275                 280                 285

Pro Gly Gly Asn Tyr Asp Phe Phe Pro Gln Gly Pro Pro Ser Ser Gln
    290                 295                 300

Ala Gln Thr Pro Val Asp Leu Pro Phe Val Pro Ser Ser Gly Pro Ser
305                 310                 315                 320

Gly Thr Pro Leu Gly Gly Leu Glu His Pro Leu Pro Gly His His Pro
                325                 330                 335

Ser Ser Glu Ala Gln Arg Phe Thr Asp Ile Leu Ala His Pro Pro Gly
            340                 345                 350

Asp Ser Pro Ser Pro Glu Pro Ser Leu Pro Gly Pro Leu His Ser Met
        355                 360                 365

Ser Ala Glu Val Phe Gly Pro Ser Pro Pro Phe Ser Ser Leu Ser Val
    370                 375                 380

Asn Gly Gly Ala Ser Tyr Gly Asn His Leu Ser His Pro Pro Glu Met
385                 390                 395                 400

Asn Glu Ala Ala Val Trp
                405

<210>  9
<211>  2405
<212>  DNA
<213>  Mus musculus

<400>  9
aggacacctg ctcgaagctg gagcgagcgc ccggtcgcga cccgtgacat gaggctgtga      60

ccgctgccgc cctccacgcc actctgggca gtgcagcgcc aggccggaga gcgtcggagg     120

acttgacccc gagaagtctt ggttgatccg taacggactc gcccctacag actcgcctac     180

agactaggaa ggctgagagc cacagcagcg gggaccgaga gggcctaagg gcccaggggc     240

cccaaggagg acgaggcggc ccgagccgcc ggggcgcgcg gctatgatgg tgcactgtgc     300

tggctgtgag cggcccatcc tcgaccgctt tctgctgaac gtactagacc gcgcgtggca     360

tatcaaatgt gttcaatgct gcgagtgcaa aaccaacctc tcggagaagt gcttctcacg     420

ggaaggcaag ctatactgta aaaacgactt tttcaggcgc tttggcacaa agtgcgccgg     480

ctgcgcgcaa ggtatctctc cgagcgacct ggtacggaag gcccggagca aagtcttcca     540

cctcaactgc ttcacctgta tggtgtgcaa taagcagcta tccaccggag aggagctcta     600

cgtgatcgac gagaacaagt ttgtgtgcaa ggacgactac ttaagctcct ctagcctcaa     660

ggaaggaagt ctcaactcgg tgtcgtcctg tacggaccgc agtttgtccc cggacctcca     720

ggatccgtta caggacgacc ccaaagagac cgacaattcg acctcatcgg acaaggaaac     780

cgctaacaac gagaatgagg aacagaactc cggcaccaaa cggcgcggcc cgcgcaccac     840

catcaaggcc aagcagctgg agacgctcaa ggcagccttc gcagccacgc ccaagcccac     900

gcgccacatt cgcgaacagc tggcacagga gacgggcctc aacatgaggg tcattcaggt     960

gtggtttcag aaccgaaggt ccaaagaacg ccgcatgaaa cagctgagcg ctctgggcgc    1020

gcggagacac gccttcttcc ggagtccgcg gcgcatgcgt cccctgggcg gccgcttgga    1080

cgagtctgag atgttggggt ctaccccata cacttattac ggagactacc aaagtgacta    1140

ctacgctccg ggaggcaatt acgatttctt cgcgcacggt ccgccgtcac aggcgcagtc    1200

ccctgccgac tccagcttcc tggcagcatc gggacctggc tcgacgccgc tgggcgcgct    1260

ggaaccgccg ctggccgggc ctcacggcgc ggacaacccc agattcaccg acatgatctc    1320

gcatccggac acgccgagcc ccgagccggg cctgcccggt gcgctgcacc ccatgccggg    1380

agaggtgttc agcggcgggc ccagcccgcc cttccccatg agcggcacca gcggctacag    1440

tggacccctg tcgcacccca accctgagct caacgaagcg gccgtatggt aaggccgagg    1500

ggctgagttg tccccctgcc accaagcccg ggacgggacg ccgcctgggt aagcctcaag 1560
agtcctctcg tgggttcgca cccaaccagg ccactcgcat caccacccct cagagctttg 1620
gcacgcgcct gcgcaatttc tcgggaccaa agtcaatatt ctgaagggtc gagattccaa 1680
gcacatctta gaagccctcc ggatccccca cccatcatca cctccttgaa ctaagagagg 1740
gggatgaggc caaggagcgg agaccatggc actacccctc cctgcgagcc gaggcattgt 1800
gaaatcctat ttctcacttt ctcttttaaa aaaagaaaga aagaaggaag gaaggaagga 1860
aggaaagaaa gaaagagagg ttgaaagggg gagagaaaga gagagagaga gagagagaga 1920
gagagagaga gagagagaga gagagagaga gagagagaga gagagagaga gagagagaga 1980
gcgcgagcga gctgaggaaa gctcagccag agaagaaaaa tgaggaggac cgccggtgaa 2040
tggtggcttt gcaggaagac aaatccacct gcgagttggc gccccctggt ggctcaatgt 2100
cagcttgtct aggaaggtgc gcggtctggg cctggccttc ctgagcccaa ctccctgctc 2160
ctccatactt tgagtctgag gggcctggca ggattcgaac cctcccaccc tgctctgacc 2220
ctgagccggg cgccaagtca ttgagcattt gcccacggat cactctccct gcccgggacc 2280
tggaggctgg gccatcagga cgaacagtat tatacttttt tggaagtcgg acgcttctag 2340
tttccttatt ttgtataaag aagaaacaaa taaagtatgt ttttgtgaaa aaaaaaaaaa 2400
aaaaa 2405

<210> 10
<211> 402
<212> PRT
<213> Mus musculus

<400> 10

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Pro Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly
            260                 265                 270

Ser Thr Pro Tyr Thr Tyr Tyr Gly Asp Tyr Gln Ser Asp Tyr Tyr Ala
        275                 280                 285

Pro Gly Gly Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Gly Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Ala Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala
385                 390                 395                 400

Val Trp

<210>  11
<211>  2084
<212>  DNA
<213>  Homo sapiens

<400>  11
aaccaggtac aagctaatac tcaacaatac tgatgccttg ttttttttgc tctgtccgga      60

cagcaacgct gtagccaatt tagatatgct ataaatttaa gaggttgcca tggccacggt     120

gcgcccattg gccgctgggc cccctacgtg cagcgccacg tcaccaaatc tgaataagga     180

tgcgcgaatt acgcggcgac cagacaaaga tgaggatccg gaccgcttga aagtggggga     240

aagtgccggc gcctccgcca ccggggaaag ccgctccgca gcgccgaggc cagcagccac     300

ccgagatacc tggggaagcc cggaacaggc gccggggcgt gcggcccgtg gcatgaggtt     360

gtgaacgcca cccgcccccc accaccccac tccgggcagc ccagcgccag gccagagatt     420

gcccaaggac tggaccggct gagtcttggt ccggaccaga ctcgccctgc agctgctgag     480

acaagaggcg aagggcagcg gagggcccgg caggcccgag ggccaggggc ccaaagggag     540

ggcaaggcgg ccgaagccgc cggggcgcgg ggctatgatg gtgcactgcg ccggttgcga     600

gcggcccatc ctcgaccgct ttctgctgaa cgtgctggac cgcgcgtggc acatcaaatg     660

tgttcagtgc tgcgagtgca aaaccaacct ctcggagaag tgcttctcgc gcgagggcaa     720

gctctactgc aaaaatgact ttttcaggcg ctttggcacg aaatgcgccg gctgcgcgca     780

aggcatctcg cccagcgacc tggtgcgcaa ggcccggagc aaagtctttc acctcaactg     840

tttcacctgc atggtgtgta acaagcagct gtccaccggc gaggagctct acgtcatcga     900

cgagaacaag ttcgtgtgca aagacgacta cctgagctca tccagcctca aggagggcag     960

cctcaactca gtgtcatcct gtacggaccg cagtttgtcc ccggacctcc aggacgcact    1020

gcaggacgac cccaaagaga cggacaactc gacctcgtcg gacaaggaga cggccaacaa    1080

cgagaacgag gagcagaact cgggcaccaa gcggcgcggc ccccgcacca ccatcaaggc    1140

caagcagctg gagacgctca aggctgcctt cgccgccacg cccaagccca cgcgccacat    1200

ccgcgagcag ctggcgcagg agaccggcct caacatgcgc gtcatccagg tgtggtttca    1260

gaaccgacgg tccaaagaac gccggatgaa acagctgagc gccctaggcg cccggaggca    1320

cgccttcttc cggagtccgc ggcgcatgcg tccgctgggc ggccgcttgg acgagtctga    1380

gatgttgggg tccaccccgt acacctacta cggagactac caaggcgact actacgcgcc    1440

gggaagcaac tacgacttct tcgcgcacgg cccgccttcg caggcgcagt ccccggccga    1500

ctccagcttc ctggcggcct ctggccccgg ctcgacgccg ctgggagcgc tggaaccgcc    1560

gctcgccggc ccgcacgccg cggacaaccc caggttcacc gacatgatct cgcacccgga    1620

cacaccgagc cccgagccag gcctgccggg cacgctgcac cccatgcccg gcgaggtatt 1680

cagcggcggg cccagcccgc ccttcccaat gagcggcacc agcggctaca gcggacccct 1740

gtcgcatccc aaccccgagc tcaacgaagc cgccgtgtgg taaggccgcc gggccgcccc 1800

ccgcgctcgg cccccggggg ccccgccccg aagcagcctc ctgaaaccaa aacgcccgac 1860

gcagacgcgg tgggagacgt gggtgtccct cgggggttct ctctcgggtc cgcactcaac 1920

tggcagctgc tcctcggctg ggcgccgagg gggggccgac ccccatctcc accccgcggg 1980

ctctccagga gcctcagccc accgccagta ctctcccagc aaccgcgagc aatttcttgg 2040

gaccaaagtc aatactccgg agggtcaaga gatttcgagc acgc 2084

<210> 12
<211> 402
<212> PRT
<213> Homo sapiens

<400> 12

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Ala Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly
            260                 265                 270

Ser Thr Pro Tyr Thr Tyr Tyr Gly Asp Tyr Gln Gly Asp Tyr Tyr Ala
        275                 280                 285

Pro Gly Ser Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Ala Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Thr Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala
385                 390                 395                 400

Val Trp


<210>  13
<211>  1914
<212>  DNA
<213>  Rattus norvegicus

<400>  13
cggcgcctcc gccaaccggg aaagccgctc gccagtgccg aagccagcag cctccgagga      60

cacctgctcg aagctggagc gagcgcccag acgcgacccg tgacatgagg ctgtgaccgc 120
cgccgccctc cacgccactc tgggcagtgc agtgccaggc cggagagcgt cggaggactt 180
gaccccgaga agtcttggtt gatctgtatc ggactcgacc ctaccgaaga ccacagacca 240
ggggggctga gagccacaga ccaggggggc tgagagccac agcagcgggg accgagaggc 300
cctaagggcc caggggcccc aaggaggacg aggcggcccg agccgccggg gcgcgcggct 360
atgatggtgc actgtgccgg ctgtgagcgg cccatcctcg accgctttct gctgaacgtg 420
ctggaccgcg cgtggcatat caaatgtgtt caatgctgcg agtgcaaaac caacctctcg 480
gagaagtgtt tctcccggga gggcaagctg tactgtaaaa acgacttctt caggcgcttt 540
ggcacaaagt gcgccggctg tgcgcaaggt atctctccca gcgacctggt gcgcaaggcc 600
cggagcaaag tcttccacct caactgcttc acctgcatgg tgtgcaacaa gcagctgtcc 660
accggagaag aactctacgt gatcgacgag aacaagtttg tgtgcaagga cgactactta 720
agctcctcta gtctcaaaga gggaagcctc aactcagtgt catcctgtac ggaccgcagt 780
ttgtctccgg acctccaaga tccgttacag gacgacccca aagagaccga caactcgacc 840
tcatcggaca aggagaccgc taacaacgag aatgaggaac agaactccgg caccaaacgg 900
cgcggcccgc gcactaccat caaggccaag cagctggaga cgctcaaggc agccttcgca 960
gccacgccca agcccacgcg ccacatccgc gaacagctgg cacaagagac cggcctcaac 1020
atgagggtca ttcaggtgtg gtttcagaac cgaaggtcca aagaacgccg catgaaacag 1080
ctgagcgctc tgggcgcccg gagacacgcc ttcttccgga gtccgcggcg catgcgtccc 1140
ctgggcggcc gcttggacga gtctgagatg ttggggtcta ccccatatac ttattatgga 1200
gactaccaaa gcgactacta cgctccggga ggcaactacg atttcttcgc gcacggcccg 1260
ccgtcgcagg cacagtctcc ggccgactca agcttcttgg cagcatcggg acctggctcg 1320
acgccgcttg gcgcgctgga accaccgctg gctgggcctc acggcgcgga caaccctagg 1380
ttcaccgaca tgatctcgca cccggacacg cccagtccgg agccaggctt gcccggagcg 1440
ctgcacccca tgccgggaga ggtgttcagc ggcgggccca gcccgccctt ccccatgagc 1500
ggcaccagcg gctacagcgg acccctgtcg caccccaatc ctgagctcaa cgaagcggcc 1560
gtatggtaag gccgaggggc cgagttgacc cctgccacca agccccggac gccgcctggg 1620
taagccacaa gagtcttctc ttgagtttgc acccaccagg caactcgcat caccacccct 1680
cagagcttcg gcacgcgcct gcacagtttc tcgggaccaa agtcaatatt ctggagggtc 1740
gagattccaa gcacacccta gaagccctcc ggacccccac ccaaccatca cctctttgaa 1800
ttaagagggg gaggggatga gacaaggaac ggagatcgtg gtactacccc tccctgcgag 1860
ccgaggcatt gtggaatcct atttctcgct ttctcttttt aaaaagggga attc 1914

<210> 14
<211> 402
<212> PRT
<213> Rattus norvegicus

<400> 14

Met Met Val His Cys Ala Gly Cys Glu Arg Pro Ile Leu Asp Arg Phe
1               5                   10                  15

Leu Leu Asn Val Leu Asp Arg Ala Trp His Ile Lys Cys Val Gln Cys
            20                  25                  30

Cys Glu Cys Lys Thr Asn Leu Ser Glu Lys Cys Phe Ser Arg Glu Gly
        35                  40                  45

Lys Leu Tyr Cys Lys Asn Asp Phe Phe Arg Arg Phe Gly Thr Lys Cys
    50                  55                  60

Ala Gly Cys Ala Gln Gly Ile Ser Pro Ser Asp Leu Val Arg Lys Ala
65                  70                  75                  80

Arg Ser Lys Val Phe His Leu Asn Cys Phe Thr Cys Met Val Cys Asn
                85                  90                  95

Lys Gln Leu Ser Thr Gly Glu Glu Leu Tyr Val Ile Asp Glu Asn Lys
            100                 105                 110

Phe Val Cys Lys Asp Asp Tyr Leu Ser Ser Ser Ser Leu Lys Glu Gly
        115                 120                 125

Ser Leu Asn Ser Val Ser Ser Cys Thr Asp Arg Ser Leu Ser Pro Asp
    130                 135                 140

Leu Gln Asp Pro Leu Gln Asp Asp Pro Lys Glu Thr Asp Asn Ser Thr
145                 150                 155                 160

Ser Ser Asp Lys Glu Thr Ala Asn Asn Glu Asn Glu Glu Gln Asn Ser
                165                 170                 175

Gly Thr Lys Arg Arg Gly Pro Arg Thr Thr Ile Lys Ala Lys Gln Leu
            180                 185                 190

Glu Thr Leu Lys Ala Ala Phe Ala Ala Thr Pro Lys Pro Thr Arg His
        195                 200                 205

Ile Arg Glu Gln Leu Ala Gln Glu Thr Gly Leu Asn Met Arg Val Ile
    210                 215                 220

Gln Val Trp Phe Gln Asn Arg Arg Ser Lys Glu Arg Arg Met Lys Gln
225                 230                 235                 240

Leu Ser Ala Leu Gly Ala Arg Arg His Ala Phe Phe Arg Ser Pro Arg
                245                 250                 255

Arg Met Arg Pro Leu Gly Gly Arg Leu Asp Glu Ser Glu Met Leu Gly

260                 265                 270

Ser Thr Pro Tyr Thr Tyr Tyr Gly Asp Tyr Gln Ser Asp Tyr Tyr Ala
        275                 280                 285

Pro Gly Gly Asn Tyr Asp Phe Phe Ala His Gly Pro Pro Ser Gln Ala
    290                 295                 300

Gln Ser Pro Ala Asp Ser Ser Phe Leu Ala Ala Ser Gly Pro Gly Ser
305                 310                 315                 320

Thr Pro Leu Gly Ala Leu Glu Pro Pro Leu Ala Gly Pro His Gly Ala
                325                 330                 335

Asp Asn Pro Arg Phe Thr Asp Met Ile Ser His Pro Asp Thr Pro Ser
            340                 345                 350

Pro Glu Pro Gly Leu Pro Gly Ala Leu His Pro Met Pro Gly Glu Val
        355                 360                 365

Phe Ser Gly Gly Pro Ser Pro Pro Phe Pro Met Ser Gly Thr Ser Gly
    370                 375                 380

Tyr Ser Gly Pro Leu Ser His Pro Asn Pro Glu Leu Asn Glu Ala Ala
385                 390                 395                 400

Val Trp

<210>  15
<211>  4156
<212>  DNA
<213>  Mus musculus

<400>  15
gagttcagag acagagaaag gcgctgtcag actacgctcc acttggggct cgcttgccgc      60
gggtttccgc gcacccacac tttctgtggt ggtggggaca ccctgcccca atccaggccc     120
ccattctgcc caccaccccc ccgccgggat ttgagcgcca ttagctcgcg ccagtaccgg     180
gatcttcgct cagctcgcct gagtcccgcc cgctggattc gaggacccgc ttggatgctt     240
cgcctccgag cgccctcgga agatgggccg gcagctacaa gcctaaagac gtcaagggct     300
taccagtctc caaggcaaac ccggagctct aagcccgctg ttcttccgtg gaggagatgg     360
cttccagccc actgccaggg cccaatgata tcctacttgc atcgccatcg agcgccttcc     420
agcccgacgc attgagccaa ccgcggccgg gtcacgccaa ccttaaaccc aaccaggtgg     480
gccaggtgat cctctatggc attcccatcg tgtccttggt gatcgacggg caggagcgcc     540
tgtgcctggc gcagatctcc aacactcttc tcaagaactt cagctataac gagatccaca     600
accgccgagt ggctctgggc atcacgtgcg tgcagtgcac acctgtccaa ctggagatcc     660
tgcggcgggc cggggccatg cccatctctt cgcggcgttg tggcatgatc accaagcgtg     720

aggccgagcg cctttgtaag tcattcctgg gcgaaaatcg gccacccaag ctgccggaca 780
acttcgcctt cgacgtgtcg cacgagtgtg cctggggctg ccgcggcagc ttcatcccgg 840
cgcgctacaa cagctctcgc gccaagtgca tcaagtgtag ctactgcaac atgtacttct 900
cccccaacaa gttcattttc cactcccacc gcaccccaga cgccaagtac acacagccag 960
acgcggctaa cttcaattcc tggcgccgcc atctcaagct cacagacaag agccctcagg 1020
acgagctagt cttcgcctgg gaggacgtca aggccatgtt caacggtggc agccgcaagc 1080
gcgcgctacc tcaacccagc gcgcacccgg cctgtcaccc actcagttcc gtcaaggctg 1140
ctgcggtggc ggccgcagcc gcagtggccg ggggcggagg cctgctgggc ccgcacctgc 1200
tgggggctcc acccccgcca ccgccgccac cacccttggc tgagctggcg ggcgcacctc 1260
acgcccatca caagcggccg cgcttcgacg acgatgacga ttccctccag gaagctgccg 1320
tggtggctgc agccagcctc tcggcagccg cagccagcct ctcggtggcc gcagccacag 1380
gcggcgccgg gccaggcgca ggtggccccg ggggtggctg cgtggccggc gtgggcgtgg 1440
gtgccagtgc gggggctggt gcagcagctg gcaccaaagg tccacgcagc tacccagtca 1500
tcccagtgcc cagcaagggt tcgttcgggg gcgtgctaca gaagttcccg ggctgcgggg 1560
gcctcttccc gcatccttac accttcccgg cagcggccgc agccttcggc ttgtgccaca 1620
agaaggagga cgcggggaca gcggccgagg ccctgggggg agcgggcgcg gggagcgcag 1680
gtgcggcgcc caaggcaggg ctgtcgggtc tcttctggcc cgcgggtcgc aaggacgcct 1740
tctaccctcc cttctgcatg ttctggccac cgcggacccc cggcgggctg cccgtgccca 1800
cctacctaca gcccccgccg cagcccccgt ctgcgctcgg ctgcgcgctg ggtgatagcc 1860
cggccctgct gcgtcaggcc ttcctggacc tggccgagcc gggcggtgca ggtggcagcg 1920
ccgaggcagc gccccctccg ggccaacctc cccccgtggt ggccaatggc cctggctccg 1980
gtcctccagc tactgggggc actggagcac gcgacacgct cttcgagtcg cccccgggcg 2040
gcagcggcgg ggactgcagc gccgggtcca cgccacccgc agagcaagga gtgacgtccg 2100
ggaccgggtc tgcgtcctcc ggagcaggct ctgtgggcac ccgagtgccg gctccccatc 2160
acccgcacct cctggaaggg cgcaaggcgg gcggcggcag ctaccaccat tccagcgcct 2220
tccgtccggt gggcggcaag gacgacgcag aaagcctggc caagctgcac ggggcgtcgg 2280
cgggcacacc ccactcagcc ccagcgcatc accatcacca ccaccatcac ccgcaccatc 2340
accaccatca ccctccgcag ccgccgtcgc cactgctgct gttgcagccc cagcccgatg 2400
agccggggtc ggagcgccac cacccagccc cgccaccccc gccaccgccg ccccctctgg 2460
ccccgcagcc gcaccaccga ggccttctgt cccccgaggg caccagctgc agctacccca 2520
gtgaggacag ctctgaagac gaggaggacg aggaggaaga gcaggaggtg gacgtggagg 2580
gccacaagcc actcgaaggc gaggaagagg aggacggtcg cgatcctgaa gatgaggagg 2640
aagaagatga ggagacccgg gtccttctag gagactccct ggttggcggt ggccggttcc 2700
tccagggccg agggctatcg gagaagggga gcggtcggga ccgcacgacg cccgccgtgg 2760

gtgctttccc tctagcgctg aactcctcca ggctgctaca agaggatggg aaactggggg 2820
actctggagg ctcggacctg ccggcgcccc cgcccccacc cctggccccc cagaaagcaa 2880
gcagcagtgg gggcagcagg ccaggcagcc ctgtccacca tccatcactg gaggaggagc 2940
cctcgtacaa agataatcag aaacctaagg aaaacaacca agttattata tctacaaagg 3000
atgacaactt ctcagataag aacaagggac atggcttctt catcacagat tctgattctt 3060
ctggagactt ttggagagaa agatcaggtg aacatacaca agaaaccaat tcacctcatt 3120
cgctcaaaaa ggatgtagaa aacatgggaa aagaggaact tcagaaggtt ttgtttgagc 3180
aaatagattt gcggaggcgg ctggagcaag aattccaagt gttaaaagga aatacgtcct 3240
tcccagtctt caataacttt caggatcaga tgaaaaggga actggcttac cgagaagaga 3300
tggtgcaaca gttacaaatc atcccctatg cagcaagctt gatcaggaaa gagaagcttg 3360
gcgcccatct cagcaaaagc taaaaggcga cagacccact cactcttgtt ctgtaagata 3420
cagcctacca ctgagcactt cggacctgca gaaagaagaa ctgcaaactg aaagggcttg 3480
ggcaccaaaa ccaggttcga gagaagccaa ggacaagtga cctcgcgcca gcatgggcaa 3540
ccatgtaaaa tagactgtgg cggccattca ttaggagggg ggggaggggg gagccaacca 3600
gagcggtcaa tgcttgtcac atcttttggt ggaaccaaag tttgaatatt tgtgttttga 3660
aagcatagct gatcccagag ggtaggaagt gctgagtggg gaaatgtttg tgtggtctct 3720
ttggcgccat acgattatcc tttgctcttc cggaagtaat tcatagtgga aagaggacag 3780
aatggggact taggtttaga caaacctgct ttccatacca agctggacag acacacatgg 3840
ccccttcctc tctggtactt tgcctgctgt atgaagattg tattcctctg gaaatatttt 3900
acagtttaat attgagtgta attaagaata taatcatgtt atcaaaaatg gtatttaact 3960
ctgttgtagt ttctttaaca ttcatgtgga taaaaagttt ataataaaaa aactatgacg 4020
taatagatgt gttcatgtag ttaagtgcat atatgcttgg gggcaactca gaaacgtaat 4080
gctttttaga gttattttgg cataaagtat ttgaatataa ttatttttga aaacaaaaaa 4140
aaaaaaaaaa aaaaaa 4156

<210> 16
<211> 1008
<212> PRT
<213> Mus musculus

<400> 16

Met Ala Ser Ser Pro Leu Pro Gly Pro Asn Asp Ile Leu Leu Ala Ser
1               5                   10                  15

Pro Ser Ser Ala Phe Gln Pro Asp Ala Leu Ser Gln Pro Arg Pro Gly
            20                  25                  30

His Ala Asn Leu Lys Pro Asn Gln Val Gly Gln Val Ile Leu Tyr Gly
        35                  40                  45

Ile Pro Ile Val Ser Leu Val Ile Asp Gly Gln Glu Arg Leu Cys Leu
    50                  55                  60

Ala Gln Ile Ser Asn Thr Leu Leu Lys Asn Phe Ser Tyr Asn Glu Ile
65                  70                  75                  80

His Asn Arg Arg Val Ala Leu Gly Ile Thr Cys Val Gln Cys Thr Pro
                85                  90                  95

Val Gln Leu Glu Ile Leu Arg Arg Ala Gly Ala Met Pro Ile Ser Ser
            100                 105                 110

Arg Arg Cys Gly Met Ile Thr Lys Arg Glu Ala Glu Arg Leu Cys Lys
        115                 120                 125

Ser Phe Leu Gly Glu Asn Arg Pro Pro Lys Leu Pro Asp Asn Phe Ala
    130                 135                 140

Phe Asp Val Ser His Glu Cys Ala Trp Gly Cys Arg Gly Ser Phe Ile
145                 150                 155                 160

Pro Ala Arg Tyr Asn Ser Ser Arg Ala Lys Cys Ile Lys Cys Ser Tyr
                165                 170                 175

Cys Asn Met Tyr Phe Ser Pro Asn Lys Phe Ile Phe His Ser His Arg
            180                 185                 190

Thr Pro Asp Ala Lys Tyr Thr Gln Pro Asp Ala Ala Asn Phe Asn Ser
        195                 200                 205

Trp Arg Arg His Leu Lys Leu Thr Asp Lys Ser Pro Gln Asp Glu Leu
    210                 215                 220

Val Phe Ala Trp Glu Asp Val Lys Ala Met Phe Asn Gly Gly Ser Arg
225                 230                 235                 240

Lys Arg Ala Leu Pro Gln Pro Ser Ala His Pro Ala Cys His Pro Leu
                245                 250                 255

Ser Ser Val Lys Ala Ala Ala Val Ala Ala Ala Ala Ala Val Ala Gly
            260                 265                 270

Gly Gly Gly Leu Leu Gly Pro His Leu Leu Gly Ala Pro Pro Pro Pro
        275                 280                 285

Pro Pro Pro Pro Pro Leu Ala Glu Leu Ala Gly Ala Pro His Ala His
    290                 295                 300

His Lys Arg Pro Arg Phe Asp Asp Asp Asp Asp Ser Leu Gln Glu Ala
305                 310                 315                 320

Ala Val Val Ala Ala Ala Ser Leu Ser Ala Ala Ala Ala Ser Leu Ser
                325                 330                 335

Val Ala Ala Ala Thr Gly Gly Ala Gly Pro Gly Ala Gly Gly Pro Gly
            340                 345                 350

Gly Gly Cys Val Ala Gly Val Gly Val Gly Ala Ser Ala Gly Ala Gly
        355                 360                 365

Ala Ala Ala Gly Thr Lys Gly Pro Arg Ser Tyr Pro Val Ile Pro Val
    370                 375                 380

Pro Ser Lys Gly Ser Phe Gly Gly Val Leu Gln Lys Phe Pro Gly Cys
385                 390                 395                 400

Gly Gly Leu Phe Pro His Pro Tyr Thr Phe Pro Ala Ala Ala Ala Ala
                405                 410                 415

Phe Gly Leu Cys His Lys Lys Glu Asp Ala Gly Thr Ala Ala Glu Ala
            420                 425                 430

Leu Gly Gly Ala Gly Ala Gly Ser Ala Gly Ala Ala Pro Lys Ala Gly
        435                 440                 445

Leu Ser Gly Leu Phe Trp Pro Ala Gly Arg Lys Asp Ala Phe Tyr Pro
    450                 455                 460

Pro Phe Cys Met Phe Trp Pro Pro Arg Thr Pro Gly Gly Leu Pro Val
465                 470                 475                 480

Pro Thr Tyr Leu Gln Pro Pro Pro Gln Pro Pro Ser Ala Leu Gly Cys
                485                 490                 495

Ala Leu Gly Asp Ser Pro Ala Leu Leu Arg Gln Ala Phe Leu Asp Leu
            500                 505                 510

Ala Glu Pro Gly Gly Ala Gly Gly Ser Ala Glu Ala Ala Pro Pro Pro
        515                 520                 525

Gly Gln Pro Pro Pro Val Val Ala Asn Gly Pro Gly Ser Gly Pro Pro
    530                 535                 540

Ala Thr Gly Gly Thr Gly Ala Arg Asp Thr Leu Phe Glu Ser Pro Pro
545                 550                 555                 560

Gly Gly Ser Gly Gly Asp Cys Ser Ala Gly Ser Thr Pro Pro Ala Glu
                565                 570                 575

Gln Gly Val Thr Ser Gly Thr Gly Ser Ala Ser Ser Gly Ala Gly Ser
            580                 585                 590

Val Gly Thr Arg Val Pro Ala Pro His His Pro His Leu Leu Glu Gly
        595                 600                 605

Arg Lys Ala Gly Gly Gly Ser Tyr His His Ser Ser Ala Phe Arg Pro
    610                 615                 620

Val Gly Gly Lys Asp Asp Ala Glu Ser Leu Ala Lys Leu His Gly Ala
625                 630                 635                 640

Ser Ala Gly Thr Pro His Ser Ala Pro Ala His His His His His His
                645                 650                 655

His His Pro His His His His His His Pro Pro Gln Pro Pro Ser Pro
            660                 665                 670

Leu Leu Leu Leu Gln Pro Gln Pro Asp Glu Pro Gly Ser Glu Arg His
        675                 680                 685

His Pro Ala Pro Pro Pro Pro Pro Pro Pro Pro Pro Leu Ala Pro Gln
    690                 695                 700

Pro His His Arg Gly Leu Leu Ser Pro Glu Gly Thr Ser Cys Ser Tyr
705                 710                 715                 720

Pro Ser Glu Asp Ser Ser Glu Asp Glu Glu Asp Glu Glu Glu Glu Gln
                725                 730                 735

Glu Val Asp Val Glu Gly His Lys Pro Leu Glu Gly Glu Glu Glu Glu
            740                 745                 750

Asp Gly Arg Asp Pro Glu Asp Glu Glu Glu Glu Asp Glu Glu Thr Arg
        755                 760                 765

Val Leu Leu Gly Asp Ser Leu Val Gly Gly Gly Arg Phe Leu Gln Gly
    770                 775                 780

Arg Gly Leu Ser Glu Lys Gly Ser Gly Arg Asp Arg Thr Thr Pro Ala
785                 790                 795                 800

Val Gly Ala Phe Pro Leu Ala Leu Asn Ser Ser Arg Leu Leu Gln Glu
                805                 810                 815

Asp Gly Lys Leu Gly Asp Ser Gly Gly Ser Asp Leu Pro Ala Pro Pro
            820                 825                 830

Pro Pro Pro Leu Ala Pro Gln Lys Ala Ser Ser Ser Gly Gly Ser Arg
        835                 840                 845

Pro Gly Ser Pro Val His His Pro Ser Leu Glu Glu Glu Pro Ser Tyr
    850                 855                 860

Lys Asp Asn Gln Lys Pro Lys Glu Asn Asn Gln Val Ile Ile Ser Thr
865                 870                 875                 880

Lys Asp Asp Asn Phe Ser Asp Lys Asn Lys Gly His Gly Phe Phe Ile
                885                 890                 895

Thr Asp Ser Asp Ser Ser Gly Asp Phe Trp Arg Glu Arg Ser Gly Glu
            900                 905                 910

His Thr Gln Glu Thr Asn Ser Pro His Ser Leu Lys Lys Asp Val Glu
        915                 920                 925

Asn Met Gly Lys Glu Glu Leu Gln Lys Val Leu Phe Glu Gln Ile Asp
    930                 935                 940

Leu Arg Arg Arg Leu Glu Gln Glu Phe Gln Val Leu Lys Gly Asn Thr
945                 950                 955                 960

Ser Phe Pro Val Phe Asn Asn Phe Gln Asp Gln Met Lys Arg Glu Leu
                965                 970                 975

Ala Tyr Arg Glu Glu Met Val Gln Gln Leu Gln Ile Ile Pro Tyr Ala
            980                 985                 990

Ala Ser Leu Ile Arg Lys Glu Lys  Leu Gly Ala His Leu  Ser Lys Ser
        995                 1000                1005

<210>  17
<211>  3045
<212>  DNA
<213>  Homo sapiens

<400>  17
atggcttcca gtccgctgcc agggcccaac gacatcctgc tggcgtcgcc gtcgagcgcc      60

ttccagcccg acacgctgag ccagccgcgg ccagggcacg ccaacctcaa acccaaccag     120

gtgggccagg tgatcctcta cggcattccc atcgtgtcgt tggtgatcga cgggcaagag     180

cgcctgtgcc tggcgcagat ctccaacact ctgctcaaga acttcagcta caacgagatc     240

cacaaccgtc gcgtggcact gggcatcacg tgtgtgcagt gcacgccggt gcaactggag     300

atcctgcggc gtgccggggc catgcccatc tcatcgcgcc gctgcggcat gatcaccaaa     360

cgcgaggccg agcgtctgtg caagtcgttc ctgggcgaaa acaggccgcc caagctgcca     420

gacaatttcg ccttcgacgt gtcacacgag tgcgcctggg gctgccgcgg cagcttcatt     480

cccgcgcgct acaacagctc gcgcgccaag tgcatcaaat gcagctactg caacatgtac     540

ttctcgccca acaagttcat tttccactcc caccgcacgc ccgacgccaa gtacactcag     600

ccagacgcag ccaacttcaa ctcgtggcgc cgtcatctca agctcaccga caagagtccc     660

caggacgagc tggtcttcgc ctgggaggac gtcaaggcca tgttcaacgg cggcagccgc     720

aagcgcgcac tgccccagcc gggcgcgcac cccgcctgcc acccgctcag ctctgtgaag     780

gcggccgccg tggccgccgc ggccgcggtg gccggaggcg ggggtctgct gggcccccac 840
ctgctgggtg cgcccccgcc gccgccgccg ccaccgccgc ccttggcaga gctggctggt 900
gccccgcacg cccatcacaa gcggccgcgc ttcgacgacg acgacgactc cttgcaggag 960
gccgccgtag tggccgccgc cagcctctcg gccgcagccg ccagcctctc tgtggctgct 1020
gcttcgggcg gcgcggggac tggtgggggc ggcgctgggg gtggctgtgt ggccggcgtg 1080
ggcgtgggcg cgggcgcggg ggcgggtgcc ggggcagggg ccaaaggccc gcgcagctac 1140
ccagtcatcc cggtgcccag caagggctcg ttcgggggcg tcctgcagaa gttcccgggc 1200
tgcggcgggc tcttcccgca cccctacacc ttccctgccg cggccgccgc cttcagcttg 1260
tgccataaga aagaggatgc gggtgccgcc gctgaggccc tggggggcgc gggcgcaggc 1320
ggcgcgggcg cggcgcccaa ggccggcttg tccggcctct tctggcccgc gggccgcaag 1380
gacgccttct atccgccctt ctgcatgttc tggccgccgc ggacccctgg cgggctcccg 1440
gtgcccacct acctgcagcc cccgcctcag ccgccctcgg cgctaggctg cgcgctaggc 1500
gaaagcccgg ccctgctgcg ccaggccttc ctggacctgg ctgagccagg cggtgctgct 1560
gggagcgccg aggccgcgcc cccgccgggg cagcccccgc aggtagtggc caacggcccg 1620
ggctccggcc cacctcctcc tgccgggggc gccggctctc gcgacgcgct cttcgagtcg 1680
cccccgggcg gcagcggcgg ggactgcagc gcgggctcca cgccgcccgc ggactctgtg 1740
gcagctgccg gggcaggggc cgcggccgcc gggtctggcc ccgcgggctc ccgggttccg 1800
gcgccccacc atccgcacct tctggagggg cgcaaagcgg gcggtggcag ctaccaccat 1860
tccagcgcct tccggccagt gggcggcaag gacgacgcgg agagcctggc caagctgcac 1920
ggggcgtcgg cgggcgcgcc ccactcggcc cagacgcatc cccaccacca tcaccaccct 1980
caccaccacc accaccacca ccaccccccg cagccgccgt cgccgcttct gctgctgccc 2040
ccgcagcccg acgagccggg ttccgagcgc caccacccgg ccccgccgcc gccgccgccg 2100
ccgcccccgc cgccccctct ggcccagcac ccgcaccacc gaggccttct gtctcccggg 2160
ggaaccagct gctgctaccc cagcgaggac agctccgagg acgaggacga cgaggaagaa 2220
gagcaggagg tggacgtgga gggccacaag ccccccgagg gcgaggaaga ggaggaaggt 2280
cgagaccctg acgacgacga ggaagaggac gaggagacgg aggtcctact cggcgacccc 2340
ttagtcgggg gcggccggtt cctccagggc cgagggccgt cggagaaggg gagcagccgg 2400
gaccgcgcgc cggccgtcgc gggcgcgttc ccgctcggcc tgaactcctc caggctgctg 2460
caggaagacg ggaaactcgg ggaccccggc tcggacctgc ccccgccccc gccgccgccc 2520
ctggcccccc agaaggcgag tggcggcggc agcagcagcc cgggcagccc agttcaccat 2580
ccatcactgg aggagcagcc ctcctacaaa gatagtcaga aaactaagga aaataaccaa 2640
gtaattgtat ctacaaagga tgacaacgtt ctagataaga acaaggagca tagctttttc 2700
atcacagact ctgatgcttc tggaggagat ttttggagag aaagatcagg tgaacataca 2760
caagaaacca actcacctca ttcactgaaa aaggatgtag aaaatatggg gaaagaagaa 2820

cttcagaagg ttttatttga acaaatagat ttacggagac gactggaaca agaattccag 2880

gtgttaaaag gaaacacatc tttcccagta ttcaataatt ttcaggatca gatgaaaagg 2940

gagctagcct accgagaaga aatggtgcaa cagttacaaa ttatccccta tgcagcaagt 3000

ttgatcagga aagaaaagct tggcgcccat ctcagcaaaa gctaa 3045

<210> 18
<211> 1014
<212> PRT
<213> Homo sapiens

<400> 18

Met Ala Ser Ser Pro Leu Pro Gly Pro Asn Asp Ile Leu Leu Ala Ser
1               5                   10                  15

Pro Ser Ser Ala Phe Gln Pro Asp Thr Leu Ser Gln Pro Arg Pro Gly
            20                  25                  30

His Ala Asn Leu Lys Pro Asn Gln Val Gly Gln Val Ile Leu Tyr Gly
        35                  40                  45

Ile Pro Ile Val Ser Leu Val Ile Asp Gly Gln Glu Arg Leu Cys Leu
    50                  55                  60

Ala Gln Ile Ser Asn Thr Leu Leu Lys Asn Phe Ser Tyr Asn Glu Ile
65                  70                  75                  80

His Asn Arg Arg Val Ala Leu Gly Ile Thr Cys Val Gln Cys Thr Pro
                85                  90                  95

Val Gln Leu Glu Ile Leu Arg Arg Ala Gly Ala Met Pro Ile Ser Ser
            100                 105                 110

Arg Arg Cys Gly Met Ile Thr Lys Arg Glu Ala Glu Arg Leu Cys Lys
        115                 120                 125

Ser Phe Leu Gly Glu Asn Arg Pro Pro Lys Leu Pro Asp Asn Phe Ala
    130                 135                 140

Phe Asp Val Ser His Glu Cys Ala Trp Gly Cys Arg Gly Ser Phe Ile
145                 150                 155                 160

Pro Ala Arg Tyr Asn Ser Ser Arg Ala Lys Cys Ile Lys Cys Ser Tyr
                165                 170                 175

Cys Asn Met Tyr Phe Ser Pro Asn Lys Phe Ile Phe His Ser His Arg
            180                 185                 190

Thr Pro Asp Ala Lys Tyr Thr Gln Pro Asp Ala Ala Asn Phe Asn Ser
        195                 200                 205

Trp Arg Arg His Leu Lys Leu Thr Asp Lys Ser Pro Gln Asp Glu Leu
    210                 215                 220

Val Phe Ala Trp Glu Asp Val Lys Ala Met Phe Asn Gly Gly Ser Arg
225                 230                 235                 240

Lys Arg Ala Leu Pro Gln Pro Gly Ala His Pro Ala Cys His Pro Leu
                245                 250                 255

Ser Ser Val Lys Ala Ala Ala Val Ala Ala Ala Ala Ala Val Ala Gly
            260                 265                 270

Gly Gly Gly Leu Leu Gly Pro His Leu Leu Gly Ala Pro Pro Pro Pro
        275                 280                 285

Pro Pro Pro Pro Pro Pro Leu Ala Glu Leu Ala Gly Ala Pro His Ala
    290                 295                 300

His His Lys Arg Pro Arg Phe Asp Asp Asp Asp Asp Ser Leu Gln Glu
305                 310                 315                 320

Ala Ala Val Val Ala Ala Ala Ser Leu Ser Ala Ala Ala Ala Ser Leu
                325                 330                 335

Ser Val Ala Ala Ala Ser Gly Gly Ala Gly Thr Gly Gly Gly Gly Ala
            340                 345                 350

Gly Gly Gly Cys Val Ala Gly Val Gly Val Gly Ala Gly Ala Gly Ala
        355                 360                 365

Gly Ala Gly Ala Gly Ala Lys Gly Pro Arg Ser Tyr Pro Val Ile Pro
    370                 375                 380

Val Pro Ser Lys Gly Ser Phe Gly Gly Val Leu Gln Lys Phe Pro Gly
385                 390                 395                 400

Cys Gly Gly Leu Phe Pro His Pro Tyr Thr Phe Pro Ala Ala Ala Ala
                405                 410                 415

Ala Phe Ser Leu Cys His Lys Lys Glu Asp Ala Gly Ala Ala Ala Glu
            420                 425                 430

Ala Leu Gly Gly Ala Gly Ala Gly Gly Ala Gly Ala Ala Pro Lys Ala
        435                 440                 445

Gly Leu Ser Gly Leu Phe Trp Pro Ala Gly Arg Lys Asp Ala Phe Tyr
    450                 455                 460

Pro Pro Phe Cys Met Phe Trp Pro Pro Arg Thr Pro Gly Gly Leu Pro
465                 470                 475                 480

Val Pro Thr Tyr Leu Gln Pro Pro Pro Gln Pro Pro Ser Ala Leu Gly
                485                 490                 495

Cys Ala Leu Gly Glu Ser Pro Ala Leu Leu Arg Gln Ala Phe Leu Asp
            500                 505                 510

Leu Ala Glu Pro Gly Gly Ala Ala Gly Ser Ala Glu Ala Ala Pro Pro
        515                 520                 525

Pro Gly Gln Pro Pro Gln Val Val Ala Asn Gly Pro Gly Ser Gly Pro
    530                 535                 540

Pro Pro Pro Ala Gly Gly Ala Gly Ser Arg Asp Ala Leu Phe Glu Ser
545                 550                 555                 560

Pro Pro Gly Gly Ser Gly Gly Asp Cys Ser Ala Gly Ser Thr Pro Pro
                565                 570                 575

Ala Asp Ser Val Ala Ala Ala Gly Ala Gly Ala Ala Ala Ala Gly Ser
            580                 585                 590

Gly Pro Ala Gly Ser Arg Val Pro Ala Pro His His Pro His Leu Leu
        595                 600                 605

Glu Gly Arg Lys Ala Gly Gly Gly Ser Tyr His His Ser Ser Ala Phe
    610                 615                 620

Arg Pro Val Gly Gly Lys Asp Asp Ala Glu Ser Leu Ala Lys Leu His
625                 630                 635                 640

Gly Ala Ser Ala Gly Ala Pro His Ser Ala Gln Thr His Pro His His
                645                 650                 655

His His His Pro His His His His His His His His Pro Pro Gln Pro
            660                 665                 670

Pro Ser Pro Leu Leu Leu Leu Pro Pro Gln Pro Asp Glu Pro Gly Ser
        675                 680                 685

Glu Arg His His Pro Ala Pro Pro Pro Pro Pro Pro Pro Pro Pro Pro
    690                 695                 700

Pro Pro Leu Ala Gln His Pro His His Arg Gly Leu Leu Ser Pro Gly
705                 710                 715                 720

Gly Thr Ser Cys Cys Tyr Pro Ser Glu Asp Ser Ser Glu Asp Glu Asp
                725                 730                 735

Asp Glu Glu Glu Glu Gln Glu Val Asp Val Glu Gly His Lys Pro Pro
            740                 745                 750

Glu Gly Glu Glu Glu Glu Glu Gly Arg Asp Pro Asp Asp Asp Glu Glu
        755                 760                 765

Glu Asp Glu Glu Thr Glu Val Leu Leu Gly Asp Pro Leu Val Gly Gly
    770                 775                 780

Gly Arg Phe Leu Gln Gly Arg Gly Pro Ser Glu Lys Gly Ser Ser Arg
785                 790                 795                 800

Asp Arg Ala Pro Ala Val Ala Gly Ala Phe Pro Leu Gly Leu Asn Ser
                805                 810                 815

Ser Arg Leu Leu Gln Glu Asp Gly Lys Leu Gly Asp Pro Gly Ser Asp
            820                 825                 830

Leu Pro Pro Pro Pro Pro Pro Pro Leu Ala Pro Gln Lys Ala Ser Gly
        835                 840                 845

Gly Gly Ser Ser Ser Pro Gly Ser Pro Val His His Pro Ser Leu Glu
    850                 855                 860

Glu Gln Pro Ser Tyr Lys Asp Ser Gln Lys Thr Lys Glu Asn Asn Gln
865                 870                 875                 880

Val Ile Val Ser Thr Lys Asp Asp Asn Val Leu Asp Lys Asn Lys Glu
                885                 890                 895

His Ser Phe Phe Ile Thr Asp Ser Asp Ala Ser Gly Gly Asp Phe Trp
            900                 905                 910

Arg Glu Arg Ser Gly Glu His Thr Gln Glu Thr Asn Ser Pro His Ser
        915                 920                 925

Leu Lys Lys Asp Val Glu Asn Met Gly Lys Glu Glu Leu Gln Lys Val
    930                 935                 940

Leu Phe Glu Gln Ile Asp Leu Arg Arg Arg Leu Glu Gln Glu Phe Gln
945                 950                 955                 960

Val Leu Lys Gly Asn Thr Ser Phe Pro Val Phe Asn Asn Phe Gln Asp
                965                 970                 975

Gln Met Lys Arg Glu Leu Ala Tyr Arg Glu Glu Met Val Gln Gln Leu
            980                 985                 990

Gln Ile Ile Pro Tyr Ala Ala Ser  Leu Ile Arg Lys Glu  Lys Leu Gly
        995                 1000                 1005

Ala His  Leu Ser Lys Ser
    1010

<210> 19
<211> 3123
<212> DNA
<213> Mus musculus

<400> 19
gctctctccg acccgcaggc ccacgggagc ctgagctccg cctccccagg gcgcggaagc 60

tggcgaagcc ccagggattc ccatttatag cttggtttcc actcagctca gtccctccag 120

gactcgggct gagcaagttt cttccattcc cttctctcct ccctccaccc ccttctcctc 180

ctccttctcc ttcttttctt cctcctcatt cccgcctccc cttcaacctc agcagggtgc 240

aggtgtccaa ctcgaacaag ggccccaact tggactcaga tgttcccact ctcagacccc 300

ctgataatgc aggggcgccc gcctgctgcg cggacagcta ccctgagcat ccgtagccgt 360

ccgcacacaa ggcgcgggag tttctcaatg ggaagaggcc gggactctag gaggcggggc 420

gaataggatt cctcccgcct agtgggtccc tcgcagtcct agggttgcaa cccttgagcg 480

gtagagaaca ccggagactg cggatgagcc agatttcggg gacataaaat cttccagccc 540

ggagagaatt gtgtgcagag aggggctcca gtccagcgtg gtgtgagagg cgtgctatca 600

agaaagaagt tggaggggaa ccagtgcaac cctaactctg cgagatcttg gggtacacac 660

actcgggatg ctggcctccg ccctcctcgt tttcctttgc tgtttcaaag gacatgcagg 720

ctcatcgccc catttcctac aacagccaga ggacatggtg gtgctgttgg gggaggaagc 780

ccggctgccc tgcgctctgg gcgcgtacag ggggctcgtg cagtggacta aggatgggct 840

ggctctaggg ggcgaaagag accttccagg gtggtcccgg tactggatat cggggaattc 900

agccagtggc cagcatgacc tccacattaa gcctgtggaa ttggaagatg aggcatcgta 960

tgagtgccag gcttcgcaag caggtctccg atcacgacca gcccaactgc acgtgatggt 1020

ccccccagaa gctccccagg tactaggcgg cccctctgtg tctctggttg ctggagttcc 1080

tggaaatctg acctgtcgga gtcgtgggga ttcacgacct gcccctgaac tactgtggtt 1140

ccgagatggg atccggctgg atgggagcag cttccaccag accacgctga aggacaaggc 1200

cactggaaca gtggaaaaca ccttattcct gaccccttcc agtcatgatg atggtgccac 1260

cttgatctgc agagcgcgaa gccaggccct gcccacaggg agggacacag ctgttacact 1320

gagccttcag tatcccccaa tggtgactct gtctgctgag ccccagactg tgcaggaggg 1380

agagaaggtg actttcctgt gtcaagccac tgcccagcct cctgtcactg gctacaggtg 1440

ggcgaagggg ggatccccgg tgcttggggc acgtgggcca aggttggagg tcgttgcaga 1500

tgccactttc ctgactgagc cggtgtcctg cgaggtcagc aacgcggtcg gaagcgccaa 1560

ccgcagcacc gcgctggaag tgttgtatgg acccattctg caggcaaaac ctaagtccgt 1620

gtccgtggac gtggggaaag atgcctcctt cagctgtgtc tggcgcggga acccacttcc 1680

acggataacc tggacccgca tgggtggctc tcaggtgctg agctccgggc ccacgctgcg 1740

gcttccgtcc gtggcactgg aggatgcggg cgactatgta tgcagggctg agccgaggag 1800

aacgggtctg ggaggcggca aagcgcaggc gaggctgact gtgaacgcac cccctgtagt 1860

gacagccctg caacctgcac cagcctttct gaggggtcct gctcgcctcc agtgtgtggt 1920

gtttgcctcc cctgccccag actcggtggt ttggtcttgg gacgagggct tcttggaggc 1980

aggctcactg ggcaggttcc tagtggaagc cttcccagcc ccggaagtgg aggggggaca 2040

gggccctggc cttatttctg tgctacacat ttccggaacc caggagtccg actttaccac 2100

cggcttcaac tgcagtgccc gcaaccggct aggagaggga cgagtccaga tccacttggg 2160

ccgtagagac ttgctgccta ctgtccggat tgtggctggt gcagcatctg cagccacctc 2220

tctccttatg gtcatcactg gagtggtcct ctgctgctgg cgccatggct ctctctctaa 2280

gcaaaagaac ttggtccgga tcccgggaag cagcgagggt tccagttcac gtggccctga 2340

ggaggagaca ggcagcagtg aggaccgggg tcccattgtg cacaccgacc acagtgattt 2400

ggttcttgag gaaaaagagg ctctggagac aaaggatcca accaacggtt actacagggt 2460

tcgaggggtc agtgtgagcc ttagccttgg ggaagctcct ggaggaggcc tcttcttgcc 2520

accgccctct ccgatcggtc tcccagggac tcctacttac tatgacttca agccacatct 2580

ggacttagtc cctccctgca gactgtacag agcgagggca ggttatctta ccacccccca 2640

tccccgtgcc ttcaccagct acatgaaacc cacatccttt ggacccccag aattgagctc 2700

tggaactccc cccttcccgt atgctacctt gtctccaccc agccaccagc gtctccagac 2760

tcatgtgtga atccatctct ccaagtgaag ggtcttggaa tcttctgttt gccatatagt 2820

gtgttgtcca gatttctggg gagtcagaac aagttgatga ccaacccctc caaaactgaa 2880

cattgaagga gggaaagatc attacaagca tcaggactgt tggtgtacac tcagttcagc 2940

caaagtggat tctccaagtg ggagcaatat ggccgctttc ccatgagaaa gacattcaag 3000

atggtgacta aatgactaaa tactttgcag agggacaaag atgggaacta gggatatgga 3060

tggaagtagt agagaagata tatgaccatc tgcatcaaga gaaaggataa cataagacaa 3120

atc 3123

<210> 20
<211> 700
<212> PRT
<213> Mus musculus

<400> 20

Met Leu Ala Ser Ala Leu Leu Val Phe Leu Cys Cys Phe Lys Gly His
1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly
        115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Gly Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys
225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys
        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu
545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Arg Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Glu Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  21
<211>  2847
<212>  DNA
<213>  Mus musculus

<400>  21
ggtccggaat tcccgggatg agaggggctc cagtccagcg tggtgtgaga ggcgtgctat      60

caagaaagaa gttggagggg aaccagtgca accctaactc tgcgagatct tggggtacac     120

acactcggga tgctggcctc cgccctcctc gttttccttt gctgtttcaa aggacatgca     180

ggctcatcgc cccatttcct acaacagcca gaggacatgg tggtgctgtt gggggaggaa     240

gcccggctgc cctgcgctct gggcgcgtac agggggctcg tgcagtggac taaggatggg     300

ctggctctag ggggcgaaag agaccttcca gggtggtccc ggtactggat atcggggaat     360

tcagccagtg gccagcatga cctccacatt aagcctgtgg aattggaaga tgaggcatcg     420

tatgagtgcc aggcttcgca agcaggtctc cgatcacgac cagcccaact gcacgtgatg     480

gtccccccag aagctcccca ggtactaggc ggcccctctg tgtctctggt tgctggagtt     540

cctggaaatc tgacctgtcg gagtcgtggg gattcacgac ctgcccctga actactgtgg     600

ttccgagatg ggatccggct ggatgggagc agcttccacc agaccacgct gaaggacaag     660

gccactggaa cagtggaaaa caccttattc ctgacccctt ccagtcatga tgatggtgcc     720

accttgatct gcagagcgcg aagccaggcc ctgcccacag ggagggacac agctgttaca     780

ctgagccttc agtatccccc aatggtgact ctgtctgctg agccccagac tgtgcaggag     840

ggagagaagg tgactttcct gtgtcaagcc actgcccagc ctcctgtcac tggctacagg     900

tgggcgaagg ggggatcccc ggtgcttggg gcacgtgggc caaggttgga ggtcgttgca     960

gatgccactt tcctgactga gccggtgtcc tgcgaggtca gcaacgcggt cggaagcgcc    1020

aaccgcagca ccgcgctgga agtgttgtat ggacccattc tgcaggcaaa acctaagtcc    1080

gtgtccgtgg acgtggggaa agatgcctcc ttcagctgtg tctggcgcgg gaacccactt    1140

ccacggataa cctggacccg catgggtggc tctcaggtgc tgagctccgg gcccacgctg 1200
cggcttccgt ccgtggcact ggaggatgcg ggcgactatg tatgcagggc tgagccgagg 1260
agaacgggtc tgggaggcgg caaagcgcag gcgaggctga ctgtgaacgc accccctgta 1320
gtgacagccc tgcaacctgc accagccttt ctgaggggtc ctgctcgcct ccagtgtgtg 1380
gtgtttgcct cccctgcccc agactcggtg gtttggtctt gggacgaggg cttcttggag 1440
gcaggctcac tgggcaggtt cctagtggaa gccttcccag ccccggaagt ggagggggga 1500
cagggccctg gccttatttc tgtgctacac atttccggaa cccaggagtc cgactttacc 1560
accggcttca actgcagtgc ccgcaaccgg ctaggagagg gacgagtcca gatccacttg 1620
ggccgtagag acttgctgcc tactgtccgg attgtggctg gtgcagcatc tgcagccacc 1680
tctctcctta tggtcatcac tggagtggtc ctctgctgct ggcgccatgg ctctctctct 1740
aagcaaaaga acttggtccg gatcccagga agcagcgagg gttccagttc acgtggccct 1800
gaggaggaga caggcagcag tgaggaccgg ggtcccattg tgcacaccga ccacagtgat 1860
ttggttcttg aggaaaaaga ggctctggag acaaaggatc caaccaacgg ttactacagg 1920
gttcgagggg tcagtgtgag ccttagcctt ggggaagctc ctggaggagg cctcttcttg 1980
ccaccgccct ctccgatcgg tctcccaggg actcctactt actatgactt caagccacat 2040
ctggacttag tccctccctg cagactgtac agagcgaggg caggttatct taccaccccc 2100
catccccgtg ccttcaccag ctacatgaaa cccacatcct ttggaccccc agaattgagc 2160
tctggaactc cccccttccc gtatgctacc ttgtctccac ccagccacca gcgtctccag 2220
actcatgtgt gaatccatct ctccaagtga agggtcttgg aatcttctgt ttgccatata 2280
gtgtgttgtc cagatttctg ggagtcagaa acaagttgat gaccaacccc tccaaaactg 2340
aacattgaag gagggaaaga tcattacaag catcaggact gttggtgtac actcagttca 2400
gccaaagtgg attctccaag tgggagcaat atggccgctt tcccatgaga aagacattca 2460
agatggtgac taaatgacta aatactttgc agagggacaa agatgggaac tagggatatg 2520
gatggaagta gtagagaaga tatatgacca tctgcatcaa gagaaaggat aacataagac 2580
aaatcaagat gaaagaaata atccacaccc ccccccccac cgcgtcctgg ccaataagta 2640
tagcctacat ggctgttcat tatctgggaa ccaaaatggc cactatcttg actccttcct 2700
taaagataca gaaagaattg aatccaagga atggggtagg gtggaaatag aagaaatgaa 2760
ggggactctt gggctaagaa tacttatgtt taataataaa agggggaggc aaagaaaaaa 2820
aaaaaaaaaa aaaaaaaaaa aaaaaaa 2847

<210> 22
<211> 700
<212> PRT
<213> Mus musculus

<400> 22

Met Leu Ala Ser Ala Leu Leu Val Phe Leu Cys Cys Phe Lys Gly His

1               5                   10                  15

Ala Gly Ser Ser Pro His Phe Leu Gln Gln Pro Glu Asp Met Val Val
            20                  25                  30

Leu Leu Gly Glu Glu Ala Arg Leu Pro Cys Ala Leu Gly Ala Tyr Arg
        35                  40                  45

Gly Leu Val Gln Trp Thr Lys Asp Gly Leu Ala Leu Gly Gly Glu Arg
    50                  55                  60

Asp Leu Pro Gly Trp Ser Arg Tyr Trp Ile Ser Gly Asn Ser Ala Ser
65                  70                  75                  80

Gly Gln His Asp Leu His Ile Lys Pro Val Glu Leu Glu Asp Glu Ala
                85                  90                  95

Ser Tyr Glu Cys Gln Ala Ser Gln Ala Gly Leu Arg Ser Arg Pro Ala
            100                 105                 110

Gln Leu His Val Met Val Pro Pro Glu Ala Pro Gln Val Leu Gly Gly
        115                 120                 125

Pro Ser Val Ser Leu Val Ala Gly Val Pro Gly Asn Leu Thr Cys Arg
    130                 135                 140

Ser Arg Gly Asp Ser Arg Pro Ala Pro Glu Leu Leu Trp Phe Arg Asp
145                 150                 155                 160

Gly Ile Arg Leu Asp Gly Ser Ser Phe His Gln Thr Thr Leu Lys Asp
                165                 170                 175

Lys Ala Thr Gly Thr Val Glu Asn Thr Leu Phe Leu Thr Pro Ser Ser
            180                 185                 190

His Asp Asp Gly Ala Thr Leu Ile Cys Arg Ala Arg Ser Gln Ala Leu
        195                 200                 205

Pro Thr Gly Arg Asp Thr Ala Val Thr Leu Ser Leu Gln Tyr Pro Pro
    210                 215                 220

Met Val Thr Leu Ser Ala Glu Pro Gln Thr Val Gln Glu Gly Glu Lys
225                 230                 235                 240

Val Thr Phe Leu Cys Gln Ala Thr Ala Gln Pro Pro Val Thr Gly Tyr
                245                 250                 255

Arg Trp Ala Lys Gly Gly Ser Pro Val Leu Gly Ala Arg Gly Pro Arg
            260                 265                 270

Leu Glu Val Val Ala Asp Ala Thr Phe Leu Thr Glu Pro Val Ser Cys

        275                 280                 285

Glu Val Ser Asn Ala Val Gly Ser Ala Asn Arg Ser Thr Ala Leu Glu
    290                 295                 300

Val Leu Tyr Gly Pro Ile Leu Gln Ala Lys Pro Lys Ser Val Ser Val
305                 310                 315                 320

Asp Val Gly Lys Asp Ala Ser Phe Ser Cys Val Trp Arg Gly Asn Pro
                325                 330                 335

Leu Pro Arg Ile Thr Trp Thr Arg Met Gly Gly Ser Gln Val Leu Ser
            340                 345                 350

Ser Gly Pro Thr Leu Arg Leu Pro Ser Val Ala Leu Glu Asp Ala Gly
        355                 360                 365

Asp Tyr Val Cys Arg Ala Glu Pro Arg Arg Thr Gly Leu Gly Gly Gly
    370                 375                 380

Lys Ala Gln Ala Arg Leu Thr Val Asn Ala Pro Pro Val Val Thr Ala
385                 390                 395                 400

Leu Gln Pro Ala Pro Ala Phe Leu Arg Gly Pro Ala Arg Leu Gln Cys
                405                 410                 415

Val Val Phe Ala Ser Pro Ala Pro Asp Ser Val Val Trp Ser Trp Asp
            420                 425                 430

Glu Gly Phe Leu Glu Ala Gly Ser Leu Gly Arg Phe Leu Val Glu Ala
        435                 440                 445

Phe Pro Ala Pro Glu Val Glu Gly Gly Gln Gly Pro Gly Leu Ile Ser
    450                 455                 460

Val Leu His Ile Ser Gly Thr Gln Glu Ser Asp Phe Thr Thr Gly Phe
465                 470                 475                 480

Asn Cys Ser Ala Arg Asn Arg Leu Gly Glu Gly Arg Val Gln Ile His
                485                 490                 495

Leu Gly Arg Arg Asp Leu Leu Pro Thr Val Arg Ile Val Ala Gly Ala
            500                 505                 510

Ala Ser Ala Ala Thr Ser Leu Leu Met Val Ile Thr Gly Val Val Leu
        515                 520                 525

Cys Cys Trp Arg His Gly Ser Leu Ser Lys Gln Lys Asn Leu Val Arg
    530                 535                 540

Ile Pro Gly Ser Ser Glu Gly Ser Ser Ser Arg Gly Pro Glu Glu Glu

545                 550                 555                 560

Thr Gly Ser Ser Glu Asp Arg Gly Pro Ile Val His Thr Asp His Ser
                565                 570                 575

Asp Leu Val Leu Glu Glu Lys Glu Ala Leu Glu Thr Lys Asp Pro Thr
            580                 585                 590

Asn Gly Tyr Tyr Arg Val Arg Gly Val Ser Val Ser Leu Ser Leu Gly
        595                 600                 605

Glu Ala Pro Gly Gly Gly Leu Phe Leu Pro Pro Pro Ser Pro Ile Gly
    610                 615                 620

Leu Pro Gly Thr Pro Thr Tyr Tyr Asp Phe Lys Pro His Leu Asp Leu
625                 630                 635                 640

Val Pro Pro Cys Arg Leu Tyr Arg Ala Arg Ala Gly Tyr Leu Thr Thr
                645                 650                 655

Pro His Pro Arg Ala Phe Thr Ser Tyr Met Lys Pro Thr Ser Phe Gly
            660                 665                 670

Pro Pro Glu Leu Ser Ser Gly Thr Pro Pro Phe Pro Tyr Ala Thr Leu
        675                 680                 685

Ser Pro Pro Ser His Gln Arg Leu Gln Thr His Val
    690                 695                 700

<210>  23
<211>  2153
<212>  DNA
<213>  Homo sapiens

<400>  23
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg cccgtcgccc catttcctgc aacagccaga ggacctggtg     300

gtgctgctgg gggaggaagc ccggctgccg tgtgctctgg gcgcctactg ggggctagtt      360

cagtggacta agagtgggct ggccctaggg ggccaaaggg acctaccagg gtggtcccgg     420

tactggatat cagggaatgc agccaatggc cagcatgacc tccacattag gcccgtggag     480

ctagaggatg aagcatcata tgaatgtcag gctacacaag caggcctccg ctccagacca     540

gcccaactgc acgtgctggt ccccccagaa gccccccagg tgctgggcgg cccctctgtg      600

tctctggttg ctggagttcc tgcgaacctg acatgtcgga gccgtgggga tgcccgccct     660

acccctgaat tgctgtggtt ccgagatggg gtcctgttgg atggagccac cttccatcag     720

accctgctga aggaagggac ccctgggtca gtggagagca ccttaaccct gacccctttc 780
agccatgatg atggagccac ctttgtctgc cgggcccgga gccaggccct gcccacagga 840
agagacacag ctatcacact gagcctgcag taccccccag aggtgactct gtctgcttcg 900
ccacacactg tgcaggaggg agagaaggtc attttcctgt gccaggccac agcccagcct 960
cctgtcacag gctacaggtg ggcaaaaggg ggctctccgg tgctcggggc ccgcgggcca 1020
aggttagagg tcgtggcaga cgcctcgttc ctgactgagc ccgtgtcctg cgaggtcagc 1080
aacgccgtgg gtagcgccaa ccgcagtact gcgctggatg tgctgtttgg gccgattctg 1140
caggcaaagc cggagcccgt gtccgtggac gtgggggaag acgcttcctt cagctgcgcc 1200
tggcgcggga acccgcttcc acgggtaacc tggacccgcc gcggtggcgc gcaggtgctg 1260
ggctctggag ccacactgcg tcttccgtcg gtggggcccg aggacgcagg cgactatgtg 1320
tgcagagctg aggctgggct atcgggcctg cggggcggcg ccgcggaggc tcggctgact 1380
gtgaacgctc ccccagtagt gaccgccctg cactctgcgc ctgccttcct gaggggccct 1440
gctcgcctcc agtgtctggt tttcgcctct cccgccccag atgccgtggt ctggtcttgg 1500
gatgagggct tcctggaggc ggggtcgcag ggccggttcc tggtggagac attccctgcc 1560
ccagagagcc gcgggggact gggtccgggc ctgatctctg tgctacacat ttcggggacc 1620
caggagtctg actttagcag gagctttaac tgcagtgccc ggaaccggct gggcgaggga 1680
ggtgcccagg ccagcctggg ccgtagagac ttgctgccca ctgtgcggat agtggccgga 1740
gtggccgctg ccaccacaac tctccttatg gtcatcactg gggtggccct ctgctgctgg 1800
cgccacagca aggcctcagc ctctttctcc gagcaaaaga acctgatgcg aatccctggc 1860
agcagcgacg gctccagttc acgaggtcct gaagaagagg agacaggcag ccgcgaggac 1920
cggggcccca ttgtgcacac tgaccacagt gatctggttc tggaggagga agggactctg 1980
gagaccaagg acccaaccaa cggttactac aaggtccgag gagtcagtcc acccgcgtct 2040
ccagactcac gtgtgacatc tttccaatgg aagagtcctg ggatctccaa cttgccataa 2100
tggattgttc tgatttctga ggagccagga caagttggcg accttactcc tcc 2153

<210> 24
<211> 633
<212> PRT
<213> Homo sapiens

<400> 24

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Pro Pro Ala Ser Pro Asp Ser Arg Val Thr Ser Phe Gln Trp
    610                 615                 620

Lys Ser Pro Gly Ile Ser Asn Leu Pro
625                 630

<210>  25
<211>  2979
<212>  DNA
<213>  Homo sapiens

<400>  25
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg cccgtcgccc catttcctgc aacagccaga ggacctggtg     300

gtgctgctgg gggaggaagc ccggctgccg tgtgctctgg gcgcctactg ggggctagtt      360

cagtggacta agagtgggct ggccctaggg ggccaaaggg acctaccagg gtggtcccgg     420

tactggatat cagggaatgc agccaatggc cagcatgacc tccacattag gcccgtggag     480

ctagaggatg aagcatcata tgaatgtcag gctacacaag caggcctccg ctccagacca     540

gcccaactgc acgtgctggt ccccccagaa gccccccagg tgctgggcgg cccctctgtg     600

tctctggttg ctggagttcc tgcgaacctg acatgtcgga gccgtgggga tgcccgccct     660

acccctgaat tgctgtggtt ccgagatggg gtcctgttgg atggagccac cttccatcag     720

accctgctga aggaagggac ccctgggtca gtggagagca ccttaaccct gacccctttc     780

agccatgatg atggagccac ctttgtctgc cgggcccgga gccaggccct gcccacagga     840

agagacacag ctatcacact gagcctgcag taccccccag aggtgactct gtctgcttcg     900

ccacacactg tgcaggaggg agagaaggtc attttcctgt gccaggccac agcccagcct     960

cctgtcacag gctacaggtg ggcaaaaggg ggctctccgg tgctcggggc ccgcgggcca    1020

aggttagagg tcgtggcaga cgcctcgttc ctgactgagc ccgtgtcctg cgaggtcagc    1080

aacgccgtgg gtagcgccaa ccgcagtact gcgctggatg tgctgtttgg gccgattctg    1140

caggcaaagc cggagcccgt gtccgtggac gtgggggaag acgcttcctt cagctgcgcc    1200

tggcgcggga acccgcttcc acgggtaacc tggacccgcc gcggtggcgc gcaggtgctg    1260

ggctctggag ccacactgcg tcttccgtcg gtggggcccg aggacgcagg cgactatgtg    1320

tgcagagctg aggctgggct atcgggcctg cggggcggcg ccgcggaggc tcggctgact    1380

gtgaacgctc ccccagtagt gaccgccctg cactctgcgc ctgccttcct gaggggccct     1440

gctcgcctcc agtgtctggt tttcgcctct cccgccccag atgccgtggt ctggtcttgg    1500

gatgagggct tcctggaggc ggggtcgcag ggccggttcc tggtggagac attccctgcc 1560
ccagagagcc gcgggggact gggtccgggc ctgatctctg tgctacacat ttcggggacc 1620
caggagtctg actttagcag gagctttaac tgcagtgccc ggaaccggct gggcgaggga 1680
ggtgcccagg ccagcctggg ccgtagagac ttgctgccca ctgtgcggat agtggccgga 1740
gtggccgctg ccaccacaac tctccttatg gtcatcactg gggtggccct ctgctgctgg 1800
cgccacagca aggcctcagc ctctttctcc gagcaaaaga acctgatgcg aatccctggc 1860
agcagcgacg gctccagttc acgaggtcct gaagaagagg agacaggcag ccgcgaggac 1920
cggggcccca ttgtgcacac tgaccacagt gatctggttc tggaggagga agggactctg 1980
gagaccaagg acccaaccaa cggttactac aaggtccgag gagtcagtgt gagcctgagc 2040
cttggcgaag cccctggagg aggtctcttc ctgccaccac cctcccccct tgggccccca 2100
gggaccccta ccttctatga cttcaaccca cacctgggca tggtcccccc ctgcagactt 2160
tacagagcca gggcaggcta tctcaccaca ccccaccctc gagctttcac cagctacatc 2220
aaacccacat cctttgggcc cccagatctg gcccccggga ctcccccctt cccatatgct 2280
gccttcccca cacctagcca cccgcgtctc cagactcacg tgtgacatct ttccaatgga 2340
agagtcctgg gatctccaac ttgccataat ggattgttct gatttctgag gagccaggac 2400
aagttggcga ccttactcct ccaaaactga acacaagggg agggaaagat cattacattt 2460
gtcaggagca tttgtataca gtcagctcag ccaaaggaga tgccccaagt gggagcaaca 2520
tggccaccca atatgcccac ctattccccg gtgtaaaaga gattcaagat ggcaggtagg 2580
ccctttgagg agagatgggg acagggcagt gggtgttggg agtttggggc cgggatggaa 2640
gttgtttcta gccactgaaa gaagatattt caagatgacc atctgcattg agaggaaagg 2700
tagcatagga tagatgaaga tgaagagcat accaggcccc accctggctc tccctgaggg 2760
gaactttgct cggccaatgg aaatgcagcc aagatggcca tatactccct aggaacccaa 2820
gatggccacc atcttgattt tactttcctt aaagactcag aaagacttgg acccaaggag 2880
tggggataca gtgagaatta ccactgttgg ggcaaaatat tgggataaaa atatttatgt 2940
ttaataataa aaaaaagtca aagaggcaaa aaaaaaaaa 2979

<210> 26
<211> 708
<212> PRT
<213> Homo sapiens

<400> 26

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly

        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro

305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly

580                 585                 590

Thr Leu Glu Thr Lys Asp Pro Thr Asn Gly Tyr Tyr Lys Val Arg Gly
        595                 600                 605

Val Ser Val Ser Leu Ser Leu Gly Glu Ala Pro Gly Gly Gly Leu Phe
    610                 615                 620

Leu Pro Pro Pro Ser Pro Leu Gly Pro Pro Gly Thr Pro Thr Phe Tyr
625                 630                 635                 640

Asp Phe Asn Pro His Leu Gly Met Val Pro Pro Cys Arg Leu Tyr Arg
                645                 650                 655

Ala Arg Ala Gly Tyr Leu Thr Thr Pro His Pro Arg Ala Phe Thr Ser
            660                 665                 670

Tyr Ile Lys Pro Thr Ser Phe Gly Pro Pro Asp Leu Ala Pro Gly Thr
        675                 680                 685

Pro Pro Phe Pro Tyr Ala Ala Phe Pro Thr Pro Ser His Pro Arg Leu
    690                 695                 700

Gln Thr His Val
705

<210>  27
<211>  2973
<212>  DNA
<213>  Homo sapiens

<400>  27
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg cccgtcgccc catttcctgc aacagccaga ggacctggtg     300

gtgctgctgg gggaggaagc ccggctgccg tgtgctctgg gcgcctactg ggggctagtt    360

cagtggacta agagtgggct ggccctaggg ggccaaaggg acctaccagg gtggtcccgg     420

tactggatat cagggaatgc agccaatggc cagcatgacc tccacattag gcccgtggag     480

ctagaggatg aagcatcata tgaatgtcag gctacacaag caggcctccg ctccagacca     540

gcccaactgc acgtgctggt ccccccagaa gccccccagg tgctgggcgg cccctctgtg     600

tctctggttg ctggagttcc tgcgaacctg acatgtcgga gccgtgggga tgcccgccct     660

acccctgaat tgctgtggtt ccgagatggg gtcctgttgg atggagccac ctttcatcag     720

accctgctga aggaagggac ccctgggtca gtggagagca ccttaaccct gacccctttc     780

agccatgatg atggagccac ctttgtctgc cgggcccgga gccaggccct gcccacagga     840

agagacacag ctatcacact gagcctgcag taccccccag aggtgactct gtctgcttcg 900
ccacacactg tgcaggaggg agagaaggtc attttcctgt gccaggccac agcccagcct 960
cctgtcacag gctacaggtg ggcaaaaggg ggctctccgg tgctcggggc ccgcgggcca 1020
aggttagagg tcgtggcaga cgcctcgttc ctgactgagc ccgtgtcctg cgaggtcagc 1080
aacgccgtgg gtagcgccaa ccgcagtact gcgctggatg tgctgtttgg gccgattctg 1140
caggcaaagc cggagcccgt gtccgtggac gtgggggaag acgcttcctt cagctgcgcc 1200
tggcgcggga acccgcttcc acgggtaacc tggacccgcc gcggtggcgc gcaggtgctg 1260
ggctctggag ccacactgcg tcttccgtcg gtggggcccg aggacgcagg cgactatgtg 1320
tgcagagctg aggctgggct atcgggcctg cggggcggcg ccgcggaggc tcggctgact 1380
gtgaacgctc ccccagtagt gaccgccctg cactctgcgc ctgccttcct gaggggccct 1440
gctcgcctcc agtgtctggt tttcgcctct cccgccccag atgccgtggt ctggtcttgg 1500
gatgagggct tcctggaggc ggggtcgcag ggccggttcc tggtggagac attccctgcc 1560
ccagagagcc gcgggggact gggtccgggc ctgatctctg tgctacacat ttcggggacc 1620
caggagtctg actttagcag gagctttaac tgcagtgccc ggaaccggct gggcgaggga 1680
ggtgcccagg ccagcctggg ccgtagagac ttgctgccca ctgtgcggat agtggccgga 1740
gtggccgctg ccaccacaac tctccttatg gtcatcactg gggtggccct ctgctgctgg 1800
cgccacagca aggcctcagc ctctttctcc gagcaaaaga acctgatgcg aatccctggc 1860
agcagcgacg gctccagttc acgaggtcct gaagaagagg agacaggcag ccgcgaggac 1920
cggggcccca ttgtgcacac tgaccacagt gatctggttc tggaggagga agggactctg 1980
gagaccaagg acccaaccaa cggttactac aaggtccgag gagtcagtgt gagcctgagc 2040
cttggcgaag cccctggagg aggtctcttc ctgccaccac cctcccccct tgggccccca 2100
gggaccccta ccttctatga cttcaaccca cacctgggca tggtcccccc ctgcagactt 2160
tacagagcca gggcaggcta tctcaccaca ccccaccctc gagctttcac cagctacatc 2220
aaacccacat cctttgggcc cccagatctg gcccccggga ctcccccctt cccatatgct 2280
gccttcccca cacctagcca cccgcgtctc cagactcacg tgtgacatct ttccaatgga 2340
agagtcctgg gatctccaac ttgccataat ggattgttct gatttctgag gagccaggac 2400
aagttggcga ccttactcct ccaaaactga acacaagggg agggaaagat cattacattt 2460
gtcaggagca tttgtataca gtcagctcag ccaaaggaga tgccccaagt gggagcaaca 2520
tggccaccca atatgcccac ctattccccg gtgtaaaaga gattcaagat ggcaggtagg 2580
ccctttgagg agagatgggg acagggcagt gggtgttggg agtttggggc cgggatggaa 2640
gttgtttcta gccactgaaa gaagatattt caagatgacc atctgcattg agaggaaagg 2700
tagcatagga tagatgaaga tgaagagcat accaggcccc accctggctc tccctgaggg 2760
gaactttgct cggccaatgg aaatgcagcc aagatggcca tatactccct aggaacccaa 2820
aatggccacc atcttgattt tactttcctt aaagactcag aaagacttgg acccaaggag 2880

tggggataca gtgagaatta ccactgttgg ggcaaaatat tgggataaaa atatttatgt 2940

ttaataataa aaaaaagtca aagagaaaaa aaa 2973

<210> 28
<211> 708
<212> PRT
<213> Homo sapiens

<400> 28

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Gly Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Ala Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Val Ser Leu Ser Leu Gly Glu Ala Pro Gly Gly Gly Leu Phe
    610                 615                 620

Leu Pro Pro Pro Ser Pro Leu Gly Pro Pro Gly Thr Pro Thr Phe Tyr
625                 630                 635                 640

Asp Phe Asn Pro His Leu Gly Met Val Pro Pro Cys Arg Leu Tyr Arg
                645                 650                 655

Ala Arg Ala Gly Tyr Leu Thr Thr Pro His Pro Arg Ala Phe Thr Ser
            660                 665                 670

Tyr Ile Lys Pro Thr Ser Phe Gly Pro Pro Asp Leu Ala Pro Gly Thr
        675                 680                 685

Pro Pro Phe Pro Tyr Ala Ala Phe Pro Thr Pro Ser His Pro Arg Leu
    690                 695                 700

Gln Thr His Val
705

<210>  29
<211>  1969
<212>  DNA
<213>  Homo sapiens

<400>  29
accttggggg acgaatgctc aggatgcggg tccccgccct cctcgtcctc ctcttctgct      60

tcagagggag agcaggcccg tcgccccatt tcctgcaaca gccagaggac ctggtggtgc     120

tgctggggga ggaagcccgg ctgccgtgtg ctctgggcgc ctactggggg ctagttcagt     180

ggactaagag tgggctggcc ctagggggcc aaagggacct accagggtgg tcccggtact 240
ggatatcagg gaatgcagcc aatggccagc atgacctcca cattaggccc gtggagctag 300
aggatgaagc atcatatgaa tgtcaggcta cacaagcagg cctccgctcc agaccagccc 360
aactgcacgt gctggtcccc ccagaagccc cccaggtgct gggcggcccc tctgtgtctc 420
tggttgctgg agttcctgcg aacctgacat gtcggagccg tggggatgcc cgccctaccc 480
ctgaattgct gtggttccga gatggggtcc tgttggatgg agccaccttc catcagaccc 540
tgctgaagga agggacccct gggtcagtgg agagcacctt aaccctgacc cctttcagcc 600
atgatgatgg agccaccctt gtctgccggg cccggagcca ggccctgccc acaggaagag 660
acacagctat cacactgagc ctgcagtacc ccccagaggt gactctgtct gcttcgccac 720
acactgtgca ggagggagag aaggtcattt tcctgtgcca ggccacagcc cagcctcctg 780
tcacaggcta caggtgggca aaagggggct ctccggtgct cggggcccgc gggccaaggt 840
tagaggtcgt ggcagacgcc tcgttcctga ctgagcccgt gtcctgcgag gtcagcaacg 900
ccgtgggtag cgccaaccgc agtactgcgc tggatgtgct gtttgggccg attctgcagg 960
caaagccgga gcccgtgtcc gtggacgtgg gggaagacgc ttccttcagc tgcgcctggc 1020
gcgggaatcc gcttccacgg gtaacctgga cccgccgcgg tggcgcgcag gtgctgggct 1080
ctggagccac actgcgtctt ccgtcggtgg ggcccgagga cgcagacgac tatgtgtgca 1140
gagctgaggc tgggctatcg ggcctgcggg gcggcgccgc ggaggctcgg ctgactgtga 1200
acgctccccc agtagtgacc gccctgcact ctgcgcctgc cttcctgagg ggccctgctc 1260
gcctccagtg tctggttttc gcctctcccg ccccagatgc cgtggtctgg tcttgggatg 1320
agggcttcct ggaggcgggg tcgcagggtc ggttcctggt ggagacattc cctgccccag 1380
agagccgcgg gggactgggt ccgggcctga tctctgtgct acacatttcg gggacccagg 1440
agtctgactt tagcaggagc tttaactgca gtgcccggaa ccggctgggc gagggaggtg 1500
cccaggccag cctgggccgt agagacttgc tgcccactgt gcggatagtg gccggagtgg 1560
tcgctgccac cacaactctc cttatggtca tcactggggt ggccctctgc tgctggcgcc 1620
acagcaaggc ctcagcctct ttctccgagc aaaagaacct gatgcgaatc cctggcagca 1680
gcgacggctc cagttcacga ggtcctgaag aagaggagac aggcagccgc gaggaccggg 1740
gccccattgt gcacactgac cacagtgatc tggttctgga ggaggaaggg actctggaga 1800
ccaaggaccc aaccaacggt tactacaagg tccgaggagt cagtccaccc gcgtctccag 1860
actcacgtgt gacatctttc caatggaaga gtcctgggat ctccaacttg ccataatgga 1920
ttgttctgat ttctgaggag ccaggacaag ttggcgacct tactcctcc 1969

<210> 30
<211> 633
<212> PRT
<213> Homo sapiens

<400> 30

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Leu Val Cys Arg Ala Arg Ser
        195                 200                 205

Gln Ala Leu Pro Thr Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Ala Gln Pro Pro Val
                245                 250                 255

Thr Gly Tyr Arg Trp Ala Lys Gly Gly Ser Pro Val Leu Gly Ala Arg
            260                 265                 270

Gly Pro Arg Leu Glu Val Val Ala Asp Ala Ser Phe Leu Thr Glu Pro
        275                 280                 285

Val Ser Cys Glu Val Ser Asn Ala Val Gly Ser Ala Asn Arg Ser Thr
    290                 295                 300

Ala Leu Asp Val Leu Phe Gly Pro Ile Leu Gln Ala Lys Pro Glu Pro
305                 310                 315                 320

Val Ser Val Asp Val Gly Glu Asp Ala Ser Phe Ser Cys Ala Trp Arg
                325                 330                 335

Gly Asn Pro Leu Pro Arg Val Thr Trp Thr Arg Arg Gly Gly Ala Gln
            340                 345                 350

Val Leu Gly Ser Gly Ala Thr Leu Arg Leu Pro Ser Val Gly Pro Glu
        355                 360                 365

Asp Ala Asp Asp Tyr Val Cys Arg Ala Glu Ala Gly Leu Ser Gly Leu
    370                 375                 380

Arg Gly Gly Ala Ala Glu Ala Arg Leu Thr Val Asn Ala Pro Pro Val
385                 390                 395                 400

Val Thr Ala Leu His Ser Ala Pro Ala Phe Leu Arg Gly Pro Ala Arg
                405                 410                 415

Leu Gln Cys Leu Val Phe Ala Ser Pro Ala Pro Asp Ala Val Val Trp
            420                 425                 430

Ser Trp Asp Glu Gly Phe Leu Glu Ala Gly Ser Gln Gly Arg Phe Leu
        435                 440                 445

Val Glu Thr Phe Pro Ala Pro Glu Ser Arg Gly Gly Leu Gly Pro Gly
    450                 455                 460

Leu Ile Ser Val Leu His Ile Ser Gly Thr Gln Glu Ser Asp Phe Ser
465                 470                 475                 480

Arg Ser Phe Asn Cys Ser Ala Arg Asn Arg Leu Gly Glu Gly Gly Ala
                485                 490                 495

Gln Ala Ser Leu Gly Arg Arg Asp Leu Leu Pro Thr Val Arg Ile Val
            500                 505                 510

Ala Gly Val Val Ala Ala Thr Thr Thr Leu Leu Met Val Ile Thr Gly
        515                 520                 525

Val Ala Leu Cys Cys Trp Arg His Ser Lys Ala Ser Ala Ser Phe Ser
    530                 535                 540

Glu Gln Lys Asn Leu Met Arg Ile Pro Gly Ser Ser Asp Gly Ser Ser
545                 550                 555                 560

Ser Arg Gly Pro Glu Glu Glu Glu Thr Gly Ser Arg Glu Asp Arg Gly
                565                 570                 575

Pro Ile Val His Thr Asp His Ser Asp Leu Val Leu Glu Glu Glu Gly
            580                 585                 590

Thr Leu Glu Thr Lys Asp Pro Thr Asn Gly Tyr Tyr Lys Val Arg Gly
        595                 600                 605

Val Ser Pro Pro Ala Ser Pro Asp Ser Arg Val Thr Ser Phe Gln Trp
    610                 615                 620

Lys Ser Pro Gly Ile Ser Asn Leu Pro
625                 630

<210>  31
<211>  2003
<212>  DNA
<213>  Homo sapiens

<400>  31
ccgcggaact ggcaggcgtt tcagagcgtc agaggctgcg gatgagcaga cttggaggac      60

tccaggccag agactaggct gggcgaagag tcgagcgtga agggggctcc gggccagggt     120

gacaggaggc gtgcttgaga ggaagaagtt gacgggaagg ccagtgcgac ggcaaatctc     180

gtgaaccttg ggggacgaat gctcaggatg cgggtccccg ccctcctcgt cctcctcttc     240

tgcttcagag ggagagcagg gtggtcccgg tactggatat cagggaatgc agccaatggc     300

cagcatgacc tccacattag gcccgtggag ctagaggatg aagcatcata tgaatgtcag     360

gctacacaag caggcctccg ctccagacca gcccaactgc acgtgctggt ccccccagaa     420

gccccccagg tgctgggcgg cccctctgtg tctctggttg ctggagttcc tgcgaacctg     480

acatgtcgga gccgtgggga tgcccgccct acccctgaat tgctgtggtt ccgagatggg     540

gtcctgttgg atggagccac cttccatcag accctgctga aggaagggac ccctgggtca     600

gtggagagca ccttaaccct gacccctttc agccatgatg atggagccac ctttgtctgc     660

cgggcccgga gccaggccct gcccacagga agagacacag ctatcacact gagcctgcag     720

taccccccag aggtgactct gtctgcttcg ccacacactg tgcaggaggg agagaaggtc     780

attttcctgt gccaggccac agcccagcct cctgtcacag gctacaggtg ggcaaaaggg     840

ggctctccgg tgctcggggc ccgcgggcca aggttagagg tcgtggcaga cgcctcgttc     900

ctgactgagc ccgtgtcctg cgaggtcagc aacgccgtgg gtagcgccaa ccgcagtact     960

gcgctggatg tgctgtttgg gccgattctg caggcaaagc cggagcccgt gtccgtggac    1020

gtgggggaag acgcttcctt cagctgcgcc tggcgcggga acccgcttcc acgggtaacc    1080

tggacccgcc gcggtggcgc gcaggtgctg ggctctggag ccacactgcg tcttccgtcg    1140

gtggggcccg aggacgcagg cgactatgtg tgcagagctg aggctgggct atcgggcctg 1200

cggggcggcg ccgcggaggc tcggctgact gtgaacgctc ccccagtagt gaccgccctg 1260

cactctgcgc ctgccttcct gaggggccct gctcgcctcc agtgtctggt tttcgcctct 1320

cccgccccag atgccgtggt ctggtcttgg gatgagggct tcctggaggc ggggtcgcag 1380

ggccggttcc tggtggagac attccctgcc ccagagagcc gcgggggact gggtccgggc 1440

ctgatctctg tgctacacat ttcggggacc caggagtctg actttagcag gagctttaac 1500

tgcagtgccc ggaaccggct gggcgaggga ggtgcccagg ccagcctggg ccgtagagac 1560

ttgctgccca ctgtgcggat agtggccgga gtggccgctg ccaccacaac tctccttatg 1620

gtcatcactg gggtggccct ctgctgctgg cgccacagca aggcctcagc ctctttctcc 1680

gagcaaaaga acctgatgcg aatccctggc agcagcgacg gctccagttc acgaggtcct 1740

gaagaagagg agacaggcag ccgcgaggac cggggcccca ttgtgcacac tgaccacagt 1800

gatctggttc tggaggagga agggactctg gagaccaagg acccaaccaa cggttactac 1860

aaggtccgag gagtcagtcc acccgcgtct ccagactcac gtgtgacatc tttccaatgg 1920

aagagtcctg ggatctccaa cttgccataa tggattgttc tgatttctga ggagccagga 1980

caagttggcg accttactcc tcc 2003

<210> 32
<211> 583
<212> PRT
<213> Homo sapiens

<400> 32

Met Leu Arg Met Arg Val Pro Ala Leu Leu Val Leu Leu Phe Cys Phe
1               5                   10                  15

Arg Gly Arg Ala Gly Trp Ser Arg Tyr Trp Ile Ser Gly Asn Ala Ala
            20                  25                  30

Asn Gly Gln His Asp Leu His Ile Arg Pro Val Glu Leu Glu Asp Glu
        35                  40                  45

Ala Ser Tyr Glu Cys Gln Ala Thr Gln Ala Gly Leu Arg Ser Arg Pro
    50                  55                  60

Ala Gln Leu His Val Leu Val Pro Pro Glu Ala Pro Gln Val Leu Gly
65                  70                  75                  80

Gly Pro Ser Val Ser Leu Val Ala Gly Val Pro Ala Asn Leu Thr Cys
                85                  90                  95

Arg Ser Arg Gly Asp Ala Arg Pro Thr Pro Glu Leu Leu Trp Phe Arg
            100                 105                 110

Asp Gly Val Leu Leu Asp Gly Ala Thr Phe His Gln Thr Leu Leu Lys

115                 120                 125

Glu Gly Thr Pro Gly Ser Val Glu Ser Thr Leu Thr Leu Thr Pro Phe
    130                 135                 140

Ser His Asp Asp Gly Ala Thr Phe Val Cys Arg Ala Arg Ser Gln Ala
145                 150                 155                 160

Leu Pro Thr Gly Arg Asp Thr Ala Ile Thr Leu Ser Leu Gln Tyr Pro
                165                 170                 175

Pro Glu Val Thr Leu Ser Ala Ser Pro His Thr Val Gln Glu Gly Glu
            180                 185                 190

Lys Val Ile Phe Leu Cys Gln Ala Thr Ala Gln Pro Pro Val Thr Gly
        195                 200                 205

Tyr Arg Trp Ala Lys Gly Gly Ser Pro Val Leu Gly Ala Arg Gly Pro
    210                 215                 220

Arg Leu Glu Val Val Ala Asp Ala Ser Phe Leu Thr Glu Pro Val Ser
225                 230                 235                 240

Cys Glu Val Ser Asn Ala Val Gly Ser Ala Asn Arg Ser Thr Ala Leu
                245                 250                 255

Asp Val Leu Phe Gly Pro Ile Leu Gln Ala Lys Pro Glu Pro Val Ser
            260                 265                 270

Val Asp Val Gly Glu Asp Ala Ser Phe Ser Cys Ala Trp Arg Gly Asn
        275                 280                 285

Pro Leu Pro Arg Val Thr Trp Thr Arg Arg Gly Gly Ala Gln Val Leu
    290                 295                 300

Gly Ser Gly Ala Thr Leu Arg Leu Pro Ser Val Gly Pro Glu Asp Ala
305                 310                 315                 320

Gly Asp Tyr Val Cys Arg Ala Glu Ala Gly Leu Ser Gly Leu Arg Gly
                325                 330                 335

Gly Ala Ala Glu Ala Arg Leu Thr Val Asn Ala Pro Pro Val Val Thr
            340                 345                 350

Ala Leu His Ser Ala Pro Ala Phe Leu Arg Gly Pro Ala Arg Leu Gln
        355                 360                 365

Cys Leu Val Phe Ala Ser Pro Ala Pro Asp Ala Val Val Trp Ser Trp
    370                 375                 380

Asp Glu Gly Phe Leu Glu Ala Gly Ser Gln Gly Arg Phe Leu Val Glu

385                 390                 395                 400

Thr Phe Pro Ala Pro Glu Ser Arg Gly Gly Leu Gly Pro Gly Leu Ile
                405                 410                 415

Ser Val Leu His Ile Ser Gly Thr Gln Glu Ser Asp Phe Ser Arg Ser
            420                 425                 430

Phe Asn Cys Ser Ala Arg Asn Arg Leu Gly Glu Gly Gly Ala Gln Ala
        435                 440                 445

Ser Leu Gly Arg Arg Asp Leu Leu Pro Thr Val Arg Ile Val Ala Gly
    450                 455                 460

Val Ala Ala Ala Thr Thr Thr Leu Leu Met Val Ile Thr Gly Val Ala
465                 470                 475                 480

Leu Cys Cys Trp Arg His Ser Lys Ala Ser Ala Ser Phe Ser Glu Gln
                485                 490                 495

Lys Asn Leu Met Arg Ile Pro Gly Ser Ser Asp Gly Ser Ser Ser Arg
            500                 505                 510

Gly Pro Glu Glu Glu Glu Thr Gly Ser Arg Glu Asp Arg Gly Pro Ile
        515                 520                 525

Val His Thr Asp His Ser Asp Leu Val Leu Glu Glu Glu Gly Thr Leu
    530                 535                 540

Glu Thr Lys Asp Pro Thr Asn Gly Tyr Tyr Lys Val Arg Gly Val Ser
545                 550                 555                 560

Pro Pro Ala Ser Pro Asp Ser Arg Val Thr Ser Phe Gln Trp Lys Ser
                565                 570                 575

Pro Gly Ile Ser Asn Leu Pro
            580

<210>  33
<211>  1819
<212>  DNA
<213>  Homo sapiens

<400>  33
accttggggg acgaatgctc tggatgcggg tccccgccct cctcgtcctc ctcttctgct      60

tcagagggag agcagggtgg tcccggtact ggatatcagg gaatgcagcc aatggccagc     120

atgacctcca cattaggccc gtggagctag aggatgaagc atcatatgaa tgtcaggcta     180

cacaagcagg cctccgctcc agaccagccc aactgcacgt gctggtcccc ccagaagccc     240

cccaggtgct gggcggcccc tctgtgtctc tggttgctgg agttcctgcg aacctgacat     300

gtcggagccg tggggatgcc cgccctaccc ctgaattgct gtggttccga gatggggtcc     360

tgttggatgg agccaccttc catcagaccc tgctgaagga agggacccct gggtcagtgg 420
agagcacctt aaccctgacc cctttcagcc atgatgatgg agccaccttt gtctgccggg 480
cccggagcca ggccctgccc acaggaagag acacagctat cacactgagc ctgcagtacc 540
ccccagaggt gactctgtct gcttcgccac acactgtgca ggagggagag aaggtcattt 600
tcctgtgcca ggccacagcc cagcctcctg tcacaggcta caggtgggca aaagggggct 660
ctccggtgct cggggcccgc gggccaaggt tagaggtcgt ggcagacgcc tcgttcctga 720
ctgagcccgt gtcctgcgag gtcagcaacg ccgtgggtag cgccaaccgc agtactgcgc 780
tggatgtgct gtttgggccg attctgcagg caaagccgga gcccgtgtcc gtggacgtgg 840
gggaagacgc ttccttcagc tgcgcctggc gcgggaatcc gcttccacgg gtaacctgga 900
cccgccgcgg tggcgcgcag gtgctgggct ctggagccac actgcgtctt ccgtcggtgg 960
ggcccgagga cgcagacgac tatgtgtgca gagctgaggc tgggctatcg ggtctgcggg 1020
gcggcgccgt ggaggctcgg ctgactgtgg acgctccccc agtagtgacc gccctgcact 1080
ctgcgcctgc cttcctgagg ggccctgctc gcctccagtg tctggttttc gcctctcccg 1140
ccccagatgc cgtggtctgg tcttgggatg agggcttcct ggaggcgggg tcgcagggtc 1200
ggttcctggt ggagacattc cctgccccag agagccgcgg gggactgggt ccgggcctga 1260
tctctgtgct acacatttcg gggacccagg agtctgactt tagcaggagc tttaactgca 1320
gtgcccggaa ccggctgggc gagggaggtg cccaggccag cctgggccgt agagacttgc 1380
tgcccactgt gcggatagtg gccggagtgg tcgctgccac cacaactctc cttatggtca 1440
tcactggggt ggccctctgc tgctggcgcc acagcaaggc ctcagcctct ttctccgagc 1500
aaaagaacct gatgcgaatc cctggcagca gcgacggctc cagttcacga ggtcctgaag 1560
aagaggagac aggcagccgc gaggaccggg gccccattgt gcacactgac cacagtgatc 1620
tggttctgga ggaggaaggg actctggaga ccaaggaccc aaccaacggt tactacaagg 1680
tccgaggagt cagtccaccc gcgtctccag actcacgtgt gacatctttc caatggaaga 1740
gtcctgggat ctccaacttg ccataatgga ttgttctgat ttctgaggag ccaggacaag 1800
ttggcgacct tactcctcc 1819

<210> 34
<211> 583
<212> PRT
<213> Homo sapiens

<400> 34

<210>  35
<211>  2959
<212>  DNA
<213>  Homo sapiens

<400>  35
atgcacttga gtgcaccttg agtctccagc ctctcaagga accgggagat caggccatca      60

gcgtctcagc cagcaaaggc ctgaaccacc agtcccttat aaccctgcgt ttcagagcgt     120

cagaggcgtg cttgagagga agaagttgac gggaaggcca gtgcgacggc aaatctcgtg     180

aaccttgggg gacgaatgct caggatgcgg gtccccgccc tcctcgtcct cctcttctgc     240

ttcagaggga gagcaggccc gtcgccccat ttcctgcaac agccagagga cctggtggtg     300

ctgctggggg aggaagcccg gctgccgtgt gctctgggcg cctactgggg gctagttcag     360

tggactaaga gtgggctggc cctagggggc caaagggacc taccagggtg gtcccggtac     420

tggatatcag ggaatgcagc caatggccag catgacctcc acattaggcc cgtggagcta     480

gaggatgaag catcatatga atgtcaggct acacaagcag gcctccgctc cagaccagcc     540

caactgcacg tgctggtccc cccagaagcc ccccaggtgc tgggcggccc ctctgtgtct     600

ctggttgctg gagttcctgc gaacctgaca tgtcggagcc gtggggatgc ccgccctacc     660

cctgaattgc tgtggttccg agatggggtc ctgttggatg gagccacctt ccatcagacc     720

ctgctgaagg aagggacccc tgggtcagtg gagagcacct taaccctgcc cacaggaaga     780

gacacagcta tcacactgag cctgcagtac cccccagagg tgactctgtc tgcttcgcca     840

cacactgtgc aggagggaga gaaggtcatt ttcctgtgcc aggccacagc ccagcctcct     900

gtcacaggct acaggtgggc aaaagggggc tctccggtgc tcggggcccg cgggccaagg     960

ttagaggtcg tggcagacgc ctcgttcctg actgagcccg tgtcctgcga ggtcagcaac    1020

gccgtgggta gcgccaaccg cagtactgcg ctggatgtgc tgtttgggcc gattctgcag    1080

gcaaagccgg agcccgtgtc cgtggacgtg ggggaagacg cttccttcag ctgcgcctgg    1140

cgcgggaacc cgcttccacg ggtaacctgg acccgccgcg gtggcgcgca ggtgctgggc    1200

tctggagcca cactgcgtct tccgtcggtg gggcccgagg acgcaggcga ctatgtgtgc    1260

agagctgagg ctgggctatc gggcctgcgg ggcggcgccg cggaggctcg gctgactgtg    1320

aacgctcccc cagtagtgac cgccctgcac tctgcgcctg ccttcctgag gggccctgct    1380

cgcctccagt gtctggtttt cgcctctccc gccccagatg ccgtggtctg gtcttgggat    1440

gagggcttcc tggaggcggg gtcgcagggc cggttcctgg tggagacatt ccctgcccca    1500

gagagccgcg ggggactggg tccgggcctg atctctgtgc tacacatttc ggggacccag    1560

gagtctgact ttagcaggag ctttaactgc agtgcccgga accggctggg cgagggaggt    1620

gcccaggcca gcctgggccg tagagacttg ctgcccactg tgcggatagt ggccggagtg    1680

gccgctgcca ccacaactct ccttatggtc atcactgggg tggccctctg ctgctggcgc    1740

cacagcaagg cctcagcctc tttctccgag caaaagaacc tgatgcgaat ccctggcagc    1800

agcgacggct ccagttcacg aggtcctgaa gaagaggaga caggcagccg cgaggaccgg 1860

ggccccattg tgcacactga ccacagtgat ctggttctgg aggaggaagg gactctggag 1920

accaaggacc caaccaacgg ttactacaag gtccgaggag tcagtgtgag cctgagcctt 1980

ggcgaagccc ctggaggagg tctcttcctg ccaccaccct ccccccttgg gcccccaggg 2040

acccctacct tctatgactt caacccacac ctgggcatgg tccccccctg cagactttac 2100

agagccaggg caggctatct caccacaccc caccctcgag ctttcaccag ctacatcaaa 2160

cccacatcct ttgggccccc agatctggcc cccgggactc cccccttccc atatgctgcc 2220

ttccccacac ctagccaccc gcgtctccag actcacgtgt gacatctttc caatggaaga 2280

gtcctgggat ctccaacttg ccataatgga ttgttctgat ttctgaggag ccaggacaag 2340

ttggcgacct tactcctcca aaactgaaca caaggggagg gaaagatcat tacatttgtc 2400

aggagcattt gtatacagtc agctcagcca aaggagatgc cccaagtggg agcaacatgg 2460

ccacccaata tgcccaccta ttccccggtg taaaagagat tcaagatggc aggtaggccc 2520

tttgaggaga gatggggaca gggcagtggg tgttgggagt ttggggccgg gatggaagtt 2580

gtttctagcc actgaaagaa gatatttcaa gatgaccatc tgcattgaga ggaaaggtag 2640

cataggatag atgaagatga agagcatacc aggccccacc ctggctctcc ctgaggggaa 2700

ctttgctcgg ccaatggaaa tgcagccaag atggccatat actccctagg aacccaagat 2760

ggccaccatc ttgattttac tttccttaaa gactcagaaa gacttggacc caaggagtgg 2820

ggatacagtg agaattacca ctgttggggc aaaatattgg gataaaaata tttatgttta 2880

ataataaaaa aaagtcaaag aggaaaaaaa aaaaaaaaaa aaaaaaaaaa aaaaaaaaaa 2940

aaaaaaaaaa aaaaaaaaa 2959

<210> 36
<211> 688
<212> PRT
<213> Homo sapiens

<400> 36

Met Leu Arg Met Arg Val Pro Ala Leu Leu Val Leu Leu Phe Cys Phe
1               5                   10                  15

Arg Gly Arg Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn
65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Ala Thr Phe His Gln Thr Leu
                165                 170                 175

Leu Lys Glu Gly Thr Pro Gly Ser Val Glu Ser Thr Leu Thr Leu Pro
            180                 185                 190

Thr Gly Arg Asp Thr Ala Ile Thr Leu Ser Leu Gln Tyr Pro Pro Glu
        195                 200                 205

Val Thr Leu Ser Ala Ser Pro His Thr Val Gln Glu Gly Glu Lys Val
    210                 215                 220

Ile Phe Leu Cys Gln Ala Thr Ala Gln Pro Pro Val Thr Gly Tyr Arg
225                 230                 235                 240

Trp Ala Lys Gly Gly Ser Pro Val Leu Gly Ala Arg Gly Pro Arg Leu
                245                 250                 255

Glu Val Val Ala Asp Ala Ser Phe Leu Thr Glu Pro Val Ser Cys Glu
            260                 265                 270

Val Ser Asn Ala Val Gly Ser Ala Asn Arg Ser Thr Ala Leu Asp Val
        275                 280                 285

Leu Phe Gly Pro Ile Leu Gln Ala Lys Pro Glu Pro Val Ser Val Asp
    290                 295                 300

Val Gly Glu Asp Ala Ser Phe Ser Cys Ala Trp Arg Gly Asn Pro Leu
305                 310                 315                 320

Pro Arg Val Thr Trp Thr Arg Arg Gly Gly Ala Gln Val Leu Gly Ser
                325                 330                 335

Gly Ala Thr Leu Arg Leu Pro Ser Val Gly Pro Glu Asp Ala Gly Asp
            340                 345                 350

Tyr Val Cys Arg Ala Glu Ala Gly Leu Ser Gly Leu Arg Gly Gly Ala
        355                 360                 365

Ala Glu Ala Arg Leu Thr Val Asn Ala Pro Pro Val Val Thr Ala Leu
    370                 375                 380

His Ser Ala Pro Ala Phe Leu Arg Gly Pro Ala Arg Leu Gln Cys Leu
385                 390                 395                 400

Val Phe Ala Ser Pro Ala Pro Asp Ala Val Val Trp Ser Trp Asp Glu
                405                 410                 415

Gly Phe Leu Glu Ala Gly Ser Gln Gly Arg Phe Leu Val Glu Thr Phe
            420                 425                 430

Pro Ala Pro Glu Ser Arg Gly Gly Leu Gly Pro Gly Leu Ile Ser Val
        435                 440                 445

Leu His Ile Ser Gly Thr Gln Glu Ser Asp Phe Ser Arg Ser Phe Asn
    450                 455                 460

Cys Ser Ala Arg Asn Arg Leu Gly Glu Gly Gly Ala Gln Ala Ser Leu
465                 470                 475                 480

Gly Arg Arg Asp Leu Leu Pro Thr Val Arg Ile Val Ala Gly Val Ala
                485                 490                 495

Ala Ala Thr Thr Thr Leu Leu Met Val Ile Thr Gly Val Ala Leu Cys
            500                 505                 510

Cys Trp Arg His Ser Lys Ala Ser Ala Ser Phe Ser Glu Gln Lys Asn
        515                 520                 525

Leu Met Arg Ile Pro Gly Ser Ser Asp Gly Ser Ser Ser Arg Gly Pro
    530                 535                 540

Glu Glu Glu Glu Thr Gly Ser Arg Glu Asp Arg Gly Pro Ile Val His
545                 550                 555                 560

Thr Asp His Ser Asp Leu Val Leu Glu Glu Glu Gly Thr Leu Glu Thr
                565                 570                 575

Lys Asp Pro Thr Asn Gly Tyr Tyr Lys Val Arg Gly Val Ser Val Ser
            580                 585                 590

Leu Ser Leu Gly Glu Ala Pro Gly Gly Gly Leu Phe Leu Pro Pro Pro
        595                 600                 605

Ser Pro Leu Gly Pro Pro Gly Thr Pro Thr Phe Tyr Asp Phe Asn Pro
    610                 615                 620

His Leu Gly Met Val Pro Pro Cys Arg Leu Tyr Arg Ala Arg Ala Gly
625                 630                 635                 640

Tyr Leu Thr Thr Pro His Pro Arg Ala Phe Thr Ser Tyr Ile Lys Pro
                645                 650                 655

Thr Ser Phe Gly Pro Pro Asp Leu Ala Pro Gly Thr Pro Pro Phe Pro
            660                 665                 670

Tyr Ala Ala Phe Pro Thr Pro Ser His Pro Arg Leu Gln Thr His Val
        675                 680                 685

<210>  37
<211>  2591
<212>  DNA
<213>  Pan troglodytes

<400>  37
atgctcagga tgcgggtccc cgccctcctc gtcctcctct tctgcttcag agggagcgca      60

ggcccatcgc cccatttcct gcaacagcca gaggacctgg tggtgctgct gggggaggaa     120

gcccggctgc cgtgtgctct gggcgcctac tgggggctag ttcagtggac taagagtggg     180

ctggccctag ggggccaaag ggacctacca gggtggtccc ggtactggat atcagggaat     240

gcagccaatg gccagcatga cctccacatt aggcccgtgg agctagagga tgaagcatca     300

tatgaatgtc aggctacaca agcaggcctc cgctccagac cagcccaact gcacgtgctg     360

gtacccccag aagcccccca ggtgctgggc ggcccctctg tgtctctggt tgctggagtt     420

cctgcgaacc tgacatgtcg gagccgtggg gatgcccgcc ctacccctga attgctgtgg     480

ttccgagatg gggtcctgtt ggatggaacc accttccatc agaccctgct gaaggaaggg     540

acccctgggt cagtggagag caccttaacc ctgacccctt tcagccatga tgatggagcc     600

acctttgtct gccgggcccg gagccaggcc ctgccctcag gaagagacac ggctatcaca     660

ctgagcctgc agtacccccc agaggtgact ctgtctgctt cgccacacac tgtgcaggag     720

ggagagaagg tcattttcct gtgccaggcc acagaccagc ctcctgtcac aggctacagg     780

gaggacgaag acccagaatc caccacaatg gggaccaggc ttcctacaaa tccggcttct     840

gacgcccctt ccctgtcgca ggtgctgggc tctggagcca cactgcatct tccgtcggta     900

gggcccgagg acgcaggcga ctatgtgtgc agagctgagc ctgggctatc gggcctgggg     960

ggcggcgccg cggaggctcg gctgactgtg aacgctcccc cagtagtgac cgccctgcac    1020

tctgcgcctg ccttcctgag gggccctgct cgcctccagt gtctggtttt cgcctctccc    1080

gccccagatg ccgtggtctg gtcttgggat gagggcttcc tggaggcggg gtcgcagggc    1140

cggttcctgg tggagacatt ccctgcccca gagagccgcg ggggactggg tccgggcctg    1200

atctctgtgc tacacatttc ggggacccag gagtctgact ttagcaggag ctttaactgc    1260

agtgcccgga accggctggg cgagggaggt gcccaggcca gcctgggccg tagagacttg    1320

ctgcccactg tgcggatagt ggccggagtg gccgctgcca ccacgactct ccttatggtc    1380

atcactgggg tggccctctg ctgctggcgc cacagcaagg cctcagcctc tttctccgag 1440
caaaagaacc tgatgcgaat ccctggcagc agcgacggct ccagttcgcg aggtcctgaa 1500
gaagaggaga caggcagccg cgaggaccgg ggccccattg tgcacactga ccacagtgat 1560
ctggttctgg aggaggaagg gactctggag accaaggacc caaccaacgg ttactacaag 1620
gtccgaggag tcagtgtgag cctgagcctt ggcgaagccc ctggaggagg cctcttcctg 1680
ccaccaccct ccccccttgg gcccccaggg acccctacct tctatgactt caacccacac 1740
ctgggcatgg tccccccctg cagactttac agagccaggg caggctatct caccacaccc 1800
caccctcgag ctttcaccag ctacatcaaa cccacatcct ttgggccccc agatctggcc 1860
cccgggactc cccccttccc atgtgctgcc ttccccacac ctagccaccc gcgtctccag 1920
actcatgtgt gacatctttc cagtggaaga gtcctgggat ctccaacttg ccataatgga 1980
ttgttctgat ttctgaggag ccaggacaag ttggcgacct tactcctcca aaactgaaca 2040
cagggggagg gaaagatcat tacatttgtc aggagcattt gtgtacagtc agctcagcca 2100
aaggagatgc cccaagtggg agcaacgtgg ccacccaata tgcccaccta ttccccggtg 2160
taaaagagat tcaagatggc aggtaggccc tttgaggaga gatggggaca gggcagtggg 2220
tgttgggagt ttggggccgg gatggaagtt gtttctagcc actgaaagaa gatatttcaa 2280
gatgaccatc tgcattgaga ggaaaggtag cataggatag atgaagatga agagcatacc 2340
aggccccacc ctggctctcc ctgaggggaa ttttgctcgg ccaatggaaa tgcagccaag 2400
atggccatat actccctagg aacccaagat ggccaccatc ttgattttac tttccttaaa 2460
gactcagaaa gacttggact caaggagtgg ggatacagtg agaattacca ctgttggggc 2520
aaaggggatt cttgggataa aaatatttat gtttaataat aaaaaaaagt caaagaggca 2580
agtgtgtctt a 2591

<210> 38
<211> 643
<212> PRT
<213> Pan troglodytes

<400> 38

Met Leu Arg Met Arg Val Pro Ala Leu Leu Val Leu Leu Phe Cys Phe
1               5                   10                  15

Arg Gly Ser Ala Gly Pro Ser Pro His Phe Leu Gln Gln Pro Glu Asp
            20                  25                  30

Leu Val Val Leu Leu Gly Glu Glu Ala Arg Leu Pro Cys Ala Leu Gly
        35                  40                  45

Ala Tyr Trp Gly Leu Val Gln Trp Thr Lys Ser Gly Leu Ala Leu Gly
    50                  55                  60

Gly Gln Arg Asp Leu Pro Gly Trp Ser Arg Tyr Trp Ile Ser Gly Asn

65                  70                  75                  80

Ala Ala Asn Gly Gln His Asp Leu His Ile Arg Pro Val Glu Leu Glu
                85                  90                  95

Asp Glu Ala Ser Tyr Glu Cys Gln Ala Thr Gln Ala Gly Leu Arg Ser
            100                 105                 110

Arg Pro Ala Gln Leu His Val Leu Val Pro Pro Glu Ala Pro Gln Val
        115                 120                 125

Leu Gly Gly Pro Ser Val Ser Leu Val Ala Gly Val Pro Ala Asn Leu
    130                 135                 140

Thr Cys Arg Ser Arg Gly Asp Ala Arg Pro Thr Pro Glu Leu Leu Trp
145                 150                 155                 160

Phe Arg Asp Gly Val Leu Leu Asp Gly Thr Thr Phe His Gln Thr Leu
                165                 170                 175

Leu Lys Glu Gly Thr Pro Gly Ser Val Glu Ser Thr Leu Thr Leu Thr
            180                 185                 190

Pro Phe Ser His Asp Asp Gly Ala Thr Phe Val Cys Arg Ala Arg Ser
        195                 200                 205

Gln Ala Leu Pro Ser Gly Arg Asp Thr Ala Ile Thr Leu Ser Leu Gln
    210                 215                 220

Tyr Pro Pro Glu Val Thr Leu Ser Ala Ser Pro His Thr Val Gln Glu
225                 230                 235                 240

Gly Glu Lys Val Ile Phe Leu Cys Gln Ala Thr Asp Gln Pro Pro Val
                245                 250                 255

Thr Gly Tyr Arg Glu Asp Glu Asp Pro Glu Ser Thr Thr Met Gly Thr
            260                 265                 270

Arg Leu Pro Thr Asn Pro Ala Ser Asp Ala Pro Ser Leu Ser Gln Val
        275                 280                 285

Leu Gly Ser Gly Ala Thr Leu His Leu Pro Ser Val Gly Pro Glu Asp
    290                 295                 300

Ala Gly Asp Tyr Val Cys Arg Ala Glu Pro Gly Leu Ser Gly Leu Gly
305                 310                 315                 320

Gly Gly Ala Ala Glu Ala Arg Leu Thr Val Asn Ala Pro Pro Val Val
                325                 330                 335

Thr Ala Leu His Ser Ala Pro Ala Phe Leu Arg Gly Pro Ala Arg Leu

            340                 345                 350

Gln Cys Leu Val Phe Ala Ser Pro Ala Pro Asp Ala Val Val Trp Ser
        355                 360                 365

Trp Asp Glu Gly Phe Leu Glu Ala Gly Ser Gln Gly Arg Phe Leu Val
    370                 375                 380

Glu Thr Phe Pro Ala Pro Glu Ser Arg Gly Gly Leu Gly Pro Gly Leu
385                 390                 395                 400

Ile Ser Val Leu His Ile Ser Gly Thr Gln Glu Ser Asp Phe Ser Arg
                405                 410                 415

Ser Phe Asn Cys Ser Ala Arg Asn Arg Leu Gly Glu Gly Gly Ala Gln
            420                 425                 430

Ala Ser Leu Gly Arg Arg Asp Leu Leu Pro Thr Val Arg Ile Val Ala
        435                 440                 445

Gly Val Ala Ala Ala Thr Thr Thr Leu Leu Met Val Ile Thr Gly Val
    450                 455                 460

Ala Leu Cys Cys Trp Arg His Ser Lys Ala Ser Ala Ser Phe Ser Glu
465                 470                 475                 480

Gln Lys Asn Leu Met Arg Ile Pro Gly Ser Ser Asp Gly Ser Ser Ser
                485                 490                 495

Arg Gly Pro Glu Glu Glu Glu Thr Gly Ser Arg Glu Asp Arg Gly Pro
            500                 505                 510

Ile Val His Thr Asp His Ser Asp Leu Val Leu Glu Glu Glu Gly Thr
        515                 520                 525

Leu Glu Thr Lys Asp Pro Thr Asn Gly Tyr Tyr Lys Val Arg Gly Val
    530                 535                 540

Ser Val Ser Leu Ser Leu Gly Glu Ala Pro Gly Gly Gly Leu Phe Leu
545                 550                 555                 560

Pro Pro Pro Ser Pro Leu Gly Pro Pro Gly Thr Pro Thr Phe Tyr Asp
                565                 570                 575

Phe Asn Pro His Leu Gly Met Val Pro Pro Cys Arg Leu Tyr Arg Ala
            580                 585                 590

Arg Ala Gly Tyr Leu Thr Thr Pro His Pro Arg Ala Phe Thr Ser Tyr
        595                 600                 605

Ile Lys Pro Thr Ser Phe Gly Pro Pro Asp Leu Ala Pro Gly Thr Pro

610                 615                 620

Pro Phe Pro Cys Ala Ala Phe Pro Thr Pro Ser His Pro Arg Leu Gln
625                 630                 635                 640

Thr His Val

<210>  39
<211>  3019
<212>  DNA
<213>  Bos taurus

<400>  39
atgggaagag ggcgggtctc ccagggggcg ggccggggcg gggcgagtgg aaaccagccc      60
tccagggtct ctccgagacc tggcgcgcct aactggcagg cgtttcagag cggcggagtc     120
tgcggcggag cagacgaggg gacctccagg tcagagagag gggagcttct agcctggggc     180
gaagagtcaa gagcggaggg gactccaggc cagagtggtg ggaagtgtgc tccgaggaag     240
gaactggcag ggaagccagt gcaacagcaa gtctgctgga tcttgggcga cacacttagg     300
atgctcgtgc ccgtactcct cgtcctctcc ttctgcctca gggggcgtgc aggcctggca     360
ccccactttc tgcaacagcc agaggaccag gtagttctgt tgggagacga ggcccgactg     420
ccctgtgccc tgggcgacta ccggggactg gttcagtgga ctaaggacgg gctggctcta     480
gggggcgaaa gggaccttcc aggctggtcc cggtactgga tatcagggaa tgcagcaagt     540
ggccagcatg acctccacat tagacccgtg gagctagagg atcaagcatc gtatgaatgt     600
caagcgacac aagccggcct ccgctctcga ccagcccaac tgcatgtgct ggtgcctcca     660
gacccccccc aggtgctggg cggcccctct gtgtctctgg ttgctggggt tcctgcgaat     720
ctgacctgtc ggagccgtgg tgatgcccac cctacccctg agctgctgtg gtttcgagat     780
gggatccgac tggacggggc catcttccgc cagactcctt tgaaggaagg aaccatcggg     840
tcagtggaga gcatcttgtc tttgacccct tccagccatg atgatggagc caccttggtt     900
tgccgggccc ggagccaggc cctgcctgca gggaaggaca cagctgtaac actaagcctg     960
cagtaccccc cagtggtgac tctgtctgca gaaccacaga cagtgcagga gggagagaag    1020
gtcactttcc tatgccaggc cacagcccag cctcctgtca ccggctatag atgggcaaag    1080
gggggctccc cggtgctcgg ggcccgaggg ccaatgctgg agatagtggc tgacgcttca    1140
ttcctgactg cgccggtgtc ctgcgaggtc agcaatgcag tgggtagcgc caaccgcagc    1200
acagcgctgg acgtgcagtt tgggccgatt ctgcaggcaa agccgaaagc cttgtcggtg    1260
gacgtaggag aagacgcctc cttcagctgt gtctggcgcg ggaatccgct cccacgggta    1320
acctggaccc gccgcgggga cgcacaggtg ctggcctccg ggcccacgct gcgtcttccc    1380
gcggtggggc ccgaggatgc aggcgactac gtgtgcaggg ccgagccggg gctctcaggc    1440
cggggcggtg gctctgcaga agctaggctg actgtgaacg ctcccccagt agtgaccgct    1500
ctgcactctg cgcccgcctt cctgaggggc cccgcccgcc tccagtgtct agtcttcgct    1560

tcccccaccc cagaagcagt ggtctggtct tgggatgagg gcttcctgac ggcggggtca 1620
cggggtcggt tcctggtgga gactttccca gccccagagg gcctcaaggg acagggtcca 1680
ggcctgatct ccgtgctaca catttcgggg acccaggagt ccgactttca ccggggcttc 1740
aactgcactg cccggaaccg attgggtgag ggaggcaccc aggtcagcct gggccgtaga 1800
gatttgctgc ccactgtgcg gattgtggcc ggagtggccg ccatggccat gactctcctt 1860
atgatcatca ctggggtggc cctctgctgc tggcgccatg gcaggggtca gtcctctttc 1920
tccaagcaaa agaacctggt gcgaatccca gggagcagtg atggctccag ttccaggggc 1980
cccgaggagg agacaggcat cagcaaagac cagggcccca tcgtgcacac ggaccacagt 2040
gacctggttc tggatgagga gggggctctg gagaccaagg acccaaccaa tggttactac 2100
aaggtccgag gagtcagtgt gagcctgagc cttggagaag cccccggagg aggtctcttc 2160
ctaccaccct cctcccctct tggacctcca ggaaccccta ccttctatga cttcaatcca 2220
catcttggca tggtccctcc ctgcagacta tatagatccc gggcaggcta tctcaccaca 2280
ccccatcctc gagcttttac cagctacatc aaacccacat cctttggacc cccagatctg 2340
gcccccagca ctcccccctt cccatatgct gccttcccca cgcccagcca cccacgtctc 2400
cagactcatg tgtgacatct ctccagctga agagtcctgg gatcttcaac ttgcacaatg 2460
gattgtcctg atttctgagt aaccagaaca aactggcaac cctatccttc caaaattgaa 2520
ccttgagggg agggaaagat cattgcagtt gcctgggtca ttgcgagaaa gccactgggc 2580
ttgcctcttc ctctgtataa aagagattca agagggcaga tgggcccttt gcagagggat 2640
ggggacaggg gttatgggag ttagggacag agaaggaaat tgcttccaaa cattgaaaga 2700
agagatttca agatggccac ccacattaag aagaaaggca acataaagca gatcaagata 2760
caggacctgt ccttgctatc cctgaggggt actttgtttc acccatggaa ttgctgccaa 2820
aatggccatt cattccctgg gaatccaaga tggccaccat cttgattctt tctaccttcc 2880
ttaaaggccc tggaagaacc ggacccaagg agtaaggaca gggtgaaagc tgttgtggtg 2940
gtgggaaggg ggaaaggagt ggtgttggaa tctgggaatg agaatattta tgttcaatta 3000
aaaaaaaaaa aagttgcaa 3019

<210> 40
<211> 804
<212> PRT
<213> Bos taurus

<400> 40

Met Gly Arg Gly Arg Val Ser Gln Gly Ala Gly Arg Gly Gly Ala Ser
1               5                   10                  15

Gly Asn Gln Pro Ser Arg Val Ser Pro Arg Pro Gly Ala Pro Asn Trp
            20                  25                  30

Gln Ala Phe Gln Ser Gly Gly Val Cys Gly Gly Ala Asp Glu Gly Thr

        35                  40                  45

Ser Arg Ser Glu Arg Gly Glu Leu Leu Ala Trp Gly Glu Glu Ser Arg
    50                  55                  60

Ala Glu Gly Thr Pro Gly Gln Ser Gly Gly Lys Cys Ala Pro Arg Lys
65                  70                  75                  80

Glu Leu Ala Gly Lys Pro Val Gln Gln Gln Val Cys Trp Ile Leu Gly
                85                  90                  95

Asp Thr Leu Arg Met Leu Val Pro Val Leu Leu Val Leu Ser Phe Cys
            100                 105                 110

Leu Arg Gly Arg Ala Gly Leu Ala Pro His Phe Leu Gln Gln Pro Glu
        115                 120                 125

Asp Gln Val Val Leu Leu Gly Asp Glu Ala Arg Leu Pro Cys Ala Leu
    130                 135                 140

Gly Asp Tyr Arg Gly Leu Val Gln Trp Thr Lys Asp Gly Leu Ala Leu
145                 150                 155                 160

Gly Gly Glu Arg Asp Leu Pro Gly Trp Ser Arg Tyr Trp Ile Ser Gly
                165                 170                 175

Asn Ala Ala Ser Gly Gln His Asp Leu His Ile Arg Pro Val Glu Leu
            180                 185                 190

Glu Asp Gln Ala Ser Tyr Glu Cys Gln Ala Thr Gln Ala Gly Leu Arg
        195                 200                 205

Ser Arg Pro Ala Gln Leu His Val Leu Val Pro Pro Asp Pro Pro Gln
    210                 215                 220

Val Leu Gly Gly Pro Ser Val Ser Leu Val Ala Gly Val Pro Ala Asn
225                 230                 235                 240

Leu Thr Cys Arg Ser Arg Gly Asp Ala His Pro Thr Pro Glu Leu Leu
                245                 250                 255

Trp Phe Arg Asp Gly Ile Arg Leu Asp Gly Ala Ile Phe Arg Gln Thr
            260                 265                 270

Pro Leu Lys Glu Gly Thr Ile Gly Ser Val Glu Ser Ile Leu Ser Leu
        275                 280                 285

Thr Pro Ser Ser His Asp Asp Gly Ala Thr Leu Val Cys Arg Ala Arg
    290                 295                 300

Ser Gln Ala Leu Pro Ala Gly Lys Asp Thr Ala Val Thr Leu Ser Leu

305                 310                 315                 320

Gln Tyr Pro Pro Val Val Thr Leu Ser Ala Glu Pro Gln Thr Val Gln
                325                 330                 335

Glu Gly Glu Lys Val Thr Phe Leu Cys Gln Ala Thr Ala Gln Pro Pro
            340                 345                 350

Val Thr Gly Tyr Arg Trp Ala Lys Gly Gly Ser Pro Val Leu Gly Ala
        355                 360                 365

Arg Gly Pro Met Leu Glu Ile Val Ala Asp Ala Ser Phe Leu Thr Ala
    370                 375                 380

Pro Val Ser Cys Glu Val Ser Asn Ala Val Gly Ser Ala Asn Arg Ser
385                 390                 395                 400

Thr Ala Leu Asp Val Gln Phe Gly Pro Ile Leu Gln Ala Lys Pro Lys
                405                 410                 415

Ala Leu Ser Val Asp Val Gly Glu Asp Ala Ser Phe Ser Cys Val Trp
            420                 425                 430

Arg Gly Asn Pro Leu Pro Arg Val Thr Trp Thr Arg Arg Gly Asp Ala
        435                 440                 445

Gln Val Leu Ala Ser Gly Pro Thr Leu Arg Leu Pro Ala Val Gly Pro
    450                 455                 460

Glu Asp Ala Gly Asp Tyr Val Cys Arg Ala Glu Pro Gly Leu Ser Gly
465                 470                 475                 480

Arg Gly Gly Gly Ser Ala Glu Ala Arg Leu Thr Val Asn Ala Pro Pro
                485                 490                 495

Val Val Thr Ala Leu His Ser Ala Pro Ala Phe Leu Arg Gly Pro Ala
            500                 505                 510

Arg Leu Gln Cys Leu Val Phe Ala Ser Pro Thr Pro Glu Ala Val Val
        515                 520                 525

Trp Ser Trp Asp Glu Gly Phe Leu Thr Ala Gly Ser Arg Gly Arg Phe
    530                 535                 540

Leu Val Glu Thr Phe Pro Ala Pro Glu Gly Leu Lys Gly Gln Gly Pro
545                 550                 555                 560

Gly Leu Ile Ser Val Leu His Ile Ser Gly Thr Gln Glu Ser Asp Phe
                565                 570                 575

His Arg Gly Phe Asn Cys Thr Ala Arg Asn Arg Leu Gly Glu Gly Gly

            580                 585                 590

Thr Gln Val Ser Leu Gly Arg Arg Asp Leu Leu Pro Thr Val Arg Ile
        595                 600                 605

Val Ala Gly Val Ala Ala Met Ala Met Thr Leu Leu Met Ile Ile Thr
    610                 615                 620

Gly Val Ala Leu Cys Cys Trp Arg His Gly Arg Gly Gln Ser Ser Phe
625                 630                 635                 640

Ser Lys Gln Lys Asn Leu Val Arg Ile Pro Gly Ser Ser Asp Gly Ser
                645                 650                 655

Ser Ser Arg Gly Pro Glu Glu Glu Thr Gly Ile Ser Lys Asp Gln Gly
            660                 665                 670

Pro Ile Val His Thr Asp His Ser Asp Leu Val Leu Asp Glu Glu Gly
        675                 680                 685

Ala Leu Glu Thr Lys Asp Pro Thr Asn Gly Tyr Tyr Lys Val Arg Gly
    690                 695                 700

Val Ser Val Ser Leu Ser Leu Gly Glu Ala Pro Gly Gly Gly Leu Phe
705                 710                 715                 720

Leu Pro Pro Ser Ser Pro Leu Gly Pro Pro Gly Thr Pro Thr Phe Tyr
                725                 730                 735

Asp Phe Asn Pro His Leu Gly Met Val Pro Pro Cys Arg Leu Tyr Arg
            740                 745                 750

Ser Arg Ala Gly Tyr Leu Thr Thr Pro His Pro Arg Ala Phe Thr Ser
        755                 760                 765

Tyr Ile Lys Pro Thr Ser Phe Gly Pro Pro Asp Leu Ala Pro Ser Thr
    770                 775                 780

Pro Pro Phe Pro Tyr Ala Ala Phe Pro Thr Pro Ser His Pro Arg Leu
785                 790                 795                 800

Gln Thr His Val

<210> 41
<211> 291
<212> DNA
<213> Artificial

<220>
<223> an artificially synthesized sequence

<400> 41

aagctttcta gagggcccgt ttaaacccgc tgatcagcct cgactgtgcc ttctagttgc 60

cagccatctg ttgtttgccc ctcccccgtg ccttccttga ccctggaagg tgccactccc 120

actgtccttt cctaataaaa tgaggaaatt gcatcgcatt gtctgagtag gtgtcattct 180

attctggggg gtggggtggg gcaggacagc aagggggagg attgggaaga caatagcagg 240

catgctgggg atgcggtggg ctctatggct tctgaggcgg aaagactcga g 291

<210> 42
<211> 32
<212> DNA
<213> Artificial

<220>
<223> an artificially synthesized sequence

<400> 42
gtaccactag tgctagcacg cgtgcggccg cg 32

<210> 43
<211> 32
<212> DNA
<213> Artificial

<220>
<223> an artificially synthesized sequence

<400> 43
gatccgcggc cgcacgcgtg ctagcactag tg 32

<210> 44
<211> 3261
<212> DNA
<213> Mus musculus

<400> 44
atcgattagg agcctatggt ggcacttgtg agcaccagca gcttgtttgt tgctgtcttc 60

cctgagcctc tctctgtgtg ccctgctttt ctgttctttc aatctctttc taactcgccc 120

ttctctcttc cctccctccc tccctccctt ccttctttct cttatcttcc taccattgtc 180

tgtgactgtc gcagtctttc tgtcccggca tcgccttttc tgcccttttc tccccctttc 240

tcccccgtta acagttcttt ctctgagctc taaccccaag accttaaaaa tgcccccttc 300

tctctgtttt tctttactat cacctccctc tcacccactc acaggtactc aaagagcacc 360

ggctgctcta agtccagcag gttcacgaac ccaaagcagt cagccacagc actgagcatt 420

gcttggaccc agtgtgaact cattctgctg cttcccccaa gtccttcctg agtctgccca 480

gccgggcaat gaaatcccct gtcagcacat gccaggaaca ttctttggtg cctccccagc 540

ccgggtgacc ccaaggcccc gggagagcat ttctgatcag ggccatctcc agctcaggat 600

ggaacgcaga gctaggcctg tgtgcgtggg aaggctacaa cccccccccc aggacatcct 660

tgctctttgc agcccagact gtggcatgta ccgcacccct gctggtgaat atgcaggagt 720

gtgttgttaa accaagagag tatgggacag agagaagcca cttctgtggg tatgggtttc 780

agagtgtgcg tgttccaggg gagacgagag cagattttca tctgtggacg tatttggagt 840

gtctcatatg ggtgtgcaca gagcatccag acagtcagca ggactgtgtc cgcagacctc 900
agtgtgtgga aacctggtgt gctcagacgc agataaacat agggtggggg gtccctggga 960
gctgaagcac ctgaatccct gggccatgtg agggccttct gtttcctcaa ctcctccatc 1020
ttcccttgtg tgtgtgtgtg tgtgtgtgtg tatgtcttgc taggatgatt tatcttctta 1080
ttcttctcca cgtctttaat tccatctccc ccctcctcct cctcctccat tcctaccccc 1140
ttctcatttt ctgtcactct tgctgccccc tctgtgtctg gtccttgaat tttagaagca 1200
tatgttgact ggcctgccct cccaacctgg gtgcacagat gaattcgcct gggataagcc 1260
cctgctgcct gcttatctcc tgcaggaatt gcaactgttc tcttttgccc catattccct 1320
acaaacctct cacctcccgg ctgcttcttc tccaggaggc agtccaggca tctggcctga 1380
gcccccctca cagctctcta ccaagggtga gccctctggt ctttctttcc cacccagccc 1440
cattattgat tcattaccct tggtccacat tcccatctcc aaacctatca acacctcctg 1500
ggcaatggat tgcatacaga ccaagccagg gagtctgcta tggggccttt gccagatggt 1560
atctttgggg gccaaggtgt agagctgtca gttgctggag cctttaatta gtgcaaacct 1620
tctacactca cctttggctg ttttccttct ctgaatgctc tggggatccc aggctctcca 1680
tttttccatc ccgtggcccc agagtgccag gaaagggaag ctgtgtcagg tgtctggaaa 1740
aacagcctct cacctgactt cctcccgggg acttaggagt cctgggctca agtctgtcct 1800
atttcaaaac tcagaagcca ggagttcaga tttaggtgtg aggcctccta ggactctctt 1860
ttccaggccc ccagcctgca atccctccac ttctacagga ctcagcacag ctgttcagtc 1920
tgttggtttt cccatcctcg ggggccctag ggggtgagtg agaacgagac tggatgtcaa 1980
atccagtagc tttaagactt aagaccagac attctgaaac tggctcaact ccaccacact 2040
gcagcctgca aggcttctca gccagcagag aagtctgaac tatccccctg gggcaagtcc 2100
aaccctctga tatttagaag taccagtctg agccccaaat gtcatttatt tctgagaggt 2160
ccaggaatgt cagacccggg gtctcaggcc cccaacctcc ccccctccag ccccttcagt 2220
gagctcaggg tccctcccac ctgctctgcc agctgcactg cgtgggaacg cccagctggg 2280
ctgcaccgga gctgtcagga caagctgtgc ggttcccagc ctccctccct gcctgccaga 2340
gccagggcac tgctgcctcc cagccgtcgc ccggcaacca ctcaccactc ggatgggcca 2400
gggactgctg ggtggagaga ggggacgggt gggtgggtac tgctgggtcc tgggaaagga 2460
ggaactcctg gaggggaggg ggcgggctag attcctagat cttaaggtac gtgtatggct 2520
tgcagggata acagagcagc agggagtatt ttgggaaata gggagtattt gggaaatagg 2580
gagcagaaac cctcttctct caaggctcct aaatggtcct tcagcacctg ggtgccctct 2640
ctccgacccg caggcccacg ggagcctgag ctccgcctcc ccagggcgcg gaagctggcg 2700
aagccccagg gattcccatt tatagcttgg tttccactca gctcagtccc tccaggactc 2760
gggctgagca agtttcttcc attcccttct ctcctccctc cacccccttc tcctcctcct 2820
tctccttctt ttcttcctcc tcattcccgc ctccccttca acctcagcag ggtgcaggtg 2880

tccaactcga acaagggccc caacttggac tcagatgttc ccactctcag accccctgat 2940

aatgccgtcc gcacacaagg cgcgggagtt tctcaatggg aagaggccgg gactctagga 3000

ggcggggcga ataggattcc tcccgcctag tgggtccctc gcagtcctag ggttgcaacc 3060

cttgagcggt agagaacacc ggagactgcg gatgagccag atttcgggga cataaaatct 3120

tccagcccgg agagaattgt gtgcagagag gggctccagt ccagcgtggt gtgagaggcg 3180

tgctatcaag aaagaagttg gaggggaacc agtgcaaccc taactctgcg agatcttggg 3240

gtacacacac tcgggggtac c 3261

<210> 45
<211> 798
<212> DNA
<213> Mus musculus

<400> 45
ggtaccatgc cgcgctcctt cctggtggat tcccttgtgc tgcgggaagc cagcgacaag 60

aaggctccgg agggcagccc gccaccgctc ttcccctacg cggtcccgcc gccgcacgcg 120

ctccacggcc tctcgccggg cgcctgccac gcgcgcaagg ccggcttgct gtgcgtgtgt 180

cccctctgtg tcaccgcttc gcagctgcac gggccccccg ggccgccggc actgccgcta 240

ctcaaggcgt ccttccctcc cttcggatcg cagtactgcc acgcacccct gggccgccag 300

cactccgtgt cccctggagt cgcccacggc ccggccgcgg ccgcagcagc tgctgcactc 360

taccagacct cctacccgct gccggatccc agacagtttc actgcatctc tgtggacagc 420

agctcgaacc agctgcccag cagcaagagg atgcggacgg cgttcaccag cacacagctc 480

ctggagctgg agcgagagtt cgcctccaac atgtacctct cccgcctgcg gcgcatcgag 540

atcgcgacct atctgaacct gtccgagaag caggtgaaga tctggtttca gaaccgccgg 600

gtgaagcaca agaaagaagg caaaggcagt aaccaccgcg gcggagctgg ggcgggggcc 660

ggcgggggcg caccgcaagg ctgcaagtgc tcttcgctct cctcagccaa atgctcagag 720

gacgacgacg aattgcccat gtctccatct tcctccggga aggatgacag agatctcaca 780

gtcactccgt aggtcgac 798

<210> 46
<211> 4828
<212> DNA
<213> Homo sapiens

<400> 46
agtggcgtcg gaactgcaaa gcacctgtga gcttgcggaa gtcagttcag actccagccc 60

gctccagccc ggcccgaccc gaccgcaccc ggcgcctgcc ctcgctcggc gtccccggcc 120

agccatgggc ccttggagcc gcagcctctc ggcgctgctg ctgctgctgc aggtctcctc 180

ttggctctgc caggagccgg agccctgcca ccctggcttt gacgccgaga gctacacgtt 240

cacggtgccc cggcgccacc tggagagagg ccgcgtcctg ggcagagtga attttgaaga 300

ttgcaccggt cgacaaagga cagcctattt ttccctcgac acccgattca aagtgggcac 360

agatggtgtg attacagtca aaaggcctct acggtttcat aacccacaga tccatttctt 420
ggtctacgcc tgggactcca cctacagaaa gttttccacc aaagtcacgc tgaatacagt 480
ggggcaccac caccgccccc cgccccatca ggcctccgtt tctggaatcc aagcagaatt 540
gctcacattt cccaactcct ctcctggcct cagaagacag aagagagact gggttattcc 600
tcccatcagc tgcccagaaa atgaaaaagg cccatttcct aaaaacctgg ttcagatcaa 660
atccaacaaa gacaaagaag gcaaggtttt ctacagcatc actggccaag gagctgacac 720
accccctgtt ggtgtcttta ttattgaaag agaaacagga tggctgaagg tgacagagcc 780
tctggataga gaacgcattg ccacatacac tctcttctct cacgctgtgt catccaacgg 840
gaatgcagtt gaggatccaa tggagatttt gatcacggta accgatcaga atgacaacaa 900
gcccgaattc acccaggagg tctttaaggg gtctgtcatg gaaggtgctc ttccaggaac 960
ctctgtgatg gaggtcacag ccacagacgc ggacgatgat gtgaacacct acaatgccgc 1020
catcgcttac accatcctca gccaagatcc tgagctccct gacaaaaata tgttcaccat 1080
taacaggaac acaggagtca tcagtgtggt caccactggg ctggaccgag agagtttccc 1140
tacgtatacc ctggtggttc aagctgctga ccttcaaggt gaggggttaa gcacaacagc 1200
aacagctgtg atcacagtca ctgacaccaa cgataatcct ccgatcttca atcccaccac 1260
gtacaagggt caggtgcctg agaacgaggc taacgtcgta atcaccacac tgaaagtgac 1320
tgatgctgat gcccccaata ccccagcgtg ggaggctgta tacaccatat tgaatgatga 1380
tggtggacaa tttgtcgtca ccacaaatcc agtgaacaac gatggcattt tgaaaacagc 1440
aaagggcttg gattttgagg ccaagcagca gtacattcta cacgtagcag tgacgaatgt 1500
ggtacctttt gaggtctctc tcaccacctc cacagccacc gtcaccgtgg atgtgctgga 1560
tgtgaatgaa gcccccatct ttgtgcctcc tgaaaagaga gtggaagtgt ccgaggactt 1620
tggcgtgggc caggaaatca catcctacac tgcccaggag ccagacacat ttatggaaca 1680
gaaaataaca tatcggattt ggagagacac tgccaactgg ctggagatta atccggacac 1740
tggtgccatt tccactcggg ctgagctgga cagggaggat tttgagcacg tgaagaacag 1800
cacgtacaca gccctaatca tagctacaga caatggttct ccagttgcta ctggaacagg 1860
gacacttctg ctgatcctgt ctgatgtgaa tgacaacgcc cccataccag aacctcgaac 1920
tatattcttc tgtgagagga atccaaagcc tcaggtcata aacatcattg atgcagacct 1980
tcctcccaat acatctccct tcacagcaga actaacacac ggggcgagtg ccaactggac 2040
cattcagtac aacgacccaa cccaagaatc tatcattttg aagccaaaga tggccttaga 2100
ggtgggtgac tacaaaatca atctcaagct catggataac cagaataaag accaagtgac 2160
caccttagag gtcagcgtgt gtgactgtga aggggccgcc ggcgtctgta ggaaggcaca 2220
gcctgtcgaa gcaggattgc aaattcctgc cattctgggg attcttggag gaattcttgc 2280
tttgctaatt ctgattctgc tgctcttgct gtttcttcgg aggagagcgg tggtcaaaga 2340
gcccttactg cccccagagg atgacacccg ggacaacgtt tattactatg atgaagaagg 2400

aggcggagaa gaggaccagg actttgactt gagccagctg cacaggggcc tggacgctcg 2460
gcctgaagtg actcgtaacg acgttgcacc aaccctcatg agtgtccccc ggtatcttcc 2520
ccgccctgcc aatcccgatg aaattggaaa ttttattgat gaaaatctga aagcggctga 2580
tactgacccc acagccccgc cttatgattc tctgctcgtg tttgactatg aaggaagcgg 2640
ttccgaagct gctagtctga gctccctgaa ctcctcagag tcagacaaag accaggacta 2700
tgactacttg aacgaatggg gcaatcgctt caagaagctg gctgacatgt acggaggcgg 2760
cgaggacgac taggggactc gagagaggcg ggccccagac ccatgtgctg ggaaatgcag 2820
aaatcacgtt gctggtggtt tttcagctcc cttcccttga gatgagtttc tggggaaaaa 2880
aaagagactg gttagtgatg cagttagtat agctttatac tctctccact ttatagctct 2940
aataagtttg tgttagaaaa gtttcgactt atttcttaaa gctttttttt ttttcccatc 3000
actctttaca tggtggtgat gtccaaaaga tacccaaatt ttaatattcc agaagaacaa 3060
ctttagcatc agaaggttca cccagcacct tgcagatttt cttaaggaat tttgtctcac 3120
ttttaaaaag aaggggagaa gtcagctact ctagttctgt tgttttgtgt atataatttt 3180
ttaaaaaaaa tttgtgtgct tctgctcatt actacactgg tgtgtccctc tgcctttttt 3240
ttttttttta agacagggtc tcattctatc ggccaggctg gagtgcagtg gtgcaatcac 3300
agctcactgc agccttgtcc tcccaggctc aagctatcct tgcacctcag cctcccaagt 3360
agctgggacc acaggcatgc accactacgc atgactaatt ttttaaatat ttgagacggg 3420
gtctccctgt gttacccagg ctggtctcaa actcctgggc tcaagtgatc ctcccatctt 3480
ggcctcccag agtattggga ttacagacat gagccactgc acctgcccag ctccccaact 3540
ccctgccatt ttttaagaga cagtttcgct ccatcgccca ggcctgggat gcagtgatgt 3600
gatcatagct cactgtaacc tcaaactctg gggctcaagc agttctccca ccagcctcct 3660
ttttattttt ttgtacagat ggggtcttgc tatgttgccc aagctggtct taaactcctg 3720
gcctcaagca atccttctgc cttggccccc caaagtgctg ggattgtggg catgagctgc 3780
tgtgcccagc ctccatgttt taatatcaac tctcactcct gaattcagtt gctttgccca 3840
agataggagt tctctgatgc agaaattatt gggctctttt agggtaagaa gtttgtgtct 3900
ttgtctggcc acatcttgac taggtattgt ctactctgaa gacctttaat ggcttccctc 3960
tttcatctcc tgagtatgta acttgcaatg ggcagctatc cagtgacttg ttctgagtaa 4020
gtgtgttcat taatgtttat ttagctctga agcaagagtg atatactcca ggacttagaa 4080
tagtgcctaa agtgctgcag ccaaagacag agcggaacta tgaaaagtgg gcttggagat 4140
ggcaggagag cttgtcattg agcctggcaa tttagcaaac tgatgctgag gatgattgag 4200
gtgggtctac ctcatctctg aaaattctgg aaggaatgga ggagtctcaa catgtgtttc 4260
tgacacaaga tccgtggttt gtactcaaag cccagaatcc ccaagtgcct gcttttgatg 4320
atgtctacag aaaatgctgg ctgagctgaa cacatttgcc caattccagg tgtgcacaga 4380
aaaccgagaa tattcaaaat tccaaatttt ttcttaggag caagaagaaa atgtggccct 4440

aaagggggtt agttgagggg tagggggtag tgaggatctt gatttggatc tctttttatt 4500

taaatgtgaa tttcaacttt tgacaatcaa agaaaagact tttgttgaaa tagctttact 4560

gtttctcaag tgttttggag aaaaaaatca accctgcaat cactttttgg aattgtcttg 4620

atttttcggc agttcaagct atatcgaata tagttctgtg tagagaatgt cactgtagtt 4680

ttgagtgtat acatgtgtgg gtgctgataa ttgtgtattt tctttggggg tggaaaagga 4740

aaacaattca agctgagaaa agtattctca aagatgcatt tttataaatt ttattaaaca 4800

attttgttaa accataaaaa aaaaaaaa 4828

<210> 47
<211> 882
<212> PRT
<213> Homo sapiens

<400> 47

<210> 48
<211> 4413
<212> DNA
<213> Mus musculus

<400> 48
actggtgtgg gagccgcggc gcactactga gttcccaaga acttctgcta gactcctgcc 60

cggcctaacc cggccctgcc cgaccgcacc cgagctcagt gtttgctcgg cgtctgccgg 120

gtccgccatg ggagcccggt gccgcagctt ttccgcgctc ctgctcctgc tgcaggtctc 180

ctcatggctt tgccaggagc tggagcctga gtcctgcagt cccggcttca gttccgaggt 240

ctacaccttc ccggtgccgg agaggcacct ggagagaggc catgtcctgg gcagagtgag 300

atttgaagga tgcactggcc ggccaaggac agccttcttt tcggaagact cccgattcaa 360

agtggcgaca gacggcacca tcacagtgaa gcggcatcta aagctccaca agctggagac 420

cagtttcctc gtccgcgccc gggactccag tcatagggag ctgtctacca aagtgacgct 480

gaagtccatg gggcaccacc atcaccggca ccaccaccgc gaccctgcct ctgaatccaa 540

cccagagctg ctcatgtttc ccagcgtgta cccaggtctc agaagacaga aacgagactg 600

ggtcatccct cccatcagct gccccgaaaa tgaaaagggc gaatttccaa agaacctggt 660
tcagatcaaa tccaacaggg acaaagaaac aaaggttttc tacagcatca ccggccaagg 720
agctgacaaa ccccccgttg gcgttttcat cattgagagg gagacaggct ggctgaaagt 780
gacacagcct ctggatagag aagccattgc caagtacatc ctctattctc atgccgtgtc 840
atcaaatggg gaagcggtgg aggatcccat ggagatagtg atcacagtga cagatcagaa 900
tgacaacagg ccagagttta cccagccggt ctttgaggga ttcgttgcag aaggcgctgt 960
tccaggaacc tccgtgatga aggtctcagc caccgatgca gacgatgacg tcaacaccta 1020
caacgctgcc atcgcctaca ccatcgtcag ccaggatcct gagctgcctc acaaaaacat 1080
gttcactgtc aatagggaca ccggggtcat cagtgtgctc acctctgggc tggaccgaga 1140
gagttaccct acatacactc tggtggttca ggctgctgac cttcaaggtg aaggcttgag 1200
cacaacagcc aaggctgtga tcactgtcaa ggatattaat gacaacgctc ctgtcttcaa 1260
cccaagcacg tatcagggtc aagtgcctga gaatgaggtc aatgcccgga tcgccacact 1320
caaagtgacc gatgatgatg cccccaacac tccggcgtgg aaagctgtgt acaccgtagt 1380
caacgatcct gaccagcagt tcgttgttgt cacagacccc acgaccaatg atggcatttt 1440
gaaaacagcc aagggcttgg attttgaggc caagcagcaa tacatccttc atgtgagagt 1500
ggagaacgag gaaccctttg aggggtctct tgtcccttcc acagccactg tcactgtgga 1560
cgtggtagac gtgaatgaag cccccatctt tatgcctgcg gagaggagag tcgaagtgcc 1620
cgaagacttt ggtgtgggtc aggaaatcac atcttatacc gctcgagagc cggacacgtt 1680
catggatcag aagatcacgt atcggatttg gagggacact gccaactggc tggagattaa 1740
cccagagact ggtgccattt tcacgcgcgc tgagatggac agagaagacg ctgagcatgt 1800
gaagaacagc acatatgtag ctctcatcat cgccacagat gatggttcac ccattgccac 1860
tggcacgggc actcttctcc tggtcctgtt agacgtcaat gacaacgctc ccatcccaga 1920
acctcgaaac atgcagttct gccagaggaa cccacagcct catatcatca ccatcttgga 1980
tccagacctt ccccccaaca cgtccccctt tactgctgag ctaacccatg gggccagcgt 2040
caactggacc attgagtata atgacgcagc tcaagaatct ctcattttgc aaccaagaaa 2100
ggacttagag attggcgaat acaaaatcca tctcaagctc gcggataacc agaacaaaga 2160
ccaggtgacc acgttggacg tccatgtgtg tgactgtgaa gggacggtca acaactgcat 2220
gaaggcggga atcgtggcag caggattgca agttcctgcc atcctcggaa tccttggagg 2280
gatcctcgcc ctgctgattc tgatcctgct gctcctactg tttctacgga ggagaacggt 2340
ggtcaaagag cccctgctgc caccagatga tgatacccgg gacaatgtgt attactatga 2400
tgaagaagga ggtggagaag aagaccagga ctttgatttg agccagctgc acaggggcct 2460
ggatgcccga ccggaagtga ctcgaaatga tgtggctccc accctcatga gcgtgcccca 2520
gtatcgtccc cgtcctgcca atcctgatga aattggaaac ttcatcgatg aaaacctgaa 2580
ggcagccgac agcgacccca cggcaccccc ttacgactct ctgttggtgt tcgattacga 2640

gggcagtggt tctgaagccg ctagcctgag ctcactgaac tcctctgagt cggatcagga 2700
ccaggactac gattatctga acgagtgggg caaccgattc aagaagctgg cggacatgta 2760
cggcggtggt gaggacgact aggggactag caagtctccc ccgtgtggca ccatgggaga 2820
tgcagaataa ttatatcagt ggtctttcag ctccttccct gagtgtgtag aagagagact 2880
gatctgagaa gtgtgcagat tgcatagtgg tctcactctc cctactggac tgtctgtgtt 2940
aggatggttt tcactgattg ttgaaatctt tttttatttt ttatttttac agtgctgaga 3000
tataaactgt gccttttttt gtttgtttgt ttctgttttt gttcttttga gctatgatct 3060
gccccagaca caacagcccc aagcccctca cacctcacta attttttaca ttgtgtactt 3120
gccctcaatt accatgtttg ctgtattcta atagtcactc atgttcctga attctgttgc 3180
cctgcccagg tgatattcta ggatgcagaa atgcctgggc ccttttatgg tgagagacag 3240
gtatcttggt gtgggtgcaa ctgcgctgga tagtgtgtgt gttcccaaga tctttcgtgg 3300
tattccctct ccacctccag agaactcatt tacagtggca ttccttgttc ggctatgtgt 3360
ctggggcaga acaaaaaaaa gggaccacta tgcatgctgc acacgtctca gattcttagg 3420
tacacacctg attcttaggt gcatgccata gtgggatatg ttgctttgat cagaacctgc 3480
agggaggttt tcgggcacca cttaagtttc ttggcgtttc tttcaaacca aaactaaaga 3540
atggttgttc tctgagagag actggagtgc caccaccaaa gacagaggag agaaaaggag 3600
agaaaccaaa cttggggaca gcaacatcag cgaacccggc tagttggcac accgatggtg 3660
agggtacaca ggcggtgaga cctatcccac aagatttctg gaagactagg cttatctcaa 3720
ccaatgtttt ctggctggaa tctttgtcca tgtattcctg aagcccagga aatgcacccc 3780
tccaatgcct gctcttgatg gtagctacag aaaatgctgg ccgatttaaa cccaagttgc 3840
ccagttctga gtagaaaact gagactatgc tgtgtgtggc ggcgcgcacc tttaagccca 3900
gcactcagga ggcagaggca gtcagatctc cctgagttcg aggccaacct ggtatatata 3960
gtatagtaag cgaattctag gacagccagg gctacacaga gaaaccctgt ctctgcaaac 4020
caaaagagaa aactgagaat attacaaatt gtgcattttc tcaggaagca ggaagagaac 4080
attctaacgg gaaaaaggag acaagacctt tgagagtttt cattcaaaat gcaaatctca 4140
gctttttgat aaccactgga aagaatttta ttgaaagttc tgtacttacc taactttgga 4200
agaaaatgat gaccacaatc aactgtgaga actgttgatt tctctgtagt ttaatcatgt 4260
aatgttgcta gagtgacctt tgtatgtagt ttgagtgtat gtgtgtgggt gctgataatt 4320
ttgtattttg tggggggtgg aaaaggtaag ccattgaaac cgttctctaa gatgcatttt 4380
tatgaatttt attaaagagt tttgttaaac tgt 4413

<210> 49
<211> 884
<212> PRT
<213> Mus musculus

<400> 49

Met Gly Ala Arg Cys Arg Ser Phe Ser Ala Leu Leu Leu Leu Leu Gln
1               5                   10                  15

Val Ser Ser Trp Leu Cys Gln Glu Leu Glu Pro Glu Ser Cys Ser Pro
            20                  25                  30

Gly Phe Ser Ser Glu Val Tyr Thr Phe Pro Val Pro Glu Arg His Leu
        35                  40                  45

Glu Arg Gly His Val Leu Gly Arg Val Arg Phe Glu Gly Cys Thr Gly
    50                  55                  60

Arg Pro Arg Thr Ala Phe Phe Ser Glu Asp Ser Arg Phe Lys Val Ala
65                  70                  75                  80

Thr Asp Gly Thr Ile Thr Val Lys Arg His Leu Lys Leu His Lys Leu
                85                  90                  95

Glu Thr Ser Phe Leu Val Arg Ala Arg Asp Ser Ser His Arg Glu Leu
            100                 105                 110

Ser Thr Lys Val Thr Leu Lys Ser Met Gly His His His His Arg His
        115                 120                 125

His His Arg Asp Pro Ala Ser Glu Ser Asn Pro Glu Leu Leu Met Phe
    130                 135                 140

Pro Ser Val Tyr Pro Gly Leu Arg Arg Gln Lys Arg Asp Trp Val Ile
145                 150                 155                 160

Pro Pro Ile Ser Cys Pro Glu Asn Glu Lys Gly Glu Phe Pro Lys Asn
                165                 170                 175

Leu Val Gln Ile Lys Ser Asn Arg Asp Lys Glu Thr Lys Val Phe Tyr
            180                 185                 190

Ser Ile Thr Gly Gln Gly Ala Asp Lys Pro Pro Val Gly Val Phe Ile
        195                 200                 205

Ile Glu Arg Glu Thr Gly Trp Leu Lys Val Thr Gln Pro Leu Asp Arg
    210                 215                 220

Glu Ala Ile Ala Lys Tyr Ile Leu Tyr Ser His Ala Val Ser Ser Asn
225                 230                 235                 240

Gly Glu Ala Val Glu Asp Pro Met Glu Ile Val Ile Thr Val Thr Asp
                245                 250                 255

Gln Asn Asp Asn Arg Pro Glu Phe Thr Gln Pro Val Phe Glu Gly Phe
            260                 265                 270

Val Ala Glu Gly Ala Val Pro Gly Thr Ser Val Met Lys Val Ser Ala
        275                 280                 285

Thr Asp Ala Asp Asp Asp Val Asn Thr Tyr Asn Ala Ala Ile Ala Tyr
    290                 295                 300

Thr Ile Val Ser Gln Asp Pro Glu Leu Pro His Lys Asn Met Phe Thr
305                 310                 315                 320

Val Asn Arg Asp Thr Gly Val Ile Ser Val Leu Thr Ser Gly Leu Asp
                325                 330                 335

Arg Glu Ser Tyr Pro Thr Tyr Thr Leu Val Val Gln Ala Ala Asp Leu
            340                 345                 350

Gln Gly Glu Gly Leu Ser Thr Thr Ala Lys Ala Val Ile Thr Val Lys
        355                 360                 365

Asp Ile Asn Asp Asn Ala Pro Val Phe Asn Pro Ser Thr Tyr Gln Gly
    370                 375                 380

Gln Val Pro Glu Asn Glu Val Asn Ala Arg Ile Ala Thr Leu Lys Val
385                 390                 395                 400

Thr Asp Asp Asp Ala Pro Asn Thr Pro Ala Trp Lys Ala Val Tyr Thr
                405                 410                 415

Val Val Asn Asp Pro Asp Gln Gln Phe Val Val Val Thr Asp Pro Thr
            420                 425                 430

Thr Asn Asp Gly Ile Leu Lys Thr Ala Lys Gly Leu Asp Phe Glu Ala
        435                 440                 445

Lys Gln Gln Tyr Ile Leu His Val Arg Val Glu Asn Glu Glu Pro Phe
    450                 455                 460

Glu Gly Ser Leu Val Pro Ser Thr Ala Thr Val Thr Val Asp Val Val
465                 470                 475                 480

Asp Val Asn Glu Ala Pro Ile Phe Met Pro Ala Glu Arg Arg Val Glu
                485                 490                 495

Val Pro Glu Asp Phe Gly Val Gly Gln Glu Ile Thr Ser Tyr Thr Ala
            500                 505                 510

Arg Glu Pro Asp Thr Phe Met Asp Gln Lys Ile Thr Tyr Arg Ile Trp
        515                 520                 525

Arg Asp Thr Ala Asn Trp Leu Glu Ile Asn Pro Glu Thr Gly Ala Ile
    530                 535                 540

Phe Thr Arg Ala Glu Met Asp Arg Glu Asp Ala Glu His Val Lys Asn
545                 550                 555                 560

Ser Thr Tyr Val Ala Leu Ile Ile Ala Thr Asp Asp Gly Ser Pro Ile
                565                 570                 575

Ala Thr Gly Thr Gly Thr Leu Leu Leu Val Leu Leu Asp Val Asn Asp
            580                 585                 590

Asn Ala Pro Ile Pro Glu Pro Arg Asn Met Gln Phe Cys Gln Arg Asn
        595                 600                 605

Pro Gln Pro His Ile Ile Thr Ile Leu Asp Pro Asp Leu Pro Pro Asn
    610                 615                 620

Thr Ser Pro Phe Thr Ala Glu Leu Thr His Gly Ala Ser Val Asn Trp
625                 630                 635                 640

Thr Ile Glu Tyr Asn Asp Ala Ala Gln Glu Ser Leu Ile Leu Gln Pro
                645                 650                 655

Arg Lys Asp Leu Glu Ile Gly Glu Tyr Lys Ile His Leu Lys Leu Ala
            660                 665                 670

Asp Asn Gln Asn Lys Asp Gln Val Thr Thr Leu Asp Val His Val Cys
        675                 680                 685

Asp Cys Glu Gly Thr Val Asn Asn Cys Met Lys Ala Gly Ile Val Ala
    690                 695                 700

Ala Gly Leu Gln Val Pro Ala Ile Leu Gly Ile Leu Gly Gly Ile Leu
705                 710                 715                 720

Ala Leu Leu Ile Leu Ile Leu Leu Leu Leu Leu Phe Leu Arg Arg Arg
                725                 730                 735

Thr Val Val Lys Glu Pro Leu Leu Pro Pro Asp Asp Asp Thr Arg Asp
            740                 745                 750

Asn Val Tyr Tyr Tyr Asp Glu Glu Gly Gly Gly Glu Glu Asp Gln Asp
        755                 760                 765

Phe Asp Leu Ser Gln Leu His Arg Gly Leu Asp Ala Arg Pro Glu Val
    770                 775                 780

Thr Arg Asn Asp Val Ala Pro Thr Leu Met Ser Val Pro Gln Tyr Arg
785                 790                 795                 800

Pro Arg Pro Ala Asn Pro Asp Glu Ile Gly Asn Phe Ile Asp Glu Asn
                805                 810                 815

Leu Lys Ala Ala Asp Ser Asp Pro Thr Ala Pro Pro Tyr Asp Ser Leu
            820                 825                 830

Leu Val Phe Asp Tyr Glu Gly Ser Gly Ser Glu Ala Ala Ser Leu Ser
        835                 840                 845

Ser Leu Asn Ser Ser Glu Ser Asp Gln Asp Gln Asp Tyr Asp Tyr Leu
    850                 855                 860

Asn Glu Trp Gly Asn Arg Phe Lys Lys Leu Ala Asp Met Tyr Gly Gly
865                 870                 875                 880

Gly Glu Asp Asp

<210>  50
<211>  4396
<212>  DNA
<213>  Rattus norvegicus

<400>  50
ctggtgtggg agccgcggcg cactactgag ttctcaagaa cttctgctag acttcagccc      60

ggcctaaccc ggctctgccc gaccgcaccc gagctcagtg tttgctcggc gtttgcccgg     120

ccagccatgg gagcccggtg ccgcagcttc tccgcgctcc tgctcctgct gcaggtctcc     180

tcgtggcttt gtcagcagcc ggagtcggag tctgactcct gccgtcccgg cttcagttcc     240

gaggtctaca ccttcctggt gccggagagg cacctggaga gaggccacat cctgggcaga     300

gtgaaatttg aaggatgcac cggccgtcca aggacagcct tcttttctga agactcccga     360

ttcaaagtgt ctacagatgg cgtcatcaca gtcaaacggc atctaaagct tcacaagctg     420

gagaccagtt ttctcgtcca tgcctgggac tccagttaca ggaagctttc taccaaagtg     480

acactgaagt ccctgggcca ccaccaccac cggcatcacc acagagaccc tgtctctgaa     540

tccaacccag agctgctcac gtttcccagc tttcaccagg gtctgagaag acagaaacga     600

gactgggtca tccctcccat caactgcccg gaaaatcaaa agggcgaatt cccccagcga     660

ctggttcaga tcaaatccaa cagggacaaa gagacaacgg ttttctacag catcaccggc     720

ccaggagctg acaaaccccc tgttggcgtt ttcatcattg agagggagac aggctggctg     780

aaagtgacgc agcctctgga cagagaagcc attgacaagt accttctcta ctctcatgct     840

gtgtcatcaa atggggaagc cgtggaggat cccatggaga tagtggtcac agtcacagat     900

cagaatgaca acaggccaga gtttatccag gaggtctttg agggatctgt tgcagaaggc     960

gctcttccag gaacctccgt gatgcaggtc tcagccactg atgcagacga tgacataaac    1020

acctacaatg ctgccatcgc ctacaccatc ctcagccaag atcctgagct gcctcacaaa    1080

aacatgttca ctgtcaaccg ggacactggg gtcatcagtg tggtcacctc cggactggac    1140

cgagagagtt accctacata tactctggtg gttcaggctg ccgaccttca aggcgaaggc    1200

ctaagcacaa cagcaaaagc tgtgatcact gtcaaggata ttaatgacaa cgctcccatc    1260

ttcaacccaa gcacgtacca gggtcaagtg cttgagaatg aggtcggtgc ccgtattgcc 1320
acactcaagg tgactgatga tgatgccccc aacactccag cgtggaatgc tgtgtacacc 1380
gtagtcaatg atcctgatca tcagttcact gtcatcacag accccaagac caacgagggc 1440
attctgaaaa cagccaaggg cttggatttt gaggccaagc agcagtacat tctgcacgtg 1500
acagtggaaa atgaggagcc ctttgagggg tctcttgtcc cttccacagc cactgtcacc 1560
gtggatgtgg tagacgtgaa tgaagccccc atttttgtgc ctgcggagaa gagagtcgag 1620
gtgcctgagg actttggtgt gggtctggag atcgcatctt acactgcgcg agagccagac 1680
acattcatgg aacagaagat cacgtatcgg atttggaggg acactgccaa ttggctggag 1740
attaacccag agactggggt catttccact cgggctgaga tggacagaga agattcggag 1800
catgtgaaga acagcacgta tacagctctc atcattgcca cagatgatgg ttcacccatt 1860
gccactggca cagggactct tctcctggtc ctgtcagacg tcaacgacaa tgctcccatc 1920
ccagaacctc gaaatatgca gttctgccag agaaacccga agccccatgt catcaccatc 1980
ttggatccag accttccccc aaacacatcc cccttcactg cagagctcac ccatggggcc 2040
agcgtcaact ggaccattga gtacaatgac gcagaacaag aatctctcat tttgcaacca 2100
agaaaggact tagagattgg cgaatacaaa atcaatctca agctctcgga taaccagaat 2160
aaagaccagg tgaccacgtt ggaggtccac gtgtgtgact gtgaagggac cgtcaacaac 2220
tgcatgaagg cgatctccct ggaagcagga ttacaagttc ccgccatcct tggaatcctg 2280
ggagggatcc tggccctcct gattctgatc ctcctgctcc tactgtttct acggaggaga 2340
acggtggtca aagagccctt gctgccacca gatgacgata cccgggacaa tgtgtattac 2400
tatgatgaag agggaggtgg agaagaagac caggactttg atttgagcca gctgcacagg 2460
ggccttgatg ccagaccgga agtgattcga aatgatgtgg ctcccaccct catgagcatg 2520
ccccagtatc gtccccgtcc agccaatcct gatgaaatcg ggaacttcat cgatgaaaac 2580
ctgaaggcag cggacagtga ccccacagcg cccccttacg actctctgtt ggtgtttgac 2640
tatgagggga gtggttctga agctgcctcc ctgagctcgc tgaactcctc tgagtcagat 2700
caggaccagg actacgatta tctgaacgaa tggggcaacc ggttcaagaa gctggccgat 2760
atgtatggtg gcggcgaaga agactagaga gtcgttcctg tgtggcacca tgggagatgc 2820
agaatcatga tgtcagtggt ctttcagctc cttccctgac tttgtagaag agagactgat 2880
ctgagaagta tgcagattgc atactggtcc cactctacct accagtctgt ctgtgttagg 2940
agggttttca ctggttgttg gaatcttttt ctaaaatgtt tttgttttta cagtgctgtg 3000
atgtgatgaa ctgtaccctc tttttgtttt tgttttgagc tatgttctgc tccggacaca 3060
cagcccccaa gcccttcacc cctcactaat ttttttacat tgtatacttt cactcaatta 3120
ccatgtttat gttctgtatt ctaatagcca ctaagttcct gaattctgtt gcctggccca 3180
ggtgctattc tgtgacacag tagtgcctgg gcccttttat ggtaagagac aggtttcttg 3240
gtgtgggtgc aactgagctg gatagtgtat gtttcaaaca cctttcctgt gttctctccc 3300

cacctccaga gtgtctttac ttattcagct gtgtgtttgg ggcagaacaa aaaaataatg 3360
ggaccactat gcaagctgcg aagattctaa ggtgcacacc tgattcttag gcagatgcca 3420
tagtgagata tgttgctttg gttctctatc caatgctgtg accgggacct gcagcgaggt 3480
tttcggacac cgtggtttct tgcgtttctt tcaaaccagc agtaaaaaat ggttttttct 3540
gagagagact ggagtgccac caccaaagat agaggagaga agccaagctt ggggacagca 3600
agcatgccag tgaacctgac cactgtcatg agtcatgtgg gtggccacat gtccgtgaac 3660
ctggccagtt ggcacactga tggtgagggt acaaggaggc tagacctcgt cccacaaaat 3720
ttctggaaga attagggttg tctcagccaa tgtttcctag ctggaatcct gtccatgtat 3780
gtgttcctga agcccaggaa atacacccct ctagtgcctg cttttgatgg tagttataga 3840
aaagaccggc tgatttggac ctgagttgcc caatcttaag tacaaataga aaactgagac 3900
tatgctgggt gtgttggtgc acgcctttaa tcccggcact cgggaggcag agacagtctc 3960
agatctctct gagttcaagg tcagcctggt atagtaagtg aattccagga cagccagggc 4020
tacacagaaa ctctgtcttg gaaaaccaaa aaagaaaact gagaatatta gagattgtgc 4080
attttctcag aaagcaggaa gaaaacacca ctctgatggg aaaagggagg caaggccctt 4140
gagacttttc attgaaattg ctgtactcac ataattttgg aagcaaatga tgactgcaat 4200
caactgtgag aactgttggt ttctctgtag tttaattgtc taatgttgat agcgtgccct 4260
ttgtatgtag tttgagtgta tatgtgtgtg ggtgctgata attttgtatt ttgtggggag 4320
tggaaaaggc aagcaatcgg aactgttctc taagatgcat ttttatgaat tttattaaag 4380
agttttgtta aactgt 4396

<210> 51
<211> 886
<212> PRT
<213> Rattus norvegicus

<400> 51
   Met Gly Ala Arg Cys Arg Ser Phe Ser Ala Leu Leu Leu Leu Leu Gln
   1 5 10 15
   Val Ser Ser Trp Leu Cys Gln Gln Pro Glu Ser Glu Ser Asp Ser Cys
   20 25 30
   Arg Pro Gly Phe Ser Ser Glu Val Tyr Thr Phe Leu Val Pro Glu Arg
   35 40 45
   His Leu Glu Arg Gly His Ile Leu Gly Arg Val Lys Phe Glu Gly Cys
   50 55 60
   Thr Gly Arg Pro Arg Thr Ala Phe Phe Ser Glu Asp Ser Arg Phe Lys
   65 70 75 80
   Val Ser Thr Asp Gly Val Ile Thr Val Lys Arg His Leu Lys Leu His
   85 90 95
   Lys Leu Glu Thr Ser Phe Leu Val His Ala Trp Asp Ser Ser Tyr Arg
   100 105 110
   Lys Leu Ser Thr Lys Val Thr Leu Lys Ser Leu Gly His His His His
   115 120 125
   Arg His His His Arg Asp Pro Val Ser Glu Ser Asn Pro Glu Leu Leu
   130 135 140
   Thr Phe Pro Ser Phe His Gln Gly Leu Arg Arg Gln Lys Arg Asp Trp
   145 150 155 160
   Val Ile Pro Pro Ile Asn Cys Pro Glu Asn Gln Lys Gly Glu Phe Pro
   165 170 175
   Gln Arg Leu Val Gln Ile Lys Ser Asn Arg Asp Lys Glu Thr Thr Val
   180 185 190
   Phe Tyr Ser Ile Thr Gly Pro Gly Ala Asp Lys Pro Pro Val Gly Val
   195 200 205
   Phe Ile Ile Glu Arg Glu Thr Gly Trp Leu Lys Val Thr Gln Pro Leu
   210 215 220
   Asp Arg Glu Ala Ile Asp Lys Tyr Leu Leu Tyr Ser His Ala Val Ser
   225 230 235 240
   Ser Asn Gly Glu Ala Val Glu Asp Pro Met Glu Ile Val Val Thr Val
   245 250 255
   Thr Asp Gln Asn Asp Asn Arg Pro Glu Phe Ile Gln Glu Val Phe Glu
   260 265 270
   Gly Ser Val Ala Glu Gly Ala Leu Pro Gly Thr Ser Val Met Gln Val
   275 280 285
   Ser Ala Thr Asp Ala Asp Asp Asp Ile Asn Thr Tyr Asn Ala Ala Ile
   290 295 300
   Ala Tyr Thr Ile Leu Ser Gln Asp Pro Glu Leu Pro His Lys Asn Met
   305 310 315 320
   Phe Thr Val Asn Arg Asp Thr Gly Val Ile Ser Val Val Thr Ser Gly
   325 330 335
   Leu Asp Arg Glu Ser Tyr Pro Thr Tyr Thr Leu Val Val Gln Ala Ala
   340 345 350
   Asp Leu Gln Gly Glu Gly Leu Ser Thr Thr Ala Lys Ala Val Ile Thr
   355                 360                 365
   Val Lys Asp Ile Asn Asp Asn Ala Pro Ile Phe Asn Pro Ser Thr Tyr
       370                 375                 380
   Gln Gly Gln Val Leu Glu Asn Glu Val Gly Ala Arg Ile Ala Thr Leu
   385                 390                 395                 400
   Lys Val Thr Asp Asp Asp Ala Pro Asn Thr Pro Ala Trp Asn Ala Val
                   405                 410                 415
   Tyr Thr Val Val Asn Asp Pro Asp His Gln Phe Thr Val Ile Thr Asp
               420                 425                 430
   Pro Lys Thr Asn Glu Gly Ile Leu Lys Thr Ala Lys Gly Leu Asp Phe
           435                 440                 445
   Glu Ala Lys Gln Gln Tyr Ile Leu His Val Thr Val Glu Asn Glu Glu
       450                 455                 460
   Pro Phe Glu Gly Ser Leu Val Pro Ser Thr Ala Thr Val Thr Val Asp
   465                 470                 475                 480
   Val Val Asp Val Asn Glu Ala Pro Ile Phe Val Pro Ala Glu Lys Arg
                   485                 490                 495
   Val Glu Val Pro Glu Asp Phe Gly Val Gly Leu Glu Ile Ala Ser Tyr
               500                 505                 510
   Thr Ala Arg Glu Pro Asp Thr Phe Met Glu Gln Lys Ile Thr Tyr Arg
           515                 520                 525
   Ile Trp Arg Asp Thr Ala Asn Trp Leu Glu Ile Asn Pro Glu Thr Gly
       530                 535                 540
   Val Ile Ser Thr Arg Ala Glu Met Asp Arg Glu Asp Ser Glu His Val
   545                 550                 555                 560
   Lys Asn Ser Thr Tyr Thr Ala Leu Ile Ile Ala Thr Asp Asp Gly Ser
                   565                 570                 575
   Pro Ile Ala Thr Gly Thr Gly Thr Leu Leu Leu Val Leu Ser Asp Val
               580                 585                 590
   Asn Asp Asn Ala Pro Ile Pro Glu Pro Arg Asn Met Gln Phe Cys Gln
           595                 600                 605
   Arg Asn Pro Lys Pro His Val Ile Thr Ile Leu Asp Pro Asp Leu Pro
       610                 615                 620
   Pro Asn Thr Ser Pro Phe Thr Ala Glu Leu Thr His Gly Ala Ser Val
   625                 630                 635                 640
   Asn Trp Thr Ile Glu Tyr Asn Asp Ala Glu Gln Glu Ser Leu Ile Leu
                   645                 650                 655
   Gln Pro Arg Lys Asp Leu Glu Ile Gly Glu Tyr Lys Ile Asn Leu Lys
               660                 665                 670
   Leu Ser Asp Asn Gln Asn Lys Asp Gln Val Thr Thr Leu Glu Val His
           675                 680                 685
   Val Cys Asp Cys Glu Gly Thr Val Asn Asn Cys Met Lys Ala Ile Ser
       690                 695                 700
   Leu Glu Ala Gly Leu Gln Val Pro Ala Ile Leu Gly Ile Leu Gly Gly
   705                 710                 715                 720
   Ile Leu Ala Leu Leu Ile Leu Ile Leu Leu Leu Leu Leu Phe Leu Arg
                   725                 730                 735
   Arg Arg Thr Val Val Lys Glu Pro Leu Leu Pro Pro Asp Asp Asp Thr
               740                 745                 750
   Arg Asp Asn Val Tyr Tyr Tyr Asp Glu Glu Gly Gly Gly Glu Glu Asp
           755                 760                 765
   Gln Asp Phe Asp Leu Ser Gln Leu His Arg Gly Leu Asp Ala Arg Pro
       770                 775                 780
   Glu Val Ile Arg Asn Asp Val Ala Pro Thr Leu Met Ser Met Pro Gln
   785                 790                 795                 800
   Tyr Arg Pro Arg Pro Ala Asn Pro Asp Glu Ile Gly Asn Phe Ile Asp
                   805                 810                 815
   Glu Asn Leu Lys Ala Ala Asp Ser Asp Pro Thr Ala Pro Pro Tyr Asp
               820                 825                 830
   Ser Leu Leu Val Phe Asp Tyr Glu Gly Ser Gly Ser Glu Ala Ala Ser
           835                 840                 845
   Leu Ser Ser Leu Asn Ser Ser Glu Ser Asp Gln Asp Gln Asp Tyr Asp
       850                 855                 860
   Tyr Leu Asn Glu Trp Gly Asn Arg Phe Lys Lys Leu Ala Asp Met Tyr
   865                 870                 875                 880
   Gly Gly Gly Glu Glu Asp
                   885

<210>  52

<211>  4877
<212>  DNA
<213>  Pan troglodytes

<400>  52
agccaatcag cggtacgggg ggcggtgcct ccggggctca cctggcggca gccgcgcacc      60

ctctctcagt ggcgtcggaa ctgcaaagca cctgtgagct tgcggaagtc agttcagact     120

ccagcccgct ccagcccggc ccgacccgac cgcacccggc gcctgccctc tctcggcgtc     180

cccggccagc catgggccct tggagccgca gcctctcagc gctgctgctg ctgctgcagg     240

tctcctcttg gctctgccag gagccggagc cctgccaccc tggctttgac gccgagagct     300

acacgttcac ggtgccccgg ctccacctgg agagaggccg cgtcctgggc agagtgaatt     360

ttgaagattg caccggtcga caaaggacag cctatttttc cctcgacacc cgattcaaag     420

tgggcacaga tggtgtgatt acagtcaaaa ggcctctacg gtttcataac ccacagatcc     480

atttcttggt ctacgcctgg gactccacct acagaaagtt ttccaccaaa gtcacgctga     540

atacagtggg gcaccaccac cgccccccgc cccatcaggc ctctgtttct ggaatccaag     600

cagaattgct cacatttccc aactcctctc ctggcctcag aagacagaag agagactggg     660

ttattcctcc catcagctgc ccagaaaatg aaaaaggccc atttcctaaa aacctggttc     720

agatcaaatc caacaaagac aaagaaggca aggttttcta cagcatcact ggccaaggag     780

ctgacacacc ccctgttggt gtctttatta ttgaaagaga aacaggatgg ctgaaggtga     840

cagagcctct ggatagagaa cgcattgcca catacactct cttctctcat gctgtgtcat     900

ccaacgggaa tgccgttgag gatccaatgg agattttgat cacggtgacc gatcagaatg     960

acaacaaacc cgaattcacc caggaggtct ttaaggggtc tgtcatggaa ggtgctcttc    1020

caggaacctc tgtgatggag gtcacagcca cagacgcgga cgatgatgtg aacacctaca    1080

atgccgccat cgcttacacc atcctcagcc aagatcctga gctgcctgac aaaaatatgt    1140

tcaccattaa caggaacaca ggagtcatca gtgtggtcac cactgggctg gaccgagaga    1200

gtttccctac gtataccctg gtggttcaag ctgctgatct tcaaggtgag gggttaagca    1260

caacagcaac agctgtgatc acagtcactg acaccaacga taaccctccg atcttcaatc    1320

ccaccacgta caagggtcag gtgcctgaga acgaggctaa cgtcataatc accacactga    1380

aagtgactga tgctgatgcc cccaataccc cagcgtggga ggctgtatac accatattga    1440

atgatgatgg tggacaattt gtcgtcacca caaatccagt gaacaacgat ggcattttga    1500

aaacagcaaa gggcttggat tttgaggcca agcagcagta cattctacac gtagcagtga    1560

cgaatgtggt accttttgag gtctctctca ccacctccac agccaccgtc accgtggatg    1620

tgctggatgt gaatgaagcc cccatctttg tgcctcctga aaagagagtg gaagtgtcgg    1680

aggactttgg cgtgggccag gaaatcacat cctacactgc ccaggagcca gacacattta    1740

tggaacagaa aataacatat cggatttgga gagacactgc caactggctg gagattaatc    1800

cggacactgg tgccatttcc actcgggctg agctggacag ggaggatttg gagcacgtga    1860

agaacagcac gtacacagcc ctaatcatag ctacagacaa tggttctcca gttgctactg    1920

gaacagggac acttctgctg atcctgtctg atgtgaatga caatgccccc ataccagaac 1980
ctcgaactat attcttctgt gagaggaatc caaagcctca ggtcataaac atcattgatg 2040
cagaccttcc tcccaataca tctcccttca cagcagaact aacacacggg gcgagtgcca 2100
actggaccat tcagtacaac gacccaaccc aagaatctat cattttgaag ccaaagatgg 2160
ccttagaggt gggtgactac aaaatcaatc tcaagctcat ggataaccag aataaagacc 2220
aagtgaccac cttagaggtc agcgtgtgtg actgtgaagg ggccgccggc gtctgtagga 2280
aggcacagcc tgtcgaagca ggattgcaaa ttcctgccat tctggggatt cttggaggaa 2340
ttcttgcttt gctaattctg attctgctgc tcttgctgtt tcttcggagg agagcggtgg 2400
tcaaagagcc cttactgccc ccagaggatg acacccggga caacgtttat tactatgatg 2460
aagaaggagg cggagaagag gaccaggact ttgacttgag ccagttgcac aggggcctgg 2520
acgctcggcc tgaagtgact cgtaacgacg ttgcaccaac cctcatgagt gtcccccggt 2580
accttccccg ccctgccaat cccgatgaaa ttggaaattt tattgatgaa aatctgaaag 2640
cggctgatac tgaccccaca gccccgcctt atgattctct tctcgtgttt gactatgaag 2700
gaagcggttc cgaagctgct agtctgagct ccctgaactc ctcagagtca gacaaagacc 2760
aggactatga ctacttgaac gaatggggca atcgcttcaa gaagctggcg gacatgtacg 2820
gaggtggcga ggacgactag gggactcgag agaggcgggc cctagacccg tgtgctggga 2880
aatgcagaaa tcatgttgct ggtggttttt cagctccctt cccttgagat gagtttctgg 2940
ggaaaagaag agactggttg gtgatgcagt tagtatagct ttatgctctc tccactttat 3000
agctctaata agtgtgtgtt agaaaagttt tgacttattt cttgaagctt tttttttttt 3060
cccatcactc tacatggtgg tgatgtccaa aagataccca aattttaata ttccagaaga 3120
acaactttag catcagaagg ttcacccagc accttgcaga ttttcttaag gaattttgtc 3180
tcacttttaa aaagaagggg agaagtcagc tactctagtt ctgttgtttt gtgtatataa 3240
ttttttaaaa aaaatttgtg tgctcctgct cattgctaca ctggtgtgtc cctctgcctt 3300
tttttttttt aagacagggt ctcattctgt cggccaggct ggagtgcagt ggtgcaatca 3360
cagctcactg cagccttgtc ctcccaggct caagctatcc ttgcacctca gcctcccaag 3420
tagctgggac cacaggcatg caccactacg catgactaat tttttaaata tttgagacgg 3480
ggtctccctg tgttacccag gctggtctca aactcctggg ctcaagtgat cctcccatct 3540
tggcctccca gagtattggg attacagaca tgagccactg cacctgccca gctccccaac 3600
tccctgccat tttttaagag acagtttcgc tccattgccc aggcctggga tgcagtgatg 3660
tgatcatagc tcactgtaac ctcaaactct ggggctcaag caattctccc accagcctcc 3720
tttttatttt tttgtacaga tggggtcttg ctatgttgcc catgctggtc ttaaactcct 3780
ggcctcaagc aatcctcctg ccttggcccc ccaaagtgct gggattgcgg gcatgagctg 3840
ctgtgcccag cctccatgtt ttaatatcaa ctctccctcc tcaattcagt tgctttgccc 3900
aagataggag ttctccgatg cagaaattat tgggctcttt tagggtaaga agctcgtgtc 3960

ttcgtctggc cacatcttga ctaggtattg tctactctga agacctttaa tggcttccct 4020

ctttcatctc ctgagtatgt aacttgcaat gggcatctat ccagtgactt gttctgagta 4080

agtgtgttcg ttaatgttta tttagctctg aagcaagagt gatatactcc aggacttaga 4140

atagtgccta aagtgctgca gccaaagaca gagcggaact atgaaaattg ggcttggaga 4200

tggcaggaga gcttgtcatt gagcctggca atttagcaaa ctgatgctga ggatgattga 4260

ggtgggtcta cctcatctct gaaaattctg gaaggaatgg aggagtctca acatgtgttt 4320

ctgacacagg atctgtggtt tgtactcaaa ggccagaatc cccaagtgcc tgcttttgat 4380

gatgtctaca gaaaatgctg gctgagctga acacatttgc ccaattccag atgtgcatag 4440

aaaactgaga atattcaaaa ttccaaattt ttttcttagg agcaagaaga aaatgtggcc 4500

ctaaaggggg ttagttgagg ggtagggggt agtgaggatc ttgatttgga tctcttttta 4560

tttaaatgtg aatttcaact tttgacaatc aaagaaaaga gttttattga aatagcttta 4620

ctgtttctca agtgttttgg agaaaaaaat caaccctgca atcacttttt ggaattgtct 4680

tgatttttcg gcagttcaag ctatatcgaa tatagttctg tgtagagaat gtcactgtag 4740

ttttgagtgt atacatgtgt gggtgctgat aattgtgtat tttctctggg ggtggaaaag 4800

gaaaacgatt caagctgaga aaagtattct caaagatgca tttttataaa ttttattaaa 4860

gaattttgtt aaaccat 4877

<210> 53
<211> 882
<212> PRT
<213> Pan troglodytes

<400> 53

Met Gly Pro Trp Ser Arg Ser Leu Ser Ala Leu Leu Leu Leu Leu Gln
1               5                   10                  15

Val Ser Ser Trp Leu Cys Gln Glu Pro Glu Pro Cys His Pro Gly Phe
            20                  25                  30

Asp Ala Glu Ser Tyr Thr Phe Thr Val Pro Arg Leu His Leu Glu Arg
        35                  40                  45

Gly Arg Val Leu Gly Arg Val Asn Phe Glu Asp Cys Thr Gly Arg Gln
    50                  55                  60

Arg Thr Ala Tyr Phe Ser Leu Asp Thr Arg Phe Lys Val Gly Thr Asp
65                  70                  75                  80

Gly Val Ile Thr Val Lys Arg Pro Leu Arg Phe His Asn Pro Gln Ile
                85                  90                  95

His Phe Leu Val Tyr Ala Trp Asp Ser Thr Tyr Arg Lys Phe Ser Thr
            100                 105                 110

Lys Val Thr Leu Asn Thr Val Gly His His His Arg Pro Pro Pro His
        115                 120                 125

Gln Ala Ser Val Ser Gly Ile Gln Ala Glu Leu Leu Thr Phe Pro Asn
    130                 135                 140

Ser Ser Pro Gly Leu Arg Arg Gln Lys Arg Asp Trp Val Ile Pro Pro
145                 150                 155                 160

Ile Ser Cys Pro Glu Asn Glu Lys Gly Pro Phe Pro Lys Asn Leu Val
                165                 170                 175

Gln Ile Lys Ser Asn Lys Asp Lys Glu Gly Lys Val Phe Tyr Ser Ile
            180                 185                 190

Thr Gly Gln Gly Ala Asp Thr Pro Pro Val Gly Val Phe Ile Ile Glu
        195                 200                 205

Arg Glu Thr Gly Trp Leu Lys Val Thr Glu Pro Leu Asp Arg Glu Arg
    210                 215                 220

Ile Ala Thr Tyr Thr Leu Phe Ser His Ala Val Ser Ser Asn Gly Asn
225                 230                 235                 240

Ala Val Glu Asp Pro Met Glu Ile Leu Ile Thr Val Thr Asp Gln Asn
                245                 250                 255

Asp Asn Lys Pro Glu Phe Thr Gln Glu Val Phe Lys Gly Ser Val Met
            260                 265                 270

Glu Gly Ala Leu Pro Gly Thr Ser Val Met Glu Val Thr Ala Thr Asp
        275                 280                 285

Ala Asp Asp Asp Val Asn Thr Tyr Asn Ala Ala Ile Ala Tyr Thr Ile
    290                 295                 300

Leu Ser Gln Asp Pro Glu Leu Pro Asp Lys Asn Met Phe Thr Ile Asn
305                 310                 315                 320

Arg Asn Thr Gly Val Ile Ser Val Val Thr Thr Gly Leu Asp Arg Glu
                325                 330                 335

Ser Phe Pro Thr Tyr Thr Leu Val Val Gln Ala Ala Asp Leu Gln Gly
            340                 345                 350

Glu Gly Leu Ser Thr Thr Ala Thr Ala Val Ile Thr Val Thr Asp Thr
        355                 360                 365

Asn Asp Asn Pro Pro Ile Phe Asn Pro Thr Thr Tyr Lys Gly Gln Val
    370                 375                 380

Pro Glu Asn Glu Ala Asn Val Ile Ile Thr Thr Leu Lys Val Thr Asp
385                 390                 395                 400

Ala Asp Ala Pro Asn Thr Pro Ala Trp Glu Ala Val Tyr Thr Ile Leu
                405                 410                 415

Asn Asp Asp Gly Gly Gln Phe Val Val Thr Thr Asn Pro Val Asn Asn
            420                 425                 430

Asp Gly Ile Leu Lys Thr Ala Lys Gly Leu Asp Phe Glu Ala Lys Gln
        435                 440                 445

Gln Tyr Ile Leu His Val Ala Val Thr Asn Val Val Pro Phe Glu Val
    450                 455                 460

Ser Leu Thr Thr Ser Thr Ala Thr Val Thr Val Asp Val Leu Asp Val
465                 470                 475                 480

Asn Glu Ala Pro Ile Phe Val Pro Pro Glu Lys Arg Val Glu Val Ser
                485                 490                 495

Glu Asp Phe Gly Val Gly Gln Glu Ile Thr Ser Tyr Thr Ala Gln Glu
            500                 505                 510

Pro Asp Thr Phe Met Glu Gln Lys Ile Thr Tyr Arg Ile Trp Arg Asp
        515                 520                 525

Thr Ala Asn Trp Leu Glu Ile Asn Pro Asp Thr Gly Ala Ile Ser Thr
    530                 535                 540

Arg Ala Glu Leu Asp Arg Glu Asp Leu Glu His Val Lys Asn Ser Thr
545                 550                 555                 560

Tyr Thr Ala Leu Ile Ile Ala Thr Asp Asn Gly Ser Pro Val Ala Thr
                565                 570                 575

Gly Thr Gly Thr Leu Leu Leu Ile Leu Ser Asp Val Asn Asp Asn Ala
            580                 585                 590

Pro Ile Pro Glu Pro Arg Thr Ile Phe Phe Cys Glu Arg Asn Pro Lys
        595                 600                 605

Pro Gln Val Ile Asn Ile Ile Asp Ala Asp Leu Pro Pro Asn Thr Ser
    610                 615                 620

Pro Phe Thr Ala Glu Leu Thr His Gly Ala Ser Ala Asn Trp Thr Ile
625                 630                 635                 640

Gln Tyr Asn Asp Pro Thr Gln Glu Ser Ile Ile Leu Lys Pro Lys Met
                645                 650                 655

Ala Leu Glu Val Gly Asp Tyr Lys Ile Asn Leu Lys Leu Met Asp Asn
            660                 665                 670

Gln Asn Lys Asp Gln Val Thr Thr Leu Glu Val Ser Val Cys Asp Cys
        675                 680                 685

Glu Gly Ala Ala Gly Val Cys Arg Lys Ala Gln Pro Val Glu Ala Gly
    690                 695                 700

Leu Gln Ile Pro Ala Ile Leu Gly Ile Leu Gly Gly Ile Leu Ala Leu
705                 710                 715                 720

Leu Ile Leu Ile Leu Leu Leu Leu Leu Phe Leu Arg Arg Arg Ala Val
                725                 730                 735

Val Lys Glu Pro Leu Leu Pro Pro Glu Asp Asp Thr Arg Asp Asn Val
            740                 745                 750

Tyr Tyr Tyr Asp Glu Glu Gly Gly Gly Glu Glu Asp Gln Asp Phe Asp
        755                 760                 765

Leu Ser Gln Leu His Arg Gly Leu Asp Ala Arg Pro Glu Val Thr Arg
    770                 775                 780

Asn Asp Val Ala Pro Thr Leu Met Ser Val Pro Arg Tyr Leu Pro Arg
785                 790                 795                 800

Pro Ala Asn Pro Asp Glu Ile Gly Asn Phe Ile Asp Glu Asn Leu Lys
                805                 810                 815

Ala Ala Asp Thr Asp Pro Thr Ala Pro Pro Tyr Asp Ser Leu Leu Val
            820                 825                 830

Phe Asp Tyr Glu Gly Ser Gly Ser Glu Ala Ala Ser Leu Ser Ser Leu
        835                 840                 845

Asn Ser Ser Glu Ser Asp Lys Asp Gln Asp Tyr Asp Tyr Leu Asn Glu
    850                 855                 860

Trp Gly Asn Arg Phe Lys Lys Leu Ala Asp Met Tyr Gly Gly Gly Glu
865                 870                 875                 880

Asp Asp

<210>  54
<211>  26
<212>  DNA
<213>  Artificial

<220>
<223>  an artificially synthesized sequence

<400>  54
cagctccaca acctacatca ttccgt                                          26


<210>  55
<211>  12
<212>  DNA
<213>  Artificial

<220>
<223>  an artificially synthesized sequence

<400>  55
acggaatgat gt                                                         12


<210>  56
<211>  26
<212>  DNA
<213>  Artificial

<220>
<223>  an artificially synthesized sequence

<400>  56
ctccaatgcc tgctcttgat ggtagc                                          26


<210>  57
<211>  26
<212>  DNA
<213>  Artificial

<220>
<223>  an artificially synthesized sequence

<400>  57
tctctgtgta gccctggctg tcctag                                          26


<210>  58
<211>  26
<212>  DNA
<213>  Artificial

<220>
<223>  an artificially synthesized sequence

<400>  58
cttcccgtat gctaccttgt ctccac                                          26


<210>  59
<211>  26
<212>  DNA
<213>  Artificial

<220>
<223>  an artificially synthesized sequence

<400>  59
ccaacagtcc tgcatgcttg taatga                                          26

## Description

### Technical Field

The present invention provides a marker gene and protein for Purkinje progenitor cells, and relates to the use of the gene and protein in identifying Purkinje progenitor cells.

### Background Art

The brain functions by forming a complex network from a great variety of neurons. Its failure may result in various neurological diseases. To treat such diseases, transplantation and regeneration therapies are currently investigated. The most important thing in these therapeutic methods is to correctly identify various types of neurons in transplantation materials. Furthermore, from the viewpoint of improving safety and therapeutic effect, it is desirable to isolate only the type of cells that are needed for transplantation.

The cerebellum works on smooth motor functions such as regulation of balance, posture, and voluntary movement. The failure of cerebellar function due to cerebellar tumor, cerebellar vermis degeneration caused by chronic alcoholism, spinocerebellar degeneration, or such results in dynamic ataxia and balance disorder. Functional recovery can be achieved by replenishing lost neurons and reconstituting the network. There are about five types of neurons in the cerebellum including Purkinje cell, and formation of a proper network of the respective neurons according to organogenic program enables neurotransmission.

Research on the development of cerebellar Purkinje cells has advanced to a close understanding of their origin. However, there are few established markers for identifying progenitor cells. Cell surface markers remain unidentified, and there is no established technique for isolating viable progenitor cells.

The Neph3 gene (hereinafter sometimes referred to as "65B13") is known to be transiently expressed in dopamine-producing neuron progenitor cells after termination of cell division (see Patent Document 1); however, there is no report published on connection of the gene with Purkinje cell. Meanwhile, it has been reported that the types of spinal cord interneurons and Purkinje cells can be identified by using the expression of the Corl1 gene and Corl2 gene as an indicator, respectively (see Patent Documents 2 and 3). At the same time, there are known methods for inducing Purkinje cells from ES cells, but they are not useful practically (Non-Patent Documents 1 and 2).

### Prior Art Documents

### Patent Documents

Patent Document 1: WO 2004/038018
Patent Document 2: WO 2006/022243
Patent Document 3: WO 2006/082826

### Non-Patent Documents

Non-Patent Document 1: Su HL, Muguruma K, Matsuo-Takasaki M, Kengaku M, Watanabe K, Sasai Y Generation of cerebellar neuron precursors from embryonic stem cells. Dev Biol. 2006 Feb 15;290(2):287-96.
Non-Patent Document 2: Salero E, Hatten ME. Differentiation of ES cells into cerebellar neurons. Proc Natl Acad Sci USA. 2007 Feb 20;104(8):2997-3002.

### Disclosure of the Invention

### [Problems to be Solved by the Invention]

The present invention was achieved in view of the above circumstances. An objective of the present invention is to provide markers that enable selective identification of Purkinje progenitor cells. Another objective of the present invention is to provide methods of using the markers as an indicator to identify Purkinje progenitor cells, and reagents for use in these methods.

### [Means for Solving the Problems]

The present inventors identified a selective marker, 65B 13, for GABA neuron progenitor cells in the cerebellum, and successfully isolated GABA neuron progenitor cells using antibodies that bind to the protein encoded by the gene. Specifically, 65B 13 was discovered to be useful as a marker to isolate GABA-producing neuron progenitor cells in the cerebellum.

Of GABA-producing neurons, only Purkinje cells transmit neural signals outwardly from the cerebellar cortex. Degeneration of Purkinje cells is involved in the onset of spinocerebellar degeneration and such. 65B 13 is a useful marker for isolating GABA neuron progenitor cells including Purkinje cells. However, to obtain high-purity Purkinje progenitor cells, Purkinje progenitor cells have to be further purified from a 65B13-positive cell population using a marker selectively expressed in Purkinje progenitor cells.

The present inventors conducted dedicated studies, and as a result revealed that E-cadherin is a useful marker for isolating Purkinje progenitor cells from a 65B13-positive cell population, thereby completing the present invention.

Specifically, E-cadherin was demonstrated to be a useful marker for isolating Purkinje progenitor cells when used in combination with 65B 13.

The present invention provides markers that enable selective detection of Purkinje progenitor cells, methods of detecting Purkinje progenitor cells using the markers as an indicator, cell populations detected by the methods, and reagents for use in the methods. More specifically, the present invention provides:
[1] a method for detecting or selecting a Purkinje progenitor cell, which comprises the steps of:
   (A) detecting a protein selected from:
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B) detecting a protein selected from:
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[2] a method for detecting or selecting a Purkinje progenitor cell, which comprises the step of detecting a marker protein by linking a polynucleotide encoding the marker protein to a promoter that controls (induces) expression of an mRNA translated into a protein selected from:
   (A) protein selected from:
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B) a protein selected from:
      (v') a protein comprising the amino acid sequence of SEQ ID NO: 47, 49, 51, or 53;
         (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
         (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
         (viii') a protein which is expressed in a Purkinje progenitor cell and comprises an amino acid sequence having 80% or higher sequence identity to the amino acid sequence of SEQ ID NO: 47, 49, 51, or 53;
[3] the method of [1] or [2], wherein the step of detecting the protein comprises the steps of:
   (d) contacting a test cell sample with an antibody that binds to the protein described in [1] or [2]; and
   (e) detecting reactivity;
[4] the method of any one of [1] to [3], wherein the detection step is followed by the step of separating a Purkinje progenitor cell from the detected sample;
[5] a method for detecting or selecting a Purkinje progenitor cell, which comprises the steps of:
   (A) detecting the expression of a polynucleotide capable of hybridizing to a polynucleotide selected from (i),(ii), (iii), and (iv) below, or a complementary sequence thereof:
      (i) a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iii) a polynucleotide which is expressed in a Purkinje progenitor cell and hybridizes under stringent conditions to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iv) a polynucleotide which is expressed in a Purkinje progenitor cell and has 80% or higher sequence identity to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
   (B) detecting the expression of a polynucleotide capable of hybridizing to a polynucleotide selected from (i'), (ii'), (iii'), and (iv') below, or a complementary sequence thereof:
      (i') a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (iii') a polynucleotide which is expressed in a Purkinje progenitor cell and hybridizes under stringent conditions to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
      (iv') a polynucleotide which is expressed in a Purkinje progenitor cell and has 80% or higher sequence identity to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or the nucleotide sequence from positions 192 to 2849 of SEQ ID NO: 52;
[6] the method of [5], wherein the step of detecting the expression of a polynucleotide comprises the steps of:
   (a) contacting a test cell sample with a polynucleotide that can hybridize to the polynucleotide described in [5] or to a complementary sequence thereof, or with a probe comprising the polynucleotide; and
   (b) detecting reactivity;
[7] the method of [6], wherein the probe is contacted with mRNA prepared from the test cell sample or a complementary DNA (cDNA) transcribed from the mRNA in step (a);
[8] the method of [5], wherein the step of detecting the expression of a polynucleotide that can hybridize to the polynucleotide described in [5] or to a complementary sequence thereof comprises the steps of:
   (a-1) conducting gene amplification using a polynucleotide derived from the test cell sample as a template, and a primer comprising a polynucleotide that can hybridize to the polynucleotide described in [5] or to a complementary sequence thereof, or a set of primers comprising a polynucleotide that can hybridize to the polynucleotide selected in [5] or to a complementary sequence thereof; and
   (b-1) detecting the resulting amplification product;
[9] the method of [8], wherein mRNA prepared from the test cell sample or a complementary DNA (cDNA) transcribed from the mRNA is used as a template in step (a-1);
[10] the method of any one of [5] to [9], wherein the detection step is followed by the step of separating a Purkinje progenitor cell from the detected sample;
[11] a kit for detecting a Purkinje progenitor cell, which comprises a probe, a primer, or a set of primers that enable detection of the expression of or selection of a polynucleotide that can hybridize to a polynucleotide selected from (A) and (B) below, or to a complementary sequence thereof:
   (A)
      (i) a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iii) a polynucleotide which is expressed in a Purkinje progenitor cell and hybridizes under stringent conditions to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iv) a polynucleotide which is expressed in a Purkinje progenitor cell and has 80% or higher sequence identity to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or the nucleotide sequence from positions 196 to 2262 of SEQ ID NO: 35;
   (B)
      (i') a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (iii') a polynucleotide which is expressed in a Purkinje progenitor cell and hybridizes under stringent conditions to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
      (iv') a polynucleotide which is expressed in a Purkinje progenitor cell and has 80% or higher sequence identity to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
[12] a kit for detecting or selecting a Purkinje progenitor cell, which comprises antibodies that bind to proteins described in:
   (A) a protein selected from:
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B) a protein selected from:
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[13] a kit for detecting or selecting a Purkinje progenitor cell, which comprises a polynucleotide comprising a polynucleotide encoding a marker protein linked to a promoter that controls (induces) expression of an mRNA translated into a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 5 1, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[14] a method for producing a Purkinje progenitor cell population, which comprises the steps of:
   (VII) obtaining a cell population potentially containing a Purkinje progenitor cell;
   (VIII) detecting a Purkinje progenitor cell using the method of any one of [1] to [10]; and
   (IX) growing the cell detected or selected in step (VIII);
[15] the production method of [14], wherein the Purkinje progenitor cell is used to treat cerebellar degeneration;
[16] a Purkinje progenitor cell population obtained by the steps of:
   (VII) obtaining a cell population potentially containing a Purkinje progenitor cell;
   (VIII) detecting or selecting a Purkinje progenitor cell using the method of any one of [1] to [10]; and
   (IX) growing the cell detected in step (VIII);
[17] a reagent for detecting or selecting a Purkinje progenitor cell, which comprises a probe, a primer, or a set of primers that enable to detect the expression of a polynucleotide that can hybridize to a polynucleotide selected from (A) and (B) below, or to a complementary sequence thereof:
   (A)
      (i) a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iii) a polynucleotide which is expressed in a Purkinje progenitor cell and hybridizes under stringent conditions to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iv) a polynucleotide which is expressed in a Purkinje progenitor cell and has 80% or higher sequence identity to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
   (B)
      (i') a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (iii') a polynucleotide which is expressed in a Purkinje progenitor cell and hybridizes under stringent conditions to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
      (iv') a polynucleotide which is expressed in a Purkinje progenitor cell and has 80% or higher sequence identity to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
[18] a reagent for detecting or selecting a Purkinje progenitor cell, which comprises an antibody that binds to a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[19] a reagent for detecting or selecting a Purkinje progenitor cell, which comprises a polynucleotide comprising a polynucleotide encoding a marker protein linked to a promoter that controls (induces) expression of an mRNA translated into a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[20] a polynucleotide for detecting or selecting a Purkinje progenitor cell for use in regeneration medicine to treat cerebellar degeneration, which can hybridize to a polynucleotide selected from (A) and (B) below, or to a complementary sequence thereof:
   (A)
      (i) a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iii) a polynucleotide which is expressed in a Purkinje progenitor cell and hybridizes under stringent conditions to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iv) a polynucleotide which is expressed in a Purkinje progenitor cell and has 80% or higher sequence identity to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or the nucleotide sequence from positions 196 to 2262 of SEQ ID NO: 35;
   (B)
      (i') a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (iii') a polynucleotide which is expressed in a Purkinje progenitor cell and hybridizes under stringent conditions to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
      (iv') a polynucleotide which is expressed in a Purkinje progenitor cell and has 80% or higher sequence identity to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
[21] an antibody for detecting or selecting a Purkinje progenitor cell for use in regeneration medicine to treat cerebellar degeneration, which binds to a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36;
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53.
   Furthermore, the present invention relates to:
[22] a method for treating cerebellar degeneration, which comprises transplanting a Purkinje progenitor cell detected or selected by using a polynucleotide that can hybridize to a polynucleotide selected from (A), and (B) below, or to a complementary sequence thereof:
   (A)
      (i) a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iii) a polynucleotide which is expressed in a Purkinje progenitor cell and hybridizes under stringent conditions to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35;
      (iv) a polynucleotide which is expressed in a Purkinje progenitor cell and has 80% or higher sequence identity to a polynucleotide comprising:
         the nucleotide sequence from positions 178 to 2280 of SEQ ID NO: 1;
         the nucleotide sequence from positions 127 to 2079 of SEQ ID NO: 3;
         the nucleotide sequence from positions 668 to 2770 of SEQ ID NO: 19;
         the nucleotide sequence from positions 130 to 2232 of SEQ ID NO: 21;
         the nucleotide sequence from positions 199 to 2100 of SEQ ID NO: 23;
         the nucleotide sequence from positions 199 to 2325 of SEQ ID NO: 25;
         the nucleotide sequence from positions 15 to 2325 of SEQ ID NO: 27;
         the nucleotide sequence from positions 15 to 1916 of SEQ ID NO: 29;
         the nucleotide sequence from positions 199 to 1950 of SEQ ID NO: 31;
         the nucleotide sequence from positions 15 to 1766 of SEQ ID NO: 33; or
         the nucleotide sequence from positions 196 to 2262 of SEQ ID NO: 35; and
   (B)
      (i') a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52;
      (iii') a polynucleotide which is expressed in a Purkinje progenitor cell and hybridizes under stringent conditions to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or
         the nucleotide sequence from positions 192 to 2849 of SEQ ID NO: 52; and
      (iv') a polynucleotide which is expressed in a Purkinje progenitor cell and has 80% or higher sequence identity to a polynucleotide comprising:
         the nucleotide sequence from positions 125 to 2773 of SEQ ID NO: 46;
         the nucleotide sequence from positions 128 to 2782 of SEQ ID NO: 48;
         the nucleotide sequence from positions 127 to 2787 of SEQ ID NO: 50; or the nucleotide sequence from positions 192 to 2849 of SEQ ID NO: 52;
[23] the method of [22] for transplanting a Purkinje progenitor cell, wherein the Purkinje progenitor cell is a spinal cord or cerebellar Purkinje progenitor cell;
[24] the method of [22] or [23] for transplanting a Purkinje progenitor cell, wherein the polynucleotide comprises at least 10 consecutive nucleotides of a polynucleotide selected from (A) and (B) mentioned above, or a complementary sequence thereof;
[25] the method of [22] or [23] for transplanting a Purkinje progenitor cell, wherein the polynucleotide comprises at least 15 consecutive nucleotides of a polynucleotide selected from (A) and (B) mentioned above, or a complementary sequence thereof; and
[26] a method for treating cerebellar degeneration, which comprises transplanting a Purkinje progenitor cell detected or selected by using an antibody that binds to a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53;
[27] the method of [26] for transplanting a Purkinje progenitor cell, wherein the Purkinje progenitor cell is a spinal cord or cerebellar Purkinje progenitor cell; and
[28] the method of [26] or [27] for transplanting a Purkinje progenitor cell, wherein the antibody binds to a protein selected from:
   (A)
      (v) a protein comprising the amino acid sequence of SEQ ID NO: 2, 4, 20, 22, 24, 26, 28, 30, 32, 34, or 36;
      (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
      (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
      (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
   (B)
      (v') a protein comprising the amino acid sequence of SEQ ID NO: 47, 49, 51, or 53;
      (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
      (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
      (viii') a protein which is expressed in a Purkinje progenitor cell and comprises an amino acid sequence having 80% or higher sequence identity to the amino acid sequence of SEQ ID NO: 47, 49, 51, or 53.

### [Effects of the Invention]

The present invention identified selective markers 65B13 and E-cadherin for Purkinje progenitor cells, and successfully isolated Purkinje progenitor cells by using antibodies against these markers. This technique can provide viable Purkinje progenitor cells, and is expected to be useful in preparing materials for transplantation therapy for degenerative diseases, search of specific genes, discovery of drugs targeting Purkinje cells, etc.

Highly pure Purkinje cells can be obtained by methods for preparing Purkinje progenitor cells using the markers of the present invention. Thus, the methods are applicable to, for example, drug discovery or regeneration medicine targeting spinocerebellar degeneration.

### Brief Description of the Drawings

Fig. 1 shows the expression pattern of the 65B 13 gene in the nervous system of fetal mouse. A, E12.5 sagittal section; B, E12.5 cerebellar primordium; C, E12.5 spinal cord; D, E10.5 spinal cord; E, E12.5 spinal cord; F, E14.5 spinal cord. CB, cerebellar primordium; SC, spinal cord.
Fig. 2 shows a comparison of the 65B13, Corl2, and Pax2 expressions in the fetal cerebellar primordium. Fig. 2A shows the result of expression analysis at E12.5; Fig. 2B shows the result of expression analysis at E14.5. In Fig. 2B, the left and right photographs show the expression of 65B 13 or Pax2, respectively.
Fig. 3 shows that 65B13-positive cells could be isolated from the cerebellar primordia of fetal mouse. Fig. 3A shows results for the E12.5 cerebella and isolated 65B13-positive cells. Fig. 3B shows results of the E14.5 cerebella and isolated 65B13-positive cells.
Fig. 4 shows differentiation of 65B13-positive cells isolated from the cerebellar primordia of E12.5 (A) and E14.5 (B) mice into GABA-producing Purkinje cells and GABA-producing Golgi cells, respectively.
Fig. 5 shows the structures of DNA constructs that can be used to select GABA-producing neuron progenitor cells.
Fig. 6 shows that a foreign gene (Gshl) could be expressed specifically in GABA-producing neuron progenitor cells by using the 65B13 promoter.
Fig. 7 shows in a photograph results of RT-PCR analysis for the mRNA expression of 65B 13 and E-cadherin in 65B13-positive cells of the cerebellar primordium, myelencephalon, and spinal cord. CB, cerebellar primordium; Mye, myelencephalon; SC, spinal cord.
Fig. 8 shows in photographs comparison of the expression of E-cadherin, 65B13, Corl2, and Pax2 in the cerebellar primordium and myelencephalon areas between E12.5 and E14.5 mice. CB, cerebellar primordium; Mye, myelencephalon; E-cad, E-cadherin.
Fig. 9 shows in diagrams results of flow cytometry analysis for the cell surface expression of 65B 13 and E-cadherin in the cerebellar primordium, myelencephalon, and spinal cord of E12.5 mice.
Fig. 10 shows in diagrams and a photograph differentiation of Purkinje cells from the E-cadherin and 65B 13-double positive cells isolated from cerebellar primordia of E12.5 mice.
A: pattern obtained by flow cytometry analysis prior to isolation. P5: isolated population. B: purity analysis following the isolation. C: staining pattern of isolated cells after two days of culture.

### Mode for Carrying Out the Invention

The 65B13 gene is known to be expressed transiently in dopamine-producing neuron progenitor cells after termination of cell division (WO 2004/038018); however, there is no report published on the connection of the gene with GABA neuron.

The present inventors discovered that GABA neuron progenitor cells could be isolated by using the expression of the 65B13 gene as an indicator. The present inventors also successfully demonstrated that the E-cadherin gene is a marker selectively expressed in Purkinje progenitor cells among 65B13-positive cells.

Specifically, the present invention provides methods for detecting or selecting Purkinje progenitor cells.

The present invention provides methods for detecting or selecting Purkinje progenitor cells, which comprise the steps of detecting a 65B 13 protein and detecting an E-cadherin protein.

The steps of detecting a 65B 13 protein and detecting an E-cadherin protein may be carried out simultaneously. Alternatively, either of the two may be followed by the other.

The 65B 13 gene of the present invention includes, for example, two types of genes named 65B13-a and 65B13-b, which are alternative isoforms of the 65B13 gene. The respective nucleotide sequences are shown in SEQ ID NOs: 1 and 3, and the amino acid sequences encoded by the genes are shown in SEQ ID NOs: 2 and 4.

The coding region of 65B 13-a starts with A at position 178 in SEQ ID NO: 1, and extends to the stop codon at positions 2278 to 2280, encoding a protein of 700 amino acids.

The 17 amino acid residues encoded by the sequence of positions 178 to 228 constitute a signal sequence, while the 17 amino acid residues encoded by the sequence of positions 1717 to 1767 constitute a transmembrane region.

On the other hand, the coding region of 65B13-b starts with A at position 127 in SEQ ID NO: 2 and extends to the stop codon at positions 2277 to 2079, encoding a protein of 650 amino acids. The 17 amino acid residues encoded by the sequence of positions 127 to 178 constitute a signal sequence, while the 17 amino acid residues encoded by the sequence of positions 1516 to 1566 constitute a transmembrane region.

Furthermore, the 65B13 gene of the present invention also includes, for example, the genes of SEQ ID NOs: 19, 21, 23, 25, 27, 29, 31, 33, and 35. The amino acid sequences encoded by the genes are shown in SEQ ID NOs: 20, 22, 24, 26, 28, 30, 32, 34, and 36, respectively.

The coding region of SEQ ID NO: 19 starts with A at position 668 and extends to the stop codon at positions 2768 to 2770, encoding a protein of 700 amino acids. The 19 amino acid residues encoded by the sequence of positions 668 to 724 constitute a signal sequence, while the 494 amino acid residues encoded by the sequence of positions 725 to 2206 constitute an extracellular domain.

The coding region of SEQ ID NO: 21 starts with A at position 130 and extends to the stop codon at positions 2230 to 2232, encoding a protein of 700 amino acids. The 19 amino acid residues encoded by the sequence of positions 130 to 186 constitute a signal sequence, while the 494 amino acid residues encoded by the sequence of positions 187 to 1668 constitute an extracellular domain.

The coding region of SEQ ID NO: 23 starts with A at position 199 and extends to the stop codon at positions 2098 to 2100, encoding a protein of 633 amino acids. The 20 amino acid residues encoded by the sequence of positions 199 to 258 constitute a signal sequence, while the 490 amino acid residues encoded by the sequence of positions 259 to 1728 constitute an extracellular domain.

The coding region of SEQ ID NO: 25 starts with A at position 199 and extends to the stop codon at positions 2323 to 2325, encoding a protein of 708 amino acids. The 20 amino acid residues encoded by the sequence of positions 199 to 258 constitute a signal sequence, while the 490 amino acid residues encoded by the sequence of positions 259 to 1728 constitute an extracellular domain.

The coding region of SEQ ID NO: 27 starts with A at position 199 and extends to the stop codon at positions 2323 to 2325, encoding a protein of 708 amino acids. The 20 amino acid residues encoded by the sequence of positions 199 to 258 constitute a signal sequence, while the 490 amino acid residues encoded by the sequence of positions 259 to 1728 constitute an extracellular domain.

The coding region of SEQ ID NO: 29 starts with A at position 15 and extends to the stop codon at positions 1914 to 1916, encoding a protein of 633 amino acids. The 20 amino acid residues encoded by the sequence of positions 15 to 74 constitute a signal sequence, while the 490 amino acid residues encoded by the sequence of positions 75 to 1544 constitute an extracellular domain.

The coding region of SEQ ID NO: 31 starts with A at position 199 and extends to the stop codon at positions 1948 to 1950, encoding a protein of 583 amino acids. The 20 amino acid residues encoded by the sequence of positions 199 to 258 constitute a signal sequence, while the 440 amino acid residues encoded by the sequence of positions 259 to 1578 constitute an extracellular domain.

The coding region of SEQ ID NO: 33 starts with A at position 15 and extends to the stop codon at positions 1764 to 1766, encoding a protein of 583 amino acids. The 20 amino acid residues encoded by the sequence of positions 15 to 74 constitute a signal sequence, while the 440 amino acid residues encoded by the sequence of positions 75 to 1394 constitute an extracellular domain.

The coding region of SEQ ID NO: 35 starts with A at position 196 and extends to the stop codon at positions 2260 to 2262, encoding a protein of 688 amino acids. The 20 amino acid residues encoded by the sequence of positions 196 to 255 constitute a signal sequence, while the 470 amino acid residues encoded by the sequence of positions 256 to 1665 constitute an extracellular domain.

In addition, the 65B13 gene of the present invention also includes, for example, the sequences of accession numbers XM_994164, AL136654, XM_512603, XR_012248, XM_541684, and XM_583222.

The coding region of SEQ ID NO: 46 starts with A at position 125 and extends to the stop codon at positions 2771 to 2773, encoding a protein of 883 amino acids. The 22 amino acid residues encoded by the sequence of positions 125 to 190 constitute a signal sequence, while the 686 amino acid residues encoded by the sequence of positions 191 to 2248 constitute an extracellular domain.

The coding region of SEQ ID NO: 48 starts with A at position 128 and extends to the stop codon at positions 2780 to 2782, encoding a protein of 885 amino acids. The 22 amino acid residues encoded by the sequence of positions 128 to 193 constitute a signal sequence, while the 688 amino acid residues encoded by the sequence of positions 194 to 2257 constitute an extracellular domain.

The coding region of SEQ ID NO: 50 starts with A at position 127 and extends to the stop codon at positions 2785 to 2787, encoding a protein of 887 amino acids. The 22 amino acid residues encoded by the sequence of positions 127 to 192 constitute a signal sequence, while the 690 amino acid residues encoded by the sequence of positions 193 to 2262 constitute an extracellular domain.

The coding region of SEQ ID NO: 52 starts with A at position 192 and extends to the stop codon at positions 2838 to 2840, encoding a protein of 883 amino acids. The 22 amino acid residues encoded by the sequence of positions 192 to 257 constitute a signal sequence, while the 686 amino acid residues encoded by the sequence of positions 258 to 1933 constitute an extracellular domain.

A preferred embodiment of the method for detecting or selecting a Purkinje progenitor cell comprises the steps of:
(A) detecting a protein selected from:
   (v) a protein comprising the amino acid sequence of SEQ ID NO: 2, 4, 20, 22, 24, 26, 28, 30, 32, 34, or 36;
   (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
   (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
   (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B) detecting a protein selected from:
   (v') a protein comprising the amino acid sequence of SEQ ID NO: 47, 49, 51, or 53;
   (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
   (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
   (viii') a protein which is expressed in a Purkinje progenitor cell and comprises an amino acid sequence having 80% or higher sequence identity to the amino acid sequence of SEQ ID NO: 47, 49, 51, or 53.

A "protein" in the present invention can also be referred to as a "polypeptide" in general. A "polypeptide" of the present invention refers to a peptide polymer encoded by a polynucleotide of the present invention. In the present invention, proteins having 65B13 protein or E-cadherin protein can be included. Preferred examples include proteins having the amino acid sequence described in SEQ ID NO: 2, 4, 20, 22, 24, 26, 28, 30, 32, 34, or 36, or proteins having the amino acid sequence described in SEQ ID NO: 47, 49, 51, or 53.

The polypeptides of the present invention may comprise naturally occurring or modified amino acid residues. Examples of amino acid residue modifications include acylation, acetylation, amidation, arginylation, GPI anchor formation, crosslinking, γ-carboxylation, cyclization, covalent crosslink formation, glycosylation, oxidation, covalent bonding of a lipid or fat derivative, cystine formation, disulfide bond formation, selenoylation, demethylation, protein fragmentation treatment, covalent bonding of a nucleotide or nucleotide derivative, hydroxylation, pyroglutamate formation, covalent bonding of a flavin, prenylation, covalent bonding with a heme portion, covalent bonding of phosphatidyl inositol, formylation, myristoylation, methylation, ubiquitination, iodination, racemization, ADP-ribosylation, sulfation, and phosphorylation.

Moreover, the polypeptides of the present invention include precursors containing a signal peptide portion, mature proteins lacking a signal peptide portion, and fusion proteins modified with other peptide sequences. Peptide sequences to be added to a polypeptide of the present invention can be selected from sequences that facilitate protein purification using, for example, pcDNA3.1/Myc-His vector (Invitrogen), or those that confer stability in recombinant protein production. Examples of such sequences are influenza agglutinin (HA), glutathione S transferase (GST), substance P, multiple histidine tag (such as 6x His and 10x His), protein C fragment, maltose-binding protein (MBP), immunoglobulin constant region, α-tubulin fragment, β-galactosidase, B-tag, c-myc fragment, E-tag (epitope on a monoclonal phage), FLAG (Hopp et al. Bio/Technol. 1988, 6:1204-10), lck tag, p18 HIV fragment, HSV-tag (human simple Herpes virus glycoprotein), SV40T antigen fragment, T7-tag (T7 gene 10 protein), and VSV-GP fragment (vesicular stomatitis virus glycoprotein).

It is well known that a mutant polypeptide comprising an amino acid sequence, in which one or more amino acids are deleted, inserted, substituted, or added, maintains the same biological activity as the original polypeptide (Mark et al. Proc. Natl. Acad. Sci. USA 1984, 81:5662-6; Zoller and Smith. Nucleic Acids Res. 1982, 10:6487-500; Wang et al. Science 1984, 224:1431-3; Dalbadie-McFarland et al. Proc. Natl. Acad. Sci. USA 1982, 79:6409-13). Such mutant polypeptides may be those, for example, having an insertion, substitution, or deletion of 1 to 30 amino acids, preferably 1 to 20 amino acids, more preferably 1 to 10 amino acids, even more preferably 1 to 5 amino acids, and still more preferably one or two amino acids, or those that have amino acids added to either or both ends. The mutant polypeptides may be those having an amino acid sequence preferably with one or more conservative amino acid substitutions (preferably one or several substitutions, more preferably one, two, or three substitutions) in the original amino acid sequence.

Here, an amino acid substitution refers to a mutation in which one or more amino acid residues in a sequence are changed to a different type of amino acid residue. When the amino acid sequence encoded by a polynucleotide of the present invention is altered by such a substitution, a conservative substitution is preferably carried out if the function of the protein is to be maintained. A conservative substitution means altering a sequence so that it encodes an amino acid that has properties similar to those of the amino acid before substitution.

Amino acids can be classified, based on their properties, into non-polar amino acids (Ala, Ile, Leu, Met, Phe, Pro, Trp, Val), non-charged amino acids (Asn, Cys, Gln, Gly, Ser, Thr, Tyr), acidic amino acids (Asp, Glu), basic amino acids (Arg, His, Lys), neutral amino acids (Ala, Asn, Cys, Gln, Gly, Ile, Leu, Met, Phe, Pro, Ser, Thr, Trp, Tyr, Val), aliphatic amino acids (Ala, Gly), branched amino acids (Ile, Leu, Val), hydroxyamino acids (Ser, Thr), amide-type amino acids (Gln, Asn), sulfur-containing amino acids (Cys, Met), aromatic amino acids (His, Phe, Trp, Tyr), heterocyclic amino acids (His, Trp), imino acids (Pro, 4Hyp), etc. In particular, substitutions among Ala, Val, Leu, and Ile; Ser and Thr; Asp and Glu; Asn and Gln; Lys and Arg; and Phe and Tyr are preferable in order to maintain protein properties. There are no particular limitations on the number and sites of the mutated amino acids, as long as the amino acids encoded by the polynucleotide have the antigenicity of 65B13 or E-cadherin.

A polynucleotide encoding an amino acid sequence, in which one or more amino acids are deleted, inserted, substituted, or added to the amino acid sequence of SEQ ID NO: 2, 4, 20, 22, 24, 26, 28, 30, 32, 34, or 36, or the amino acid sequence of SEQ ID NO: 47, 49, 51, or 53 can be prepared according to methods such as site-directed mutagenesis described in "Molecular Cloning, A Laboratory Manual 2nd ed." (Cold Spring Harbor Press (1989)), "Current Protocols in Molecular Biology" (John Wiley & Sons (1987-1997), Sections 8.1-8.5), Hashimoto-Goto et al. (Gene 1995, 152:271-5), Kunkel (Proc. Natl. Acad. Sci. USA 1985, 82:488-92), Kramer and Fritz (Method. Enzymol. 1987, 154:350-67), Kunkel (Method. Enzymol. 1988, 85:2763-6), etc.

The above-described proteins of the present invention also include proteins encoded by polynucleotides that hybridize under stringent conditions to a polynucleotide encoding the amino acid sequence of SEQ ID NO: 2, 4, 20, 22, 24, 26, 28, 30, 32, 34, or 36, or the amino acid sequence of SEQ ID NO: 47, 49, 51, or 53 which are functionally equivalent to a protein comprising the amino acid sequence of SEQ ID NO: 2, 4, 20, 22, 24, 26, 28, 30, 32, 34, or 36, or the amino acid sequence of SEQ ID NO: 47, 49, 51, or 53.

"Equivalent function to a protein comprising the amino acid sequence of SEQ ID NO: 2, 4, 20, 22, 24, 26, 28, 30, 32, 34, or 36" preferably refers to the selective expression in GABA-producing neuron progenitor cells. "Equivalent function to a protein comprising the amino acid sequence of SEQ ID NO: 47, 49, 51, or 53" preferably refers to the selective expression in Purkinje progenitor cells.

Examples of hybridization conditions in the present invention include "2x SSC, 0.1% SDS, 50°C", "2x SSC, 0.1% SDS, 42°C", and "1x SSC, 0.1% SDS, 37°C". Examples of conditions of higher stringency include "2x SSC, 0.1% SDS, 65°C", "0.5x SSC, 0.1% SDS, 42°C", and "0.2x SSC, 0.1% SDS, 65°C". More specifically, a method that uses the Rapid-hyb buffer (Amersham Life Science) can be carried out by performing pre-hybridization at 68°C for 30 minutes or more, adding a probe to allow hybrid formation at 68°C for one hour or more, washing three times in 2x SSC, 0.1% SDS at room temperature for 20 minutes each, washing three times in 1x SSC, 0.1% SDS at 37°C for 20 minutes each, and finally washing twice in 1x SSC, 0.1% SDS at 50°C for 20 minutes each. This can also be carried out using, for example, the Expresshyb Hybridization Solution (CLONTECH), by performing pre-hybridization at 55°C for 30 minutes or more, adding a labeled probe and incubating at 37°C to 55°C for one hour or more, washing three times in 2x SSC, 0.1% SDS at room temperature for 20 minutes each, and washing once at 37°C for 20 minutes with 1x SSC, 0.1% SDS. Here, conditions of higher stringency can be achieved by increasing the temperature for pre-hybridization, hybridization, or second wash. For example, the pre-hybridization and hybridization temperature can be raised to 60°C, and to 68°C for higher stringency. In addition to conditions such as salt concentration of the buffer and temperature, a person with ordinary skill in the art can also integrate other conditions such as probe concentration, probe length, and reaction time, to obtain 65B 13 isoforms, allelic mutants, and corresponding genes derived from other organisms.

References such as "Molecular Cloning, A Laboratory Manual 2nd ed." (Cold Spring Harbor Press (1989), Sections 9.47-9.58), "Current Protocols in Molecular Biology" (John Wiley & Sons (1987-1997), Sections 6.3-6.4), "DNA Cloning 1: Core Techniques, A Practical Approach 2nd ed." (Oxford University (1995), Section 2.10 for conditions) can be referred to for detailed information on hybridization procedures. Examples of hybridizing polynucleotides include polynucleotides containing a nucleotide sequence that has at least 50% or more, preferably 70%, more preferably 80%, and even more preferably 90% (for example, 95% or more, 98% or more, or 99%) identity with a polynucleotide encoding the amino acid sequence of SEQ ID NO: 2, 4, 20, 22, 24, 26, 28, 30, 32, 34, or 36, or SEQ ID NO: 47, 49, 51, or 53. Such identities can be determined by the BLAST algorithm (Altschul. Proc. Natl. Acad. Sci. USA 1990, 87:2264-8; Karlin and Altschul. Proc. Natl. Acad. Sci. USA 1993, 90:5873-7). Examples of programs that have been developed based on this algorithm include the BLASTX program for determining the identity of amino acid sequences, and the BLASTN program for nucleotide sequences (Altschul et al. J. Mol. Biol. 1990, 215:403-10). These programs can be used for the sequences of the present invention. One can refer to, for example, http://www.ncbi.nlm.nih.gov for a specific example of analysis methods.

Alternatively, in the present invention, Purkinje progenitor cells may be detected or selected by using promoters (including modified promoters) (see, for example, Japanese Patent Application Kokai Publication No. (JP-A) 2002-51775 (unexamined, published Japanese patent application)) that control (induce) expression of an mRNA to be translated into the protein described in (A) or (B) below:
(A) a protein selected from (v), (vi), (vii), and (viii) above; and
(B) a protein selected from (v'), (vi'), (vii'), and (viii') above.

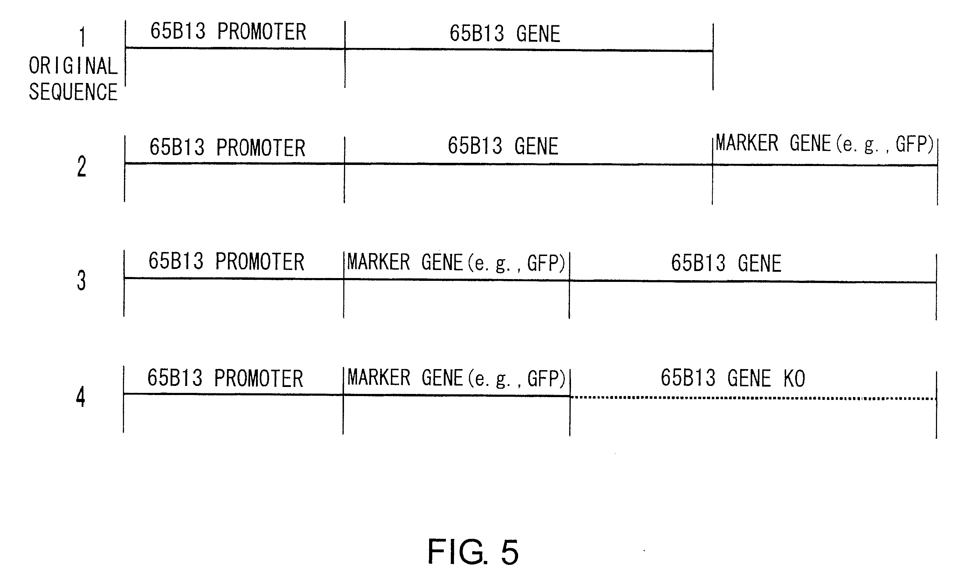

For example, it is possible to transfect cells with a vector carrying a construct in which a gene encoding a detectable marker such as GFP is linked to a promoter portion obtained by analyzing the expression regulatory region. The construct may have a structure where the gene is linked upstream or downstream of the marker gene under the control of the expression regulatory sequence (including promoters, enhancers, etc.). Alternatively, the maker gene can be knocked-in at the locus. In a preferred embodiment using 65B13, the construct includes, for example, constructs having any one of Structures 2 to 4 schematically illustrated in Fig. 5. Similar constructs are also included for E-cadherin. The expression of a marker gene specific to GABA-producing neuron progenitor cells including Purkinje cells is detected by using (A). Meanwhile, the expression of a marker gene specific to Purkinje progenitor cells is detected by using (B). The combined use of the two enables specific selection of Purkinje progenitor cells.

Specifically, the step of detecting such a target protein includes the steps of:
(d) contacting a test cell sample with an antibody that binds to the protein of (A) or (B); and
(e) detecting reactivity.

The proteins of the present invention can be detected by contacting an antibody which binds to the protein of (A) or (B) of the present invention with cell samples that may contain Purkinje progenitor cells, and detecting reactivity. The antibody may be immobilized onto appropriate carriers for use before contact with the cells. Alternatively, cells bound to the antibody can be selectively collected through affinity purification using the antibody after contacting and binding the cells with the antibody. For example, when an antibody of the present invention is linked to biotin, the cells can be purified by adding the cell sample to a plate or column immobilized with avidin or streptavidin.

The detection step may be followed by the step of isolating Purkinje progenitor cells from the detected sample. In the present invention, Purkinje progenitor cells can be efficiently separated by flow cytometry using an antibody that binds to 65B 13 (anti-65B 13 antibody) and an antibody that binds to E-cadherin (anti-E-cadherin antibody) which will be mentioned later.

The cell samples used in the methods are culture cells containing *in vitro* differentiated Purkinje cells. Purkinje cells can be differentiated *in vitro* by using known ES cells, iPS cells, dedifferentiated cells, or the like as a starting material. In general, Purkinje cells can be differentiated by co-culturing nerve tissue-derived supporting cell layer with brain tissues obtained from an area containing Purkinje cells. The cell sample used for selection of Purkinje progenitor cells of the present invention may be a group of cells separated or cultured by any method.

The present invention also relates to methods for detecting or selecting Purkinje progenitor cells, which comprise the steps of:
(A) detecting the expression of a polynucleotide encoding a 65B 13 protein; and
(B) detecting the expression of a polynucleotide encoding an E-cadherin protein.

A preferred embodiment of the polynucleotide of step (A) mentioned above includes a polynucleotide for detecting or selecting a Purkinje progenitor cell, which is capable of hybridizing to a polynucleotide selected from (i),(ii), (iii), and (iv) below, or a complementary sequence thereof:
(i) a polynucleotide comprising:
   the nucleotide sequence from positions 178 to 2280 of SEQ ID NO: 1;
   the nucleotide sequence from positions 127 to 2079 of SEQ ID NO: 3;
   the nucleotide sequence from positions 668 to 2770 of SEQ ID NO: 19;
   the nucleotide sequence from positions 130 to 2232 of SEQ ID NO: 21;
   the nucleotide sequence from positions 199 to 2100 of SEQ ID NO: 23;
   the nucleotide sequence from positions 199 to 2325 of SEQ ID NO: 25;
   the nucleotide sequence from positions 15 to 2325 of SEQ ID NO: 27;
   the nucleotide sequence from positions 15 to 1916 of SEQ ID NO: 29;
   the nucleotide sequence from positions 199 to 1950 of SEQ ID NO: 31;
   the nucleotide sequence from positions 15 to 1766 of SEQ ID NO: 33; or
   the nucleotide sequence from positions 196 to 2262 of SEQ ID NO: 35;
(ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
   the nucleotide sequence from positions 178 to 2280 of SEQ ID NO: 1;
   the nucleotide sequence from positions 127 to 2079 of SEQ ID NO: 3;
   the nucleotide sequence from positions 668 to 2770 of SEQ ID NO: 19;
   the nucleotide sequence from positions 130 to 2232 of SEQ ID NO: 21;
   the nucleotide sequence from positions 199 to 2100 of SEQ ID NO: 23;
   the nucleotide sequence from positions 199 to 2325 of SEQ ID NO: 25;
   the nucleotide sequence from positions 15 to 2325 of SEQ ID NO: 27;
   the nucleotide sequence from positions 15 to 1916 of SEQ ID NO: 29;
   the nucleotide sequence from positions 199 to 1950 of SEQ ID NO: 31;
   the nucleotide sequence from positions 15 to 1766 of SEQ ID NO: 33; or
   the nucleotide sequence from positions 196 to 2262 of SEQ ID NO: 35;
(iii) a polynucleotide which is expressed in a Purkinje progenitor cell and hybridizes under stringent conditions to a polynucleotide comprising:
   the nucleotide sequence from positions 178 to 2280 of SEQ ID NO: 1;
   the nucleotide sequence from positions 127 to 2079 of SEQ ID NO: 3;
   the nucleotide sequence from positions 668 to 2770 of SEQ ID NO: 19;
   the nucleotide sequence from positions 130 to 2232 of SEQ ID NO: 21;
   the nucleotide sequence from positions 199 to 2100 of SEQ ID NO: 23;
   the nucleotide sequence from positions 199 to 2325 of SEQ ID NO: 25;
   the nucleotide sequence from positions 15 to 2325 of SEQ ID NO: 27;
   the nucleotide sequence from positions 15 to 1916 of SEQ ID NO: 29;
   the nucleotide sequence from positions 199 to 1950 of SEQ ID NO: 31;
   the nucleotide sequence from positions 15 to 1766 of SEQ ID NO: 33; or
   the nucleotide sequence from positions 196 to 2262 of SEQ ID NO: 35;
(iv) a polynucleotide which is expressed in a Purkinje progenitor cell and has 80% or higher sequence identity to a polynucleotide comprising:
   the nucleotide sequence from positions 178 to 2280 of SEQ ID NO: 1;
   the nucleotide sequence from positions 127 to 2079 of SEQ ID NO: 3;
   the nucleotide sequence from positions 668 to 2770 of SEQ ID NO: 19;
   the nucleotide sequence from positions 130 to 2232 of SEQ ID NO: 21;
   the nucleotide sequence from positions 199 to 2100 of SEQ ID NO: 23;
   the nucleotide sequence from positions 199 to 2325 of SEQ ID NO: 25;
   the nucleotide sequence from positions 15 to 2325 of SEQ ID NO: 27;
   the nucleotide sequence from positions 15 to 1916 of SEQ ID NO: 29;
   the nucleotide sequence from positions 199 to 1950 of SEQ ID NO: 31;
   the nucleotide sequence from positions 15 to 1766 of SEQ ID NO: 33; or
   the nucleotide sequence from positions 196 to 2262 of SEQ ID NO: 35.

A preferred embodiment of the polynucleotide of step (B) mentioned above includes a polynucleotide for detecting or selecting a Purkinje progenitor cell, which is capable of hybridizing to a polynucleotide selected from (i'), (ii'), (iii'), and (iv') below, or a complementary sequence thereof:
(i') a polynucleotide comprising:
   the nucleotide sequence from positions 125 to 2773 of SEQ ID NO: 46;
   the nucleotide sequence from positions 128 to 2782 of SEQ ID NO: 48;
   the nucleotide sequence from positions 127 to 2787 of SEQ ID NO: 50; or
   the nucleotide sequence from positions 192 to 2849 of SEQ ID NO: 52;
(ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
   the nucleotide sequence from positions 125 to 2773 of SEQ ID NO: 46;
   the nucleotide sequence from positions 128 to 2782 of SEQ ID NO: 48;
   the nucleotide sequence from positions 127 to 2787 of SEQ ID NO: 50; or
   the nucleotide sequence from positions 192 to 2849 of SEQ ID NO: 52;
(iii') a polynucleotide which is expressed in a Purkinje progenitor cell and hybridizes under stringent conditions to a polynucleotide comprising:
   the nucleotide sequence from positions 125 to 2773 of SEQ ID NO: 46;
   the nucleotide sequence from positions 128 to 2782 of SEQ ID NO: 48;
   the nucleotide sequence from positions 127 to 2787 of SEQ ID NO: 50; or
   the nucleotide sequence from positions 192 to 2849 of SEQ ID NO: 52; and
(iv') a polynucleotide which is expressed in a Purkinje progenitor cell and has 80% or higher sequence identity to a polynucleotide comprising:
   the nucleotide sequence from positions 125 to 2773 of SEQ ID NO: 46;
   the nucleotide sequence from positions 128 to 2782 of SEQ ID NO: 48;
   the nucleotide sequence from positions 127 to 2787 of SEQ ID NO: 50; or
   the nucleotide sequence from positions 192 to 2849 of SEQ ID NO: 52.

In the present invention, the polynucleotides of (A) and (B) above may be referred to as "polynucleotides of the present invention".

In the present invention, a "polynucleotide" refers to a polymer comprising nucleotides or nucleotide pairs of multiple deoxyribonucleic acids (DNA) or ribonucleic acids (RNA), and includes DNA, cDNA, genomic DNA, chemically synthesized DNA, and RNA. If needed, polynucleotides can also contain non-naturally occurring nucleotides such as 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, β-D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, β-D-mannosylqueuosine, 5-methoxycarbonylmethyl-2-thiouridine, 5-methoxycarbonylmethyluridine, 5-methoxyuridine, 2-methylthio-N6-isopentenyladenosine, N-((9-β-D-ribofuranosyl-2-methylthiopurin-6-yl)carbamoyl)threonine, N-((9-β-D-ribofuranosylpurin-6-yl)N-methylcarbamoyl)threonine, uridine-5-oxyacetic acid-methyl ester, uridine-5-oxyacetic acid, wybutoxosine, pseudouridine, queuosine, 2-thiocytidine, 5-methyl-2-thiouridine, 2-thiouridine, 4-thiouridine, 5-methyluridine, N-((9-β-D-ribofuranosylpurin-6-yl)carbamoyl)threonine, 2'-O-methyl-5-methyluridine, 2'-O-methyluridine, wybutosine, and 3-(3-amino-3-carboxy propyl)uridine.

The polynucleotides of the present invention can also be produced by chemical synthesis based on the known sequences of 65B13 and E-cadherin. Alternatively, such polynucleotides can be prepared from 65B13 gene- and E-cadherin gene-expressing cells using hybridization, PCR, etc.

The polynucleotides of the present invention also include polynucleotides that hybridize under stringent conditions to a polynucleotide comprising the nucleotide sequence from positions 178 to 2280 of SEQ ID NO: 1, the nucleotide sequence from positions 127 to 2079 of SEQ ID NO: 3, the nucleotide sequence from positions 668 to 2770 of SEQ ID NO: 19, the nucleotide sequence from positions 130 to 2232 of SEQ ID NO: 21, the nucleotide sequence from positions 199 to 2100 of SEQ ID NO: 23, the nucleotide sequence from positions 199 to 2325 of SEQ ID NO: 25, the nucleotide sequence from positions 15 to 2325 of SEQ ID NO: 27, the nucleotide sequence from positions 15 to 1916 of SEQ ID NO: 29, the nucleotide sequence from positions 199 to 1950 of SEQ ID NO: 31, the nucleotide sequence from positions 15 to 1766 of SEQ ID NO: 33, or the nucleotide sequence from positions 196 to 2262 of SEQ ID NO: 35, and each of which encodes a protein that is functionally equivalent (a preferred function is the selective expression in Purkinje progenitor cells) to a protein comprising the amino acid sequence of SEQ ID NO: 2, 4, 20, 22, 24, 26, 28, 30, 32, 34, or 36. The polypeptides also include isoforms, alternative isoforms, and allelic mutants of 65B 13.

Such polynucleotides can be obtained from cDNA libraries and genomic libraries of animals such as humans, mice, rats, rabbits, hamsters, chickens, pigs, cows, goats, sheep, monkeys, and dogs by known hybridization methods such as colony hybridization, plaque hybridization, and Southern blotting using as a probe a polynucleotide comprising the nucleotide sequence from positions 1 to 2876 of SEQ ID NO: 1, the nucleotide sequence from positions 1 to 2243 of SEQ ID NO: 3, the nucleotide sequence from positions 1 to 3123 of SEQ ID NO: 19, the nucleotide sequence from positions 1 to 3247 of SEQ ID NO: 21, the nucleotide sequence from positions 1 to 2153 of SEQ ID NO: 23, the nucleotide sequence from positions 1 to 2979 of SEQ ID NO: 25, the nucleotide sequence from positions 1 to 2973 of SEQ ID NO: 27, the nucleotide sequence from positions 1 to 1969 of SEQ ID NO: 29, the nucleotide sequence from positions 1 to 2003 of SEQ ID NO: 31, the nucleotide sequence from positions 1 to 1819 of SEQ ID NO: 33, or the nucleotide sequence from positions 1 to 2959 of SEQ ID NO: 35; preferably the nucleotide sequence from positions 178 to 2280 of SEQ ID NO: 1, the nucleotide sequence from positions 127 to 2079 of SEQ ID NO: 3, the nucleotide sequence from positions 668 to 2770 of SEQ ID NO: 19, the nucleotide sequence from positions 130 to 2232 of SEQ ID NO: 21, the nucleotide sequence from positions 199 to 2100 of SEQ ID NO: 23, the nucleotide sequence from positions 199 to 2325 of SEQ ID NO: 25, the nucleotide sequence from positions 15 to 2325 of SEQ ID NO: 27, the nucleotide sequence from positions 15 to 1916 of SEQ ID NO: 29, the nucleotide sequence from positions 199 to 1950 of SEQ ID NO: 31, the nucleotide sequence from positions 15 to 1766 of SEQ ID NO: 33, or the nucleotide sequence from positions 196 to 2262 of SEQ ID NO: 35. Regarding methods for constructing cDNA libraries, one can refer to "Molecular Cloning, A Laboratory Manual 2nd ed." (Cold Spring Harbor Press (1989)). It is also possible to use cDNA libraries and genomic libraries available on the market.

The polynucleotides of the present invention also include polynucleotides that hybridize under stringent conditions to a polynucleotide comprising the nucleotide sequence from positions 125 to 2773 of SEQ ID NO: 46, the nucleotide sequence from positions 128 to 2782 of SEQ ID NO: 48, the nucleotide sequence from positions 127 to 2787 of SEQ ID NO: 50, or the nucleotide sequence from positions 192 to 2840 of SEQ ID NO: 52, and each of which encodes a protein that is functionally equivalent (a preferred function is the selective expression in Purkinje progenitor cells) to a protein comprising the amino acid sequence of SEQ ID NO: 47, 49, 51, or 53. The polypeptides also include isoforms, alternative isoforms, and allelic mutants of E-cadherin.

Such polynucleotides can be obtained from cDNA libraries and genomic libraries of animals such as humans, mice, rats, rabbits, hamsters, chickens, pigs, cows, goats, sheep, monkeys, and dogs by known hybridization methods such as colony hybridization, plaque hybridization, and Southern blotting using as a probe a polynucleotide comprising the nucleotide sequence from positions 1 to 4828 of SEQ ID NO: 46, the nucleotide sequence from positions 1 to 4413 of SEQ ID NO: 48, the nucleotide sequence from positions 1 to 4396 of SEQ ID NO: 50, or the nucleotide sequence from positions 1 to 4877 of SEQ ID NO: 52; preferably the nucleotide sequence from positions 125 to 2773 of SEQ ID NO: 46, the nucleotide sequence from positions 128 to 2782 of SEQ ID NO: 48, the nucleotide sequence from positions 127 to 2787 of SEQ ID NO: 50, or the nucleotide sequence from positions 192 to 2840 of SEQ ID NO: 52. Regarding methods for constructing cDNA libraries, one can refer to "Molecular Cloning, A Laboratory Manual 2nd ed." (Cold Spring Harbor Press (1989)). It is also possible to use cDNA libraries and genomic libraries available on the market.

Meanwhile, the phrase "functionally equivalent" means that a target protein has the same biological property as a 65B13 protein (for example, the amino acid sequence of SEQ ID NO: 2, 4, 20, 22, 24, 26, 28, 30, 32, 34, or 36) or an E-cadherin protein (for example, the amino acid sequence of SEQ ID NO: 47, 49,51, or 53). The biological property of a 65B13 protein (for example, the amino acid sequence of SEQ ID NO: 2, 4, 20, 22, 24, 26, 28, 30, 32, 34, or 36) includes, for example, the olfactory nerve network pattern. Furthermore, the selective expression in Purkinje progenitor cells can also be regarded as a function (biological property). The biological property (function) of an E-cadherin (for example, the amino acid sequence of SEQ ID NO: 47, 49, 51, or 53) includes, for example, selective expression in Purkinje progenitor cells.

Accordingly, whether a target protein has the equivalent biological property as the 65B13 protein (amino acid sequence of SEQ ID NO: 2, 4, 20, 22, 24, 26, 28, 30, 32, 34, or 36) identified by the present inventors can be assessed by those skilled in the art, for example, by testing for the olfactory nerve network pattern or selective expression in Purkinje progenitor cells.

Similarly, whether a target protein has the equivalent biological property as the E-cadherin protein (amino acid sequence of SEQ ID NO: 47, 49, 51, or 53) can be assessed by those skilled in the art, for example, by testing for selective expression in Purkinje progenitor cells.

More specifically, in constructing a cDNA library, total RNA is first prepared from cells, organs, tissues, or such that express a polynucleotide of the present invention, by known techniques such as guanidine ultracentrifugation (Chirwin et al. Biochemistry 1979, 18:5294-5299) or AGPC (Chomczynski and Sacchi Anal. Biochem. 1987, 162:156-159), followed by mRNA purification using an mRNA purification kit (Pharmacia), etc. A kit for direct mRNA preparation, such as the QuickPrep mRNA Purification Kit (Pharmacia), may also be used. Next, cDNA is synthesized from the resulting mRNA using reverse transcriptase. cDNA synthesis kits such as the AMV Reverse Transcriptase First-strand cDNA Synthesis Kit (Seikagaku Corporation) are also available commercially. Other methods that use the 5'-RACE method to synthesize and amplify cDNA by PCR may also be used (Frohman et al. Proc. Natl. Acad. Sci. USA 1988, 85:8998-9002; Belyavsky et al. Nucleic Acids Res. 1989, 17:2919-32). In addition, in order to construct cDNA libraries containing a high percentage of full-length clones, known techniques such as the oligo-capping method (Maruyama and Sugano. Gene 1994, 138:171-4; Suzuki. Gene 1997, 200:149-56) can also be employed. The cDNA obtained in this manner is then incorporated into a suitable vector.

Examples of hybridization conditions in the present invention include "2x SSC, 0.1% SDS, 50°C", "2x SSC, 0.1% SDS, 42°C", and "1x SSC, 0.1% SDS, 37°C". Examples of conditions of higher stringency include "2x SSC, 0.1% SDS, 65°C", "0.5x SSC, 0.1% SDS, 42°C", and "0.2x SSC, 0.1% SDS, 65°C". More specifically, a method that uses the Rapid-hyb buffer (Amersham Life Science) can be carried out by performing pre-hybridization at 68°C for 30 minutes or more, adding a probe to allow hybrid formation at 68°C for one hour or more, washing three times in 2x SSC, 0.1% SDS at room temperature for 20 minutes each, washing three times in 1x SSC, 0.1% SDS at 37°C for 20 minutes each, and finally washing twice in 1x SSC, 0.1% SDS at 50°C for 20 minutes each. This can also be carried out using, for example, the Expresshyb Hybridization Solution (CLONTECH), by performing pre-hybridization at 55°C for 30 minutes or more, adding a labeled probe and incubating at 37°C to 55°C for one hour or more, washing three times in 2x SSC, 0.1% SDS at room temperature for 20 minutes each, and washing once at 37°C for 20 minutes with 1x SSC, 0.1% SDS. Here, conditions of higher stringency can be achieved by increasing the temperature for pre-hybridization, hybridization, or second wash. For example, the pre-hybridization and hybridization temperature can be raised to 60°C, and to 68°C for higher stringency. In addition to conditions such as salt concentration of the buffer and temperature, a person with ordinary skill in the art can also integrate other conditions such as probe concentration, probe length, and reaction time, to obtain isoforms of 65B13 and E-cadherin of the present invention, allelic mutants, and corresponding genes derived from other organisms.

References such as "Molecular Cloning, A Laboratory Manual 2nd ed." (Cold Spring Harbor Press (1989), Sections 9.47-9.58), "Current Protocols in Molecular Biology" (John Wiley & Sons (1987-1997), Sections 6.3-6.4), "DNA Cloning 1: Core Techniques, A Practical Approach 2nd ed." (Oxford University (1995), Section 2.10 for conditions in particular) can be referred to for detailed information on hybridization procedures.

Examples of hybridizing polynucleotides include polynucleotides containing a nucleotide sequence that has at least 50% or more, preferably 70%, more preferably 80%, and even more preferably 90% (for example, 95% or more, or 99%) identity with a nucleotide sequence comprising the nucleotide sequence from positions 178 to 2280 of SEQ ID NO: 1, the nucleotide sequence from positions 127 to 2079 of SEQ ID NO: 3, the nucleotide sequence from positions 668 to 2770 of SEQ ID NO: 19, the nucleotide sequence from positions 130 to 2232 of SEQ ID NO: 21, the nucleotide sequence from positions 199 to 2100 of SEQ ID NO: 23, the nucleotide sequence from positions 199 to 2325 of SEQ ID NO: 25, the nucleotide sequence from positions 15 to 2325 of SEQ ID NO: 27, the nucleotide sequence from positions 15 to 1916 of SEQ ID NO: 29, the nucleotide sequence from positions 199 to 1950 of SEQ ID NO: 31, the nucleotide sequence from positions 15 to 1766 of SEQ ID NO: 33, or the nucleotide sequence from positions 196 to 2262 of SEQ ID NO: 35; or the nucleotide sequence from positions 125 to 2773 of SEQ ID NO: 46, the nucleotide sequence from positions 128 to 2782 of SEQ ID NO: 48, the nucleotide sequence from positions 127 to 2787 of SEQ ID NO: 50, or the nucleotide sequence from positions 192 to 2840 of SEQ ID NO: 52.

Such identities can be determined by the BLAST algorithm (Altschul. Proc. Natl. Acad. Sci. USA 1990, 87:2264-8; Karlin and Altschul. Proc. Natl. Acad. Sci. USA 1993, 90:5873-7). Examples of programs that have been developed based on this algorithm include the BLASTX program for determining the identity of amino acid sequences, and the BLASTN program for nucleotide sequences (Altschul et al. J. Mol. Biol. 1990, 215:403-10). These programs can be used for the sequences of the present invention. One can refer to, for example, http://www.ncbi.nlm.nih.gov for a specific example of analysis methods.

The isoforms or allelic mutants of 65B 13 and E-cadherin, and other genes with a 65B13- or E-cadherin-like structure and function can be obtained from cDNA libraries and genomic libraries of animals such as humans, mice, rats, rabbits, hamsters, chickens, pigs, cows, goats, sheep, monkeys, and dogs by designing primers based on the nucleotide sequence from positions 178 to 2280 of SEQ ID NO: 1, the nucleotide sequence from positions 127 to 2079 of SEQ ID NO: 3, the nucleotide sequence from positions 668 to 2770 of SEQ ID NO: 19, the nucleotide sequence from positions 130 to 2232 of SEQ ID NO: 21, the nucleotide sequence from positions 199 to 2100 of SEQ ID NO: 23, the nucleotide sequence from positions 199 to 2325 of SEQ ID NO: 25, the nucleotide sequence from positions 15 to 2325 of SEQ ID NO: 27, the nucleotide sequence from positions 15 to 1916 of SEQ ID NO: 29, the nucleotide sequence from positions 199 to 1950 of SEQ ID NO: 31, the nucleotide sequence from positions 15 to 1766 of SEQ ID NO: 33, or the nucleotide sequence from positions 196 to 2262 of SEQ ID NO: 35; or the nucleotide sequence from positions 125 to 2773 of SEQ ID NO: 46, the nucleotide sequence from positions 128 to 2782 of SEQ ID NO: 48, the nucleotide sequence from positions 127 to 2787 of SEQ ID NO: 50, or the nucleotide sequence from positions 192 to 2840 of SEQ ID NO: 52, using gene amplification technology (PCR) (Current Protocols in Molecular Biology (John Wiley & Sons (1987), Sections 6.1-6.4)).

The polynucleotide sequences of the present invention can be confirmed by using conventional sequence determination methods. For example, the dideoxynucleotide chain termination method (Sanger et al. Proc. Natl. Acad. Sci. USA 1977, 74:5463) can be used. In addition, sequences can also be analyzed using a suitable DNA sequencer.

Specifically, in a preferred embodiment, the methods of the present invention include methods for detecting or selecting Purkinje progenitor cells, which comprise the steps of:
(A) detecting the expression of a polynucleotide capable of hybridizing to a polynucleotide selected from (i), (ii), (iii), and (iv) above or a complementary sequence thereof; and
(B) detecting the expression of a polynucleotide encoding an E-cadherin protein.

The Purkinje progenitor cells of the present invention preferably include, but are not limited to, Purkinje progenitor cells of the cerebellum.

The length of a polynucleotide of the present invention is not particularly limited, as long as it allows for detection or selection of Purkinje progenitor cells. The polynucleotides of the present invention also include the so-called "oligonucleotides". In general, the polynucleotides of the present invention comprise at least ten consecutive nucleotides in the nucleotide sequence of the present invention or a complementary sequence thereof, and preferably comprise at least 15 consecutive nucleotides.

The "step of detecting the expression of a polynucleotide" in the above-described methods of the present invention preferably comprises the steps of:
(a) contacting a test cell sample with a polynucleotide that can hybridize to a polynucleotide selected by the above-described methods of the present invention or to a complementary sequence thereof, or with a probe comprising the polynucleotide; and
(b) detecting reactivity.

Alternatively, in a preferred embodiment of the methods of the present invention, first, the test cell sample is contacted with a polynucleotide of the present invention or with a probe comprising the polynucleotide. For example, it is possible to contact such a probe with mRNA prepared from the test cell sample or with a complementary DNA (cDNA) transcribed from the mRNA.

The absence and presence of reactivity is then determined in these methods. Herein, the presence of reactivity generally means that the contacted polynucleotide hybridizes (reacts) with the target sequence.

The steps of the above-described methods of the present invention may include, for example, the steps of:
(a-1) conducting gene amplification using as a template a polynucleotide derived from the test cell sample, and primers comprising polynucleotides that are capable of hybridizing to a polynucleotide of the present invention or a complementary sequence thereof, or a set of primers comprising polynucleotides that are capable of hybridizing to a polynucleotide selected in the present invention or a complementary sequence thereof; and
(b-1) detecting the resulting amplification products.

The "gene amplification method" in the above step includes known methods, for example, PCR. Furthermore, the amplification products generated by the amplification method can also be detected by known methods.

Meanwhile, for example, mRNA prepared from a test cell sample or complementary DNA (cDNA) transcribed from the mRNA may be used as a template in step (a-1) described above.

The detection step may be followed by the step of isolating Purkinje progenitor cells from the detected sample.

Since the protein encoded by the 65B13 gene of the present invention is a membrane protein, viable GABA-producing neuron progenitor cells can be isolated (separated) by using the protein as an indicator.

Similarly, since the protein encoded by the E-cadherin gene is a membrane protein, viable Purkinje progenitor cells can be isolated (separated) by using the protein as an indicator.

In addition, the methods of the present invention may comprise, in addition to the above-described step, the step of detecting or selecting Purkinje progenitor cells using the expression of a gene selected from the group consisting of Lim1/2 and Corl2 genes as an indicator.

Furthermore, the present invention also provides nucleotide chains complementary to a polynucleotide selected from (A) and (B) above for detecting or selecting Purkinje progenitor cells of the present invention, which comprise at least 15 consecutive nucleotides. Such polynucleotides comprising a nucleotide sequence that contains at least 15 consecutive nucleotides are useful as probes for detecting the generation of Purkinje progenitor cells or as primers for detecting Purkinje progenitor cells.

The nucleotide chain normally consists of 15 to 100, and preferably 15 to 35 nucleotides and the polynucleotide is appropriately labeled with a radioisotope, non-radioactive compound, or the like when used as a probe. The nucleotide chain preferably consists of at least 15 and preferably 30 nucleotides when used as a primer. A primer can be designed to have a restriction enzyme recognition sequence, a tag or such, added to the 5'-end side thereof, and at the 3' end, a sequence complementary to a target sequence. A nucleotide chain of the present invention can hybridize with a polynucleotide of the present invention. Moreover, mutations of a polynucleotide of the present invention within cells can be detected using these probes or primers. In some cases, such mutations may cause abnormalities in the activity or expression of the polypeptides of the present invention; therefore, nucleotide chains of the present invention are thought to be useful for disease diagnosis, etc.

Here, a "complementary sequence" refers to not only cases where at least 15 consecutive nucleotides of the nucleotide sequence completely pair with the template, but also includes those that have at least 70%, preferably 80%, more preferably 90%, and even more preferably 95% or more (for example, 97% or 99%) of the consecutive nucleotides paired with the template. Pair formation refers to the formation of a chain, in which T (U in the case of an RNA) corresponds to A, A corresponds to T or U, G corresponds to C, and C corresponds to G in the nucleotide sequence of the template polynucleotide. Identities can be determined by methods similar to that used in the aforementioned polynucleotide hybridization.

The present invention also provides primer sets comprising two or more polynucleotides selected from (A) and (B) above for detecting or selecting Purkinje progenitor cells of the present invention.

Furthermore, the present invention provides kits for detecting or selecting Purkinje progenitor cells. The kits of the present invention may comprise, for example, probes, primers, or primer sets that enable detection of the expression of a polynucleotide that can hybridize to a polynucleotide selected from (A) and (B) described above, or to a complementary sequence thereof. The kits may also comprise appropriate buffers, etc. Furthermore, the packages may contain instruction manuals containing a description of how to use the kits.

In an alternative embodiment, the kits of the present invention for detecting or selecting Purkinje progenitor cells include kits comprising antibodies that bind to either of the proteins of (A) and (B) below:
(A) a protein selected from (v), (vi), (vii), and (viii) above; and
(B) a protein selected from (v'), (vi'), (vii'), and (viii') above.

In an alternative embodiment, the kits of the present invention include kits comprising a polynucleotide encoding a marker protein under the control of a promoter that controls (induces) expression of an mRNA to be translated into a protein selected from (A) and (B) below:
(A) a protein selected from (v), (vi), (vii), and (viii) above; and
(B) a protein selected from (v'), (vi'), (vii'), and (viii') above.

The kits of the present invention may further comprise polynucleotides that hybridize to transcripts of one or more genes selected from the group consisting of the Lim1/2 and Corl2 genes.

The kits may further contain in combination antibodies that bind to proteins encoded by one or more genes selected from the group consisting of Lim1/2 and Cor12 genes.

The kits of the present invention may further comprise Purkinje progenitor cells as a positive control.

The present invention also provides methods for preparing a Purkinje progenitor cell population. Such methods include, for example, methods comprising the steps of:
(VII) preparing a cell population that potentially contains Purkinje progenitor cells;
(VIII) detecting Purkinje progenitor cells using a method of the present invention for detecting or selecting Purkinje progenitor cells; and
(IX) expanding cells detected or selected in step (VIII).

The Purkinje progenitor cells obtained by the production methods described above are used, for example, in treating cerebellar degeneration.

The Purkinje progenitor cells obtained by the production methods described above are also included in the present invention. It is preferable that cells produced by the above-described methods of the present invention are viable cells.

Since cells obtained in the present invention are Purkinje progenitor cells, they are preferable in transplant therapy for degenerative diseases and such in terms of their safety, survival rate, and network formation ability, compared to mixed cell populations or Purkinje progenitor cells carrying an exogenous gene. Moreover, since cells (or cell populations) of the present invention obtained according to the methods are progenitor cells, they can be differentiated into a suitable stage by selecting *in vitro* conditions such as media, and are preferable materials for various types of neural transplant therapy. When Purkinje progenitor cells obtained using the methods of the present invention are used in transplants, preferably 1 x 10³ to 1 x 10⁶ Purkinje cells, and more preferably 5 x 10⁴ to 6 x 10⁴ Purkinje cells are transplanted. The primary method is stereotaxic surgery in which a cell suspension is transplanted into the brain. In addition, cells may also be transplanted by microsurgery. See, Backlund et al. (J. Neurosurg. 1985, 62:169-73), Lindvall et al. (Ann. Neurol. 1987, 22:457-68), or Madrazo et al. (New Engl. J. Med. 1987, 316:831-4) for methods of transplanting Purkinje tissues.

Moreover, the cells of the present invention can also be used to isolate genes specific to Purkinje progenitor cells, and genes specific to each stage of the maturation from progenitor cells into Purkinje cells. They can also be used for searching therapeutic targets for degenerative diseases, elucidating the maturation process of Purkinje cells, and in screenings using maturation as an indicator.

The present invention also provides reagents for detecting or selecting Purkinje progenitor cells.

In an embodiment, the reagents of the present invention include, for example, reagents for detecting or selecting Purkinje progenitor cells, which comprise probes, primers, or primer sets that enable detection of the expression of a polynucleotide that can hybridize to a polynucleotide selected from (A) and (B) described above, or to a complementary sequence thereof.

In an alternative embodiment, such reagents of the present invention for detecting or selecting Purkinje progenitor cells include, for example, reagents for detecting or selecting Purkinje progenitor cells, which contain an antibody that binds to a protein selected from (A) and (B) below:
(A) a protein selected from (v), (vi), (vii), and (viii) above; and
(B) a protein selected from (v'), (vi'), (vii'), and (viii') above.

In an alternative embodiment, such reagents of the present invention for detecting or selecting Purkinje progenitor cells include, for example, reagents for detecting or selecting Purkinje progenitor cells, which contain a polynucleotide to be used to link a marker protein-encoding polynucleotide under the control of a promoter that controls (induces) expression of an mRNA to be translated into a protein selected from (A) and (B) below:
(A) a protein selected from (v), (vi), (vii), and (viii) above; and
(B) a protein selected from (v'), (vi'), (vii'), and (viii') above.

Herein, the "cell type identification" means not only when target cells are identified to be of a specific cell type, but also when target cells are judged not to be of a specific cell type. For example, when the 65B13 gene and E-cadherin gene are substantially expressed in target cerebellar cells, the cells can be identified to be "possibly Purkinje progenitor cells".

The present invention also provides reagents for detecting or selecting cerebellar cells. For the reagents, the above-described reagents for detecting or selecting Purkinje progenitor cells may be appropriately combined with other known markers. Such reagents enable thorough cell type identification.

Thus, in a preferred embodiment, the present invention provides reagents for detecting or selecting cerebellar cells, which comprise a combination of the above-described reagents for detecting or selecting Purkinje progenitor cells and polynucleotides that hybridize to the transcripts of one or more genes selected from the group consisting of Lim1/2 and Corl2 genes.

The sequences of the above-described marker genes are known as listed below.

The nucleotide sequence of mouse Lim1 is shown in SEQ ID NO: 5 and the amino acid sequence is shown in SEQ ID NO: 6; the nucleotide sequence of human Lim1 is shown in SEQ ID NO: 7 and the amino acid sequence is shown in SEQ ID NO: 8.

The nucleotide sequence of mouse Lim2 is shown in SEQ ID NO: 9 and the amino acid sequence is shown in SEQ ID NO: 10; the nucleotide sequence of human Lim2 is shown in SEQ ID NO: 11 and the amino acid sequence is shown in SEQ ID NO: 12; the nucleotide sequence of rat Lim2 is shown in SEQ ID NO: 13 and the amino acid sequence is shown in SEQ ID NO: 14.

The nucleotide sequence of mouse Corl2 is shown in SEQ ID NO: 15 and the amino acid sequence is shown in SEQ ID NO: 16; the nucleotide sequence of human Corl2 is shown in SEQ ID NO: 17 and the amino acid sequence is shown in SEQ ID NO: 18.

The above-described reagents for detecting or selecting Purkinje progenitor cells may further comprise in combination antibodies that bind to proteins encoded by one or more genes selected from the group consisting of Lim1/2 and Corl2 genes.

Cerebellar cells that can be identified using the above-described reagents include Purkinje cells.

The above-described reagents may further comprise cerebellar cells.

The present invention also provides polynucleotides for detecting or selecting Purkinje progenitor cells for use in regeneration medicine to treat cerebellar degeneration.

In a preferred embodiment, such polynucleotides of the present invention include polynucleotides capable of hybridizing to a polynucleotide selected from (A) and (B) below or a complementary sequence thereof:
(A) a polynucleotide selected from (i), (ii), (iii), and (iv) above; and
(B) a polynucleotide selected from (i'), (ii'), (iii'), and (iv') above.

The present invention also provides antibodies that bind to a translation product of the 65B13 gene or E-cadherin gene for use in regeneration medicine for cerebellar degeneration. In a preferred embodiment, such antibodies of the present invention include, for example, antibodies for detecting or selecting Purkinje progenitor cells for use in regeneration medicine for cerebellar degeneration, which bind to a protein selected from (A) and (B) below:
(A)
   (v) a protein comprising the amino acid sequence of SEQ ID NO: 2, 4, 20, 22, 24, 26, 28, 30, 32, 34, or 36;
   (vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
   (vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
   (viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B)
   (v') a protein comprising the amino acid sequence of SEQ ID NO: 47, 49, 51, or 53;
   (vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
   (vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
   (viii') a protein which is expressed in a Purkinje progenitor cell and comprises an amino acid sequence having 80% or higher sequence identity to the amino acid sequence of SEQ ID NO: 47, 49, 51, or 53.

In a preferred embodiment, the antibodies of the present invention include antibodies that bind to a polypeptide selected from (A) and (B) below:
(A) an extracellular domain polypeptide of GABA-producing neuron progenitor cells; and
(B) an extracellular domain polypeptide of Purkinje progenitor cells.

The extracellular domains of polypeptides used in the present invention can be searched using the program, PSORT (http://psort.ims.u-tokyo.ac.jp/), etc. Specifically, the extracellular domains of a polypeptide encoding the 65B13 protein obtained using the PSORT program are: the amino acid sequences from positions 21 to 510 of SEQ ID NO: 2, 4, 24, 26, 28, or 30; positions 20 to 513 of SEQ ID NO: 20 or 22; positions 21 to 460 of SEQ ID NO: 32 or 34; and positions 21 to 490 of SEQ ID NO: 36. Meanwhile, the extracellular domains of a polypeptide encoding the E-cadherin protein are: the amino acid sequences from positions 23 to 708 of SEQ ID NO: 47; positions 23 to 710 of SEQ ID NO: 49; positions 23 to 712 of SEQ ID NO: 51; and positions 23 to 708 of SEQ ID NO: 53.

Antibodies of the present invention also include polyclonal antibodies, monoclonal antibodies, chimeric antibodies, single-chain antibodies (scFV) (Huston et al. Proc. Natl. Acad. Sci. USA 1988, 85:5879-83; The Pharmacology of Monoclonal Antibody, vol.113, Rosenburg and Moore ed., Springer Verlag (1994) pp.269-315), humanized antibodies, multispecific antibodies (LeDoussal et al. Int. J. Cancer Suppl. 1992, 7:58-62; Paulus. Behring Inst. Mitt. 1985, 78:118-32; Millstein and Cuello. Nature 1983, 305:537-9; Zimmermann Rev. Physiol. Biochem. Pharmacol. 1986, 105:176-260; Van Dijk et al. Int. J. Cancer 1989, 43:944-9), and antibody fragments such as Fab, Fab', F(ab')2, Fc, and Fv. Moreover, an antibody of the present invention may also be modified by PEG and such, as necessary. An antibody of the present invention may also be produced in the form of a fusion protein with β-galactosidase, maltose-binding protein, GST, green fluorescent protein (GFP), or such, to allow detection without the use of a secondary antibody. In addition, an antibody may be modified by labeling with biotin or such to allow recovery using avidin, streptoavidin, etc.

An antibody of the present invention can be produced using a polypeptide of the present invention, a fragment thereof, or a cell in which a polypeptide or polypeptide fragment of the present invention is expressed, as a sensitized antigen. In addition, a short polypeptide of the present invention or a fragment thereof may also be used as an immunogen by coupling to a carrier such as bovine serum albumin, Keyhole Limpet Hemocyanin, and ovalbumin. In addition, a polypeptide of the present invention or a fragment thereof may be used in combination with a known adjuvant such as aluminum adjuvant, Freund's complete (or incomplete) adjuvant, or pertussis adjuvant, to enhance the immune response to an antigen.

Polyclonal antibodies can be obtained from, for example, the serum of an immunized animal after immunizing a mammal with a polypeptide of the present invention or a fragment thereof, together with an adjuvant as necessary. Although there are no particular limitations on the mammals used, typical examples include rodents, lagomorphs, and primates. Specific examples include rodents such as mice, rats, and hamsters; lagomorphs such as rabbits; and primates such as monkeys including cynomolgus monkeys, rhesus monkeys, baboons, and chimpanzees. Animal immunization is carried out by suitably diluting and suspending a sensitized antigen in phosphate-buffered saline (PBS) or physiological saline, mixing with an adjuvant as necessary until emulsified, and injecting into an animal intraperitoneally or subcutaneously. The sensitized antigen mixed with Freund's incomplete adjuvant is preferably administered several times, every 4 to 21 days. Antibody production can be confirmed by measuring the level of an antibody of interest in the serum using conventional methods. Finally, the serum itself may be used as a polyclonal antibody, or it may be further purified. See, for example, "Current Protocols in Molecular Biology" (John Wiley & Sons (1987), Sections 11.12-11.13), for specific methods.

A monoclonal antibody can be produced by removing the spleen from an animal immunized in the manner described above, separating immunocytes from the spleen, and fusing with a suitable myeloma cell using polyethylene glycol (PEG) or such to establish hybridomas. Cell fusion can be carried out according to the Milstein method (Galfre and Milstein. Methods Enzymol. 1981, 73:3-46). Here, suitable myeloma cells are exemplified particularly by cells that allow chemical selection of fused cells. When using such myeloma cells, fused hybridomas are selected by culturing in a culture medium (HAT culture medium) that contains hypoxanthine, aminopterin, and thymidine, which destroy cells other than the fused cells. Next, a clone that produces an antibody against a polypeptide of the present invention or a fragment thereof is selected from the established hybridomas. Subsequently, the selected clone is introduced into the abdominal cavity of a mouse or such, and ascite is collected to obtain a monoclonal antibody. See, in addition, "Current Protocols in Molecular Biology" (John Wiley & Sons (1987), Sections 11.4-11.11) for information on specific methods.

Hybridomas can also be obtained by first sensitizing human lymphocytes that have been infected by EB virus with an immunogen *in vitro,* and fusing the sensitized lymphocytes with human myeloma cells (such as U266) to obtain hybridomas that produce human antibodies (JP-A (Kokai) S63-17688). In addition, human antibodies can also be obtained by using antibody-producing cells generated by sensitizing a transgenic animal with a human antibody gene repertoire (WO 92/03918; WO 93/02227; WO 94/02602; WO 94/25585; WO 96/33735; WO 96/34096; Mendez et al. Nat. Genet. 1997, 15:146-156, etc.). Methods that do not use hybridomas can be exemplified by a method in which a cancer gene is introduced to immortalize immunocytes such as antibody-producing lymphocytes.

In addition, antibodies can also be produced by genetic recombination techniques (see Borrebaeck and Larrick (1990) Therapeutic Monoclonal Antibodies, MacMillan Publishers Ltd., UK). First, a gene that encodes an antibody is cloned from hybridomas or antibody-producing cells (such as sensitized lymphocytes). The resulting gene is then inserted into a suitable vector, the vector is introduced into a host, and the host is then cultured to produce the antibody. This type of recombinant antibody is also included in the antibodies of the present invention. Typical examples of recombinant antibodies include chimeric antibodies comprising a non-human antibody-derived variable region and a human antibody-derived constant region, and humanized antibodies comprising a non-human-derived antibody complementarity determining region (CDR), human antibody-derived framework region (FR), and human antibody constant region (Jones et al. Nature 1986, 321:522-5; Reichmann et al. Nature 1988, 332: 323-9; Presta. Curr. Op. Struct. Biol. 1992, 2:593-6; Methods Enzymol. 1991, 203:99-121).

Antibody fragments of the present invention can be produced by treating the aforementioned polyclonal or monoclonal antibodies with enzymes such as papain or pepsin. Alternatively, an antibody fragment can be produced by genetic engineering techniques using a gene that encodes an antibody fragment (see Co et al. J. Immunol. 1994, 152:2968-76; Better and Horwitz. Methods Enzymol. 1989, 178:476-96; Pluckthun and Skerra. Methods Enzymol. 1989,178:497-515; Lamoyi. Methods Enzymol. 1986, 121:652-63; Rousseaux *et al.* 1986, 121:663-9; Bird and Walker. Trends Biotechnol. 1991, 9:132-7).

The multispecific antibodies of the present invention include bispecific antibodies (BsAb), diabodies (Db), etc. Multispecific antibodies can be produced by methods such as (1) chemically coupling antibodies having different specificities with different types of bifunctional linkers (Paulus Behring Inst. Mill. 1985, 78:118-32), (2) fusing hybridomas that secrete different monoclonal antibodies (Millstein and Cuello. Nature 1983, 305:537-9), or (3) transfecting eukaryotic cell expression systems, such as mouse myeloma cells, with a light chain gene and a heavy chain gene of different monoclonal antibodies (four types of DNA), followed by the isolation of a bispecific monovalent portion (Zimmermann. Rev. Physio. Biochem. Pharmacol. 1986, 105:176-260; Van Dijk et al. Int. J. Cancer 1989, 43:944-9). On the other hand, diabodies are dimer antibody fragments comprising two bivalent polypeptide chains that can be constructed by gene fusion. They can be produced using known methods (see Holliger et al. Proc. Natl. Acad. Sci. USA 1993, 90:6444-8; EP404097; WO 93/11161).

Recovery and purification of antibodies and antibody fragments can be carried out using Protein A and Protein G, or according to known protein purification techniques (Antibodies: A Laboratory Manual, Ed. Harlow and David Lane, Cold Spring Harbor Laboratory (1988)). For example, when using Protein A to purify an antibody of the present invention, known Protein A columns such as Hyper D, POROS, or Sepharose F.F. (Pharmacia) can be used. The concentration of the resulting antibody can be determined by measuring the absorbance or by enzyme linked immunoadsorbent assay (ELISA).

Antigen-binding activity of an antibody can be determined by absorbance measurement, or by using fluorescent antibody methods, enzyme immunoassay (EIA) methods, radioimmunoassay (RIA) methods, or ELISA. When ELISA is used, an antibody of the present invention is first immobilized onto a support such as a plate. A polypeptide of the present invention is added, and then a sample containing the antibody of interest is added. Here, samples containing an antibody of interest include, for example, culture supernatants of antibody-producing cells, purified antibodies, etc. Next, a secondary antibody that recognizes an antibody of the present invention is added, followed by the incubation of the plate. Subsequently, the plate is washed and the label attached to the secondary antibody is detected. Namely, if a secondary antibody is labeled with alkaline phosphatase, the antigen binding activity can be determined by adding an enzyme substrate such as p-nitrophenyl phosphate, and measuring the absorbance. In addition, a commercially available system such as BIAcore (Pharmacia) can also be used to evaluate antibody activities.

The antibodies of the present invention can recognize or detect a polypeptide of the present invention or a fragment thereof. Furthermore, since the antibodies recognize a polypeptide of the present invention or a fragment thereof, they can recognize or detect cells or the like expressing the polypeptide or a fragment thereof. In addition, the antibodies can be used to purify a polypeptide of the present invention or a fragment thereof. The antibodies can also be used to purify cells or the like expressing the polypeptide of the present invention or a fragment thereof.

The antibodies of the present invention preferably bind to a polypeptide comprising the entire or at least six consecutive amino acid residues of the amino acid sequence of (A') and (B') described below, and more preferably bind to a polypeptide comprising at least six consecutive amino acid residues of the above amino acid sequence.
(A') the amino acid sequences from positions 21 to 510 of SEQ ID NO: 2, 4, 24, 26, 28, or 30; positions 20 to 513 of SEQ ID NO: 20 or 22; positions 21 to 460 of SEQ ID NO: 32 or 34; and positions 21 to 490 of SEQ ID NO: 36.
(B') the amino acid sequences from positions 23 to 708 of SEQ ID NO: 47; positions 23 to 710 of SEQ ID NO: 49; positions 23 to 712 of SEQ ID NO: 51; and positions 23 to 708 of SEQ ID NO: 53.

In addition, it is necessary that a support used in immobilizing an antibody or a polypeptide of the present invention is safe to cells. Examples of such a support include synthetic or naturally occurring organic polymer compounds, inorganic materials such as glass beads, silica gel, alumina, and activated charcoal, and those that have their surfaces coated with a polysaccharide or synthetic polymer. There are no particular limitations on the form of the support, examples of which include films, fibers, granules, hollow fibers, non-woven fabric, porous supports, or honeycombed supports, and the contact surface area can be controlled by changing its thickness, surface area, width, length, shape, and size in various ways.

Marker proteins for Purkinje progenitor cells other than the proteins selected by the methods of the present invention include, for example, proteins encoded by genes selected from the group consisting of the Lim1/2 and Corl2 genes.

In the methods of the present invention, transcripts of the 65B 13 gene and E-cadherin gene can be detected by contacting a polynucleotide of the present invention with nucleic acid extract derived from a cell sample, and detecting a nucleic acid that hybridizes to the polynucleotide in the nucleic acid extract.

The polynucleotide probe is preferably labeled with radioisotope or non-radioactive compound to detect transcripts of the 65B13 gene and E-cadherin gene. The radioisotopes to be used as a label include, for example, ³⁵S and ³H. When a radiolabeled polynucleotide probe is used, RNA that binds to a marker can be detected by detecting silver particles by emulsion autoradiography. Meanwhile, for conventional non-radioisotopic compounds used to label polynucleotide probes, biotin and digoxigenin are known. The detection of biotin-labeled markers can be achieved, for example, using fluorescently labeled avidin or avidin labeled with an enzyme such as alkaline phosphatase or horseradish peroxidase. On the other hand, the detection of digoxigenin-labeled markers can be achieved by using fluorescently labeled anti-digoxigenin antibody or anti-digoxigenin antibody labeled with an enzyme such as alkaline phosphatase or horseradish peroxidase. When enzyme labeling is used, the detection can be made by allowing stable dye to deposit at marker positions by incubation with an enzyme substrate.

When polynucleotide primers are used for detection of transcripts of the 65B13 gene and E-cadherin gene, their transcripts can be detected by amplifying nucleic acid that hybridizes to the polynucleotide primers, for example, using techniques such as RT-PCR.

Detection of the translation products of the 65B 13 gene and E-cadherin gene with the methods of the present invention can be made by contacting the antibody described above with a protein extract of cell samples and then detecting proteins bound to the antibody. As described above, methods for assaying the antigen binding activity of an antibody include absorbance measurement, fluorescent antibody method, enzyme immunoassay (EIA), radioimmunoassay (RIA), ELISA, etc.

In the context of the present invention, highly accurate identification can be achieved by detecting, in addition to transcripts or translation products of the 65B 13 gene and E-cadherin gene, the transcripts or translation products of one or more genes selected from the group consisting of Lim1/2 and Corl2. Such methods are also included in the present invention.

Furthermore, the expression of the polynucleotides of 65B 13 and E-cadherin of the present invention can be used as an indicator to screen for substances that are effective for induction of differentiation into Purkinje progenitor cells.

The present invention provides methods of screening for substances that are effective for induction of differentiation into Purkinje progenitor cells. Since compounds obtained through screening by the methods of the present invention have the activity of differentiating Purkinje progenitor cells, they are potential candidate compounds useful for treating diseases caused by defects in Purkinje cells. Target diseases (illnesses) of treatment using a compound obtained by the screening methods include, for example, cerebellar degeneration.

In a preferred embodiment, the above-described screening methods of the present invention comprise the steps of:
(I) contacting a test compound with cells that can differentiate into Purkinje progenitor cells; and
(II) detecting in Purkinje progenitor cells the expression of a polynucleotide that can hybridize to a polynucleotide sequence selected from (A) and (B) described above or to a complementary sequence thereof.

Here, the "test substance" may be any type of compound, examples of which include the expression products of gene libraries, synthetic low-molecular-weight compound libraries, synthetic peptide libraries, antibodies, substances released by bacteria, cell (microbial, plant, or animal) extracts, cell (microbial, plant, or animal) culture supernatants, purified or partially purified polypeptides, marine organisms, plant or animal extracts, soil, random phage peptide display libraries, etc.

Furthermore, since 65B13 and E-cadherin are expressed selectively in differentiated Purkinje progenitor cells, it can be used to screen for reagents that induce differentiation into Purkinje progenitor cells. Specifically, whether a test sample has the ability to induce differentiation into Purkinje progenitor cells can be assessed by inducing the differentiation into Purkinje progenitor cells from cells having the ability to differentiate into Purkinje progenitor cells in the presence of the test sample, and detecting the expression of E-cadherin in the differentiated cells.

Thus, the present invention provides methods of screening for candidate compounds as a reagent that induces differentiation into Purkinje progenitor cells, which comprise the steps of:
(f) inducing the differentiation into Purkinje progenitor cells from cells having the ability to differentiate into Purkinje progenitor cells in the presence of a test sample;
(g) detecting transcripts or translation products of the 65B13 gene and E-cadherin gene in the differentiated cells; and
(h) selecting a compound that increases the level of the transcripts or translation products as compared to those detected in the absence of the test sample.

In the above methods, the preferred "cells having the ability to differentiate into Purkinje progenitor cells" are cell samples containing cells that can be differentiated into Purkinje progenitor cells, such as multipotent ES cells.

The transcripts or translation products of the 65B 13 gene and E-cadherin gene of the present invention can be detected using polynucleotides that hybridize to a transcript of the 65B 13 gene or E-cadherin gene, or antibodies that bind to a translation product of the 65B 13 gene or E-cadherin gene, as described above.

In the present invention, cell growth and differentiation can be detected by comparing the cell condition with that when the test substance is not contacted. Cell growth and differentiation can be assessed through morphological observation under a microscope, or detecting or quantifying substances produced upon cell differentiation.

Cell differentiation can be assessed by comparing the expression levels of the 65B 13 gene and E-cadherin gene with those in the absence of a test sample. Specifically, when a test sample increases the level of transcript or translation product of the 65B13 gene or E-cadherin gene as compared to that in the absence of the test sample, the test sample is assessed to have the ability to differentiate nerve cells. "Increase" means that, for example, the level becomes twice, preferably five times, and more preferably ten or more times.

In a preferred embodiment of the present invention, the screening methods of the present invention further comprise the step of selecting compounds in which the expression of the polynucleotide is detected in step (II).

In another preferred embodiment of the present invention, the screening methods of the present invention also include, for example, methods comprising:
(IV) the step of contacting a test compound with cells having the ability to differentiate into Purkinje progenitor cells; and
(V) the steps comprising (A) and (B) below:
   (A) detecting a protein selected from (v), (vi), (vii), and (viii) described above; and
   (B) detecting a protein selected from (v'), (vi'), (vii'), and (viii') described above.

In a preferred embodiment, the above-described methods further comprise step (VI) of selecting compounds in which the protein is detected in step (V).

The above methods may be based on the use of promoters (including modified promoters) (as a means) that control (induce) expression of an mRNA translated into the protein of (A) or (B) below:
(A) a protein selected from (v), (vi), (vii), and (viii) above; or
(B) a protein selected from (v'), (vi'), (vii'), and (viii').

For example, it is possible to transfect cells with a vector carrying a construct in which a gene encoding a detectable marker such as green fluorescent protein (GFP) is linked to a promoter portion obtained by analyzing the expression regulatory regions. Alternatively, a marker gene can be knocked in at the gene locus. The expression of a marker gene specific to GABA-producing neuron progenitor cells comprising Purkinje cells is detected by (A) and the expression of a marker gene specific to Purkinje progenitor cells is detected by (B). Combined use of the two enables detection of marker genes specific to Purkinje progenitor cells and thus enables detection of the protein. The protein expression can be detected by methods (means) for detecting protein expression, and therefore the methods can also be used as methods (means) for detecting the protein-encoding gene.

In this case, "the gene encoding a marker is linked to a promoter portion" means that the gene encoding the marker is linked to the promoter portion in an expressible manner. The gene may be directly linked to the promoter, or the gene may be linked distantly to but still under the control of the promoter. Furthermore, the promoter portion obtained by analyzing the 65B13 expression regulatory region may be replaced with another promoter, as long as the promoter for 65B13 enables the expression of the 65B13 region. The promoter portion obtained by analyzing the E-cadherin expression region may also be replaced with another promoter, as long as the promoter for E-cadherin enables the expression of the E-cadherin region.

All prior-art documents cited in the specification have been incorporated herein by reference.

### Examples

Hereinbelow, the present invention is specifically described with reference to the Examples; however, it should not be construed as being limited thereto.

### [Example 1 Analysis of 65B13 expression in cerebellar primordium and isolation of 65B13-positive cells

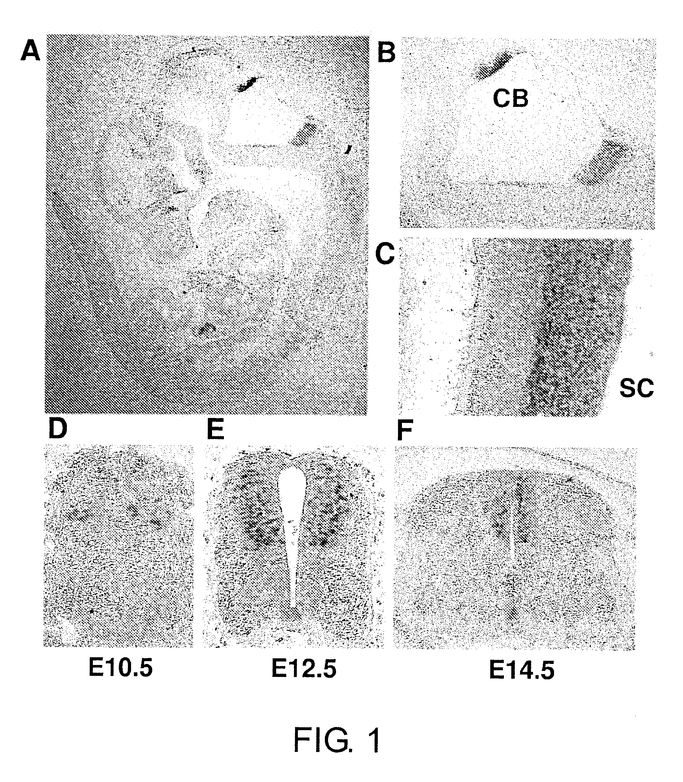

65B13 (Neph3) is selectively expressed in several areas of fetal brain (Fig. 1; WO 2004/038018; Minaki Y, Mizuhara E, Morimoto K, Nakatani T, Sakamoto Y, Inoue Y, Satoh K, Imai T, Takai Y, Ono Y. Migrating postmitotic neural precursor cells in the ventricular zone extend apical processes and form adherens junctions near the ventricle in the developing spinal cord. Neurosci Res. 2005, 52(3):250-62). In the midbrain, dopaminergic neuron progenitor cells have been demonstrated to express 65B13 (WO 2004/038018); however, the types of cells that express 65B13 remain unidentified in other areas. The cerebellum is constituted of glutamic acid-producing granule cells and GABA-producing neurons such as Purkinje cells, Golgi cells, stellate cells, and basket cells (Wang VY, Zoghbi HY Genetic regulation of cerebellar development. Nat Rev Neurosci. 2001, 2(7):484-91). The granule cells are known to develop in the rhombic lip region at E12.5 to E14.5 (Wang VY, Zoghbi HY Genetic regulation of cerebellar development. Nat Rev Neurosci. 2001, 2(7):484-91). By contrast, the development of GABA-producing neurons is still poorly understood. However, recent findings suggest that Purkinj cells are generated at E11.5 to E13.5 in the dorsal rhombomere 1 (cerebellar primordium area) (Chizhikov VV, Lindgren AG, Currle DS, Rose MF, Monuki ES, Millen KJ. The roof plate regulates cerebellar cell-type specification and proliferation. Development. 2006, 133(15):2793-804). Although there is no detailed report on Golgi cells, they are thought to be generated at a late stage (E13.5 to E15.5) of development in the same region. The stellate cells and basket cells are thought to be generated from progenitor cells in the white matter after birth (Zhang L, Goldman JE. Generation of cerebellar interneurons from dividing progenitors in white matter. Neuron. 1996, 16(1):47-54).

To identify 65B 13-expressing cells in the cerebellar primordium, the spatial expression pattern of 65B13 was compared with those of various markers. Mouse E10.5 embryos were collected and fixed in 4% PFA/PBS(-) at 4°C for two hours. After replacing with 20% sucrose/PBS(-) at 4°C overnight, the embryos were embedded in OCT. Sections of 12-µm thickness were prepared and placed onto slide glasses. Then, the sections were dried at room temperature for 30 minutes, and again wetted with PBS(-). Next, after 30 minutes of blocking (BlockAce) at room temperature, a primary antibody was reacted at room temperature for one hour. The reaction was followed by incubation at 4°C overnight. The sections were washed three times with 0.1% Tween-20/PBS(-) at room temperature for 15 minutes each, and then incubated with a fluorescently labeled secondary antibody at room temperature for one hour. After washing in the same way, the sections were washed with PBS(-) at room temperature for ten minutes, and then mounted. The primary antibodies used were: 65B13 (see WO 2004/038018; Minaki Y, Mizuhara E, Morimoto K, Nakatani T, Sakamoto Y, Inoue Y, Satoh K, Imai T, Takai Y, Ono Y. Migrating postmitotic neural precursor cells in the ventricular zone extend apical processes and form adherens junctions near the ventricle in the developing spinal cord. Neurosci Res. 2005, 52(3):250-62), Pax2 (purchased from Zymed), and Cor12 (see WO 2006/082826).

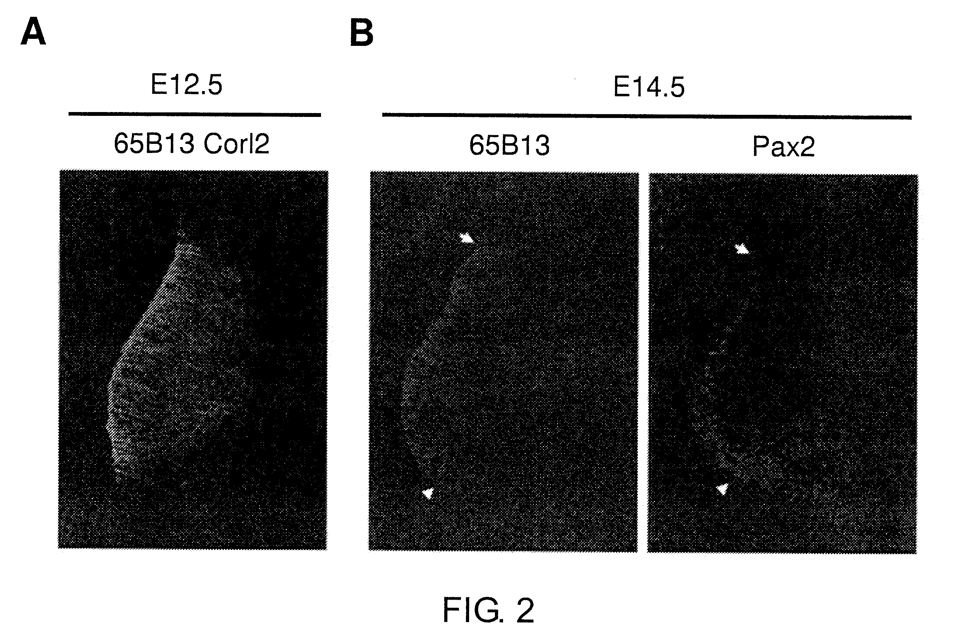

The result showed that in the cerebellar primordium of E12.5, 65B13 was selectively expressed in VZ where Cor12-positive Purkinje cells develop (Fig. 2A). In addition, neurons that are thought to be Pax2-positive Golgi cells revealed to start to emerge at E14.5 in the same 65B13-positive area (Maricich SM, Herrup K. Pax-2 expression defines a subset of GABAergic interneurons and their precursors in the developing murine cerebellum. J Neurobiol. 1999, 41(2):281-94) (Fig. 2B).

Thus, experiments were carried out to isolate and culture 65B13-positive cells for the purpose of confirming that the 65B13-positive cells are progenitor cells of Purkinje and Golgi cells.

The cerebellar primordium areas at E12.5 and E14.5 were excised, and dispersed using Cell Dissociation Buffer (Invitrogen). Then, without fixation and permeability treatment, the cells were stained at 4°C for 20 minutes using an anti-65B13 monoclonal antibody (100 times diluted purified antibody, 1% bovine fetal serum, 1 mM EDTA/SDIA differentiation medium (Kawasaki et al. Neuron 2000, 28(1):31-40)). After washing three times with 1 mM EDTA/PBS containing 1% bovine fetal serum at 4°C for three minutes, the cells were stained with a PE-labeled anti-hamster IgG antibody (Jackson; 10 µg/ml, 1% bovine fetal serum, 1 mM EDTA/SDIA differentiation medium) at 4°C for 30 minutes and washed in the same way as described above. After staining, 65B 13-expressing cells were separated with a cell sorter. The isolated cells were placed onto slide glasses coated with poly-L-ornithine (Sigma, 0.002% in PBS), laminin (Invitrogen, 5 µg/ml in PBS), and fibronectin (Sigma, 5 µg/ml in PBS) and cultured at 37°C for two days in SDIA differentiation medium supplemented with Knockout Serum Replacement (Gibco, 5%), N2 (Invitrogen, 1x), B27 (Invitrogen, 1x), ascorbic acid (Sigma, 200 µM), and BDNF (Invitrogen, 20 ng/ml). The cultured cells were fixed with 2% PFA/PBS at 4°C for 20 minutes, and then washed twice with PBS at 4°C for 10 minutes. Then, cell permeability treatment was performed using 0.3% Triton X-100/PBS at room temperature for 30 minutes, and the cells were blocked with 10% normal donkey serum/BlockAce at room temperature for 20 minutes. Next, the cells were incubated with a primary antibody (10% normal donkey serum, 2.5% BlockAce, 0.1% Triton X-100/PBS) at room temperature for one hour and then at 4°C overnight. On the next day, after washing three times with 0.1% Triton X-100/PBS at room temperature for ten minutes, the cells were incubated with a fluorescently labeled secondary antibodies (all from Jackson, 10 µg/ml, 10% normal donkey serum, 2.5% BlockAce, 0.1% Triton X-100/PBS) at room temperature for 30 minutes. After washing in the same way as described above, the cells were washed with PBS at room temperature for five minutes, mounted, and observed. An anti-HuC/D antibody was purchased from Molecular Probe.

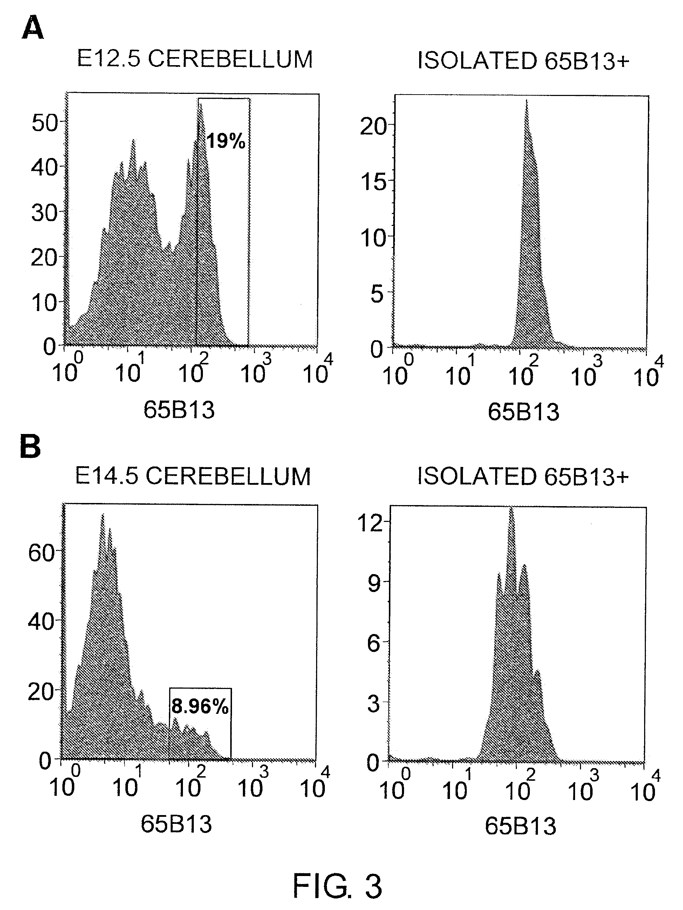

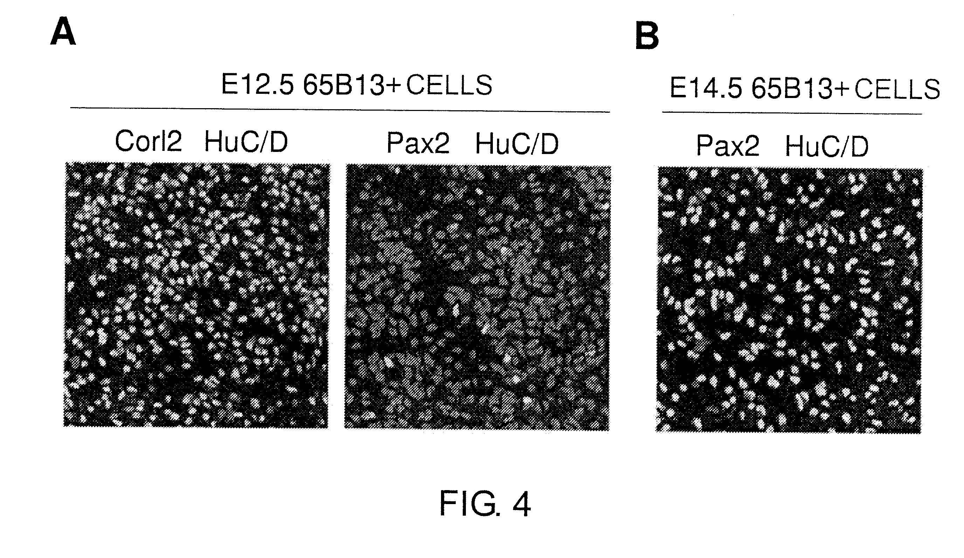

The result showed that 65B13-positive cells could be isolated alive from the cerebellar primordium areas at both of the developmental stages (Fig. 3). Furthermore, it was demonstrated that almost the entire population of the cells differentiated into neurons after two days of culturing, and nearly all of the E12.5 65B13-positive cells differentiated into Cor12-positive Purkinje cells (Fig. 4A) while nearly all of the E14.5 65B13-positive cells differentiated into Pax2-positive Golgi cell-like neurons (Fig. 4B). Thus, it was revealed that in the fetal cerebellum, 65B13 was selectively expressed in progenitor cells of Purkinje and Golgi cells, and these progenitor cells could be separated by using an anti-65B13 antibody. Specifically, 65B13 was demonstrated to be useful as a marker for separating GABA-producing neuron progenitor cells in the cerebellum.

### [Example 2] Specific expression of foreign genes using the 65B13 promoter in GABA-producing neuron progenitor cells

Next, whether foreign genes can be expressed in a GABA-producing neuron progenitor cell-specific manner using the 65B13 promoter was assessed by creating transgenic mice and analyzing the expression of foreign genes according to the protocol described below.

First, the poly A addition sequence of bovine growth hormone (SEQ ID NO: 41; derived from Invitrogen pcDNA3.1+ vector) was amplified and inserted into the *Hind*III/*Xho*I site of pSP73 (Promega) to construct pSP73-polyA. Then, the synthetic DNAs of SEQ ID NOs: 42 and 43 were annealed to each other and inserted into the *Asp*718I/*Bam*HI site of pSP73-polyA to construct pSP73-polyA II. A mouse genomic fragment (SEQ ID NO: 44) located about 3.2 kb upstream of the translation initiation codon of 65B 13 was inserted into the *Cla*I/*Asp*718I site of pSP73-polyAII to construct pN3. Finally, mouse Gsh1 cDNA (SEQ ID NO: 45) was inserted as a foreign gene into the *Asp*718I/*Sal*I site of pN3 to construct pN3-Gsh1. After linearized with *Cla*I, pN3-Gsh1 was injected into the pronuclei of mouse fertilized eggs according to the method of Gordon *et al.* (Gordon JW, Scangos GA, Plotkin DJ, Barbosa JA, Ruddle FH. Genetic transformation of mouse embryos by microinjection of purified DNA. Proc Natl Acad Sci USA. 1980 Dec;77(12):7380-4), and the eggs were transplanted into foster mothers. The fetuses were recovered at embryonic day 12.5, and the expression of Neph3 and Gsh1 in the cerebellar primordia was analyzed by the methods described in Example 1. An anti-Gsh1 antibody was prepared by the method described below. First, an expression vector was constructed for a GST fusion protein with amino acids 1 to 72 of Gsh1 as an immunization antigen. After the resulting vector was introduced into *E. coli* (JM109 strain), the expression was induced with IPTG The fusion protein was collected using glutathione beads. After the rats were immunized with the collected fusion protein, lymphocytes were collected and fused with myeloma cell P3U1. Thus, anti-Gsh1 antibody-producing hybridomas were obtained (hybridoma preparation was outsourced to Kohjin Bio Co.).

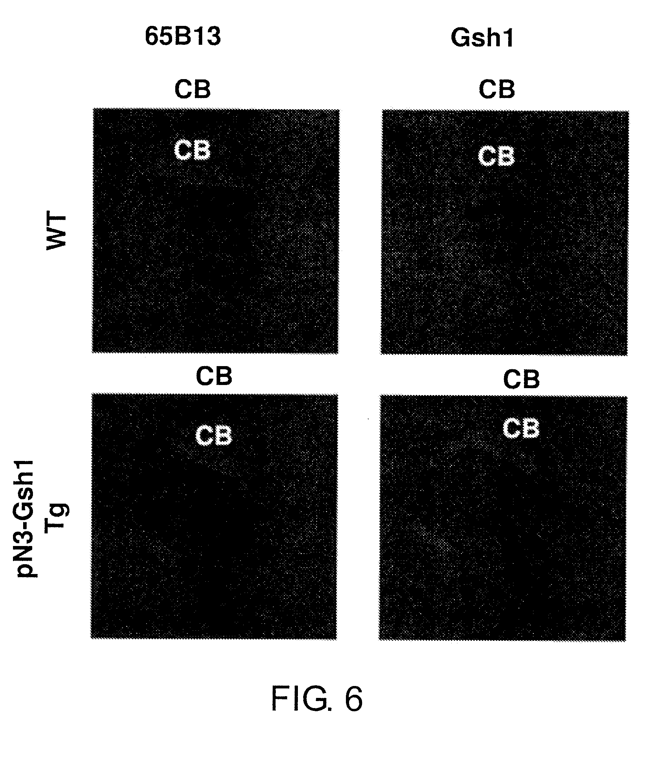

The result showed that the wild-type cerebellum expressed Gsh1 only in a very small ventral fraction of 65B 13-positive GABA-producing neuron progenitor cells, while the transgenic mice expressed Gsh1 specifically in the entire 65B13-positive area (Fig. 6). This finding demonstrates that foreign genes can be expressed in a GABA-producing neuron progenitor cell-specific manner by using the 65B13 promoter.

### [Example 3] Identification of Purkinje progenitor cell-specific genes

The 65B13 gene is known to be expressed transiently in dopamine-producing neuron progenitor cells after termination of cell division (see Patent Document 1); however, there is no report published on the connection between the gene and GABA neuron (WO 2004/038018; Minaki Y, Mizuhara E, Morimoto K, Nakatani T, Sakamoto Y, Inoue Y, Satoh K, Imai T, Takai Y, Ono Y. Migrating postmitotic neural precursor cells in the ventricular zone extend apical processes and form adherens junctions near the ventricle in the developing spinal cord. Neurosci Res. 2005, 52(3):250-62). The cerebellum is constituted of GABA-producing neurons such as Purkinje cells, Golgi cells, stellate cells, and basket cells, and glutamic acid-producing granule cells (Wang VY, Zoghbi HY Genetic regulation of cerebellar development. Nat Rev Neurosci. 2001, 2(7):484-91). The granule cells are known to develop in the rhombic lip region at E12.5 to E14.5 (Wang VY, Zoghbi HY Genetic regulation of cerebellar development. Nat Rev Neurosci. 2001, 2(7):484-91). By contrast, the development of GABA-producing neurons is still poorly understood. However, recent findings suggest that Purkinje cells are generated at E11.5 to E13.5 in the dorsal rhombomere 1 (cerebellar primordium area) (Chizhikov VV, LindgrenAG, Currle DS, Rose MF, Monuki ES, Millen KJ. The roof plate regulates cerebellar cell-type specification and proliferation. Development 2006, 133(15):2793-804; Minaki Y, Nakatani T, Mizuhara E, Inoue T, Ono Y. Identification of a novel transcriptional corepressor, Cor12, as a cerebellar Purkinje cell-selective marker. Gene Expr. Patterns, 2008, 8(6):418-423). Although there is no detailed report on Golgi cells, they are assumed to be generated at a late developmental stage (E13.5 to E15.5) in the same region. The stellate cells and basket cells are speculated to originate from progenitor cells in the white matter after birth (Zhang L, Goldman JE. Generation of cerebellar interneurons from dividing progenitors in white matter. Neuron. 1996, 16(1):47-54). Of these neurons, only Purkinje cells transmit neural signals outwardly from the cerebellar cortex. Degeneration of Purkinje cells is involved in the onset of spinocerebellar degeneration and such. 65B13 is a useful marker for isolation of GABA neuron progenitor cells including Purkinje cells. However, it was suggested that to obtain Purkinje progenitor cells of higher purity, Purkinje progenitor cells had to be further purified from a 65B13-positive cell population using a marker that is selectively expressed by Purkinje progenitor cells. Thus, the present inventors identified such selective markers according to the following protocol.

First, cerebellar primordia, myelencephalons, and spinal cords were excised from E12.5 mice, and also cerebellar primordia were excised from E14.5 mice. The cells were dispersed in accumax (MS TECHONOSYSTEMS), and without fixation or permeability treatment, they were stained at 4°C for 30 minutes using an anti-65B13 monoclonal antibody (100 times diluted purified antibody (described in Minaki *et al.,* 2005) in D-MEM-F12 containing 1% fetal bovine serum and 1 mM EDTA). Then, the cells were washed three times with D-MEM:F12 medium containing 1 % fetal bovine serum and 1 mM EDTA at 4°C for three minutes. After staining with a PE-labeled anti-hamster IgG antibody (BD pharmingen; 8 µg/ml in D-MEM:F12 medium containing 1% fetal bovine serum and 1 mM EDTA) at 4°C for 30 minutes, the cells were washed in the same way as described above. After staining, 65B 13-expressing cells were isolated by a cell sorter. Immediately after cell isolation, total RNA was prepared from the cells using the RNeasy Mini Kit (Qiagen), and double-stranded cDNA was synthesized using a cDNA synthesis kit (TAKARA). Then, the synthesized cDNA was digested with restriction enzyme *Afa*I (TAKARA). After attaching ad2, the cDNA was amplified by PCR using ad2S as a primer.

The amplification conditions were: five minutes of incubation at 72°C; and 20 cycles of 94°C for 30 seconds, 65°C for 30 seconds, and 72°C for two minutes; followed by two minutes of final incubation at 72°C.
ad2S: CAGCTCCACAACCTACATCATTCCGT (SEQ ID NO: 54)
ad2A: ACGGAATGATGT (SEQ ID NO: 55)

PCR was carried out using a reaction solution containing the following components:

| | |
|---|---|
| 10x ExTaq | 5 µl |
| 2.5 mM dNTP | 4 µl |
| ExTaq | 0.25 µl |
| 100 µM primer | 0.5 µl |
| cDNA | 2 µl |
| distilled water | 38.25 µl |

Next, search of the samples was performed using a subtraction (N-RDA) method (described in WO 2004/065599) for genes specific to 65B13-positive cells derived from E12.5 cerebellar primordium. One of the isolated cDNA fragments was found to encode E-cadherin. Then, the expression of E-cadherin was confirmed by the following RT-PCR method using the primers listed below.

PCR was carried out in the following reaction system using as a 3-ng or 0.3-ng aliquot of the amplified cDNA as template.

| | |
|---|---|
| 10x ExTaq | 1 µl |
| 2.5 mM dNTP | 0.8 µl |
| ExTaq | 0.05 µl |
| 100 µM primer | 0.1 µl each |
| cDNA | 1 µl |
| distilled water | 6.95 µl |

After two minutes of incubation at 94°C, PCR amplification was carried out with 26 cycles of 94°C for 30 seconds, 65°C for 30 seconds, and 72°C for two minutes, followed by two minutes of final incubation at 72°C.

PCR was carried out using the following primers:
E-cadherin: CTCCAATGCCTGCTCTTGATGGTAGC (SEQ ID NO: 56)
   TCTCTGTGTAGCCCTGGCTGTCCTAG (SEQ ID NO: 57)
65B13: CTTCCCGTATGCTACCTTGTCTCCAC (SEQ ID NO: 58)
   CCAACAGTCCTGCATGCTTGTAATGA (SEQ ID NO: 59)

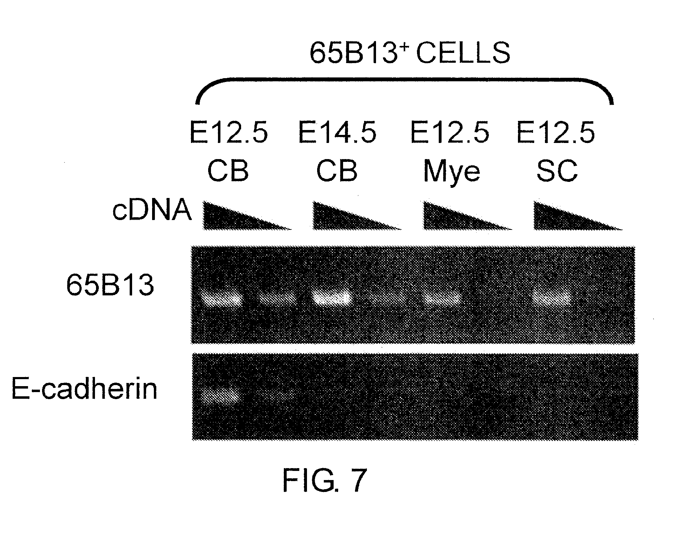

The result showed that among the 65B13-positive cells derived from various brain areas, E-cadherin was specific to 65B13-positive cells derived from the cerebellar primordium which produces Purkinje cells (Fig. 7). It was also demonstrated that E-cadherin was expressed in cerebellar primordium-derived 65B13-positive cells specifically at the stage of Purkinje cell differentiation (E12.5).

### [Example 4] Expression analysis of E-cadherin in fetal brain tissues

Next, to assess the expression of E-cadherin in fetal brain, in particular, 65B13-positive cells, the expression of E-cadherin and 65B13 was compared with the expression of the Purkinje cell-specific marker Cor12 (Minaki Y, Nakatani T, Mizuhara E, Inoue T, Ono Y. Identification of a novel transcriptional corepressor, Cor12, as a cerebellar Purkinje cell-selective marker. Gene Expr. Patterns, 2008, 8(6):418-423) and another GABA neural marker, Pax2, according to the protocol described below.

Brain tissues were excised from day-12.5 and day-14.5 mouse embryos, and fixed in 4% PFA/PBS(-) at 4°C for two hours and one hour, respectively. After fixation, the solution was replaced with 10% sucrose/PBS(-) at 4°C for five hours, and then with 20% sucrose/PBS(-) at 4°C overnight. The tissues were embedded in OCT, and sliced into 14-µm sections. The sections were placed onto glass slides, dried at room temperature for 30 minutes, and again wetted with 0.1% Triton X-100/PBS(-). Then, the tissues were blocked with BlockAce at room temperature for 30 minutes, and incubated with primary antibodies at 4°C overnight. The glass slides were washed three times with 0.1% TritonX-100/PBS(-) at room temperature for ten minutes. Then, the tissues were incubated with fluorescently labeled secondary antibodies at room temperature for 40 minutes. After washing in the same way as described above, the glass slides were rinsed twice with PBS(-), and the sections were mounted. The primary antibodies used are listed below: 65B13, as described in Example 3; Cor12, as described in Minaki Y, Nakatani T, Mizuhara E, Inoue T, Ono Y. Identification of a novel transcriptional corepressor, Cor12, as a cerebellar Purkinje cell-selective marker. Gene Expr. Patterns, 2008, 8(6):418-423; Pax2, purchased from Zymed (500 times diluted); and E-cadherin, purchased from TAKARA (100 times diluted).

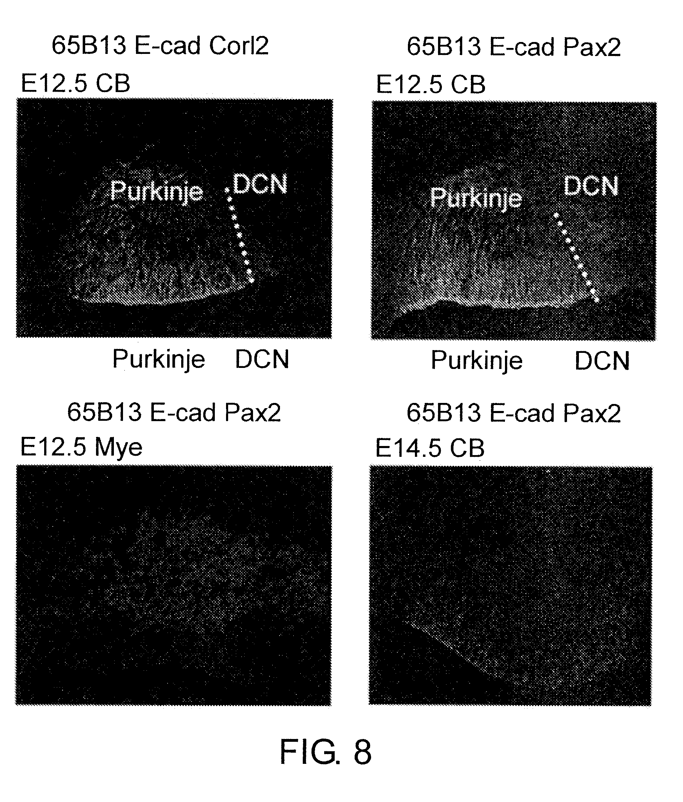

The result showed that E-cadherin was expressed selectively in the cerebellar primordium but not in the myelencephalon (Fig. 8). Furthermore, it was demonstrated that E-cadherin was expressed selectively in the 65B13-positive area of cerebellar primordium and specifically at the stage of Purkinje cell differentiation (E12.5). Furthermore, the expression level of E-cadherin within the 65B13-positive area of E12.5 cerebellar primordium was higher in the dorsal region, from which Cor12-positive Purkinje cells originate, than in the ventral region, where Pax2-positive cerebellar nucleus GABA neurons are generated.

### [Example 5] FACS analysis of E-cadherin and 65B13 expression in fetal brain cells

Next, to confirm that E-cadherin is a useful marker in FACS-based isolation of Purkinje progenitor cells, FACS analysis was carried out according to the protocol described below.

First, cerebellar primordia, myelencephalons, and spinal cords were excised from E12.5 mice, and also cerebellar primordia were excised from E14.5 mice. The cells were dispersed using the NeuroCult Chemical Dissociation Kit (StemCell Technologies Inc), and without fixation or permeability treatment, they were stained at 4°C for 30 minutes using an anti-65B13 monoclonal antibody (purified antibody described in Example 3; 100 times diluted) or an anti-E-cadherin antibody (100 times diluted). Then, the cells were washed three times with PBS containing 1 mM EDTA and 1% fetal bovine serum at 4°C for three minutes. After staining with a PE-labeled hamster IgG antibody (BD pharmingen; 8 µg/ml, in PBS containing 1% fetal bovine serum and 1 mM EDTA) or APC-labeled anti-rat IgG antibody (Jackson; 10 µg/ml, in PBS containing 1% fetal bovine serum and 1 mM EDTA) at 4°C for 30 minutes, the cells were washed in the same way as described above. After staining, E-cadherin-expressing and 65B13-expressing cells were detected using a flow cytometer.

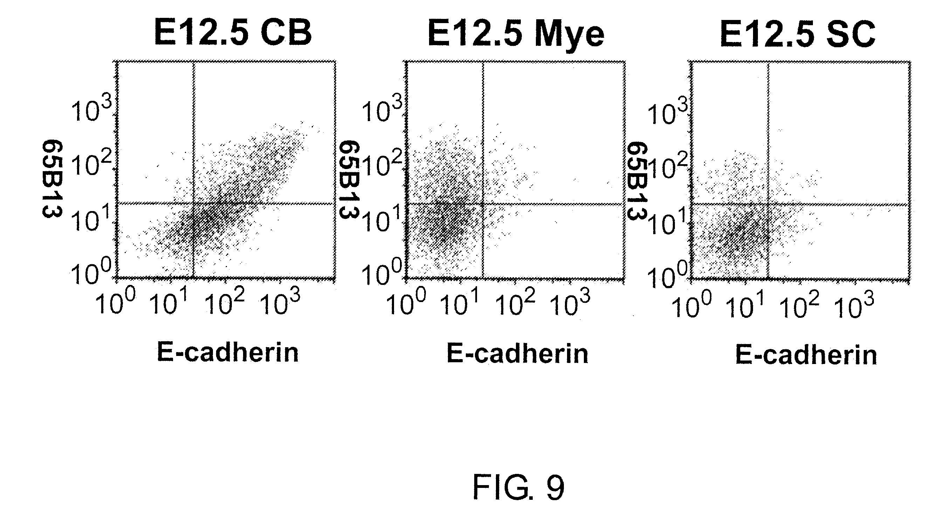

In correlation with the result of tissue expression analysis described in Example 4, the result demonstrates that only cerebellum-derived 65B13-positive cells express E-cadherin on their cell surface (Fig. 9). Thus, E-cadherin was shown to be a useful marker in the FACS-based isolation of Purkinje progenitor cells from a 65B13-positive cell population.

### [Example 6] Isolation and culture of E-cadherin and 65B13-double positive cells

Next, to confirm that E-cadherin and 65B13-double positive cells are Purkinje progenitor cells, isolation and culture experiments were carried out according to the protocol described below.

First, cells from cerebellar primordia of E12.5 mice were stained by the same method described in Example 5. E-cadherin and 65B13-double positive cells were isolated using a cell sorter. The isolated cells were placed onto glass slides coated with poly-L-ornithine (Sigma; 0.002% in PBS), laminin (Invitrogen; 2.5 µg/ml in PBS), and fibronectin (Sigma; 5 µg/ml in PBS), and cultured at 37°C for two days in D-MEM:F12 medium supplemented with N2 (Invitrogen; 1x), B27 (Invitrogen; 1x), and BDNF (Invitrogen; 20 ng/ml). The cultured cells were fixed in 2% PFA/PBS at 4°C for 20 minutes, and washed twice with PBS at 4°C for ten minutes. Then, cell permeability treatment was performed using 0.3% Triton X-100/PBS at room temperature for 30 minutes, and the cells were blocked with 10% normal donkey serum/BlockAce at room temperature for 20 minutes. Next, the cells were incubated with a primary antibodies (10% normal donkey serum, 2.5% BlockAce, 0.1% Triton X-100/PBS) at room temperature for one hour and then at 4°C overnight. On the next day, after the cells were washed three times with 0.1% Triton X-100/PBS at room temperature for ten minutes, they were incubated with fluorescently labeled secondary antibodies (all from Jackson; 3 µg/ml, 10% normal donkey serum, 2.5% BlockAce, 0.1% Triton X-100/PBS) at room temperature for 30 minutes. After washing in the same way as described above, the cells were washed with PBS at room temperature for five minutes, mounted, and observed. The primary antibodies used are listed below: HuC/D, purchased from Molecular Probe; and Cor12, as described in Example 1.

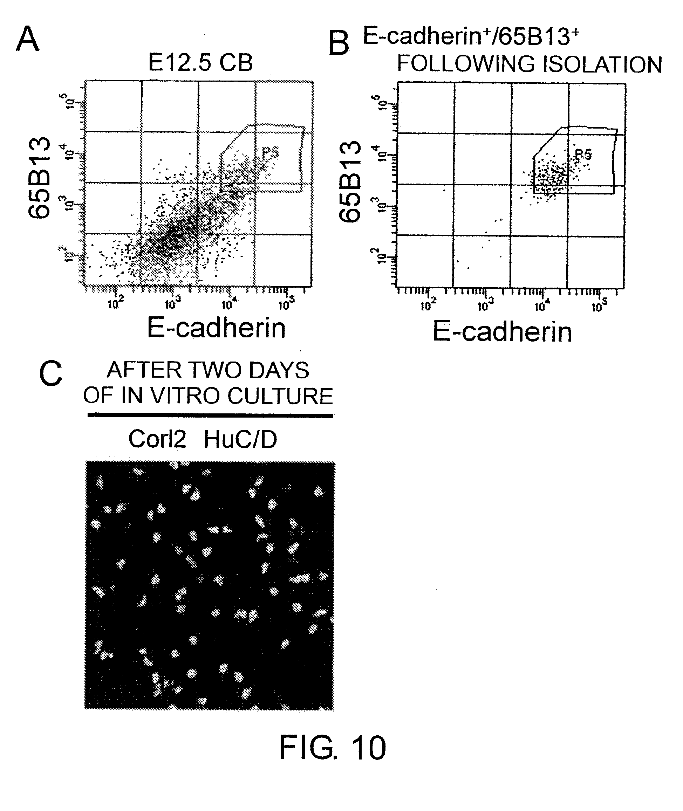

The result showed that most of the isolated E-cadherin and 65B13 double-positive cells differentiated into Cor12-positive Purkinje cells after two days of culture (Fig. 10). Thus, E-cadherin used in combination with 65B13 was demonstrated to serve as a useful maker for isolating Purkinje progenitor cells.

### Sequence Listing Free Text

SEQ ID NO: 19 Mouse 65B13 NM_172898 extracellular: 20-513 a.a.
SEQ ID NO: 21 Mouse 65B13 BC052773 extracellular: 20-513 a.a.
SEQ ID NO: 23 Human 65B13 NM_032123 extracellular: 21-510 a.a.
SEQ ID NO: 25 Human 65B13 NM_199180 extracellular: 21-510 a.a.
SEQ ID NO: 27 Human 65B13 AY358742 extracellular: 21-510 a.a.
SEQ ID NO: 29 Human 65B13 AY305301 extracellular: 21-510 a.a.
SEQ ID NO: 31 Human 65B13 NM_199179 extracellular: 21-460 a.a.
SEQ ID NO: 33 Human 65B13 AY305302 extracellular: 21-460 a.a.
SEQ ID NO: 35 Human 65B13 BC064925 extracellular: 21-490 a.a.
SEQ ID NO: 37 Chimpanzee 65B13 (predicted) XM_512603 extracellular: 21-445 a.a.
SEQ ID NO: 39 Cattle 65B13 (predicted) XM_583222 extracellular: 44-607 a.a.
SEQ ID NO: 46 Human E-cadherin NM_004360 ORF: 125-2773
SEQ ID NO: 47 Human E-cadherin amino acids SS (signal sequence): 1-22 TM (transmembrane domain):709-731
SEQ ID NO: 48 Mouse E-cadherin NM_009864 ORF: 128-2782
SEQ ID NO: 49 Mouse E-cadherin amino acids SS: 1-22 TM:711-733
SEQ ID NO: 50 Rat E-cadherin NM_031334 ORF: 127-2787
SEQ ID NO: 51 Rat E-cadherin amino acids SS: 1-22 TM:713-735
SEQ ID NO: 52 Chimpanzee E-cadherin XM_001168150 ORF: 192-2840
SEQ ID NO: 53 Chimpanzee E-cadherin amino acids SS: 1-22 TM:709-731

## Claims

1. A method for detecting or selecting a Purkinje progenitor cell, which comprises the steps of:
(A) detecting a protein selected from:
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B) detecting a protein selected from:
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

2. A method for detecting or selecting a Purkinje progenitor cell, which comprises the step of detecting a marker protein by linking a polynucleotide encoding the marker protein to a promoter that controls expression of an mRNA translated into a protein selected from:
(A) protein selected from:
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B) a protein selected from:
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

3. The method of claim 1 or 2, wherein the step of detecting the protein comprises the steps of: (d) contacting a test cell sample with an antibody that binds to the protein described in claim 1 or 2; and
(e) detecting reactivity.

4. The method of any one of claims 1 to 3, wherein the detection step is followed by the step of separating a Purkinje progenitor cell from the detected sample.

5. A method for detecting or selecting a Purkinje progenitor cell, which comprises the steps of:
(A) detecting the expression of a polynucleotide capable of hybridizing to a polynucleotide selected from (i),(ii), (iii), and (iv) below, or a complementary sequence thereof:
(i) a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iii) a polynucleotide which is expressed in a Purkinje progenitor cell and hybridizes under stringent conditions to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iv) a polynucleotide which is expressed in a Purkinje progenitor cell and has 80% or higher sequence identity to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(B) detecting the expression of a polynucleotide capable of hybridizing to a polynucleotide selected from (i'), (ii'), (iii'), and (iv') below, or a complementary sequence thereof:
(i') a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(iii') a polynucleotide which is expressed in a Purkinje progenitor cell and hybridizes under stringent conditions to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52; and
(iv') a polynucleotide which is expressed in a Purkinje progenitor cell and has 80% or higher sequence identity to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52.

6. The method of claim 5, wherein the step of detecting the expression of a polynucleotide comprises the steps of:
(a) contacting a test cell sample with a polynucleotide that can hybridize to the polynucleotide described in claim 5 or to a complementary sequence thereof, or with a probe comprising the polynucleotide; and
(b) detecting reactivity.

7. The method of claim 6, wherein the probe is contacted with mRNA prepared from the test cell sample or a complementary DNA (cDNA) transcribed from the mRNA in step (a).

8. The method of claim 5, wherein the step of detecting the expression of a polynucleotide that can hybridize to the polynucleotide described in claim 5 or to a complementary sequence thereof comprises the steps of:
(a-1) conducting gene amplification using a polynucleotide derived from the test cell sample as a template, and a primer comprising a polynucleotide that can hybridize to the polynucleotide described in claim 5 or to a complementary sequence thereof, or a set of primers comprising a polynucleotide that can hybridize to the polynucleotide selected in claim 5 or to a complementary sequence thereof; and
(b-1) detecting the resulting amplification product.

9. The method of claim 8, wherein mRNA prepared from the test cell sample or a complementary DNA (cDNA) transcribed from the mRNA is used as a template in step (a-1).

10. The method of any one of claims 5 to 9, wherein the detection step is followed by the step of separating a Purkinje progenitor cell from the detected sample.

11. A kit for detecting a Purkinje progenitor cell, which comprises a probe, a primer, or a set of primers that enable detection of the expression of or selection of a polynucleotide that can hybridize to a polynucleotide selected from (A) and (B) below, or to a complementary sequence thereof:
(A)
(i) a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iii) a polynucleotide which is expressed in a Purkinje progenitor cell and hybridizes under stringent conditions to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iv) a polynucleotide which is expressed in a Purkinje progenitor cell and has 80% or higher sequence identity to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(B)
(i') a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(iii') a polynucleotide which is expressed in a Purkinje progenitor cell and hybridizes under stringent conditions to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52; and
(iv') a polynucleotide which is expressed in a Purkinje progenitor cell and has 80% or higher sequence identity to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52.

12. A kit for detecting or selecting a Purkinje progenitor cell, which comprises antibodies that bind to proteins described in:
(A) a protein selected from:
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B) a protein selected from:
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

13. A kit for detecting or selecting a Purkinje progenitor cell, which comprises a polynucleotide comprising a polynucleotide encoding a marker protein linked to a promoter that controls expression of an mRNA translated into a protein selected from:
(A)
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B)
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

14. A method for producing a Purkinje progenitor cell population, which comprises the steps of:
(VII) obtaining a cell population potentially containing a Purkinje progenitor cell;
(VIII) detecting a Purkinje progenitor cell using the method of any one of claims 1 to 10; and
(IX) growing the cell detected or selected in step (VIII).

15. The production method of claim 14, wherein the Purkinje progenitor cell is used to treat cerebellar degeneration.

16. A Purkinje progenitor cell population obtained by the steps of:
(VII) obtaining a cell population potentially containing a Purkinje progenitor cell;
(VIII) detecting or selecting a Purkinje progenitor cell using the method of any one of claims 1 to 10; and
(IX) growing the cell detected in step (VIII).

17. A reagent for detecting or selecting a Purkinje progenitor cell, which comprises a probe, a primer, or a set of primers that enable to detect the expression of a polynucleotide that can hybridize to a polynucleotide selected from (A) and (B) below, or to a complementary sequence thereof:
(A)
(i) a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iii) a polynucleotide which is expressed in a Purkinje progenitor cell and hybridizes under stringent conditions to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iv) a polynucleotide which is expressed in a Purkinje progenitor cell and has 80% or higher sequence identity to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(B)
(i') a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(iii') a polynucleotide which is expressed in a Purkinje progenitor cell and hybridizes under stringent conditions to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52; and
(iv') a polynucleotide which is expressed in a Purkinje progenitor cell and has 80% or higher sequence identity to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52.

18. A reagent for detecting or selecting a Purkinje progenitor cell, which comprises an antibody that binds to a protein selected from:
(A)
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B)
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

19. A reagent for detecting or selecting a Purkinje progenitor cell, which comprises a polynucleotide comprising a polynucleotide encoding a marker protein linked to a promoter that controls expression of an mRNA translated into a protein selected from:
(A)
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36; and
(B)
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.

20. A polynucleotide for detecting or selecting a Purkinje progenitor cell for use in regeneration medicine to treat cerebellar degeneration, which can hybridize to a polynucleotide selected from (A) and (B) below, or to a complementary sequence thereof:
(A)
(i) a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(ii) a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iii) a polynucleotide which is expressed in a Purkinje progenitor cell and hybridizes under stringent conditions to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(iv) a polynucleotide which is expressed in a Purkinje progenitor cell and has 80% or higher sequence identity to a polynucleotide comprising:
the nucleotide sequence from positions 178 to 2280 of SEQ ID NO: 1;
the nucleotide sequence from positions 127 to 2079 of SEQ ID NO: 3;
the nucleotide sequence from positions 668 to 2770 of SEQ ID NO: 19;
the nucleotide sequence from positions 130 to 2232 of SEQ ID NO: 21;
the nucleotide sequence from positions 199 to 2100 of SEQ ID NO: 23;
the nucleotide sequence from positions 199 to 2325 of SEQ ID NO: 25;
the nucleotide sequence from positions 15 to 2325 of SEQ ID NO: 27;
the nucleotide sequence from positions 15 to 1916 of SEQ ID NO: 29;
the nucleotide sequence from positions 199 to 1950 of SEQ ID NO: 31;
the nucleotide sequence from positions 15 to 1766 of SEQ ID NO: 33; or
the nucleotide sequence from positions 196 to 2262 of SEQ ID NO: 35;
(B)
(i') a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(ii') a polynucleotide which is expressed in a Purkinje progenitor cell and encodes a polypeptide comprising an amino acid sequence with an insertion, substitution, or deletion of one or more amino acids, and/or an addition of one or more amino acids to either or both ends of the amino acid sequence encoded by a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52;
(iii') a polynucleotide which is expressed in a Purkinje progenitor cell and hybridizes under stringent conditions to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52; and
(iv') a polynucleotide which is expressed in a Purkinje progenitor cell and has 80% or higher sequence identity to a polynucleotide comprising:
the nucleotide sequence from positions 125 to 2773 of SEQ ID NO: 46;
the nucleotide sequence from positions 128 to 2782 of SEQ ID NO: 48;
the nucleotide sequence from positions 127 to 2787 of SEQ ID NO: 50; or
the nucleotide sequence from positions 192 to 2849 of SEQ ID NO: 52.

21. An antibody for detecting or selecting a Purkinje progenitor cell for use in regeneration medicine to treat cerebellar degeneration, which binds to a protein selected from:
(A)
(v) a protein comprising the amino acid sequence of SEQ ID NO: 2, 4, 20, 22, 24, 26, 28, 30, 32, 34, or 36;
(vi) a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 2, 4, 20, 22, 24, 26, 28, 30, 32, 34, or 36;
(vii) a protein which is expressed in a Purkinje progenitor cell and encoded by a polynucleotide that hybridizes under stringent conditions to a polynucleotide encoding the amino acid sequence of SEQ ID NO: 2, 4, 20, 22, 24, 26, 28, 30, 32, 34, or 36; and
(viii) a protein which is expressed in a Purkinje progenitor cell and comprises an amino acid sequence having 80% or higher sequence identity to the amino acid sequence of SEQ ID NO: 2, 4, 20, 22, 24, 26, 28, 30, 32, 34, or 36;
(B)
(v') a protein comprising the amino acid sequence of SEQ ID NO: 47, 49, 51, or 53;
(vi') a protein which is expressed in a Purkinje progenitor cell and comprises an amino acid sequence with an insertion, substitution, or deletion of one or more amino acids, and/or an addition of one or more amino acids to either or both ends of the amino acid sequence of SEQ ID NO: 47, 49, 51, or 53;
(vii') a protein which is expressed in a Purkinje progenitor cell and encoded by a polynucleotide that hybridizes under stringent conditions to a polynucleotide encoding the amino acid sequence of SEQ ID NO: 47, 49, 51, or 53; and
(viii') a protein which is expressed in a Purkinje progenitor cell and comprises an amino acid sequence having 80% or higher sequence identity to the amino acid sequence of SEQ ID NO: 47, 49, 51, or 53.
